# EUROPEAN PATENT APPLICATION

(11) **EP 2 910 565 A2**
(43) Date of publication of application: **26.08.2015**
(21) Application number: 14200166.8
(22) Date of filing: 09.02.2011
(51) Int. Cl.: C07K 14/00, A61K 38/00

(54) **Modified ADAM disintegrin domain polypeptides and uses**

(30) Priority: 11.02.2010 US 303631 P
(62) Divisional of application: 11742753.4
(71) Applicant: University of Southern California, Los Angeles, CA 90015 (US)
(72) Inventor: Minea, Radu O., Arcadia, CA 91007 (US); Swensen, Stephen D., Glendale, CA 91206-3119 (US); Markland, Francis S. Jr., Manhattan Beach, CA 90266 (US)
(74) Representative: Gates, Marie Christina Esther

(57) **Abstract**

Modified ADAM (A Disintegrin and Metalloproteinase) Polypeptides (MAPs) are provided. Methods are provided for administering MAPs for anti-angiogenesis and anti-tumor growth activity. Compositions of the invention are also useful for treating endothelial cell dysfunction and for diagnosis of integrin-related conditions.

## Description

### CROSS-REFERENCE TO RELATED APPLICATIONS

This application claims the benefit of priority under 35 U.S.C. § 119(e) to U.S. Provisional Application Serial No. 61/303,631, filed February 11, 2010, which is incorporated by reference herein in its entirety including all figures and tables.

### FIELD OF INVENTION

The invention relates to a class of engineered polypeptides that are derived from the ADAM (A Disintegrin and Metalloproteinase) mammalian family of multirole proteases and methods of making same. The invention also relates to the use of these engineered polypeptides for anti-angiogenesis and anti-tumor growth activity. The invention also relates to administering the engineered polypeptides for endothelial cell dysfunction and for diagnosis of integrin-related conditions.

### BACKGROUND OF THE INVENTION

The invention is related to U.S. Publication no. 20060246541 by Minea et al., and titled "Method of expressing proteins with disulfide bridges" and to PCT Patent Application No. PCT/US09/64256, filed November 12, 2009, and titled "Method of expressing proteins with disulfide bridges with enhanced yields and activity." The contents of both are incorporated herein by reference thereto including all figures.

The ADAMs are class of multidomain mammalian transmembrane or secreted proteins that are involved in every step of embryonic development (where they control cell proliferation, cell migration, cell specification, axon elongation and organ morphogenesis) as well as in multiple physiological and pathological processes in the adult life (from wound healing to various inflammatory processes and from angiogenesis and metastasis to organ repair and regeneration) [1-5]. Numerous reports show that many proteolytic members of ADAM family appear to be overexpressed in human malignancies which indicates that these proteases may play important roles in tumor progression [6-15]. Interestingly, while the overexpression of catalytically active ADAMs appears to be generally linked to poor outcomes in cancers, the non-proteolytic ones seem to play a role as tumor inhibitors. For instance, the overexpression of ADAM22 in human gliomas has been shown to correlate with tumor growth inhibition [16].

The structure of ADAM proteins closely resembles that of their orthologues, the PIII-class snake venom metalloproteinases. Like the PIII-class of snake venom metalloproteinases (SVMPs), the ADAMs are multidomain proteins that possess metalloprotease, disintegrin and cysteine-rich domains [17, 28]. The ADAM scaffold contains several inter- and intra-domain cysteine residues, among which is a very important one located in the disintegrin domain at the tip of a structural element called the disintegrin loop. The inter-domain cysteine residues are located in the spacer regions between the metalloprotease and disintegrin domains and between the disintegrin and cysteine-rich domains [20]. These cysteine residues, which are found in ADAMs and PIII-SVMPs but generally not in snake venom disintegrins, form the disulfide bridges that link the inter-domain (spacer) regions with the metalloprotease, disintegrin and cysteine-rich domains in ADAMs and PIII-SVMPs [20]. These spacer-domain disulfide bridges in the ADAM scaffold represent the structural elements that lock the three domains together in a tightly folded structure allowing these multidomain proteins to better survive in the extracellular environment (i.e., more resistant to a proteolytic attack etc). This stabilized ADAM scaffold naturally evolved through disulfide bond engineering into a newer fold, the PII-class SVMPs, that gives rise to free disintegrins through a mechanism that involves a proteolytic attack. As a result of molecular evolution, several of the cysteine residues characteristic of the ADAM scaffold (i.e., the ones that participate in spacer-domain disulfide bridges) were either mutated to a different residue or deleted. The consequence of these mutations was that disulfide bridges could no longer form between the spacer region and both the metalloprotease and disintegrin domains in these newer proteins (the PIIclass SVMPs) which renders their inter-domain regions susceptible to proteolysis and makes possible the release of the individual domains as free domains of snake venom disintegrins [20]. Therefore, the PII snake venom disintegrins emerged as a class of free disintegrin domain polypeptides that have become the most potent natural platelet aggregation inhibitors through their high-affinity interaction with the platelet specific integrin alphaIIbbeta3. In contrast to the ADAM scaffold disintegrin domains, these later evolving PII class SVMPs are released as free polypeptides in the venom of hematotoxic snakes and possess a novel 11-amino acid disintegrin loop, the unique structural element characteristic to this class of molecules and naturally engineered to act as a potent soluble integrin ligand by mimicking the action of extracellular matrix protein motifs (ECM-mimetic) [19]. This loop freely protrudes from the disulfide stabilized polypeptide core and interacts with integrin receptors via a tripeptide motif (usually an Arg-Gly-Asp motif) that is displayed at the tip of the loop [21].

There are 24 members in the human integrin family of which 23 have a complex expression pattern, tissue distribution and physiological functions whereas the alphaIIbbeta3 integrin is a platelet specific receptor instrumental to blood clot formation. When deregulated in different tissues or organs, by being either abnormally expressed, or mislocalized at the plasma membrane or inappropriately activated or a combination of these mechanisms, the abnormal functioning of many of these receptors is linked to a diverse pathology ranging from neoplasia to inflammatory diseases and to complex physiological responses such as wound healing and tissue regeneration [22-25].

Snake venom disintegrins have therapeutic potential as anticancer agents when delivered in a liposomal formulation [21, 26, 27]. Aside from their natural biological activity (i.e., the inhibition of platelet aggregation through a high affinity interaction with integrin alphaIIbbeta3), it has been shown that snake venom disintegrins disrupt tumor-associated processes such as metastasis and angiogenesis by their ability to engage a defined set of integrins (e.g., alphavbeta3, alphavbeta5, and alpha5beta1) that are mechanistically involved in the pathogenesis of these processes [21]. Despite these favorable attributes, these polypeptides still possess potentially negative immunological characteristics due to their derivation from snake venom. Free disintegrins are not known to be present in humans or any other mammals - the only human disintegrins identified so far exist as subdomains buried within the larger sequence of the ADAM family protein members [17]. There are over 30 ADAM proteins indentified in the mammalian kingdom (of which humans possess 20 genes and 3 pseudogenes) and all of them possess disintegrin domains [1]. Of 23 ADAM transcripts identified in humans, 3 are from pseudogenes (ADAM 1, 3 and 6) which do not translate into functional proteins.

### DESCRIPTION OF THE FIGURES

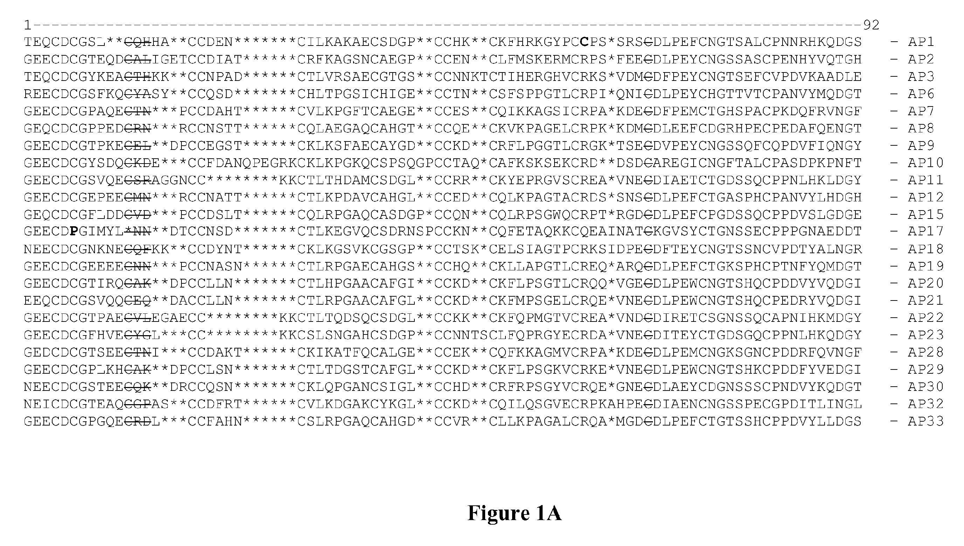

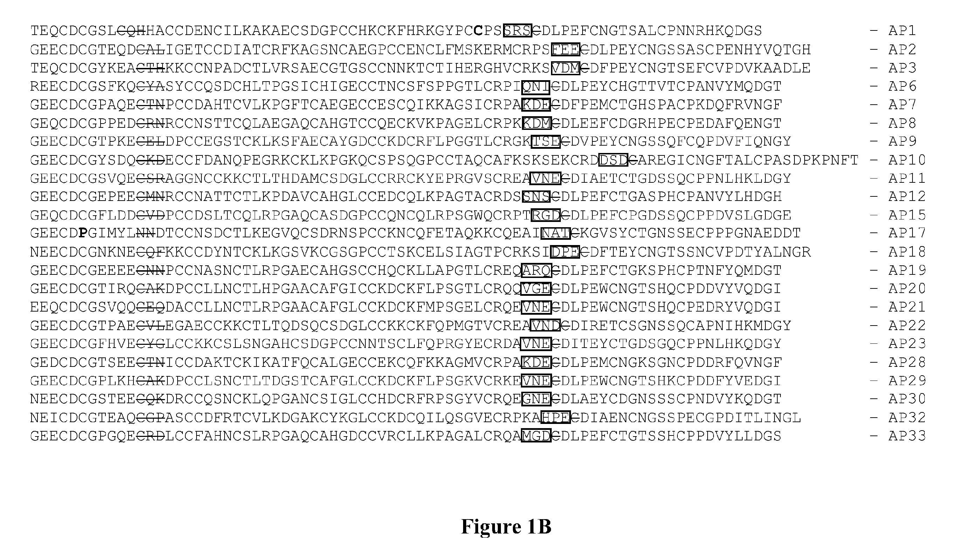

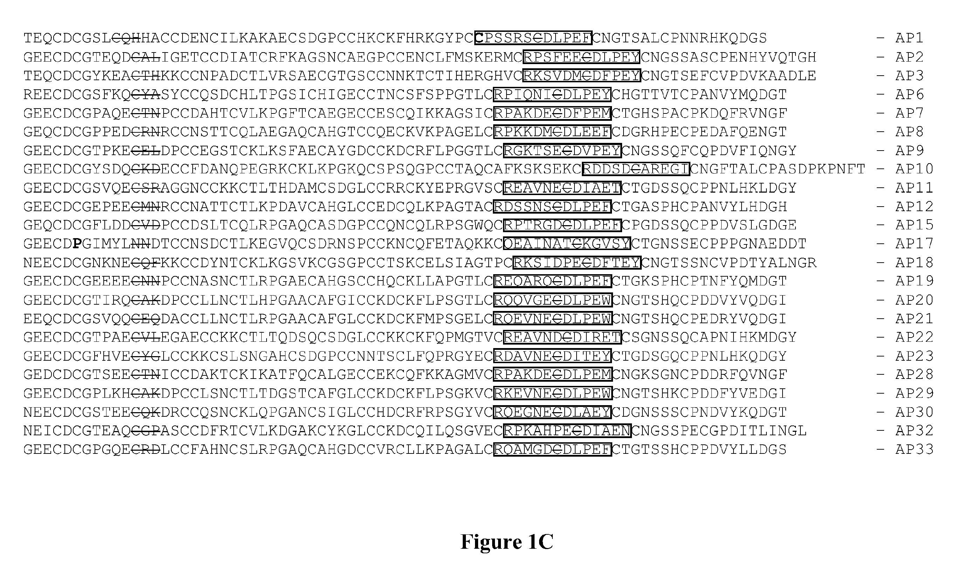

FIG. 1 shows the amino acid sequences of the disintegrin-like domains of the 23 human ADAM proteins (APs). FIG. 1A shows the sequence alignment of disintegrin-like domains of human ADAM proteins. The amino acid residues that are crossed out indicate amino acids that are removed in the corresponding MAP polypeptides. The amino acids in bold in APs 1 and 17 were replaced with another amino acid in the corresponding MAPs to conserve the medium-sized snake venom disintegrin cysteine pattern. FIG. 1B shows the tripeptide motifs (boxed amino acid residues) that are displayed at the tips of each of the APs' disintegrin loops of the disintegrin-like domain. FIG. 1C shows the disintegrin loops (boxed amino acid residues) of the disintegrin-like domains.

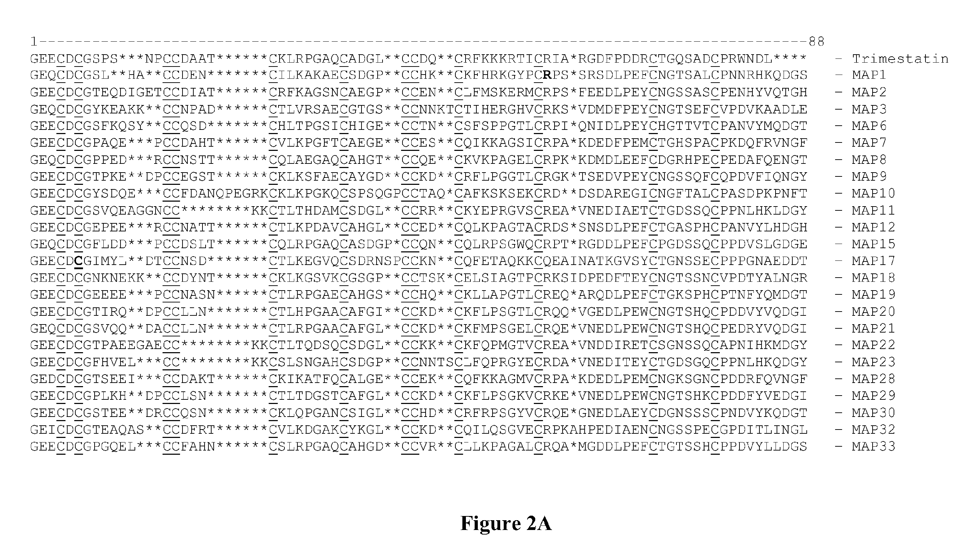

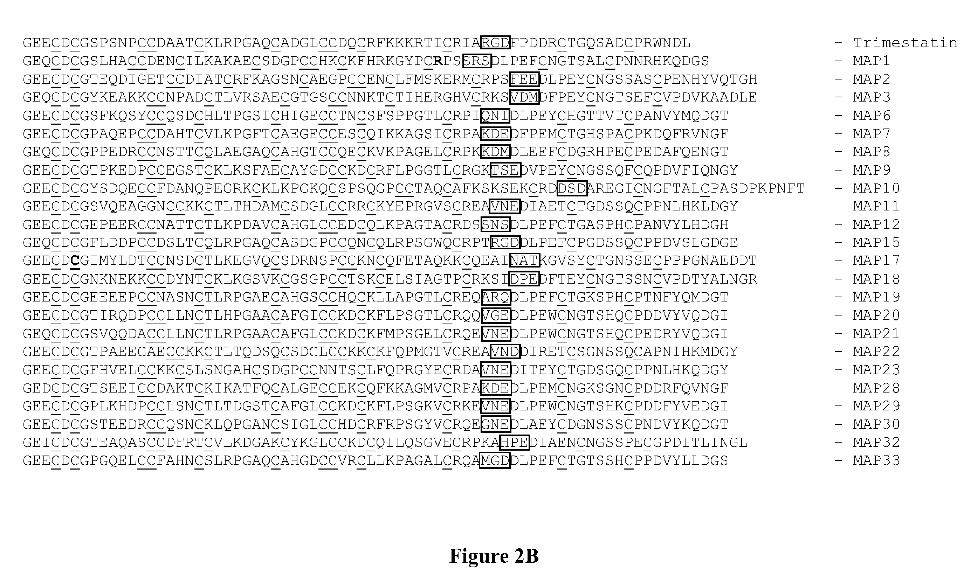

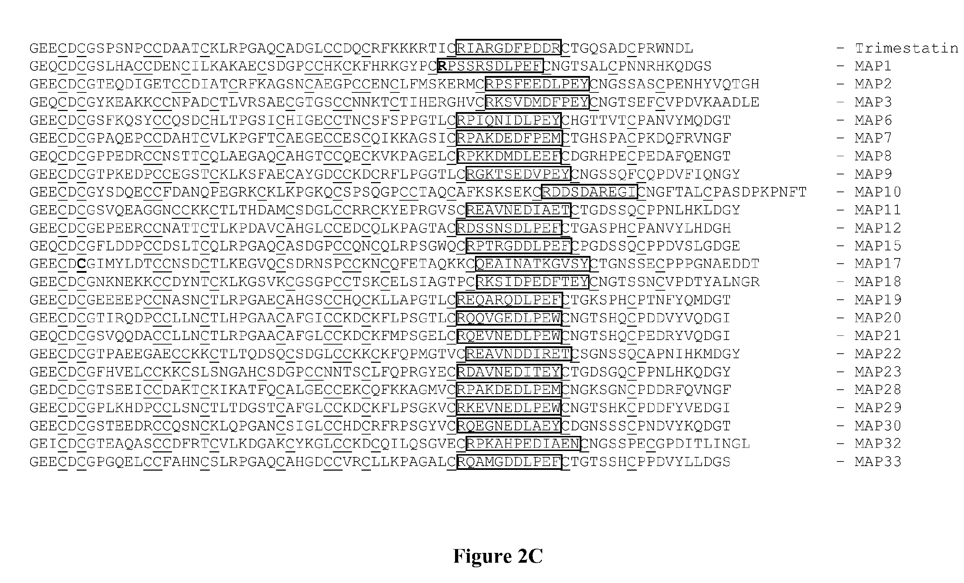

FIG. 2 shows the amino acid sequences of the MAP polypeptides. FIG. 2A shows the amino acid sequences of the 23 MAP polypeptides aligned against the sequence of trimestatin, a prototypical medium-size snake venom disintegrin (purified from the venom of *Trimeresurus flavoviridis* snake). The cysteine residues are aligned and underlined. The replacement amino acid residues corresponding to those in AP1 and AP 17 for MAP1 and MAP 17, respectively are shown in bold. In addition, the N-terminus residue in APs 1,3,6, 18, 21, 30, and 32 was replaced by a glycine residue in their corresponding MAPs. FIG. 2B shows the tripeptide motifs (boxed amino acids) that are displayed at the tips of each of the MAPs' disintegrin loops of the disintegrin-like domain and by medium-size snake venom trimestatin. FIG. 2C identifies the disintegrin loops (boxed amino acids) of MAPs and trimestatin.

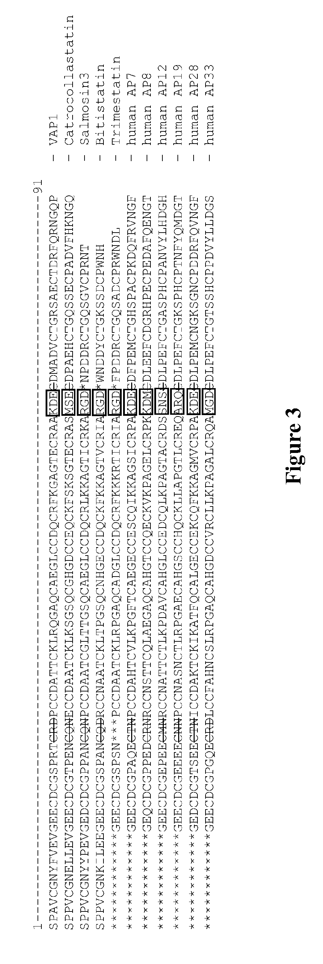

FIG. 3 shows sequence alignment of snake venom disintegrins with the disintegrin-like domains of human ADAM proteins. The disintegrin-like sequences of PIII-class snake venom metalloproteases (VAP1 and Catrocollastatin) were aligned with long-size snake venom disintegrin sequences (Salmosin3 and Bitistatin), a prototypical medium-size snake venom disintegrin (Trimestatin) and several human ADAM disintegrin-like domains (AP7, AP8, AP12, AP19, AP28, and AP33). The amino acid residues in disintegrin-like sequences (from both snake and human origin) that can be modified (e.g. deleted) in order to generate the corresponding MAP polypeptide are shown crossed out. The tripeptide amino acid motifs displayed at the tip of disintegrin loops in these disintegrin and disintegrin-like sequences are shown in a box.

FIG. 4 compares expression of Trx-D9 (native human ADAM9 disintegrin-like domain sequence-thioredoxin fusion polypeptide) to Trx-MAP9 (MAP9-thioredoxin fusion polypeptide) in different E. coli hosts (BL21 versus Origami B) analyzed by SDS-PAGE. From left to right, lanes represent: PageRuler™ Plus Prestained Protein Ladder (Fermentas, Burlington, ON), lysates from Trx-D9-transformed BL21 (DE3) cells grown and induced in Carbenicillin, lysates from Trx-D9-transformed Origami B (DE3) cells initially plated on 3 AB (Carbenicillin, Kanamycin and Tetracycline), but further expanded and induced in Carbenicillin only, lysates from Trx-MAP9-transformed BL21 (DE3) cells grown and induced in Carbenicillin, and lysates from Trx-MAP9-transformed Origami B (DE3) cells initially plated on 3 AB (Carbenicillin, Kanamycin and Tetracycline), but further expanded and induced in Carbenicillin only.

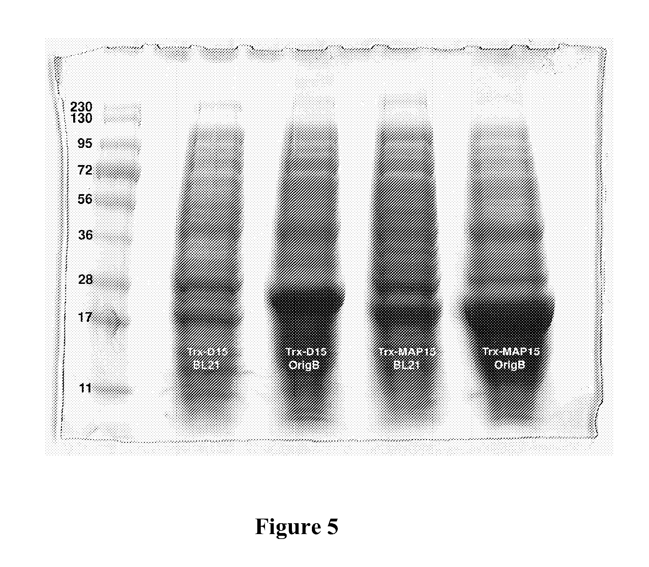

FIG. 5 compares expression of Trx-D15 (native human ADAM 15 disintegrin-like domain sequence-thioredoxin fusion polypeptide) to Trx-MAP15 (MAP15-thioredoxin fusion polypeptide) in different E. coli hosts (BL21 versus Origami B) analyzed by SDS-PAGE. From left to right, lanes represent: PageRuler™ Plus Prestained Protein Ladder (Fermentas, Burlington, ON), lysates from Trx-D15-transformed BL21 (DE3) cells grown and induced in Carbenicillin, lysates from Trx-D15-transformed Origami B (DE3) cells initially plated on 3 AB (Carbenicillin, Kanamycin and Tetracycline), but further expanded and induced in Carbenicillin only, lysates from Trx-MAP15-transformed BL21 (DE3) cells grown and induced in Carbenicillin, and lysates from Trx-MAP15-transformed Origami B (DE3) cells initially plated on 3 AB (Carbenicillin, Kanamycin and Tetracycline), but further expanded and induced in Carbenicillin only.

FIG. 6 shows the nucleic acid sequences of MAPs inserted into a pET32a expression vector 3' to bacterial TrxA. FIG. 6A shows the DNA sequences of MAPs 1, 2, 3, and 6. FIG. 6B shows the nucleic acid sequences of MAPs 7, 8, 9, and 10. FIG. 6C shows the nucleic acid sequences of MAPs 11, 12, 15, and 17. FIG. 6D shows the nucleic acid sequences of MAPs 18, 19, 20, and 21. FIG. 6E shows the nucleic acid sequences of MAPs 22, 23, 28, and 29. FIG. 6F shows the nucleic acid sequences of MAPs 30, 32, and 33.

FIG. 7 shows the oligonucleotide primer sequences used for cloning of MAP constructs into a pET32a expression vector. FIG. 7A shows the oligonucleotide primers used for cloning of MAPs 1, 2, 3, 6, 7, 8, 9, 10, 11, 12, 15, 17, and 18. FIG. 7B shows the oligonucleotide primers used for cloning of MAPs 19, 20, 21, 22, 23, 28, 29, 30, 32, and 33.

FIG. 8 shows the amino acid sequences of all TrxA-MAP constructs that were expressed in *E. coli* Origami B (DE3). The active site of TrxA (CGPC) is *italicized,* the tripeptide motif at the tip of the disintegrin loop is underlined, the TEV cleavage recognition site is highlighted in a box and the linker region between TrxA and various MAP constructs is in ***bold and italicized.*** The amino acid residues introduced to replace the residues in AP1 and AP 17 in the corresponding MAP1 and MAP 17 are highlighted in **bold double-underlined.**

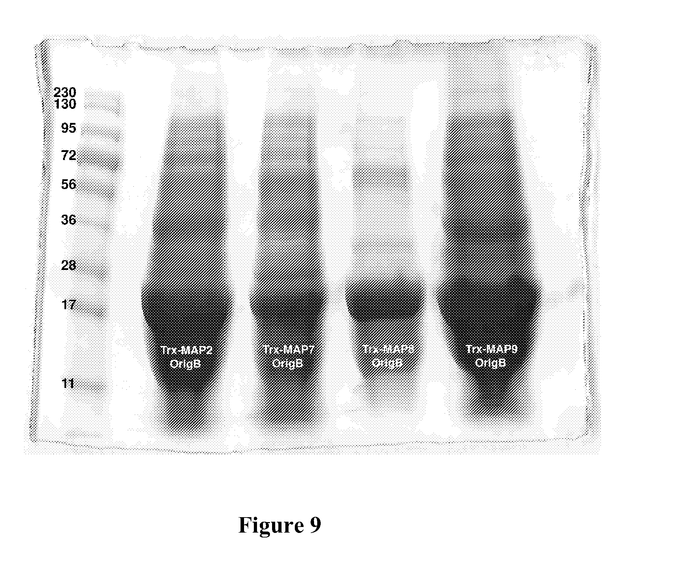

FIG. 9 shows expression of Trx-MAP2 (MAP2-thioredoxin fusion polypeptide), Trx-MAP7 (MAP7-thioredoxin fusion polypeptide), Trx-MAP8 (MAP8-thioredoxin fusion polypeptide), and Trx-MAP9 (MAP9-thioredoxin fusion polypeptide) expression levels in Origami B host analyzed by SDS-PAGE. From left to right, lanes represent: PageRuler™ Plus Prestained Protein Ladder (Fermentas, Burlington, ON), lysates from Trx-MAP2-transformed Origami B (DE3) cells, lysates from Trx-MAP7-transformed Origami B (DE3) cells, lysates from Trx-MAP8-transformed Origami B (DE3) cells, and lysates from Trx-MAP9-transformed Origami B (DE3). All transformants were initially plated on 3 antibiotics (Carbenicillin, Kanamycin and Tetracycline), but further expanded and induced in Carbenicillin only.

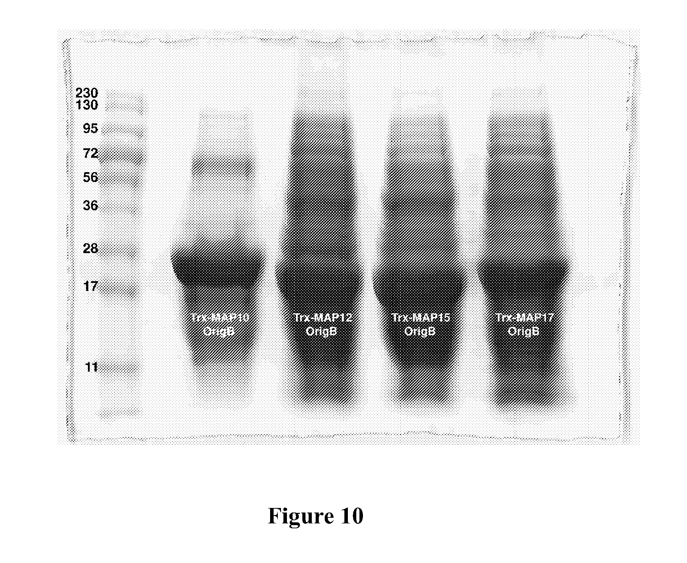

FIG. 10 shows expression of Trx-MAP10 (MAP10-thioredoxin fusion polypeptide), Trx-MAP12 (MAP12-thioredoxin fusion polypeptide), Trx-MAP15 (MAP15-thioredoxin fusion polypeptide), and Trx-MAP17 (MAP17-thioredoxin fusion polypeptide) expression levels in Origami B host analyzed by SDS-PAGE. From left to right, lanes represent: PageRuler™ Plus Prestained Protein Ladder (Fermentas, Burlington, ON), lysates from Trx-MAP10-transformed Origami B (DE3) cells, lysates from Trx-MAP12-transformed Origami B (DE3) cells, lysates from Trx-MAP15-transformed Origami B (DE3) cells, and lysates from Trx-MAP 17-transformed Origami B (DE3). All transformants were initially plated on 3 antibiotics (Carbenicillin, Kanamycin and Tetracycline), but further expanded and induced in Carbenicillin only.

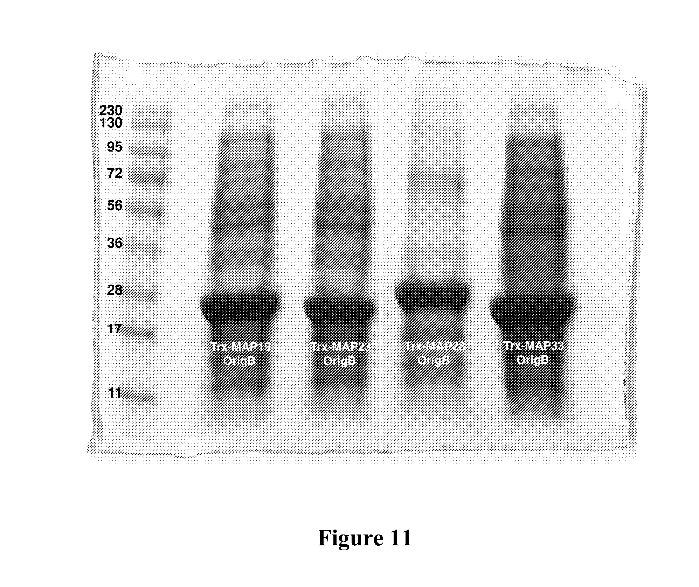

FIG. 11 shows expression of Trx-MAP 19 (MAP19-thioredoxin fusion polypeptide), Trx-MAP23 (MAP23-thioredoxin fusion polypeptide), Trx-MAP28 (MAP28-thioredoxin fusion polypeptide), and Trx-MAP33 (MAP33-thioredoxin fusion polypeptide) expression levels in Origami B host analyzed by SDS-PAGE. From left to right, lanes represent: PageRuler™ Plus Prestained Protein Ladder (Fermentas, Burlington, ON), lysates from Trx-MAP19-transformed Origami B (DE3) cells, lysates from Trx-MAP23-transformed Origami B (DE3) cells, lysates from Trx-MAP28-transformed Origami B (DE3) cells, and lysates from Trx-MAP33-transformed Origami B (DE3). All transformants were initially plated on 3 antibiotics (Carbenicillin, Kanamycin and Tetracycline), but further expanded and induced in Carbenicillin only.

FIG. 12 shows flow cytometry detection of binding of various Trx-MAPs, as indicated in the figure, to MDA-MB-231 cells (human breast carcinoma).

FIG. 13 shows flow cytometry detection of binding of various Trx-MAPs, as indicated in the figure, to a bone-homing subclone of MDA-MB-231 cells (human breast carcinoma).

FIG. 14 shows flow cytometry detection of binding of various Trx-MAPs, as indicated in the figure, to a brain-homing subclone of MDA-MB-231 cells (human breast carcinoma).

FIG. 15 shows flow cytometry detection of binding of various Trx-MAPs, as indicated in the figure, to a Jurkat cells (human T-cell leukemia cell line).

FIG. 16 shows flow cytometry detection of binding of MAP9 and MAP 15 to HUVEC (Human Umbilical Vein Endothelial Cells), MDA-MB-435 (human breast carcinoma), MDA-MB-231 (human breast carcinoma), and a glioblastoma multiforme cancer stem cell line (GBM-CSC).

FIG. 17 shows HUVEC tube formation assays in the presence of 10nM MAP9 or MAP15. Panel A - untreated control; panel B - 100µM Suramin; panel C - 10nM MAP9; panel D - 10nM MAP 15. Cells were stained with Calcein AM and imaged using confocal microscopy. All images were taken at the same magnification (scale bar = 50µm).

FIG. 18 shows tumor growth inhibition (panel A) and survival (panel B) in MDA-MB-231 xenografts by MAP 15 and a liposomal formulation of MAP 15 treatment as compared to other anticancer treatments (Avastin and Docetaxel).

FIG. 19 shows microvessel inhibition in a tumor angiogenesis assay in photomicrographs (panel A) and by quantitation of microvessel density from random photomicrographs (panel B) in MDA-MB-231 xenografts by a liposomal formulation of MAP 15 treatments as compared to other anticancer treatments (Avastin and Docetaxel) and in combination with other anticancer treatments.

FIG. 20 shows the nucleic acid sequence corresponding to the ADAM1 transcript.

FIG. 21 shows the nucleic acid sequence corresponding to the ADAM2 transcript.

FIG. 22 shows the nucleic acid sequence corresponding to the ADAM3 transcript, variant 1.

FIG. 23 shows the nucleic acid sequence corresponding to the ADAM6 transcript.

FIG. 24 shows the nucleic acid sequence corresponding to the ADAM7 transcript.

FIG. 25 shows the nucleic acid sequence corresponding to the ADAM8 transcript, variant 1.

FIG. 26 shows the nucleic acid sequence corresponding to the ADAM9 transcript, variant 1.

FIG. 27 shows the nucleic acid sequence corresponding to the ADAM10 transcript.

FIG. 28 shows the nucleic acid sequence corresponding to the ADAM11 transcript.

FIG. 29 shows the nucleic acid sequence corresponding to the ADAM 12 transcript, variant 1.

FIG. 30 shows the nucleic acid sequence corresponding to the ADAM15 transcript, variant 6.

FIG. 31 shows the nucleic acid sequence corresponding to the ADAM17 transcript.

FIG. 32 shows the nucleic acid sequence corresponding to the ADAM18 transcript, variant 1.

FIG. 33 shows the nucleic acid sequence corresponding to the ADAM19 transcript.

FIG. 34 shows the nucleic acid sequence corresponding to the ADAM20 transcript.

FIG. 35 shows the nucleic acid sequence corresponding to the ADAM21 transcript.

FIG. 36 shows the nucleic acid sequence corresponding to the ADAM22 transcript, variant 1.

FIG. 37 shows the nucleic acid sequence corresponding to the ADAM23 transcript.

FIG. 38 shows the nucleic acid sequence corresponding to the ADAM28 transcript, variant 1.

FIG. 39 shows the nucleic acid sequence corresponding to the ADAM29 transcript, variant 1.

FIG. 40 shows the nucleic acid sequence corresponding to the ADAM30 transcript.

FIG. 41 shows the nucleic acid sequence corresponding to the ADAM32 transcript.

FIG. 42 shows the nucleic acid sequence corresponding to the ADAM33 transcript, variant 1.

FIG. 43 shows the amino acid sequences for ADAM2, ADAM7, ADAM8, ADAM9, ADAM10, ADAM11, ADAM12, ADAM 15, ADAM 17, ADAM18, ADAM 19, ADAM20, ADAM21, ADAM22, ADAM23, ADAM28, ADAM29, ADAM30, ADAM32, and ADAM33 polypeptides.

### SUMMARY OF THE INVENTION

Provided herein compositions, and methods related thereto, of Modified ADAM-derived Polypeptides (MAPs), having an ADAM-derived Polypeptide (AP) that is modified at the following amino acid residues:
a) a first cysteine C-terminal to a Cys-Asp-Cys (CDC) motif;
b) two contiguous amino acids C-terminal to the first cysteine; and,
c) a cysteine C-terminal to a tripeptide motif,
   and wherein the modification of the amino acid residues is independently selected from the following:
   a) deletion;
   b) substitution; and,
   c) chemical modification,
and, wherein, the AP has a distintegrin-like domain from an ADAM having (i) the Cys-Asp-Cys (CDC) motif, (ii) the tripeptide motif as indicated in FIG. 1 and (iii) lacking all or substantially all of the ADAM metalloprotease domain, cysteine-rich domains, and interdomain segments, and wherein the ADAM is not ADAM17, as ADAM 17 has a CDP rather than a CDC motif. Provided herein are nucleic acids that encode these MAPs.

The MAP can be derived from an ADAM (A Disintegrin and Metalloproteinase) which includes ADAM15, ADAM28, ADAM1, ADAM2, ADAM3, ADAM6, ADAM7, ADAM 8, ADAM9, ADAM10, ADAM11, ADAM12, ADAM18, ADAM 19, ADAM20, ADAM21, ADAM22, ADAM23, ADAM29, ADAM30, ADAM32, ADAM33. These corresponding MAPs can be MAP15, MAP28, MAP1, MAP2, MAP3, MAP6, MAP7, MAP8, MAP9, MAP10, MAP11, MAP12, MAP18, MAP 19, MAP20, MAP21, MAP22, MAP23, MAP29, MAP30, MAP32, MAP33.

Also provided herein are fusion proteins of MAPs with an N-terminal segment of thioredoxin and nucleic acids that code these fusions.

The MAPs can have the properties of inhibiting the movement of HUVEC or MDA-MB-435 cells through a reconstituted basement membrane, increasing the level of phosphorylation of FAK in MDA-MB-435 cells or inhibiting tube formation of HUVECs in culture.

Also provided herein are expression vectors that express MAPs and prokaryotic host cells transformed with these expression vectors. The expression vector can be under inducible control, such as where the host also carries stable mutations in thioredoxin reductase B (*trxB*) gene and/or the glutathione reductase (*gor*) gene.

Also provided herein are methods of treating an individual suffering from cancer by administering an effective amount of at least one MAP. The cancer can be an integrin expressing cancer. The cancer can be breast cancer, colorectal cancer, basal cell carcinoma, adenocarcinoma, gastrointestinal cancer, colon cancer, liver cancer, bladder cancer, pancreatic cancer, ovarian cancer, cervical cancer, lung cancer, skin cancer, prostate cancer, renal cell carcinoma, central nervous system (CNS) cancer, and leukemia. The gastrointestinal cancer can be lip cancer, mouth cancer, esophageal cancer, small bowel cancer or stomach cancer. The skin cancer can be squamous cell or basal cell cancer.

Also provided herein are methods of inhibiting the binding of an integrin to a ligand by contacting a cell that expresses the integrin with an effective amount of a MAP or fusion thereof.

Also provided herein are methods of determining the presence of cancer cells in an individual by contacting the cancer cells with at least one MAP and detecting the at least one MAP. The MAP can be labeled and the label can be a Positron Emmission Tomography (PET) probe or a fluorescent probe.

Also provided herein are methods of preparing an artificial ECM scaffold by coating an artificial ECM scaffold with a MAP. The artificial ECM scaffold can further include stem cell precursors. The artificial ECM scaffold can be a urinary bladder scaffold, an esophageal scaffold or an anal scaffold.

Also provided are stents coated with a composition that includes at least one MAP.

Also provided are MAPs that are an AP having a distintegrin-like domain from an ADAM having (i) the Cys-Asp-Cys (CD) motif, (ii) the tripeptide motif as indicated in FIG. 1 and (iii) lacking all or substantially all of the ADAM metalloprotease domain, cysteine-rich domains, and interdomain segments, and further comprising amino acid modification that results in disruption of the interdomain disulfide linkages with the first cysteine C-terminal to the CDC motif and interdomain disulfide linkages with the cysteine C-terminal to the tripeptide motif.

### DETAILED DESCRIPTION OF THE INVENTION

Provided herein are a class of uniquely designed polypeptides, designated MAPs (Modified ADAM-derived Polypeptides), and encoding nucleic acids. As used herein, MAPs refer to a modified form of the native disintegrin domain of an ADAM protein. See Appendix for listing of ADAM encoding nucleic acid and amino acid sequences. As used herein, a "disintegrin domain of an ADAM protein" which may be referred to herein as "AP" ("ADAM derived Polypeptide") is a disintegrin domain of the ADAM lacking all or substantially all of its metalloprotease and cysteine-rich domains and interdomain segments. Lacking substantially all means that the remaining amino acid sequence no longer retains the function of that domain. Examples of APs are shown in FIG. 1. The N-terminal end of the AP starts at the position 3 amino acid residues N-terminal from the CDC motif up to but not including the first cysteine N-terminal to the CDC. The C-terminal end of the AP ends at the position 10 amino acid residues C-terminal from the 12th cysteine residue from the CDC motif up to but not including the next cysteine C-terminal to said 12^{th} cysteine residue. There are two exceptions: (1) the C-terminal end of AP1 ends at the position 10 amino acid residues C-terminal from the 13th cysteine residue from the CDC motif up to but not including the next cysteine C-terminal to said 13^{th} cysteine residue, and (2) ADAM 17 has a CDP motif rather than a CDC motif from which the ends of the corresponding AP (AP 17) are delineated.

A "MAP" is a "modified" form of an AP, the modifications involving an alteration(s) in the amino acid sequence of the AP to achieve the beneficial properties described herein. MAPs, therefore, have sequences which are modified relative to the sequence normally present in the AP and corresponding sequence of the ADAM polypeptide. As used herein, "modified" means that the amino acid is deleted, substituted or chemically modified and, in an embodiment, the modification results in disruption of interdomain disulfide linkage. Exemplary MAPs are shown in FIG. 2. The MAP sequences are shown aligned with trimestatin, a prototypical medium-size snake venom disintegrin. All MAP constructs were modeled after medium-size snake venom disintegrins and had their sequences modified to fold similarly to these native snake venom molecules. The MAPs (except for MAP 17) were constructed such that the first cysteine C-terminal to the CDC motif and two amino acids C-terminal to said cysteine as well as the cysteine C-terminal to the tripeptide motif of the corresponding AP are deleted. Alternatively, the cysteine residues can be substituted with alternate amino acids or the cysteine amino acid residues can be chemically modified so as to prevent disulfide bond formation. The amino acid substitutions can be conservative, e.g. the first cysteine C-terminal to the CDC motif of the AP can be substituted with a serine residue, the amino acid residues C-terminal to the cysteine can be substituted with a charged amino acid, or the cysteine C-terminal to the tripeptide motif can be substituted with a charged amino acid. Such mutational approaches and chemical modifications of amino acid residues are well known in the art. With regard to chemical modifications, an example is the use alkylating agents to react with cysteine residues to prevent formation of disulfide bonds. Except for MAP 10, 17, 18 and 32, MAPs display an 11 amino acid disintegrin loop, similar to the native loop of snake venom disintegrins. MAP 10 displays a 10 amino acid integrin loop and MAP17, MAP18, and MAP32 display a 12 amino acid disintegrin loop.

MAPs can be expressed and further purified as stand alone biologically active molecules in a bacterial system that supports both the generation of active soluble disulfide-rich polypeptides and high expression yields for these products. While not wishing to be bound by theory, the MAPs were designed from the native APs so that they could adopt a snake venom disintegrin fold rather than their native ADAM conformations. The MAPs can be expressed with high yields in the Origami B (DE3) *E. coli* strain and further purified as stable and active free polypeptides that can interact with a class of mammalian cell surface receptors, the integrins, in a manner that is similar to that of native snake venom disintegrins. The MAPs can also retain some of the signaling properties that are characteristic of the APs or disintegrin domain activities from the ADAM polypeptide from which the MAP was derived. For instance, retained characteristics may include signaling attributes related to the putative ability of the ADAM disintegrin domains to engage integrin receptors by utilizing amino acid residues located outside the classical disintegrin loop. Cellular functions of ADAMs are well known [1-5, 34].

Although not wishing to be bound by theory, it is believed that the PII-class SVMPs that give rise to the prototypical medium-sized snake venom disintegrins (e.g., Trimestatin, Kistrin, Flavoridin etc) fail to form a critical disulfide bridge between the upstream spacer region and the disintegrin domain and thus the proteolytic attack happens in the residues located immediately N-terminal to where the disintegrin domain starts, the consequence of this being that the released medium-sized disintegrins are complete disintegrin domains containing no portion of the upstream spacer region. In contrast, it is believed that the PII-class SVMPs that give rise to the long-sized snake venom disintegrins (e.g., Bitistatin, Salmosin3 etc) fail to form a critical disulfide bridge between the metalloprotease domain and the downstream spacer region and consequently a proteolytic attack happens further N-terminal in the spacer region with the release of a longer disintegrin having a portion of the spacer region attached N-terminally to the freed disintegrin domain (see the sequence alignment of various disintegrin and disintegrin domains in FIG. 3). Moreover, it is also believed that when the PII-SVMPs contain even more mutations and/or deletions, the disulfide bridges fail to form in the same spacer region but also in the N-terminal part of the disintegrin domain and even shorter variants of snake venom disintegrins are released (e.g., either partially truncated disintegrins domains that dimerize like Contortrostatin or, more rarely, extremely truncated polypeptides like Echistatin or Eristostatin). It is further believed that, in almost all cases, the free disintegrin domains display a conserved 11-amino acid disintegrin loop in the C-terminal half of their molecule, which is the hallmark of snake venom disintegrins.

The 23 different ADAM transcripts that have been identified in the human genome (3 of them being pseudogenes that are not normally translated into a protein product) have been used as the basis for creating the encoded MAPs as described herein that adopt the snake venom disintegrin fold.

Several ADAM transcripts encode a number of isoforms. Nonetheless, inside the isoforms of different ADAMs the disintegrin domain's sequence is conserved and therefore there are only 23 different disintegrin domains in the human family of ADAM proteins. When produced recombinantly, the MAPs of the invention can interact in a high affinity manner with a defined integrin set. This property makes these mutant polypeptides broad spectrum integrin ligands for clinical and therapeutic use.

Similar to the other human ADAM member transcripts, the non-functional transcripts do contain complete disintegrin sequences that, if artificially translated in a recombinant system, can generate active polypeptides with novel biological functions. The disintegrin domains of human ADAMs have between 76 to 86 amino acids (the disintegrin domain of ADAM1 is the shortest, whereas that of ADAM10 is the longest), and, with 2 exceptions (ADAMs 1 and 17), they all contain 14 canonical cysteine residues of the ADAM scaffold (see the aligned sequences of human ADAMs below). See FIG. 1. Unlike the snake venom disintegrins, that evolved to function as platelet aggregation inhibitors, most of which contain an RGD tripeptide motif at the tip of their disintegrin loop, the disintegrin loops of ADAMs display much different tripeptide motifs at their tips and therefore are expected to engage a broader range of integrins and in a different manner than their snake venom counterparts. In fact, each of the APs is believed to bind to a defined and unique set of integrin receptors thus signaling in a unique manner (see FIG. 3 for the sequence alignment of ADAM and snake venom disintegrins illustrating the differences in the disintegrin loops). Not wishing to be bound by theory, it is believed that combinations of 2 or more MAPs can be used to determine an "integrin signature" that is characteristic of a particular cell type or disease state for a cell type. An integrin signature means a combination of integrins present on the surface of a cell that is unique to that cell type or the disease state for that cell type.

The disintegrin domain of human ADAM 15 contains a RGD tripeptide motif in its disintegrin loop which supports the hypothesis that human ADAM 15 plays important regulatory roles in the cardiovascular system. This RGD tripeptide motif in ADAM 15 is shown in AP15 in FIG. 1.

MAPs for each AP portion of all 23 known human ADAM members were generated. The human ADAM disintegrin domain sequences were modified according to the rationale presented above, which includes removing the residues (among which include 2 cysteine residues) in the ADAM disintegrin domain that normally participate in interdomain-disintegrin domain disulfide bridge formation in the native ADAM proteins. Not wishing to be bound by theory, the apparent function of these disulfide bridges is to keep the disintegrin loops in ADAMs tightly packed and unavailable to integrin receptors. By modifying the residues that participate in the formation of these disulfide bridges, such as by deletion, these MAPs acquire the mobility of the canonical 11-amino acid loop and the disintegrin-fold characteristic of snake venom disintegrins. Among the 23 members of the human ADAMs, 6 members perfectly fit the above-mentioned scheme (ADAMs 7, 8, 12, 19, 28 and 33) when aligned with long- and medium-sized snake venom disintegrins as well as with PIII-class SVMPs (see FIG. 3 for an alignment of snake venom disintegrins and human ADAM disintegrin domains). Nonetheless, by introducing these modifications, with the exception of 4 ADAMs (10, 17, 18 and 32), all human ADAM members were converted to MAPs that display a 11-amino acid disintegrin loop. Regarding the 4 exceptions, 3 (ADAMs 17, 18 and 32) were converted to MAPs displaying a slightly longer, 12-amino acid loop, while 1 member (ADAM 10) was converted to a MAP carrying a slightly shorter 10-amino acid disintegrin loop (see AP10 in FIG. 2 for the MAP sequence alignment). Moreover, in the case of 2 APs (ADAMs 1 and 17), one additional native residue in each sequence was replaced with either an arginine residue (to generate MAP1) or a cysteine residue (to generate MAP 17) to restore the cysteine pattern characteristic of snake venom disintegrin domains (see FIG. 2 for the MAP sequence alignment).

As used herein, "interdomain regions" or "spacer regions" means the polypeptide portion of an ADAM between the metalloprotease and disintegrin domain (the "MD interdomain region") and between the disintegrin domain and the cysteine-rich domain (the "DC interdomain region"), respectively, wherein the MD interdomain region starts at least 10 amino acid residues N-terminal to the AP and the DC interdomain region starts at least 10 amino acid residues C-terminal to the AP. Each interdomain is 5 to 15 amino acids in length.

The DNA sequences of all 23 MAPs were *de novo* synthesized and cloned into the pET32a expression vector [30] downstream of bacterial thioredoxin A (TrxA). The MAPs were produced in the Origami B (DE3) bacterial strain as described in PCT Patent Application No. PCT/US09/64256, filed November 12, 2009, and titled "Method of expressing proteins with disulfide bridges with enhanced yields and activity." This application describes an improvement upon the expression system disclosed in U.S. Publication no. 20060246541, which includes, as an embodiment, expression of a chimeric snake venom disintegrin Vicrostatin (VCN) in the Origami B (DE3)/pET32a system. The improved method was used to generate increased amounts of correctly-folded active MAPs. This is achieved by growing the Origami B cells in a less selective environment and thus allowing for the generation and expansion of VCN-transformants that display a more optimal redox environment during the induction of the heterologous recombinant protein production. Unlike other *E. coli* strains, the Origami B is unique in that, by carrying mutations in two key genes, thioredoxin reductase (*trxB*) and glutathione reductase (*gor*), that are critically involved in the control of the two major oxido-reductive pathways in *E. coli*, this bacterium's cytoplasmic microenvironment is artificially shifted to a more oxidative redox state, which is the catalyst state for disulfide bridge formation in proteins [18, 29].

The Origami B strain has growth rates and biomass yields similar to those obtained with wild-type *E. coli* strains, which makes it an attractive and scalable production alternative for difficult-to-express recombinant proteins like VCN. This strain is also derived from a *lacZY* mutant of BL21. The *lacY1* deletion mutants of BL21 (the original Tuner strains) enable adjustable levels of protein expression by all cells in culture. The lac permease (*lacY1*) mutation allows uniform entry of IPTG (a lactose derivative) into all cells in the population, which produces a controlled, more homogenous induction. By adjusting the concentration of IPTG, the expression of target proteins can be optimized and theoretically maximal levels could be achieved at significantly lower levels of IPTG. Thus the Origami B combines the desirable characteristics of BL21 (deficient in *ompT* and *lon* proteases), Tuner (*lacZY* mutant) and Origami (*trxB*/*gor* mutant) hosts in one strain. As mentioned above, the mutations in both the thioredoxin reductase (*trxB*) and glutathione reductase (*gor*) greatly promote disulfide bond formation in the cytoplasm [29].

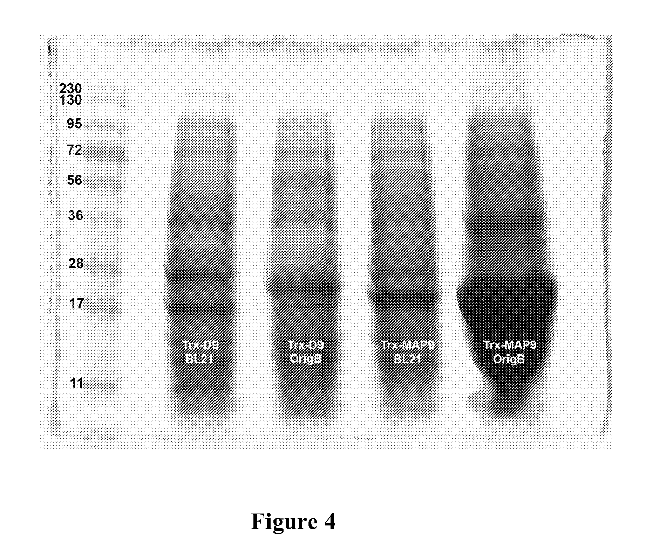

Although the Origami B strain offers a clear advantage over E. coli strains with reducing cytoplasmic environments like BL21 (FIGS. 4 and 5 show a comparison in expression levels between strains), the mere usage of the Origami B strain and the pET32a expression vector does not automatically guarantee the generation of a soluble and/or active product. The generation of disulfide-rich polypeptides in Origami B appears to be sequence dependent. For example, MAPs (e.g. MAP9 and MAP15) can be expressed in Origami B with significantly higher expression yields compared to their corresponding AP versions of human ADAMs 9 and 15 despite the fact that the same system and production technique were employed (FIGS. 4 and 5). Consequently, the modification of APs into MAPs can result in polypeptides having a disintegrin domain with greater expression yield in Origami B cells.

Furthermore, after purifying expressed disintegrin domains (APs) of ADAM 9 and 15, in a process that involves TEV protease treatment and RP-HPLC purification, the collected free polypeptides appeared to be unstable and to precipitate out of solution after reconstitution from lyophilized powder. In contrast, the corresponding MAP polypeptides, generated by employing the same purification steps, appear to be more soluble and stable when reconstituted in water after lyophilization.

MAPs of the invention are prepared so as to be substantially isolated or substantially purified. As used herein, the term "substantially purified" (or isolated) in reference to a MAP does not require absolute purity. Instead, it represents an indication that the MAP is preferably greater than 50% pure, more preferably at least 75% pure, and most preferably at least 95% pure, at least 99% pure and most preferably 100% pure. MAPs can be prepared synthetically or prepared by recombinant expression as described herein.

The term "substantially" as used herein means plus or minus 10% unless otherwise indicated.

Pharmaceutical compositions containing MAPs should comprise at a minimum an amount of the MAP effective to achieve the desired effect (e.g. inhibit cancer growth or prevent or inhibit cancer metastasis) and include a buffer, salt, and/or suitable carrier or excipient. Generally, in these compositions, MAPs are present in an amount sufficient to provide about 0.01 mg/kg to about 50 mg/kg per day, preferably about 0.1 mg/kg to about 5.0 mg/kg per day, and most preferably about 0.1 mg/kg to about 0.5 mg/kg per day.

MAPs may be administered by a variety of heretofore known means suitable for delivery thereof into the body of a subject (e.g. the blood stream) in substantial amounts. Intravenous administration of MAPs in a suitable liquid vehicle or excipient is presently contemplated as the preferred route of administration. MAPs are soluble in water, and may therefore be effectively administered in a suitable aqueous solution (e.g., phosphate buffered saline). Alternatively, MAPs may be administered orally (in the form of tablets or capsules formulated with a suitable binder or excipient material, or in the form of aqueous or oily suspensions, solutions, emulsions, syrups or elixirs) or as a parenteral suspension. MAPs can also be delivered intraarterially or intraductally, or may be introduced locally at the intended site of action. As is well known in the art, adjuvants or excipients such as local anesthetics, preservatives, buffering agents, lubricants, wetting agents, colorants, flavorings, fillers and diluents may suitably be included in any of these formulations.

MAPs may be delivered by way of liposomes. Liposomal delivery is well known in the art and has been described for delivery of disintegrins. For example, Swenson et al. describes use of intravenous delivery of contortrostatin in liposomes for therapy of breast cancer [26].

MAPs, such as MAP9, display pro-angiogenic effects (see below). Not wishing to be bound by theory, this could represent a more general characteristic of the members of the meltrin class of ADAM family (ADAMs 9, 12, and 19). The meltrins are mesenchymal ADAMs that were originally described to be expressed in the course of differentiation induction of muscle cells where they are involved in cell fusion and other processes and may play an important role in the stabilization of neovessels [33]. Consequently, recombinant polypeptides derived from the meltrin class of ADAM proteins can be used therapeutically to stimulate the formation of new capillary networks and maintain the collateral capillary growth in diseases such as coronary artery disease (CAD) or other ischemic conditions. Therefore, for example, MAPs may be coated on or chemically coupled to stents for use in therapy of CAD and related diseases that utilize stents.

MAPs can also be directly or indirectly conjugated to drugs, toxins, radionuclides and the like, and these conjugates used for diagnostic or therapeutic applications. For instance, a MAP can be used to identify or treat tissues or organs that express a cognate integrin. MAPs or bioactive fragments or portions thereof, can be coupled to detectable or cytotoxic molecules and delivered to a mammal having cells, tissues or organs that express the cognate integrin or integrins.

Suitable detectable molecules may be directly or indirectly attached to a MAP, and include radionuclides, enzymes, substrates, cofactors, inhibitors, fluorescent markers, chemiluminescent markers, magnetic particles and the like. Suitable cytotoxic molecules may be directly or indirectly attached to the polypeptide or antibody, and include bacterial or plant toxins (for instance, diphtheria toxin, Pseudomonas exotoxin, ricin, abrin and the like), as well as therapeutic radionuclides, such as iodine-131, rhenium-188 or yttrium-90 (either directly attached to the polypeptide or antibody, or indirectly attached through means of a chelating moiety, for instance). MAPs may also be conjugated to cytotoxic drugs, such as adriamycin. For indirect attachment of a detectable or cytotoxic molecule, the detectable or cytotoxic molecule can be conjugated with a member of a complementary/anticomplementary pair, where the other member is bound to the polypeptide or antibody portion, such as biotin/streptavidin.

The disclosed compositions can be used either singly or in combination for the treatment of diseases related to endothelial cell dysfunction. In an embodiment, the disclosed compositions are useful for cancer treatment. The cancer can be of epithelial origin. The cancer can be breast cancer, colorectal cancer, basal cell carcinoma, adenocarcinoma, gastrointestinal cancer, such as, for example, lip cancer, mouth cancer, esophageal cancer, small bowel cancer and stomach cancer, colon cancer, liver cancer, bladder cancer, pancreatic cancer, ovarian cancer, cervical cancer, lung cancer, and skin cancer, such as squamous cell and basal cell cancers, prostate cancer, renal cell carcinoma, central nervous system (CNS) cancer, leukemia and other known cancers that effect epithelial cells throughout the body. Not wishing to be bound by theory, in many forms of cancer a pathogenic cross-talk exists between ADAMs (membrane-tethered and/or secreted forms) and their integrin counter-receptors, a process that is mechanistically important for cancer progression. These pathological interactions serve in cancer for the recruitment of ADAM metalloproteinases via integrins into multiprotein complexes (i.e., invadosomes) that are assembled by highly migratory cells (cancer, inflammatory or endothelial cells) in the process of executing some critical integrin-driven steps in tumor progression: the infiltration of tumor stroma by inflammatory cells where they are shown to play important supportive roles, the migratory steps associated with tumor angiogenesis where endothelial cells from preexisting vessels are recruited to assemble neovessels, and, finally, the migratory and invasive events associated with metastasis. The disruption of these pathological processes by MAPs reduce malignancies. In addition to their potential antiangiogenic, antiinflammatory and antimetastatic effects, the broad spectrum anti-integrin MAPs can also impact tumor differentiation. The ability to send the right differentiation signals to cancer stem cells, by forcing them to acquire a more stable and differentiated phenotype during the course of therapy, remains one of the main goals of cancer therapy. For instance, Yuan et al. showed that a cell population with stem characteristics that was isolated from human glioblastoma multiforme (a highly-aggressive form of brain cancer) could be manipulated to differentiate *in vitro* and adopt more benign features [32]. Differentiation signals coming from soluble integrin ligands (e.g., either single or combinations of MAPs), cancer stem cells from various human malignancies may be induced to fully commit along their differentiation lineages and stay differentiated.

Composition of the invention can also be used for screening a candidate compounds for MAP-specific binding by comparing the relative binding of said candidate compound to an integrin in the presence and in the absence of a MAP, wherein a decrease of integrin binding in the presence of said MAP indicates that said MAP-specific integrin binding molecule is MAP-specific.

Compositions of the invention can be used for early detection of cancer by taking advantage of specific "integrin signatures" displayed by cancer cells. An "integrin signature" or "integrin profile" is the one or more integrins expressed on the surface of a cell. For example, carcinomas and sarcomas can display specific 'integrin signatures' based on a tumors' state of differentiation and organ localization. MAP-specific binding can be employed to identify and diagnose various cancer types based on integrin signatures either as *in vivo* cancer imaging agents (diagnostic imaging) and/or *ex vivo* molecular tools for tumor specimen staining (diagnostic pathology). In the latter case, staining cancer specimens with different MAPs can lead to characteristic staining patterns depending on various tumors' origin and grading. Compositions of the invention can be used for cancer staging, monitoring cancer cancer progression or therapy. The cancer can be of epithelial origin. The cancer can be breast cancer, colorectal cancer, basal cell carcinoma, adenocarcinoma, gastrointestinal cancer, such as, for example, lip cancer, mouth cancer, esophageal cancer, small bowel cancer and stomach cancer, colon cancer, liver cancer, bladder cancer, pancreatic cancer, ovarian cancer, cervical cancer, lung cancer, and skin cancer, such as squamous cell and basal cell cancers, prostate cancer, renal cell carcinoma, and other known cancers that effect epithelial cells throughout the body. MAPs can be used with PET (Positron Emission Tomography) probes to take advantage of the unique ability of MAPs with known integrin affinities to differentially bind to primary tumors, metastatic foci, as well as the tumor neovasculature based on the specific integrin expression of each particular solid tumor. For example, MAPs can be labeled with ¹⁸F through the amino group using N-succinimidyl-4-¹⁸F-fluorobenzoate (¹⁸F-SFB) under optimized fluorination reaction conditions or by conjugation with the metal chelator DOTA/NOTA for ⁶⁴Cu/^{6g}Ga-labeling. Furthermore, fluorescently-labeled MAPs may be used *in vivo* and *ex vivo* (on biopsy specimens) for tumor integrin expression profile analysis.

Compositions of the invention may be also utilized as molecular tools in regenerative medicine. For example, by coating artificial ECM scaffolds (organ molds) with a MAP or combinations of MAPs, stem cell precursors can be induced into populating these scaffolds and differentiate into desirable epithelial and mesenchymal layers. For example, a urinary bladder scaffold coated with such a composition containing one or more MAPs can be used to direct stem cell progenitors to commit into both urothelium and muscular layers. Other examples include esophageal and rectal scaffolds coated with such a composition containing one or more MAPs can be used to direct stem cell progenitors to commit into the relevant tissue layers. Compositions of the invention can also be used to create defined tissue culture plate coatings that could either support the growth and maintain the stemness of embryonic stem cells *in vitro* or guide the commitment of these totipotent cells along their differentiation lineages in the same system.

Compositions of the invention may be used as novel drugs for supporting the collateral growth of capillaries in various ischemic conditions, such as drug-eluting stents. For example, MAPs can be conjugated with a radionuclide, such as with a beta-emitting radionuclide, to reduce restenosis. Such therapeutic approach poses less danger to clinicians who administer the radioactive therapy.

### Examples

### Example 1

### Preparation and Purification of MAPs

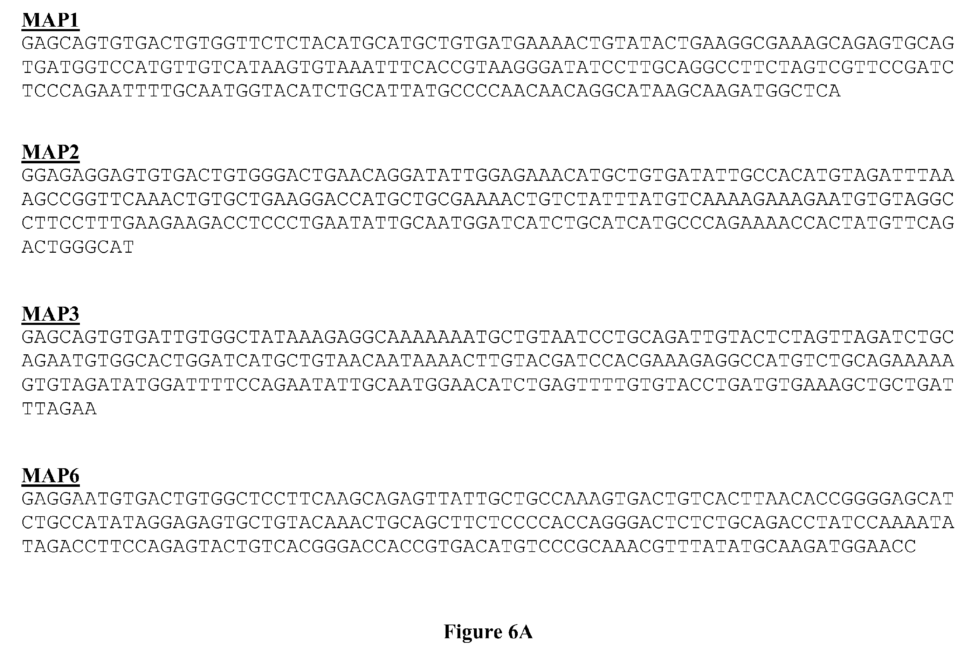

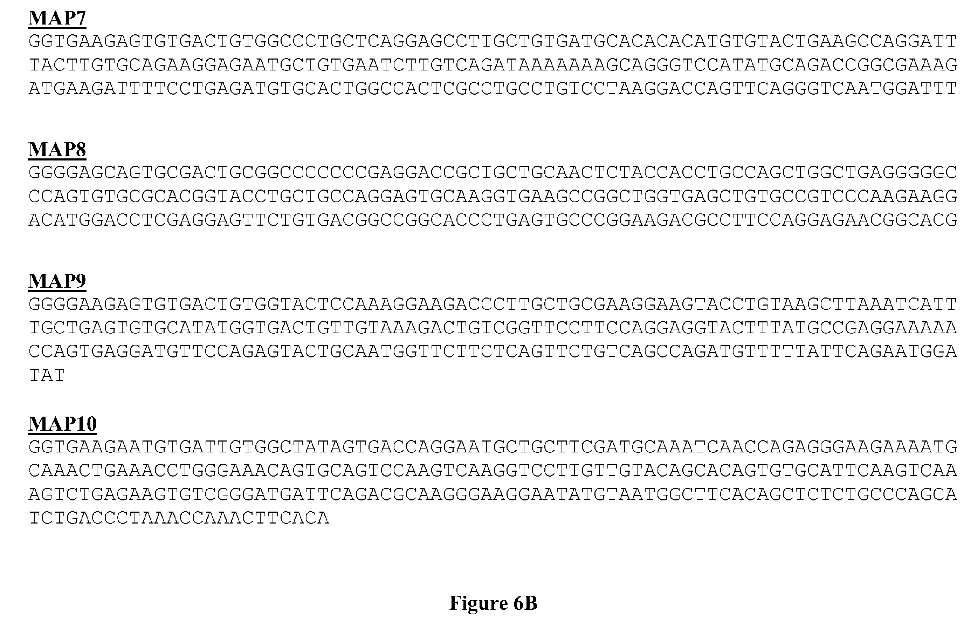

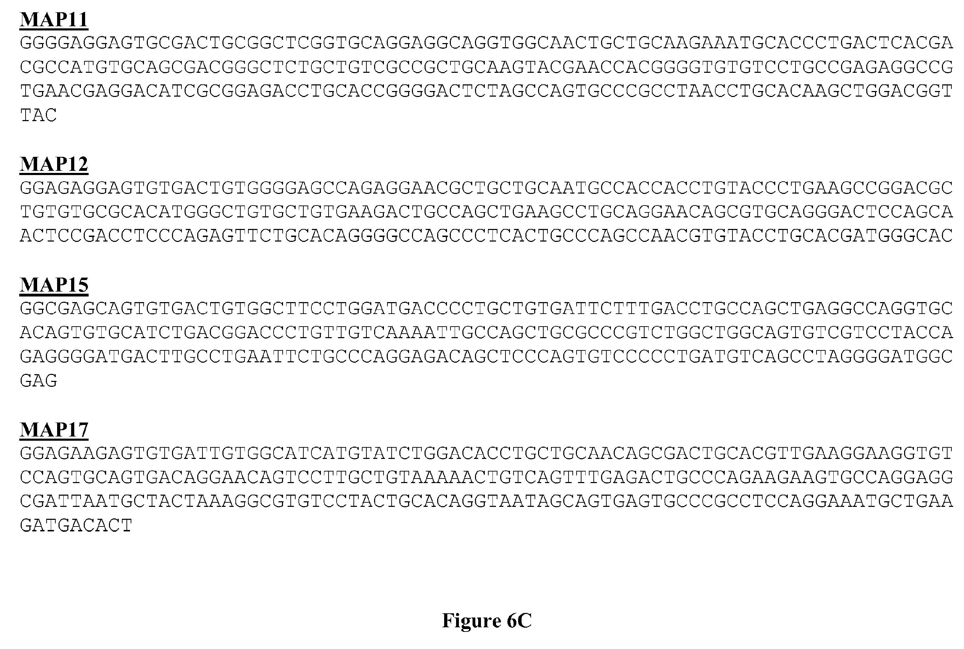

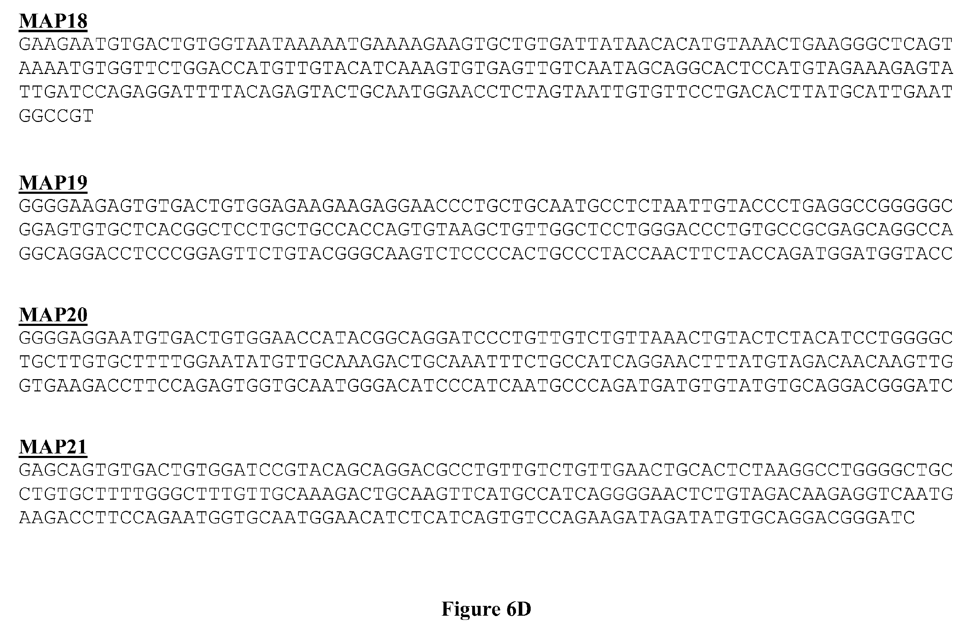

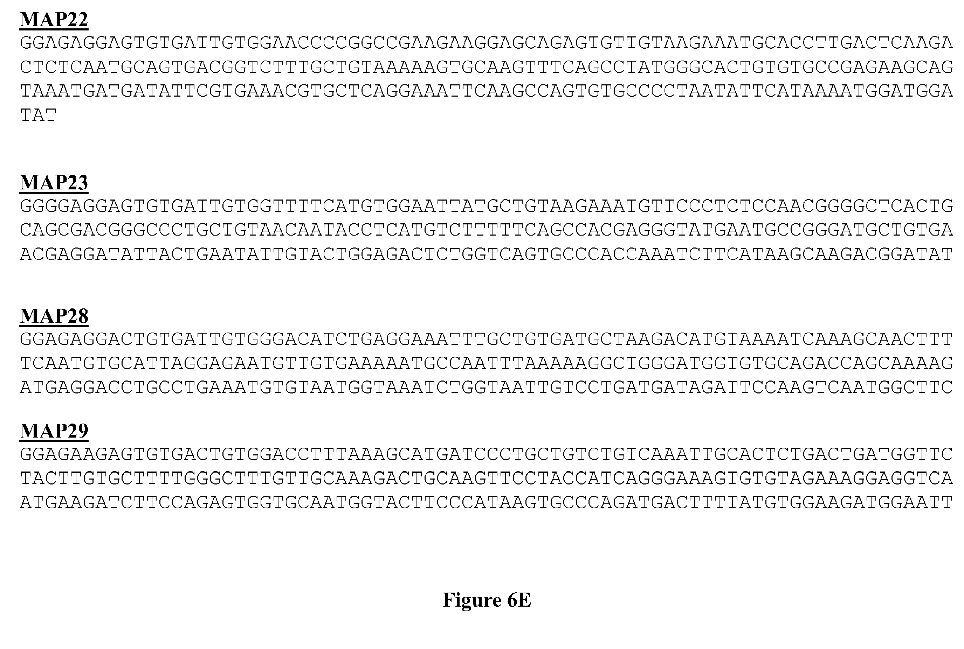

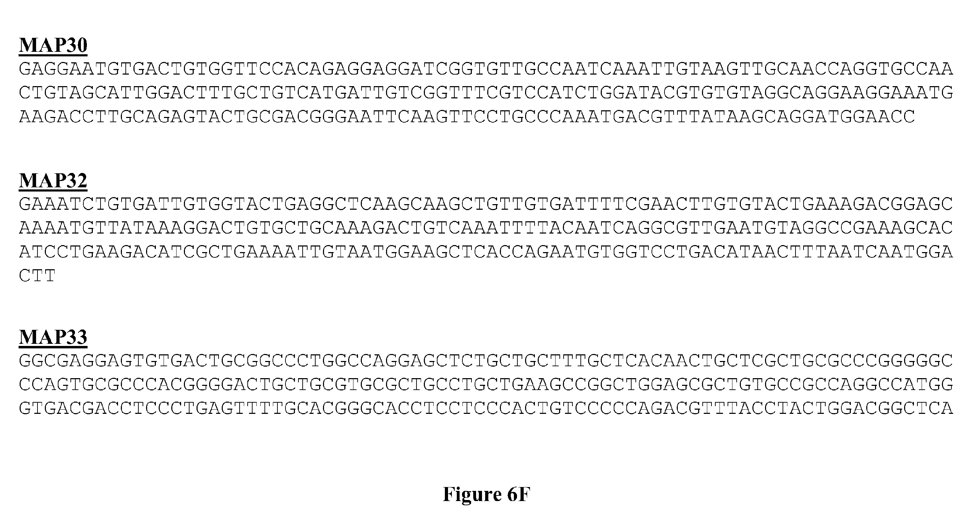

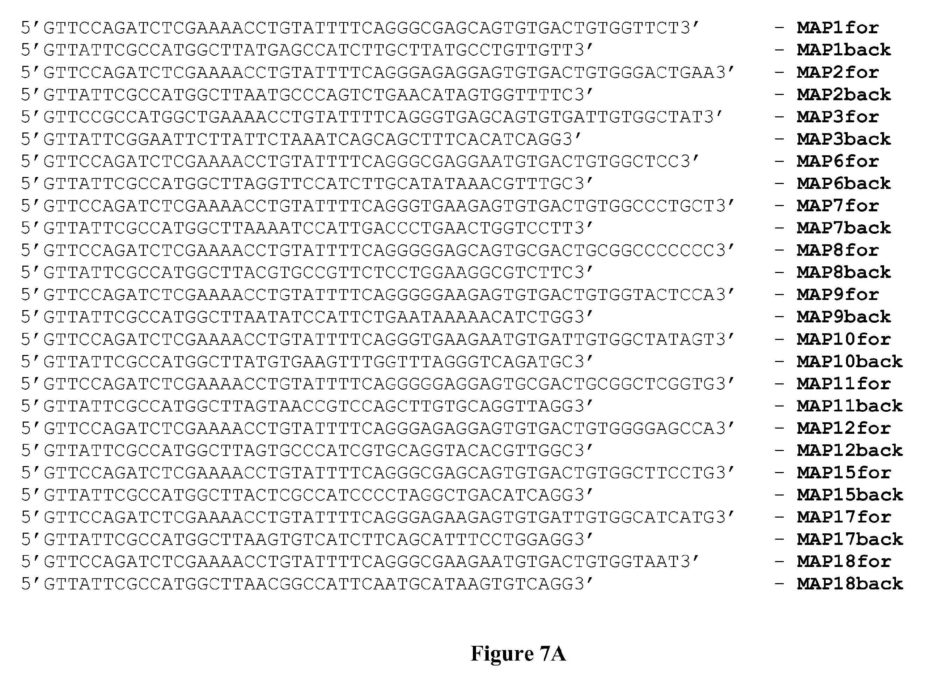

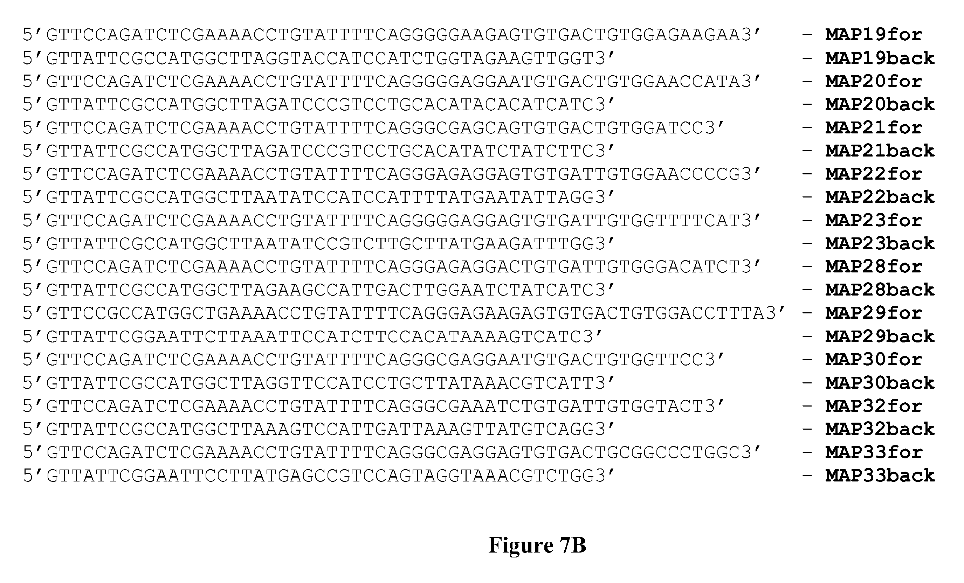

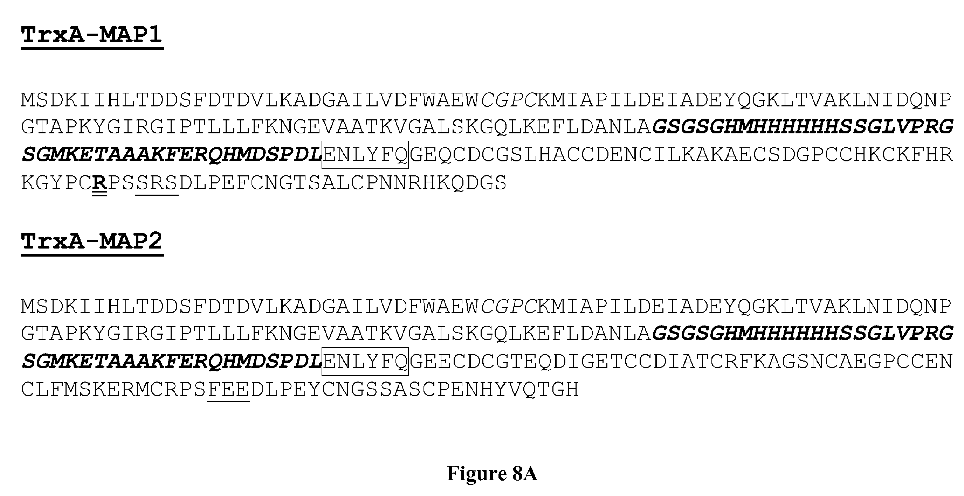

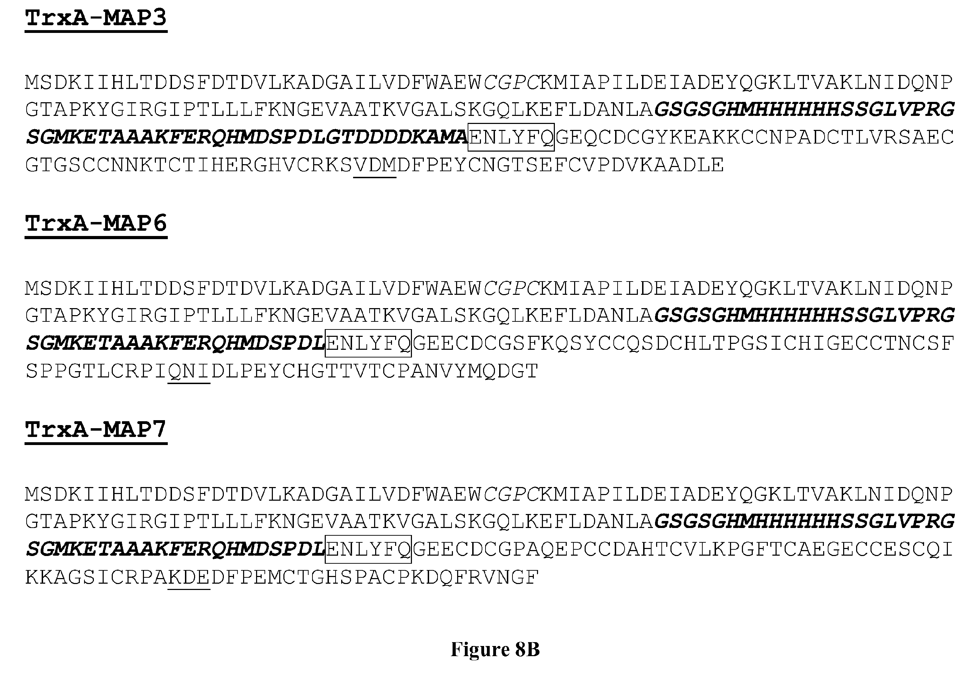

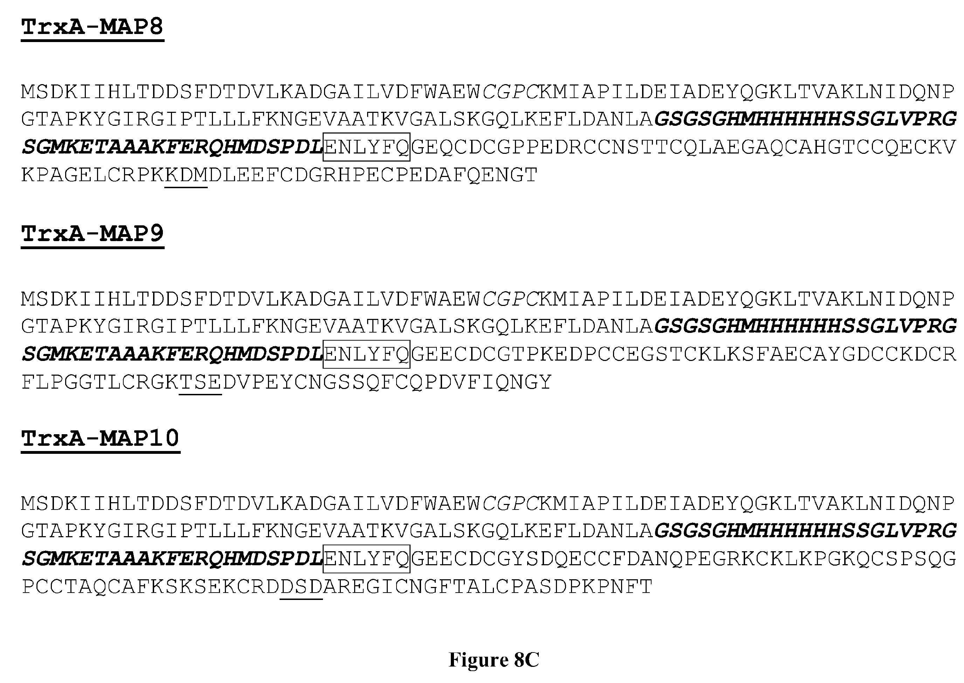

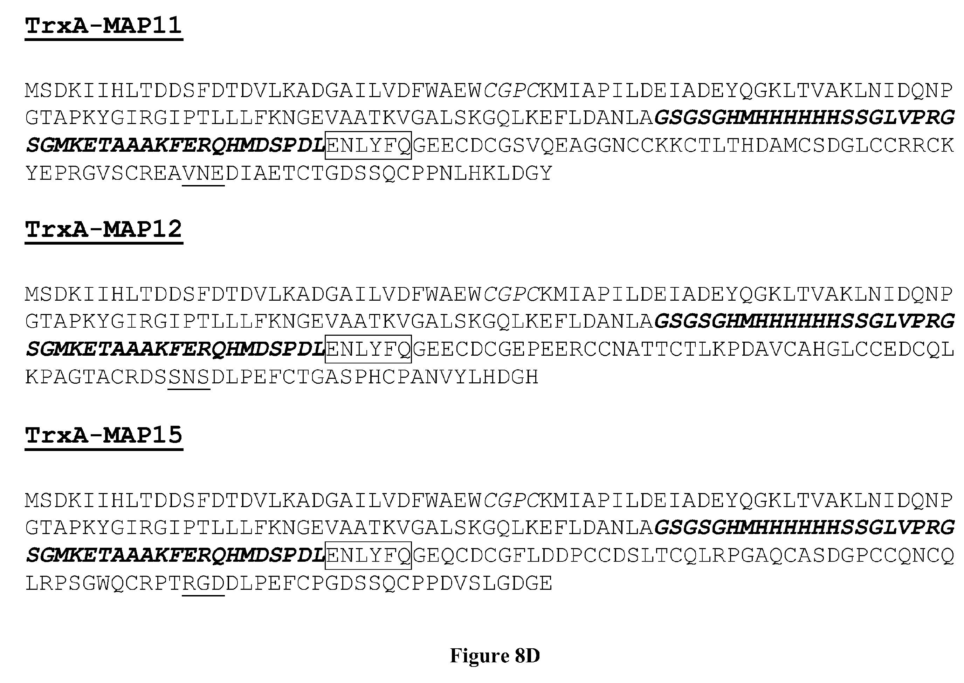

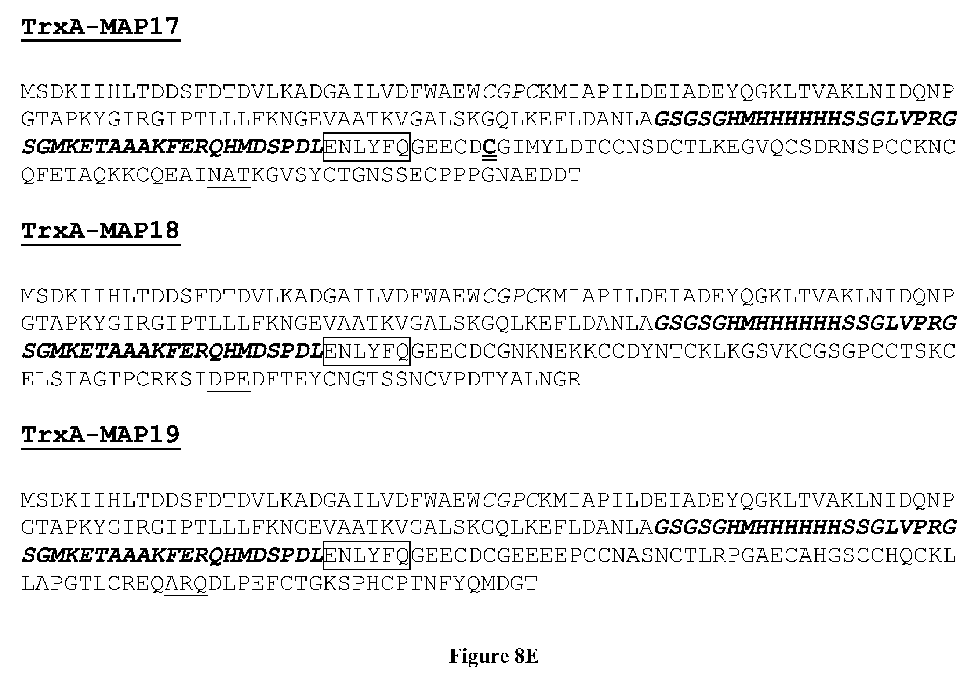

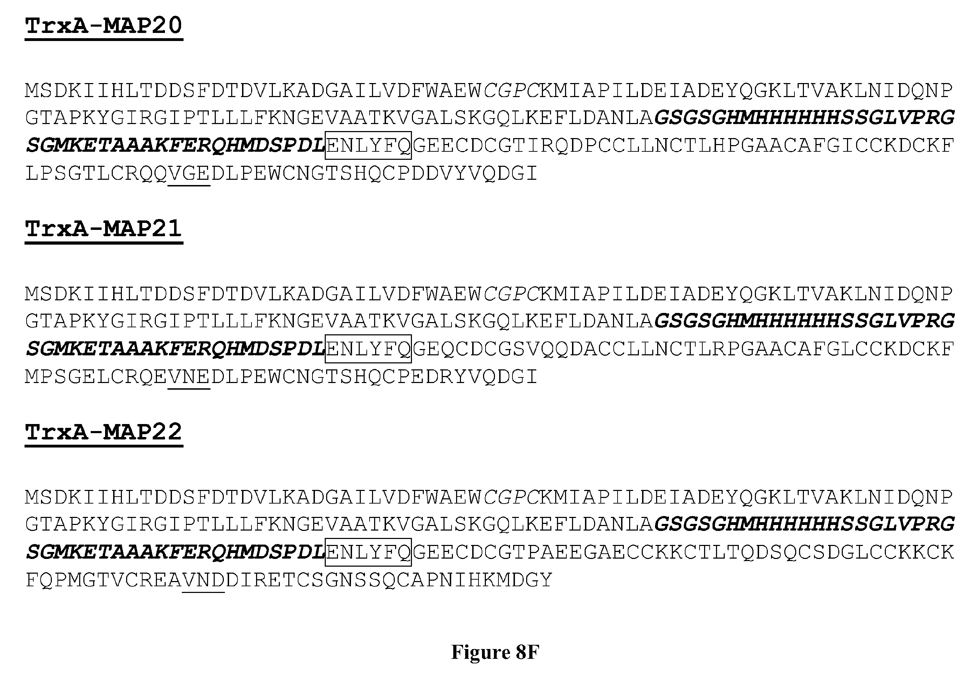

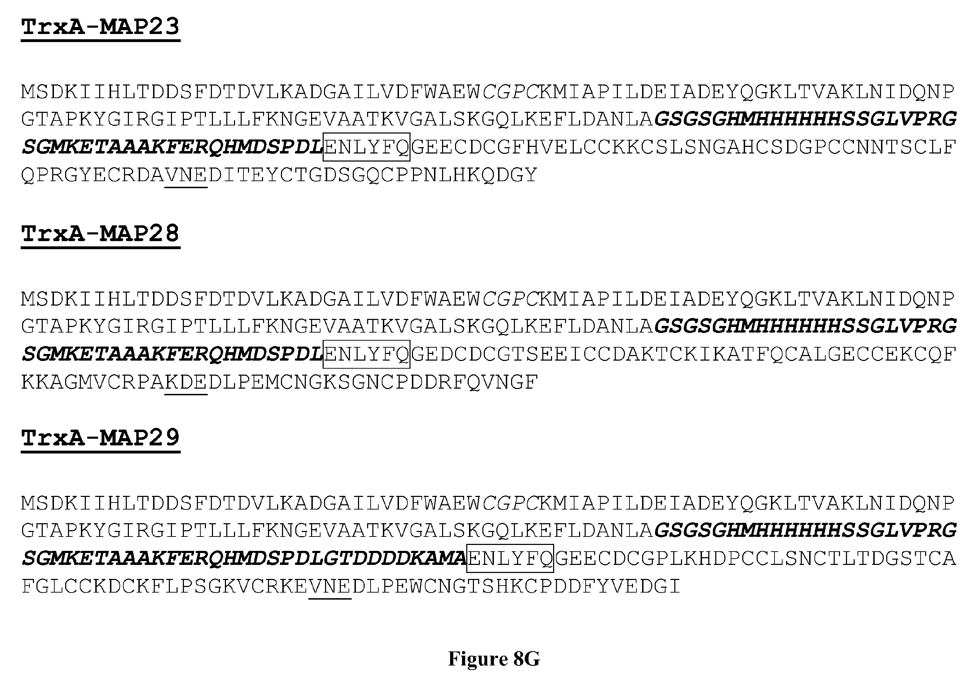

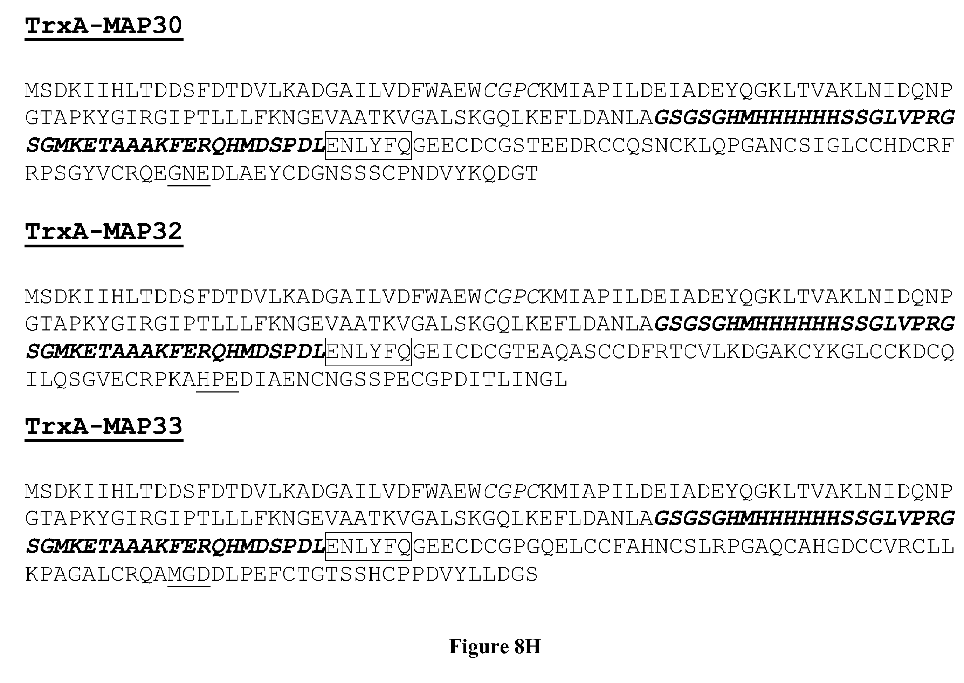

FIG. 6 shows a listing of synthetic MAPs DNA sequences that were cloned into pET32a expression vector. FIG. 7 shows the corresponding list of oligonucleotide primers utilized for MAPs cloning into pET32a vector. FIG. 8 shows the amino acid sequences of all TrxA-MAP constructs that were expressed in Origami B (DE3). The active site of TrxA and the tripeptide motif at the tip of the disintegrin loop are underlined, the TEV cleavage site is highlighted in a box and the linker region between TrxA and various MAP constructs is in ***bold black and italicized.*** The new residues introduced to replace the native residues in MAPs 1 and 17 are highlighted in **bold double-underlined.**

Bacterial cells and reagents. The Origami B (DE3) E. coli strain and pET32a expression vector carrying the bacterial thioredoxin A gene (trxA) were purchased from Novagen (San Diego, CA). All 23 MAP DNA sequences were *de novo* synthesized and inserted into a plasmid by Epoch Biolabs, Inc. (Sugar Land, TX). The AP DNA sequences were PCR amplified from cDNA libraries built from several mammalian cell lines including HUVEC (PromoCell GmbH, Heidelberg, Germany), MDA-MB-435 (ATCC, Manassas, VA), MDA-MB-231 (ATCC, Manassas, VA), and Jurkat (ATCC, Manassas, VA). The oligonucleotide primers used for further cloning the APs and MAPs DNA sequences into pET32a expression vector were synthesized by Operon Biotechnologies, Inc. (Huntsville, AL). All restriction enzymes and ligases used for cloning the APs and MAPs DNA sequences into pET32a expression vector were purchased from New England Biolabs, Inc. (Ipswich, MA). The recombinant TEV protease was purchased from Invitrogen (Carlsbad, CA).

Construction of MAP expression vectors and recombinant production. The synthetic MAPs DNA sequences that were cloned into pET32a expression vector downstream of TrxA are listed in FIG. 6A-F. The oligonucleotide primers used for MAPs cloning are listed in FIG. 7A-B. The generated pET32a plasmids carrying the DNA sequences of MAPs cloned downstream of TrxA gene were initially amplified in DH5α E. coli, purified and sequenced before being transferred into Origami B (DE3) E. coli. The transformed cells for each MAP construct were then plated on LB-Agar supplemented with carbenicillin (50µg/mL), tetracycline (12.5µg/mL), and kanamycin (15µg/mL) and grown overnight at 37°C. From these plates, multiple cultures were established for each MAP construct from individual colonies of transformed Origami B by transferring these colonies into LB media containing carbenicillin (50µg/mL). These initial cultures were grown overnight and further used for the inoculation of bigger volumes of LB media containing carbenicillin (50µg/mL) that were grown at 37°C and 250rpm in a shaker-incubator until they reached an OD600 of 0.6-1. At this point, the cells from individual MAP cultures were induced using 1mM IPTG and incubated for another 4-5 hours at 37°C and 250 rpm. At the end of the induction period, the cells from individual MAP cultures were pelleted at 4000xg and lysed in a microfluidizer (Microfluidics M-110L, Microfluidics, Newton, MA). The operating conditions of the microfluidizer included applied pressures of 14,000-18,000 psi, bacterial slurry flow rates of 300-400 ml per minute and multiple passes of the slurry through the processor. The insoluble cellular debris from lysates processed from individual MAP cultures was removed by centrifugation (40,000xg) and the soluble material containing Trx-MAPs for each MAP culture was collected. The expressed fusion proteins (i.e., Trx-MAPs) in the collected soluble lysates were then proteolysed by incubation with recombinant TEV protease overnight at room temperature which efficiently cleaved off each individual MAP from its TrxA fusion partner as monitored by SDS-PAGE. When proteolysis was complete, the proteolyzed lysates were passed through a 0.22µm filter, diluted 1:100 in double distilled H₂O, ultrafiltrated through a 50,000 MWCO cartridge (Biomax50, Millipore) and then reconcentrated against a 5,000 MWCO cartridge (Biomax5, Millipore) using a tangential flow ultrafiltration device (Labscale TFF system, Millipore).

The APs were cloned into pET32a, transformed into Origami B, and expressed using the same procedures described above for MAPs.

Purification of recombinant MAPs. The MAPs were purified from filtrated lysates by employing a high-performance liquid chromatography (HPLC) procedure according to a protocol previously established for snake venom disintegrins [2]. Purification was performed by C18-reverse phase HPLC using the standard elution conditions previously employed for the purification of native CN [2]. Individual filtrated lysates processed as described above were loaded onto a Vydac C18 column (218TP54, Temecula, CA). A ten-minute rinse (at 5 ml/min) of the column with an aqueous solution containing 0.1%TFA was followed by a linear gradient (0-100%) elution over 150 min in a mobile phase containing 80% acetonitrile and 0.1%TFA. The MAPs start eluting in 35-40% acetonitrile.

Expression analysis of MAPs. *E. coli* transformants were grown overnight at 37°C in shaker flasks were induced in 1mM IPTG for 5 hours at 37°C and 250rpm. At the end of the induction period, the cells were pelleted at 4,000xg, lysed by multiple freeze-thaw cycles, and further centrifuged at 40,000xg to remove the insoluble cell debris. 5µl of soluble cell lysates from various *E. coli* hosts were loaded under reducing conditions on a precast 4-20% NuSep iGel (NuSep Inc., Lawrenceville, GA) and then Coomassie stained.

The resultant expression yield of AP9 and AP15 was lower than that compared to their corresponding MAPs (MAP9 and MAP15) (FIGS. 4 and 5). Both MAP9 and MAP15 were generated in Origami B (DE3) with batch-to-batch expression yields ranging from 200mg to 350mg of HPLC-purified protein per liter of bacterial culture. These high yields of purified recombinant MAPs were achieved by lysis of the pelleted bacterial transformants at the end of the induction step with a microfluidizer. A similar production technique generated recombinant MAPs with similar expression yields in the Origami B (DE3) system (FIGS. 9-11). In contrast, the expression of MAPs in closely related bacterial hosts that also support the formation of disulfide bridges (e.g., the AD494 (DE3), a K12 derivative that carries the trxB mutation only, or the Rosetta-gami B (DE3), an Origami B derivative optimized for rare codon usage) appeared to offer no additional advantage over the production in Origami B (DE3).

### Example 2

### Differential Cell Binding of MAPs

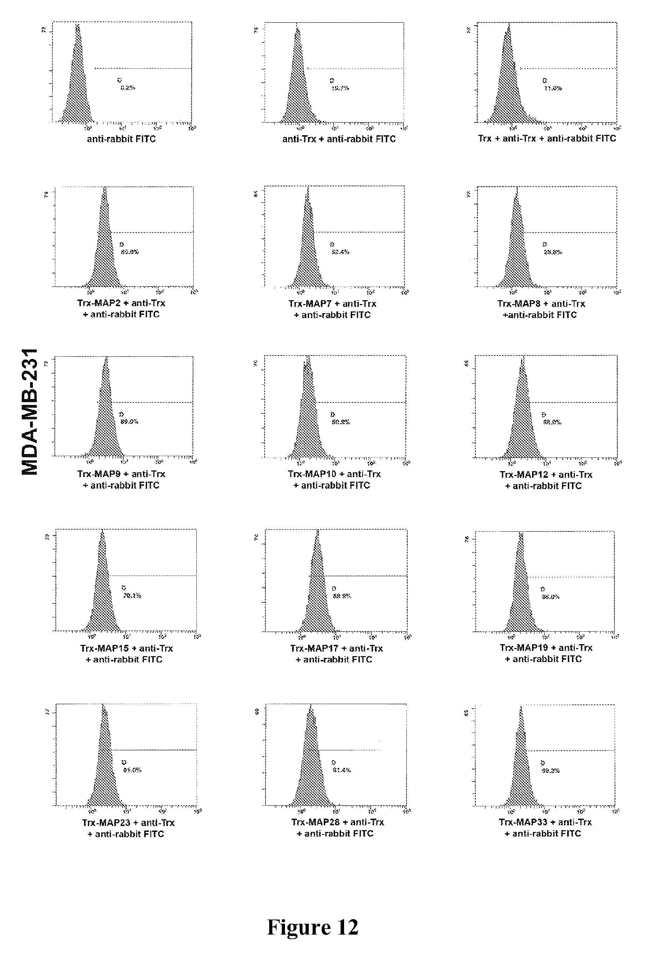

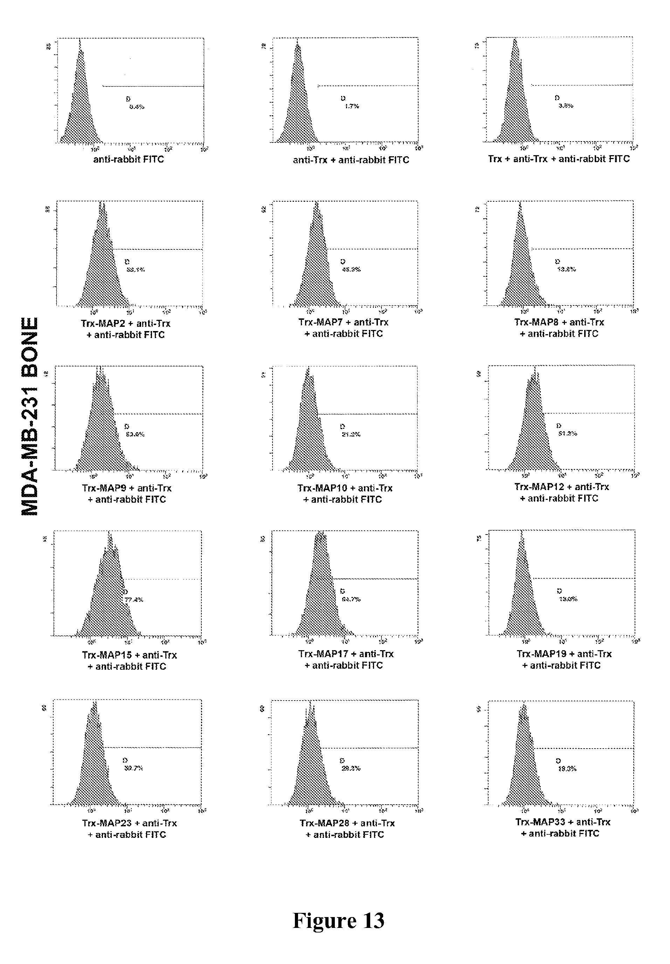

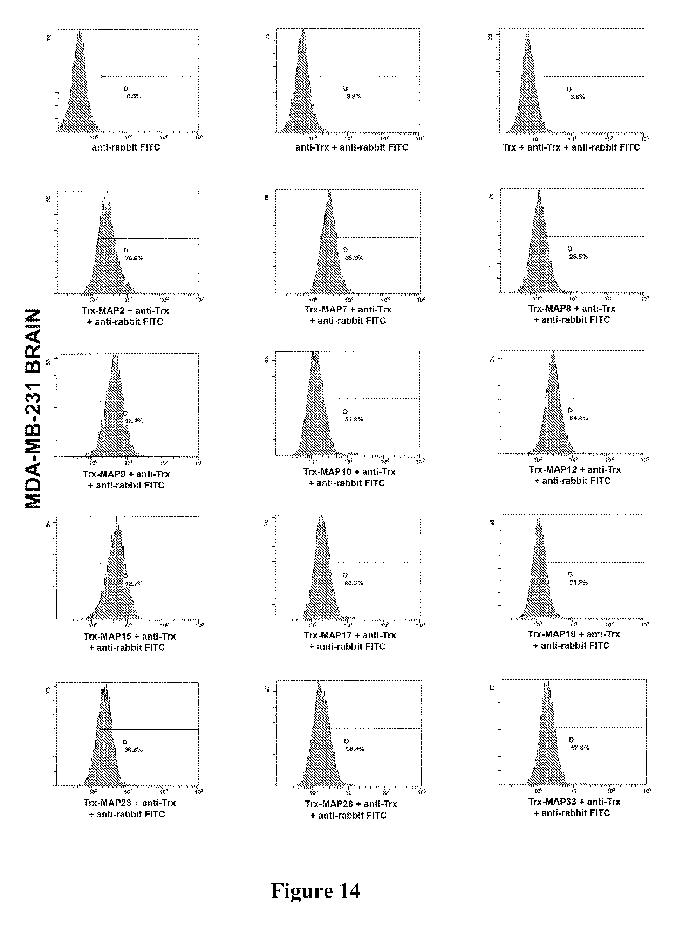

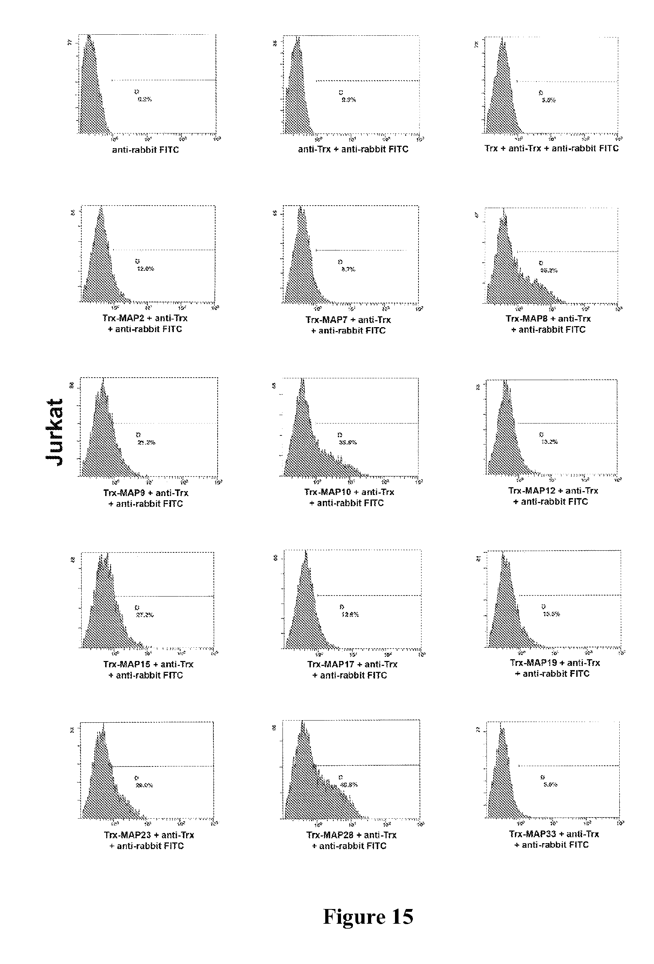

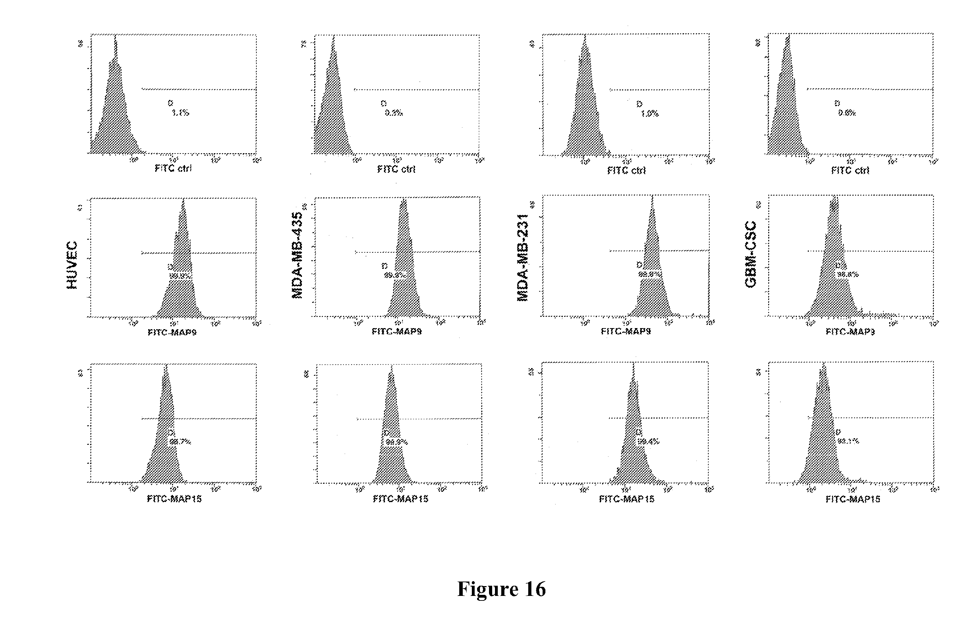

The Trx-MAPs produced in Origami B (DE3) using the method described above were analyzed by flow cytometry for differential binding to multiple cell lines (FIGS. 12-15), which included a human breast carcinoma line (MDA-MB-231) and two metastatic subclones of this line with tropism for different organs based on differential integrin profiles - a bone-homing subclone (MDA-MB-231 BONE) and a brain-homing subclone (MDA-MB-231 BRAIN). The 2 subclones of the MDA-MB-231 line were a gift from the investigators who originally isolated them [31]. Cells were incubated with the indicated Trx-MAPs and probed with the corresponding anti-Trx polyclonal antisera (Sigma-Aldrich, Inc., St. Louis, MO). The bound molecules were further detected with an anti-rabbit FITC-labeled antibody. Cells incubated with either the secondary FITC-labeled antibody only or the anti-Trx antisera plus the secondary antibody were used as controls. The Trx-MAPs 8 and 28, which correspond to ADAMs 8 and 28 that have been previously shown to be expressed on different lineages of immune cells and participate in immune responses, preferentially bind to Jurkat cells (a T-cell leukemia cell line) as shown by flow cytometry analysis (FIG. 15). Moreover, two HPLC-purified MAPs (MAP9 and MAP15) were FITC-labeled and analyzed by flow cytometry for direct binding to cancer cells lines (MDA-MB-231, MDA-MB-435), a cancer stem cell population isolated [32] from human glioblastoma (GBM-CSC) as well as human umbilical vein endothelial cells (HUVEC). Flow cytometry data (FIG. 16) shows that the purified MAP9 and MAP 15 bind avidly to different cell lines that are expected to display vastly different integrin profiles.

### Example 3

### Antiangiogenic Effect of MAPs

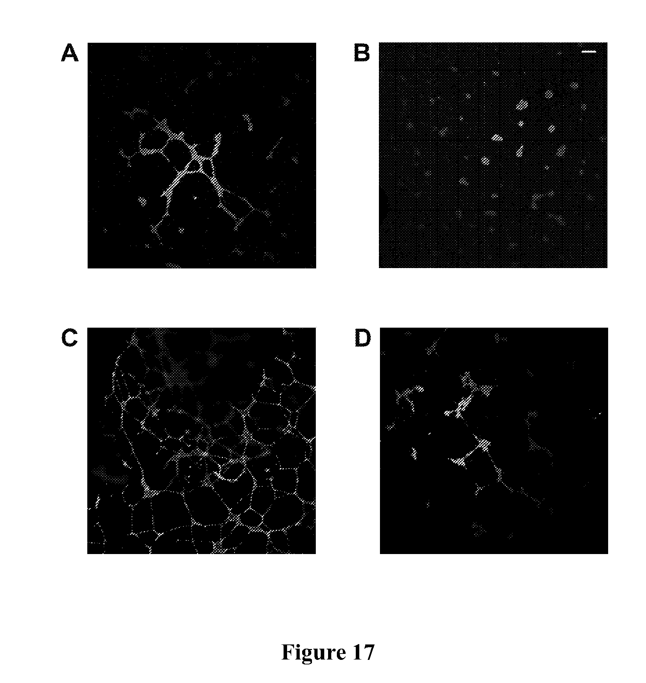

MAPs were then tested for angiogenic activity using the *in vitro* HUVEC tube formation assay. HUVEC cells were plated on 'Endothelial Cell Tube Formation' plates (BD Biosciences) in the presence of 10nM of either MAP9 or MAP 15. A known tube formation inhibitor (Suramin) was used as a negative control. See FIG. 17: Panel A - untreated control; panel B - 100µM Suramin; panel C - 10nM MAP9; panel D - 10nM MAP15. Cells were stained with Calcein AM and imaged using confocal microscopy. All images were taken at the same magnification (scale bar = 50µm). MAP15 showed significant anti-angiogenic activity in this assay. MAP9 appeared to have a pro-angiogenic effect by leading to the formation of an increased number of tubes when compared to the untreated control in this assay. This pro-angiogenic effect of MAP9 is supported by *in vivo* observations showing that a liposomal formulation of MAP9 promotes tumor growth (i.e., faster tumor growth, bigger tumors and a decreased survival compared to an untreated control) when administered intravenously in the MDA-MB-231 xenograft animal model.

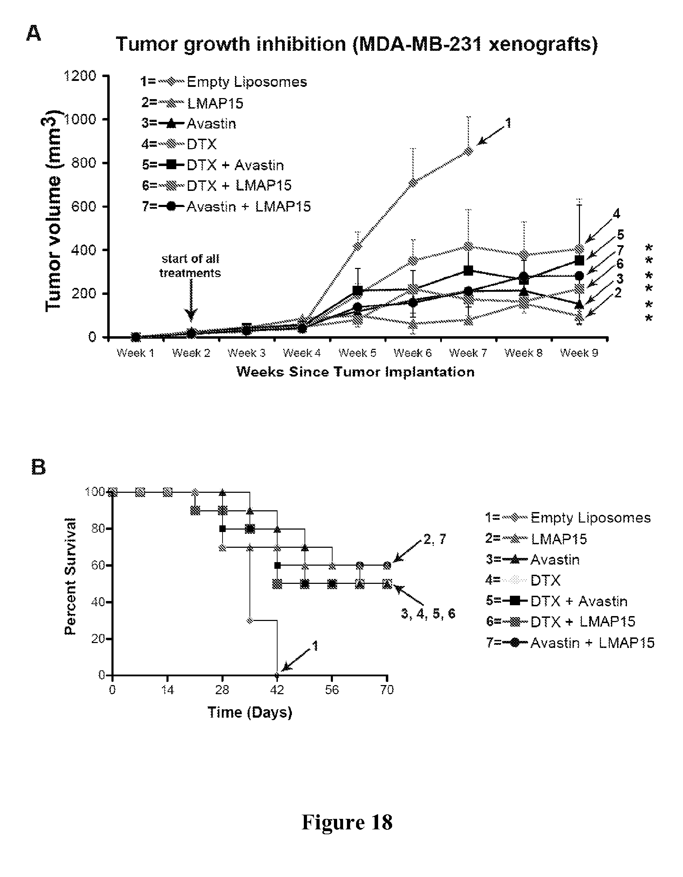

FIG. 18A shows inhibition of tumor growth induced by different treatments in the MDA-MB-231 model. Nude mice inoculated orthotopically (mammary fat pads; 2.5x10⁶ MDA-MB-231 cells per mouse in complete Matrigel) were allowed to grow palpable tumors before treatment was commenced (indicated by the arrow). Groups of animals (n=10) were treated intravenously with LMAP15, the dose-equivalent of 100µg of MAP 15 per injection, administered twice a week, or Avastin (400µg per injection; approx. 20µg/gr) administered intravenously once per week, or docetaxel (DTX, 160µg) administered intraperitoneally once per week, or combinations of these agents. The control group received empty liposomes only. When compared to the control group, a significant delay in tumor growth was observed in all treated groups. The statistical analysis was done using ANOVA with Dunnett's *post-hoc* multiple comparison tests (* signifies a P<0.001). FIG. 18B shows animal survival data. Treatment groups showed increased survival compared to the control group (all control animals died by week 7). Either LMAP15 or Avastin plus LMAP15 groups had the highest survival. The in vivo efficacy data from this animal model indicates that LMAP15 exhibits similar antitumor potency to either Avastin or Docetaxel. The animals treated with liposomal MAP 15 alone showed a better survival rate (i.e., the number of animals still alive at the end of the study) when compared to those treated with either Avastin or Docetaxel or combination therapy in the same xenograft model.

### Example 4

### Antiangiogenic Effect of LMAP15

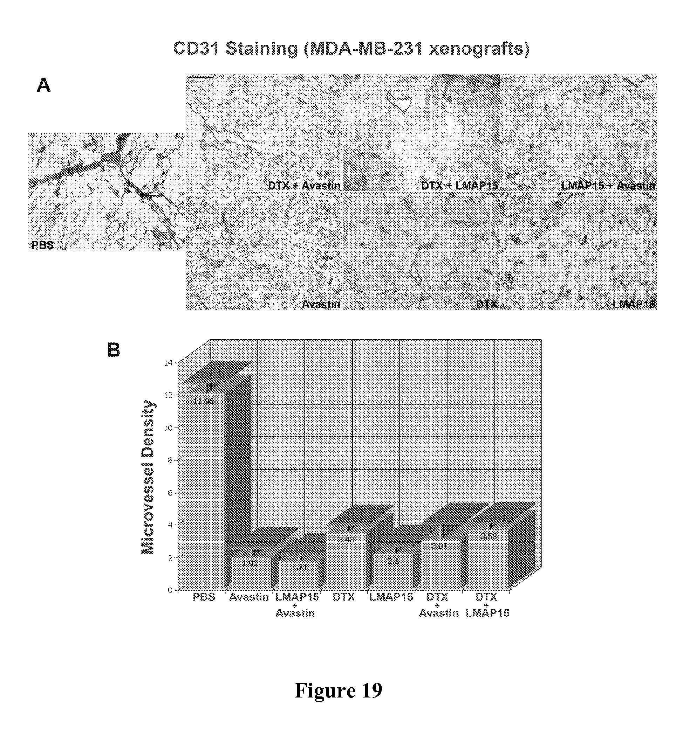

To assess the antiangiogenic effect of LMAP15, administered either alone or in combination, tumors from each group in the MDA-MB-231 study were dissected and extracted from dead or sacrificed animals and subsequently analyzed for microvessel density by immunohistochemistry. The extracted tumors were embedded in Tissue-Tek O.C.T ('Optimal Cutting Temperature' compound, Sakura Finetek USA) then frozen in dry ice, cut into 5 micron sections, fixed in acetone and stored at 4°C until stained. For CD31 staining, the acetone-fixed slides were washed in PBS and blocked in PBS containing 5% goat serum then incubated overnight at room temperature with 200 µl of a rat polyclonal anti-CD31 antibody (BD Biosciences, San Diego, CA) diluted 1:50 in PBS and applied to the slides according to the manufacturer's protocol. This was followed by multiple washings in PBS (7 minutes/wash) and addition of 200 µl of a biotinylated secondary goat anti-rat antibody diluted 1:100 and applied for 45 minutes at room temperature. After 3 more washings in PBS, 200 µl of Avidin Binding Complex (Vector Laboratories, Burlingame, CA) diluted 1 drop in 2ml PBS was applied to each slide for 30 minutes at room temperature after which the 3-amino-9-ethylcarbazole chromogen was added to visualize the antibody-stained microvessels. After three more washings in PBS, the slides were counterstained with Myers Hematoxylin and then mounted. To quantitate the CD31-stained microvessels, the slides were subjected to 'random field' analysis [35, 36]. Images were captured blindly from random fields on each slide at 200x using an Olympus E20N digital camera (Olympus America, Melville, NY) attached to a microscope. For each group, 4 tumors were stained and 40 random fields analyzed. The CD31-positive areas were computed for each random field as % of total stained area using the 'SimplePCI' advanced imaging software (C-Imaging Systems, Cranberry Township, PA) and then averaged for each group. To eliminate bias, the random field image capture and the subsequent processing and analysis of the captured images were carried out in a blind fashion. LMAP15 was shown to significantly reduce microvessel density in this xenograft model (FIG. 19) when administered as either monotherapy or in combination with other anti-angiogenics with a different mechanism of action (e.g., Avastin) or chemotherapeutics (e.g., Docetaxel).

### Example 5

### Metastatic Breast Cancer Model

To evaluate the MAPs therapeutic efficacy as anti-invasive/anti-metastatic agents the optical luciferase imaging approach (in vivo bioluminescence) is employed in several animal models of spontaneous metastatic breast cancer. The following cell lines are stably infected with an adenoviral transduction system: a human breast cancer cell line (the MDA-MB-231, a triple-negative cell line), and two murine breast cancer lines (the 4T1, a HER2-negative line, and the D2F2, a HER2-positive line) with both luciferase and green fluorescence protein (GFP) reporter genes. In the human xenograft MDA-MB-231 model, an inoculum of 2x10⁶ cells suspended in complete Matrigel is injected in the mammary fat pads of nude mice and allowed to grow until the formed tumors are palpable (approx. 2 weeks after implantation). The following treatment groups (5 animals per group) are formed: group 1 control (animals receiving no treatments or manipulations), group 2 control (animals receiving empty liposomes intravenously), group 3 control (animals receiving PBS intravenously), group 1 treated (animals receiving Avastin intravenously), group 2 treated (animals receiving Docetaxel intraperitoneally), group 3 treated (animals receiving a combination of Avastin intravenously and Docetaxel intraperitoneally), group 4 treated (animals receiving MAP 15 intravenously), group 5 treated (animals receiving a liposomal formulation of MAP 15 intravenously), group 6 treated (animals receiving a combination of MAP 15 intravenously, Avastin intravenously and Docetaxel intraperitoneally), and group 7 treated (animals receiving a combination of a liposomal formulation of MAP 15 intravenously, Avastin intravenously and Docetaxel intraperitoneally). The treatments are administered as follows: Avastin and Docetaxel are administered weekly using the maximum dosages previously reported in the literature as efficacious against primary tumors and metastatic foci in this model, MAP 15 is administered at the dose of 100µg polypeptide/injection every other day, and liposomal MAP 15 is administered at the dose equivalent of 100µg polypeptide/injection twice weekly.

Similar to the MDA-MB-231 model, the MAP 15 efficacy as an anti-invasive/antimetastatic agent is also determined in two murine breast cancer models (the 4T1 and the D2F2 models). In these models an inoculum of 5x10⁵ cells (either 4T1 or D2F2) in PBS is injected in the mammary fat pads of immunocompetent BALB/c mice and allowed to grow tumors that become palpable (approx. 1 to 1.5 weeks after implantation). Once tumors become palpable, the following treatment groups (5 animals per group) are started: group 1 control (animals receiving no treatments or manipulations), group 2 control (animals receiving empty liposomes intravenously), group 3 control (animals receiving PBS intravenously), group 1 treated (animals receiving Avastin intravenously), group 2 treated (animals receiving Lapatinib intravenously), group 3 treated (animals receiving Herceptin intravenously), group 4 treated (animals receiving a combination of Avastin, Lapatinib and Herceptin intravenously), group 5 treated (animals receiving MAP 15 intravenously), group 6 treated (animals receiving a liposomal formulation of MAP 15 intravenously), group 7 treated (animals receiving a combination of MAP 15, Avastin, Lapatinib, and Herceptin intravenously), and group 8 treated (animals receiving a combination of a liposomal formulation of MAP15, Avastin, Lapatinib, and Herceptin intravenously). The treatments are administered as following: Avastin, Lapatinib and Herceptin are administered weekly using the maximum dosages previously reported in the literature as efficacious against primary tumors and metastatic foci in these models, MAP 15 is administered at the dose of 100µg polypeptide/injection every other day, and liposomal MAP 15 is administered at the dose equivalent of 100µg polypeptide/injection twice weekly.

The tumor growth before and after initiating the treatments in mammary fat pads and at distant sites is monitored in all models by weekly Xenogen bioluminescence imaging. Primary tumor size is also measured by caliper and volumes calculated on the basis of the formula: 1 x w2 x0.5. For the weekly bioluminescence (luciferase) imaging, mice are injected via an intraperitoneal route with a Luciferin solution (15 mg/mL or 30 mg/kg, in PBS, dose of 5-50 mg/kg) which is allowed to distribute in non-anesthetized animals for about 5-15 minutes. The mice are then placed into a clear Plexiglas anesthesia box (2-4% isofluorane) which allows unimpeded visual inspection of the animals and monitoring of their breathing status. In this setting, the anesthesia delivery tube that supplies the anesthesia to the box is split so that the same concentration of anesthesia is delivered to the anesthesia manifold located inside the imaging chamber on the Xenogen IVIS 100 imaging instrument. After the mice are fully anesthetized, they are transferred from the anesthesia box to the anesthesia nose cones attached to the manifold in the imaging chamber, the door is closed, and images are acquired using Xenogen instrument. The imaging time is between one to five minutes per side (dorsal/ventral), depending on the experiment. When the animals are turned from dorsal to ventral (or vice versa) they are monitored for any signs of distress or changes in vitality. The acquired images are evaluated by comparing the level luciferase activity at the site of the control and treated tumors, and by comparing the distribution area under the curve associated with the luminescence from tumors in each treatment group. By employing the above approach the efficacy of MAP 15 against primary tumors and bioluminescent metastatic foci is determined. Animal weights are also measured twice weekly and animals are observed visually for any signs of stress response or malaise. Animals that showed severe signs of impairment are excluded from the experiment.

At the conclusion of these studies (after 8 weeks of treatment in the MDA-MB-231 model, and after 3 weeks of treatment in the 4T1 and D2F2 models) a determination of whether MAP 15 alone is as efficacious as or better than any of the monotherapies tested (either Avastin or Docetaxel or Lapatinib or Herceptin) at inhibiting primary tumor growth is made, reducing the number and size or both of metastatic foci, and prolonging animal survival in these tumor models. In addition, when administered in combination with either Avastin plus Docetaxel in the MDA-MB-231 model or in combination with Avastin plus Lapatinib in the 4T1 model or in combination with Avastin plus Lapatinib plus Herceptin in the D2F2 model, MAP 15 proved to be more efficacious than any monotherapy or other combinations at inhibiting primary tumor growth, reducing the number and size or both of metastatic foci, and prolonging survival in these tumor models.

### Appendix

### ADAM Nucleic Acid and Amino Acid Sequences

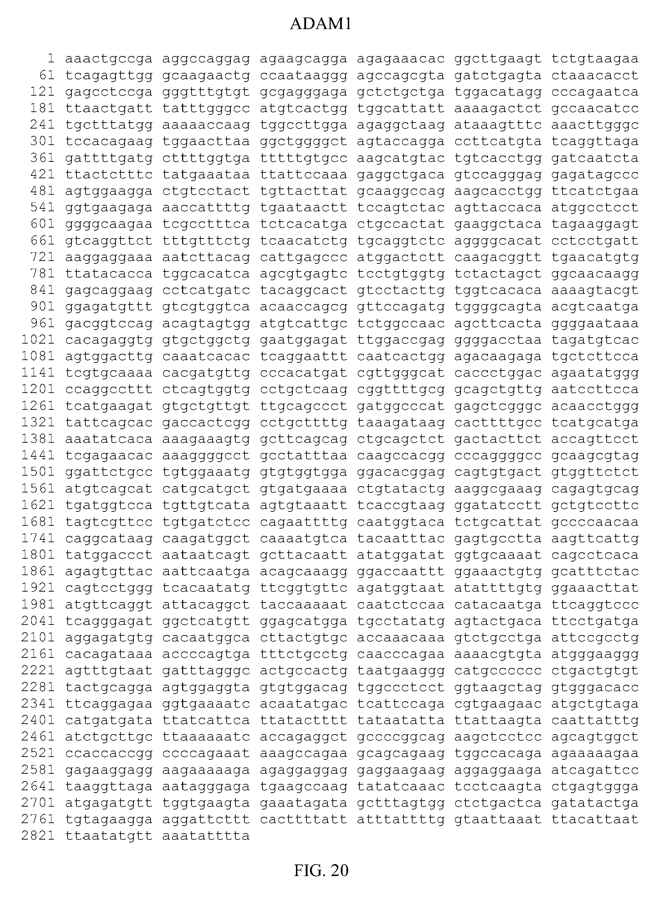

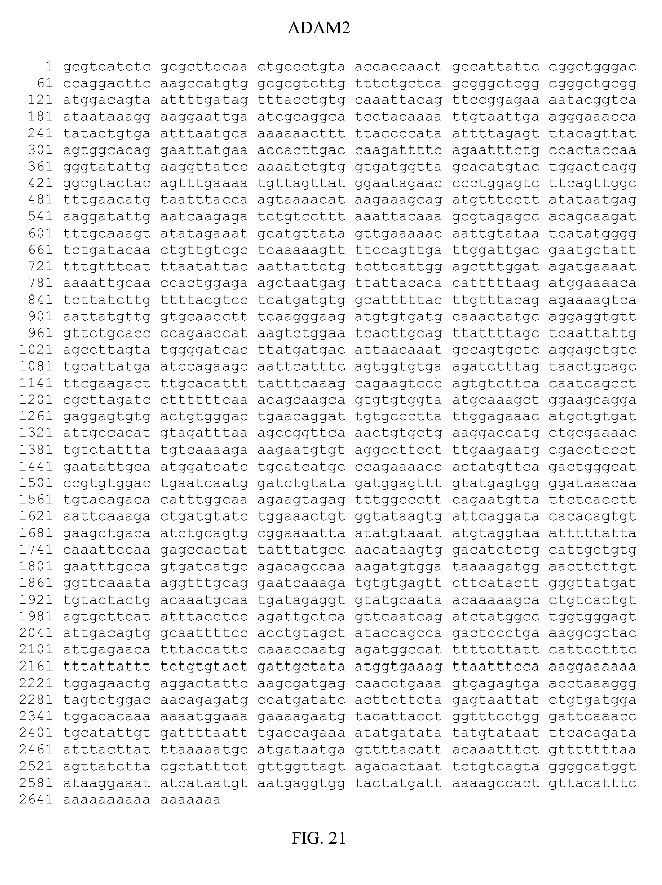

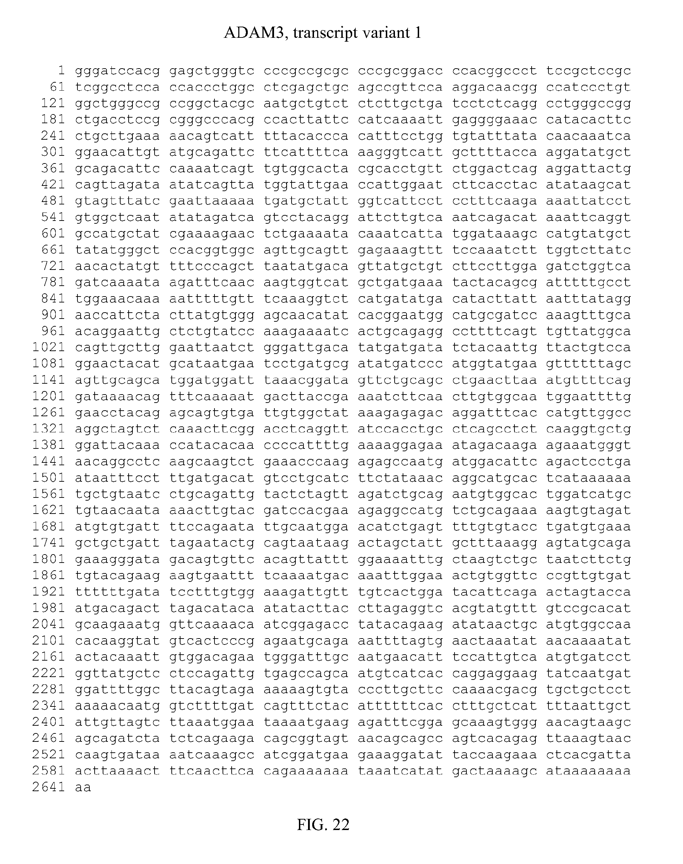

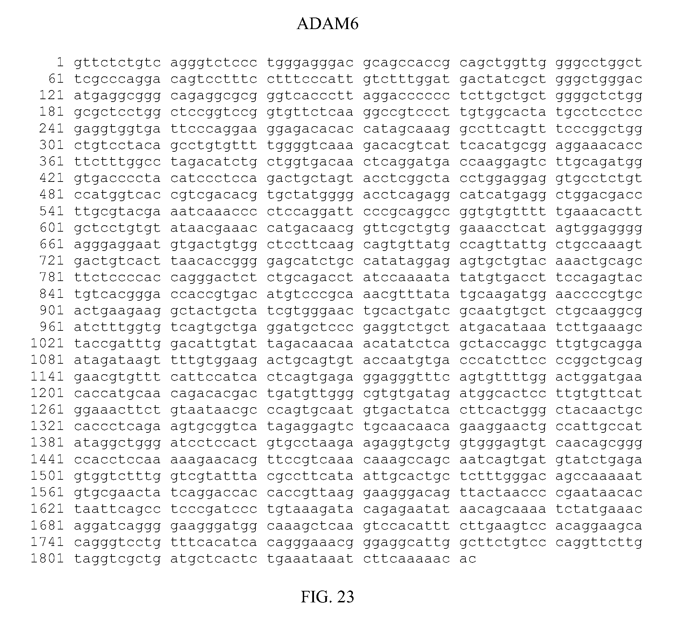

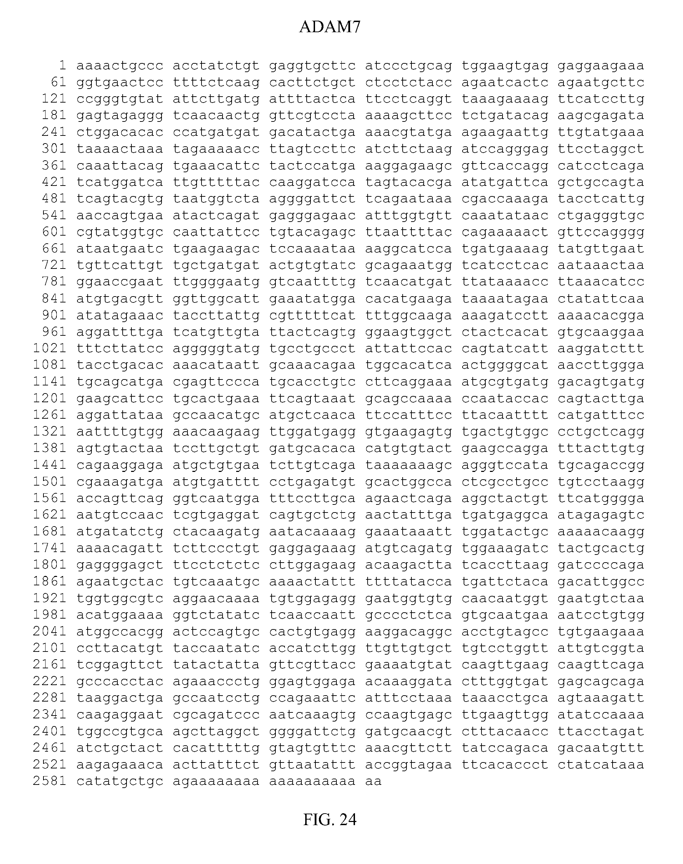

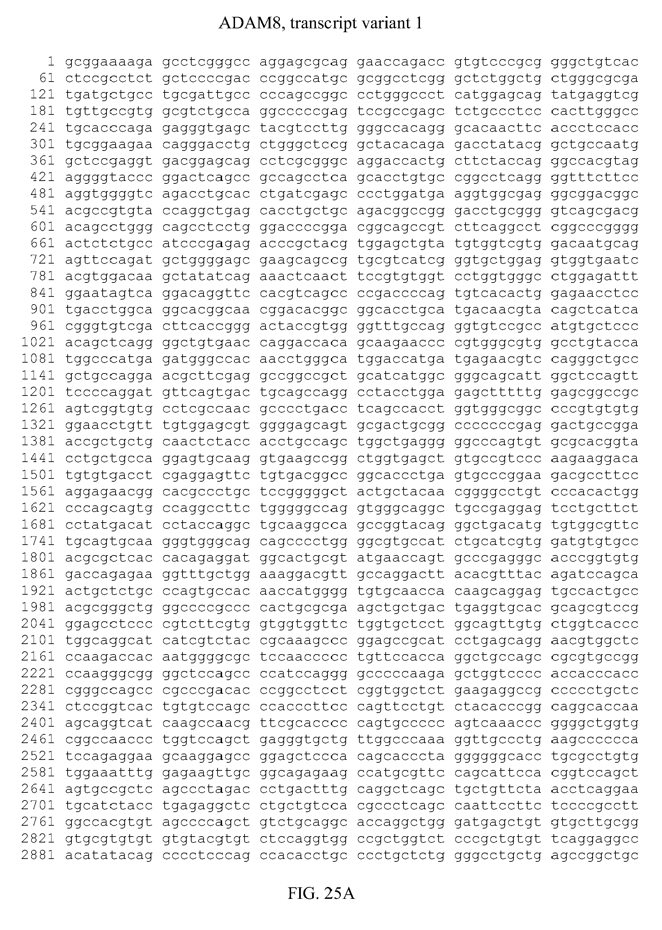

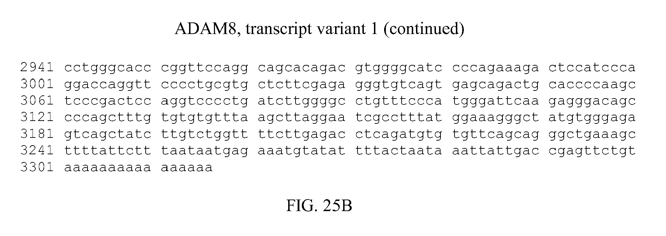

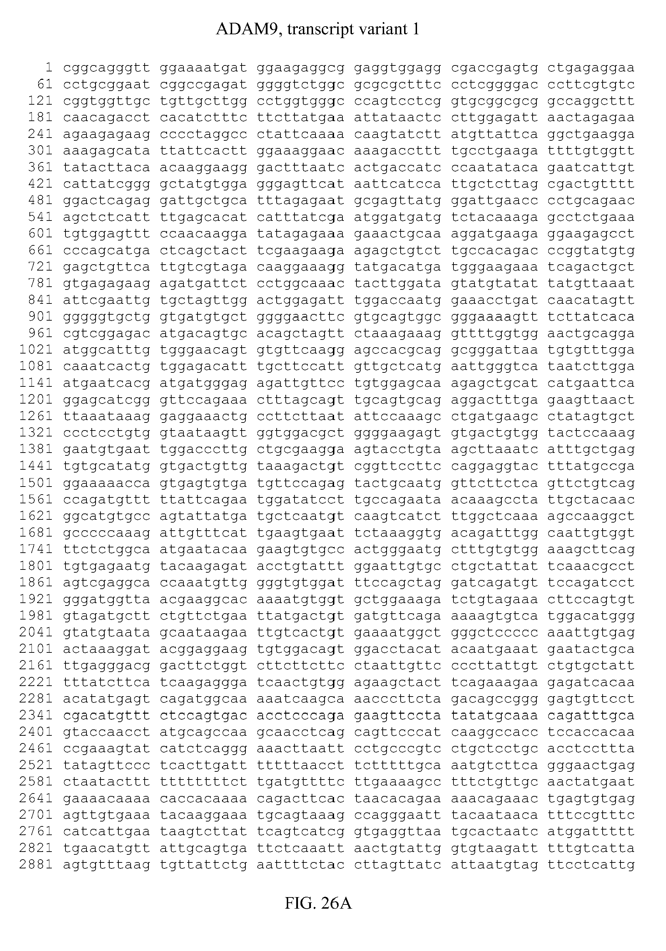

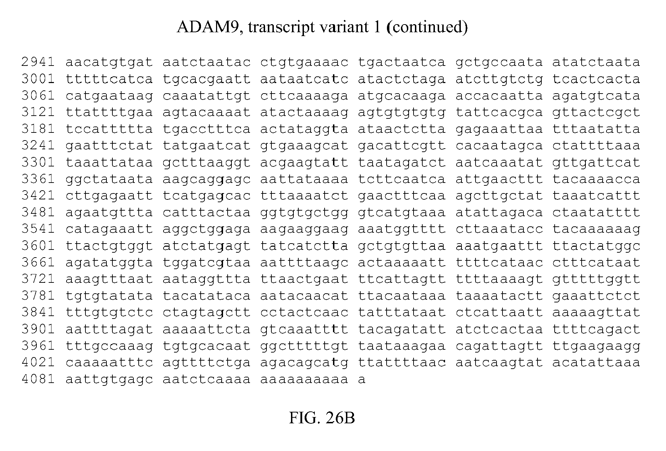

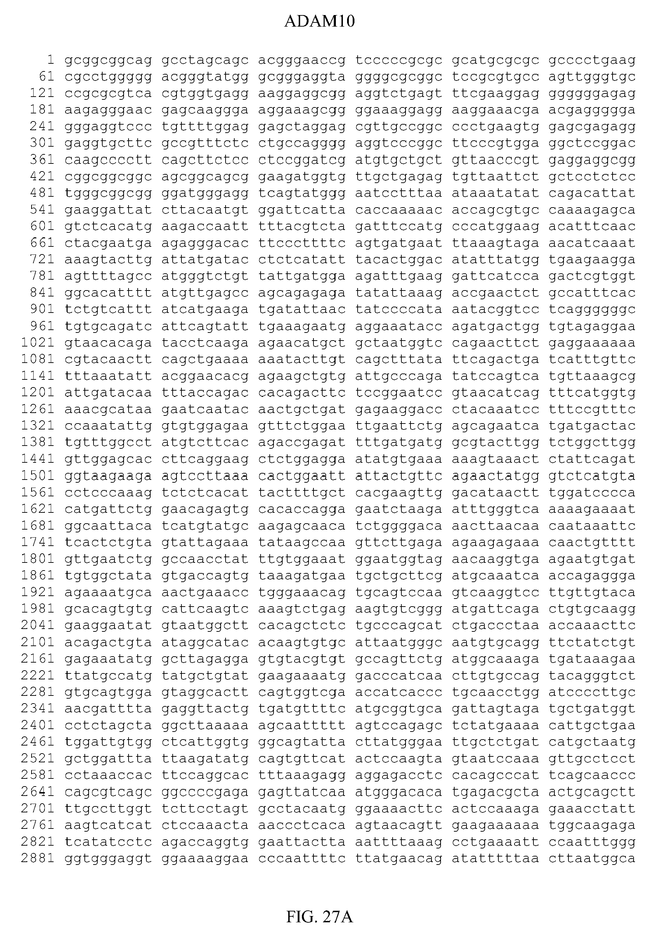

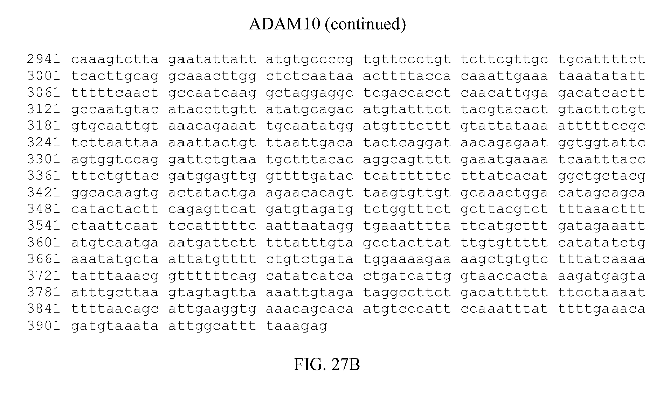

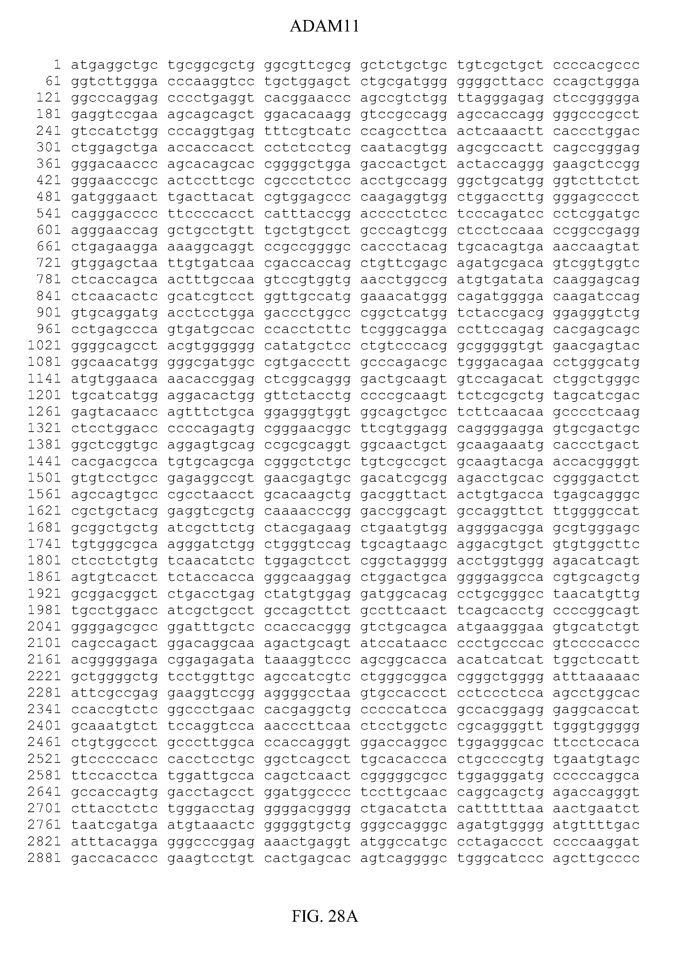

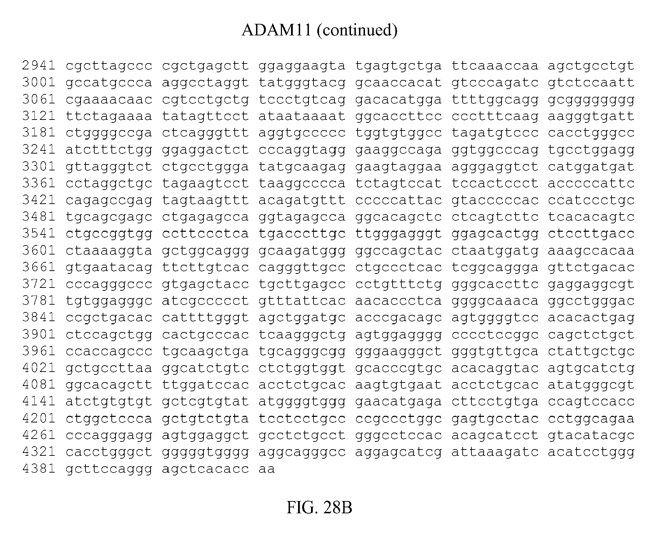

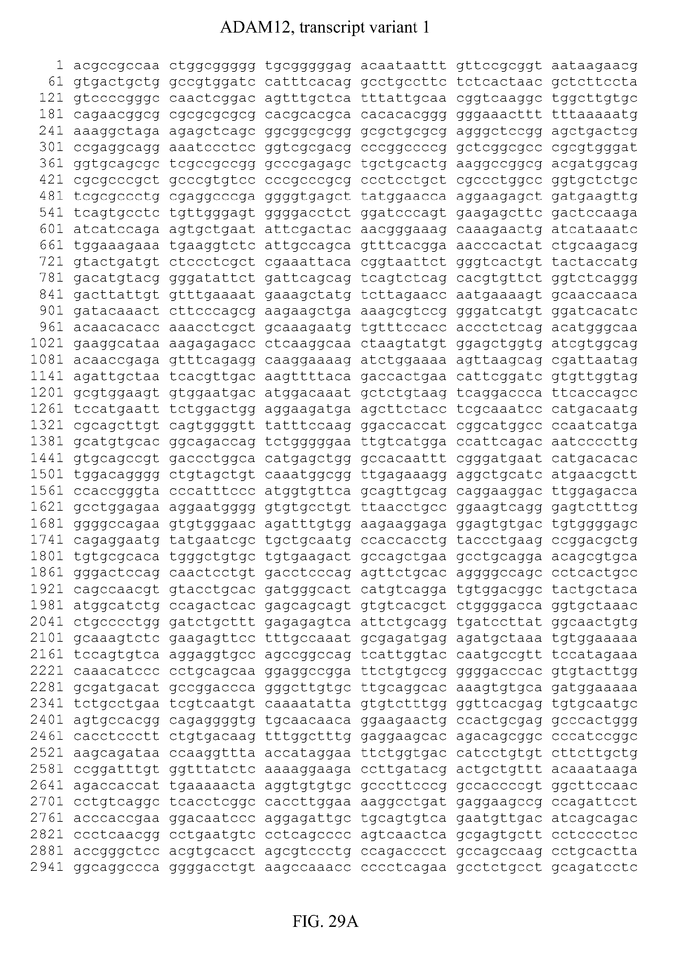

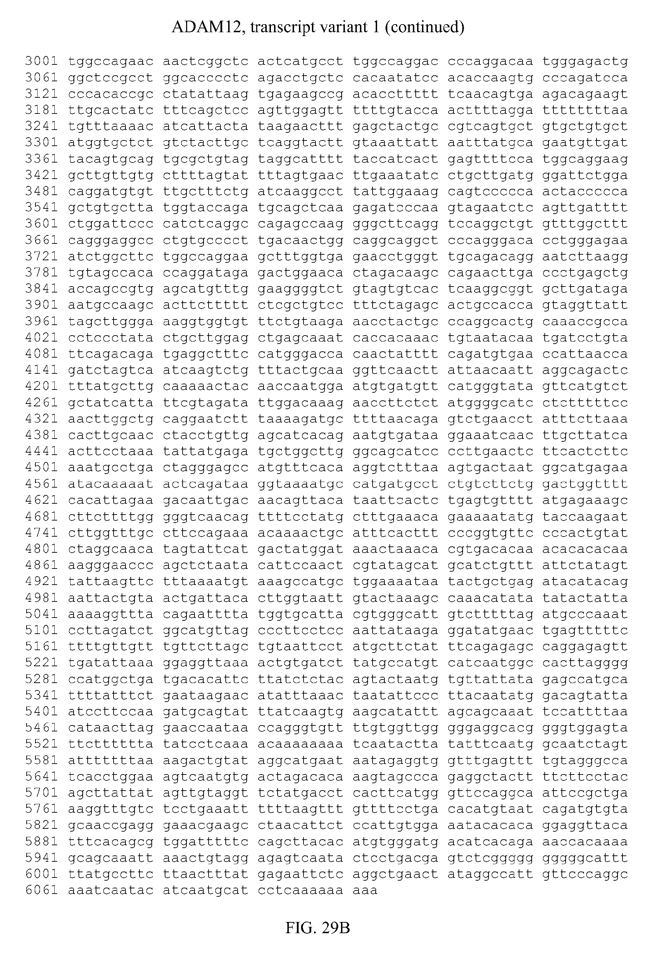

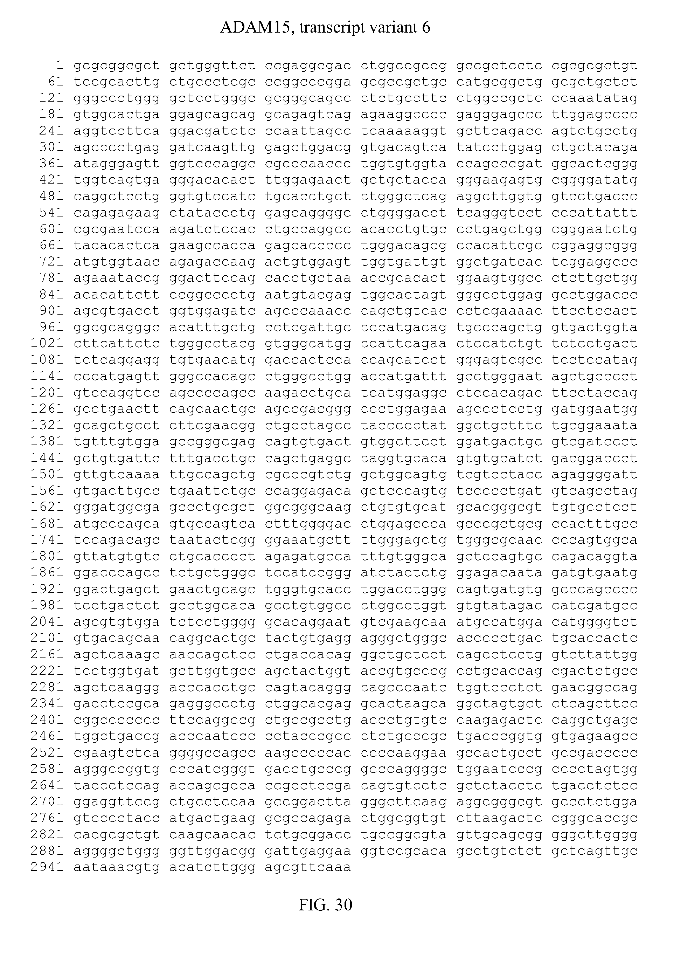

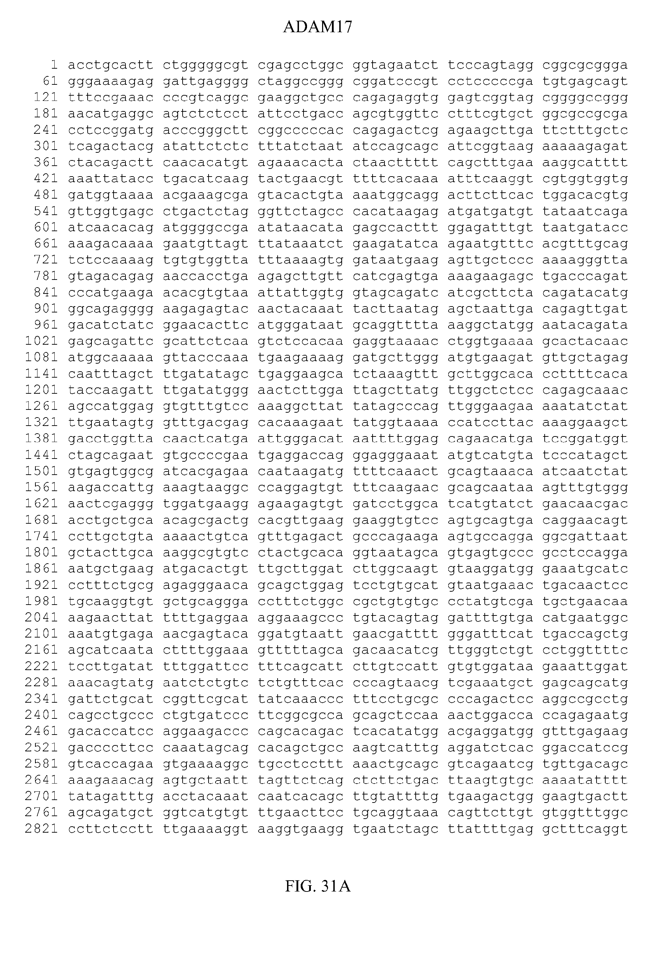

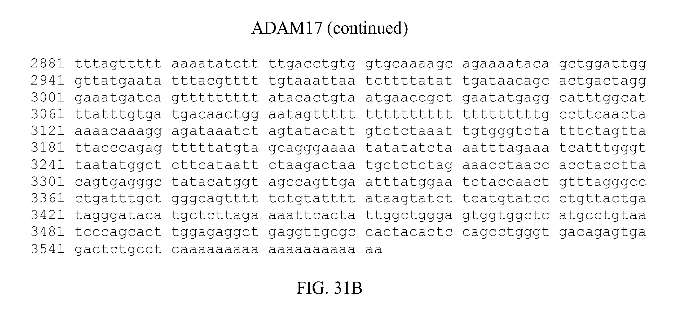

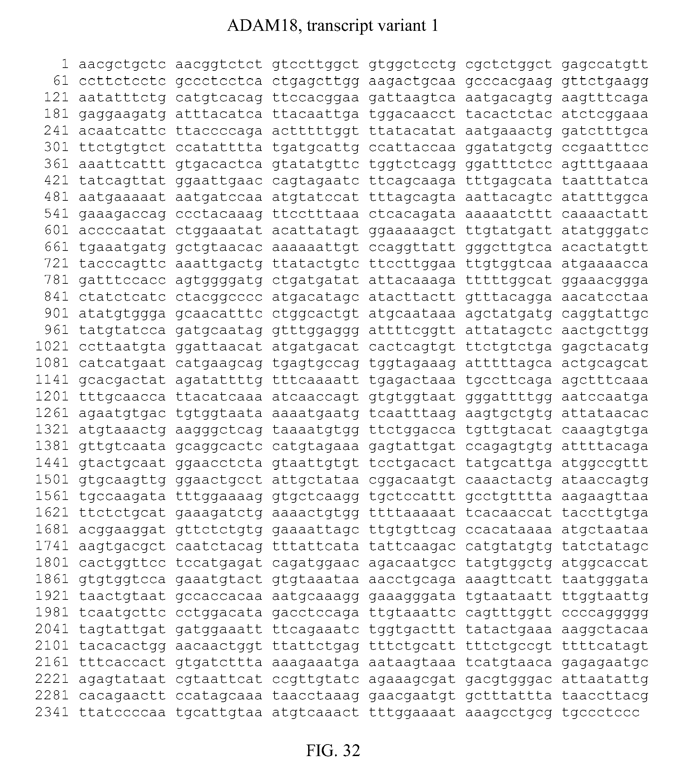

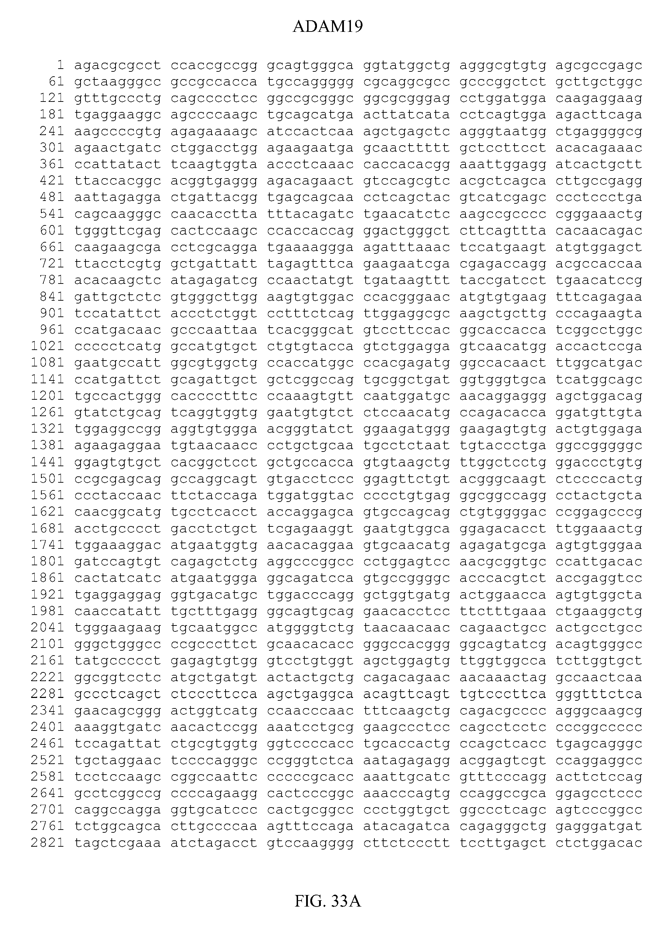

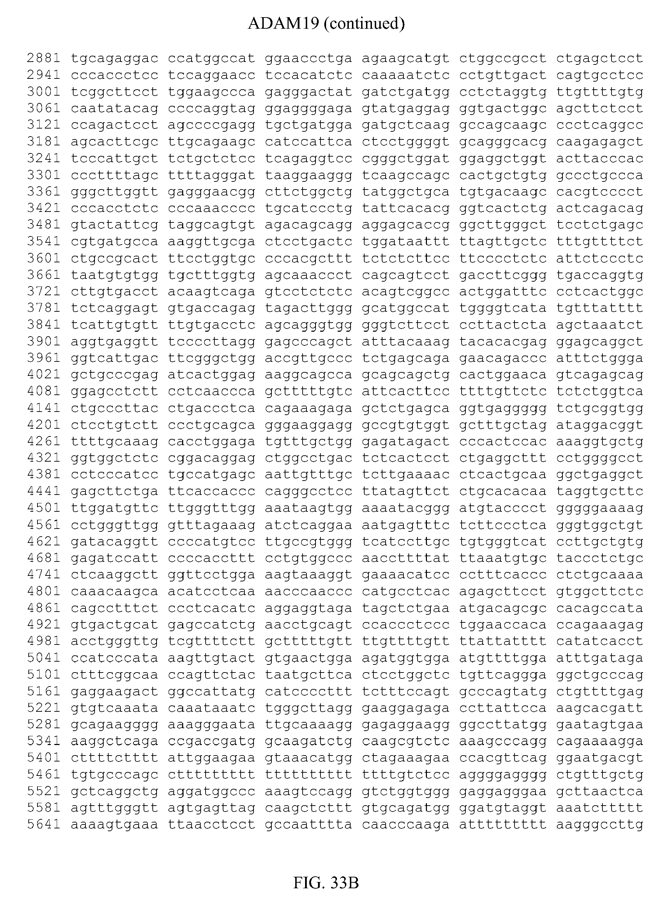

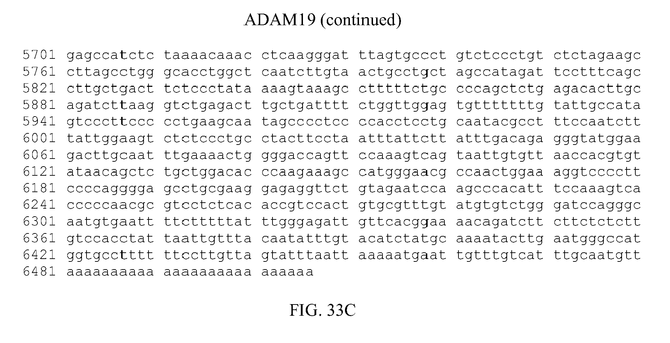

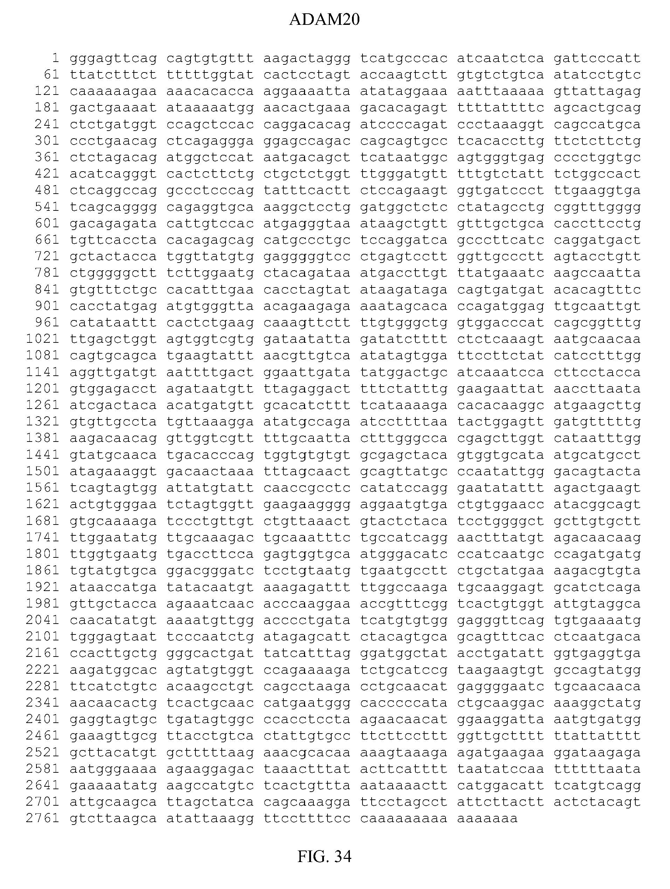

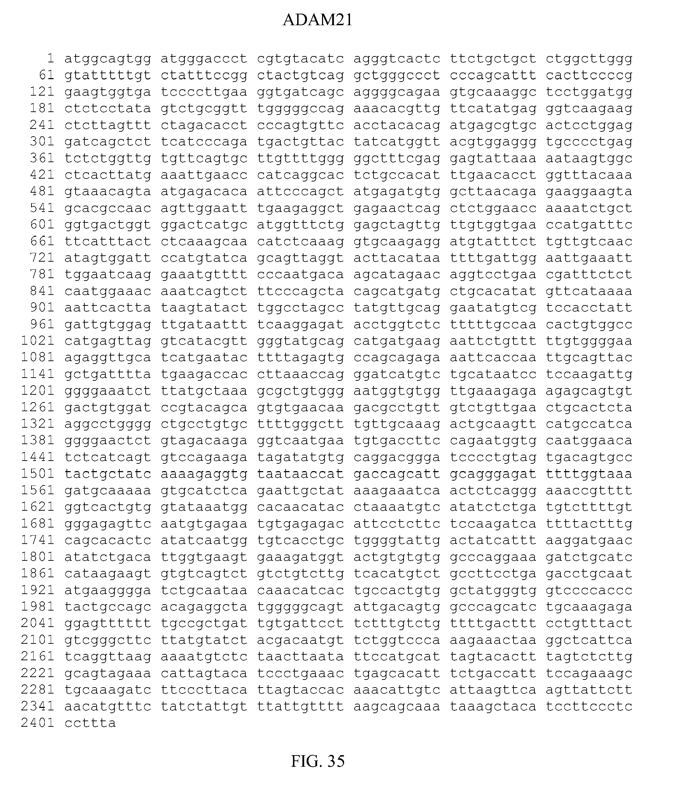

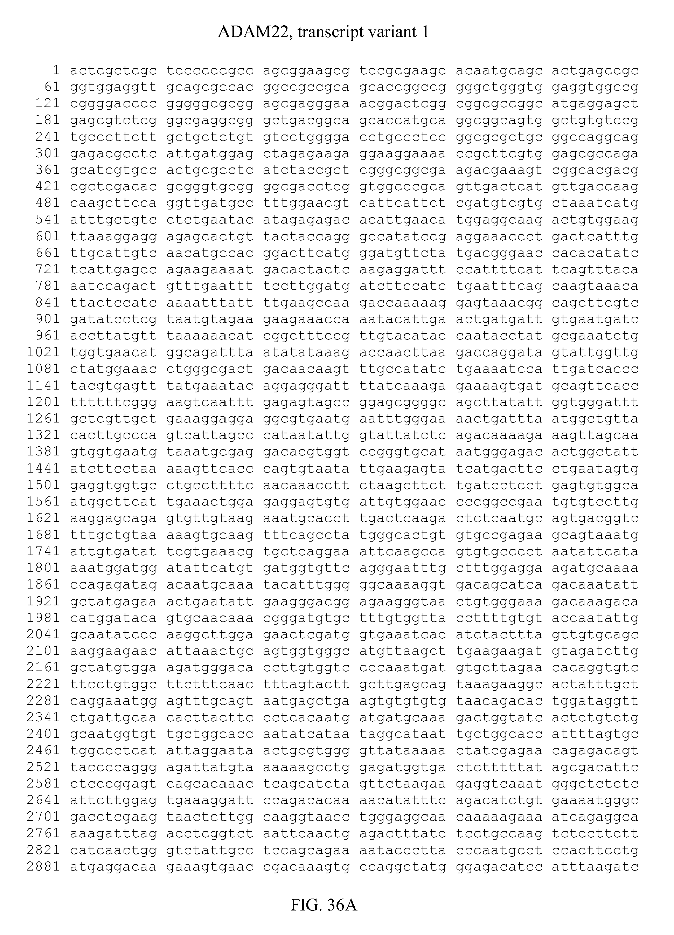

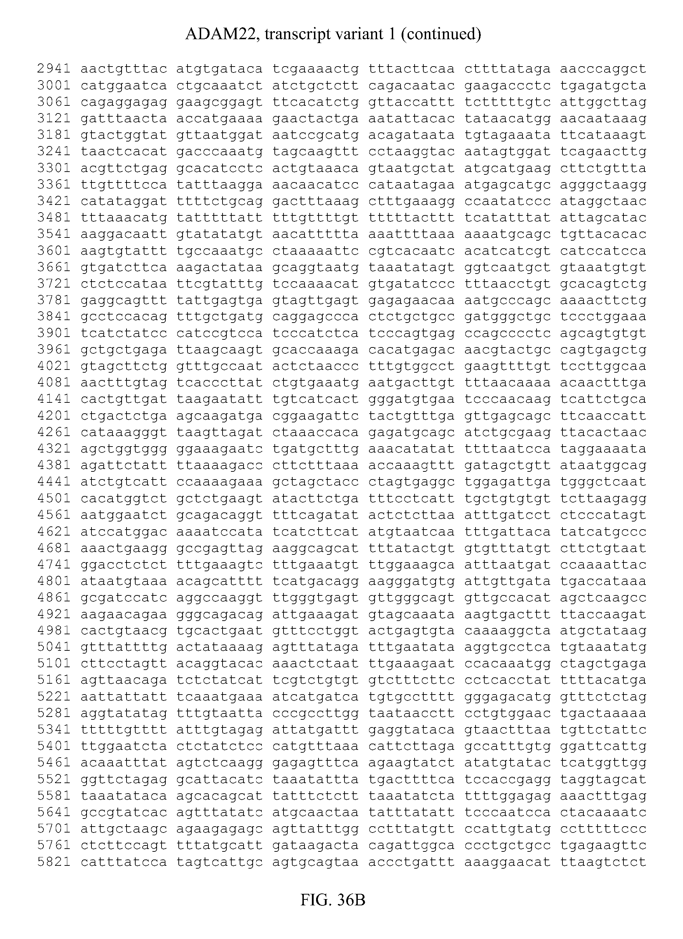

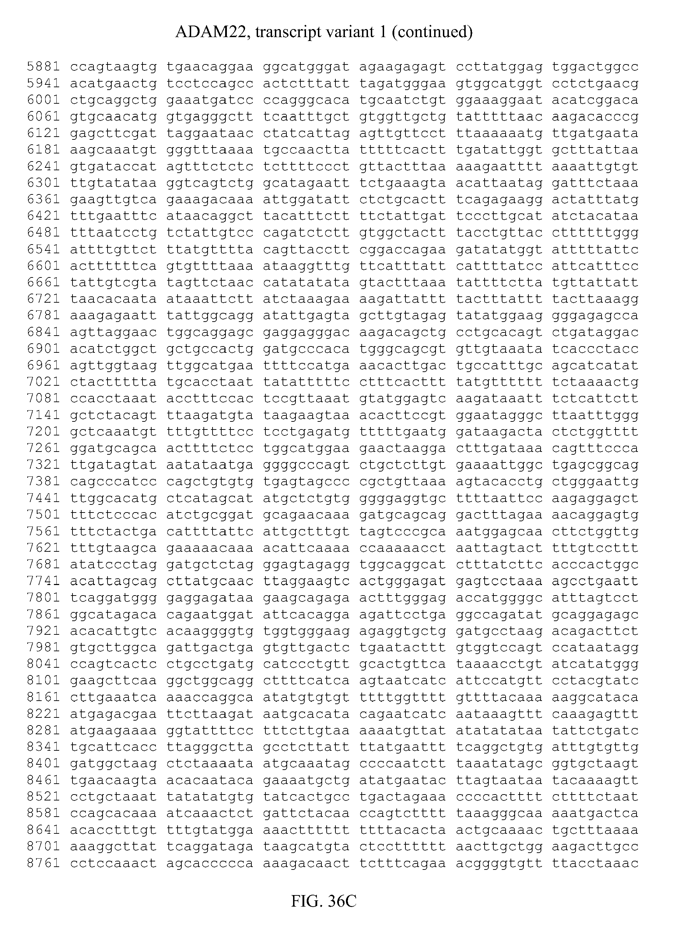

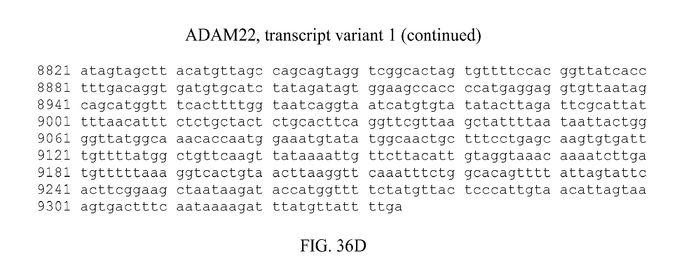

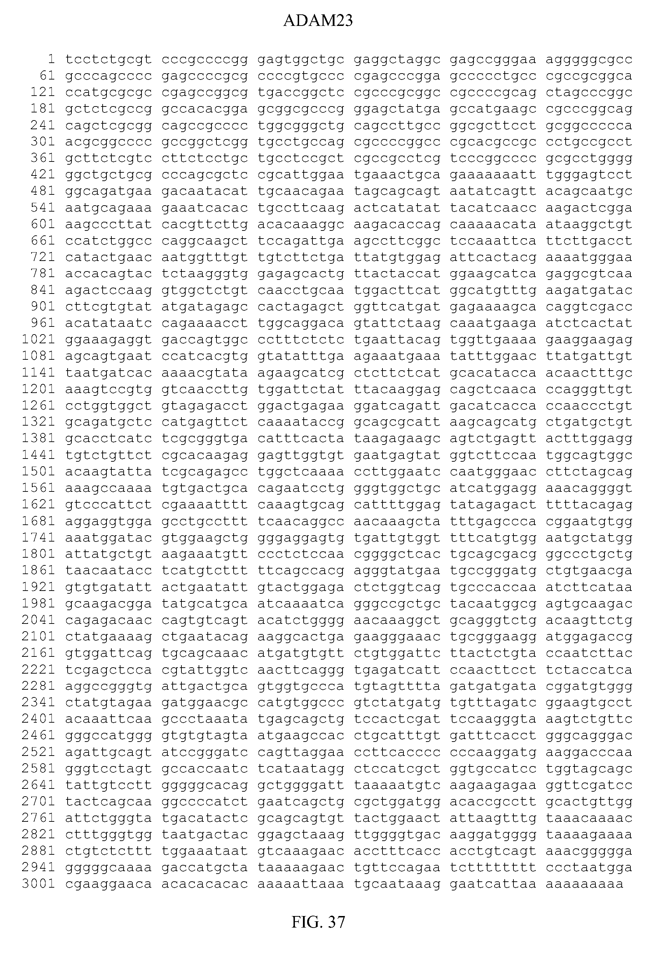

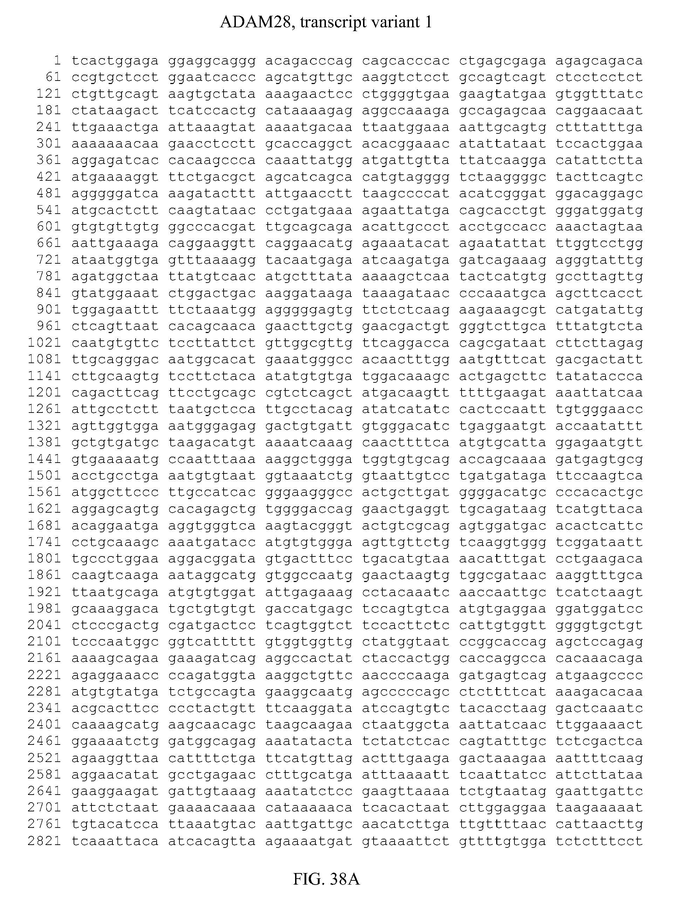

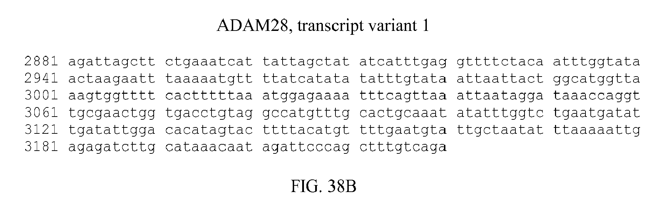

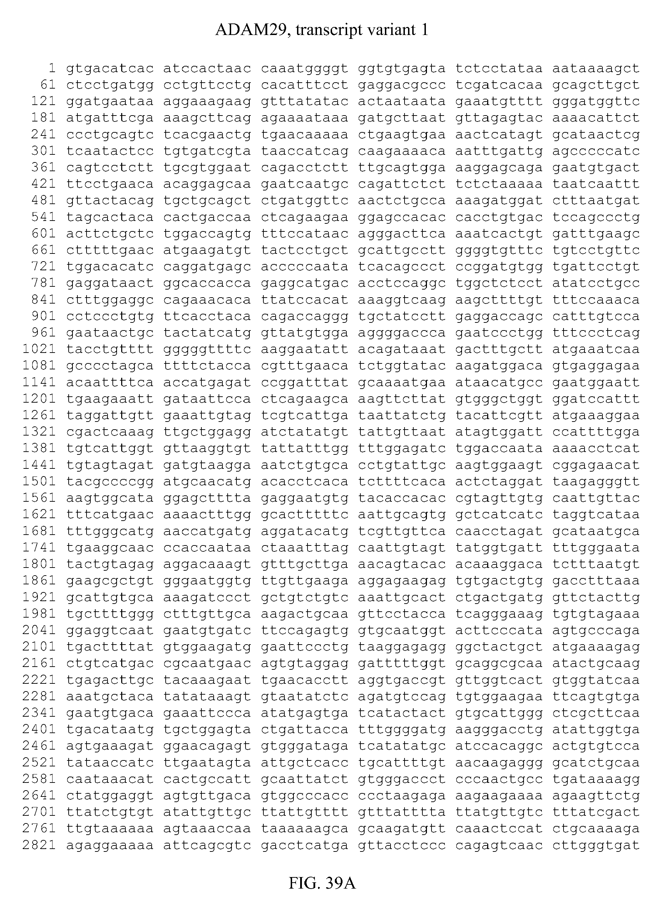

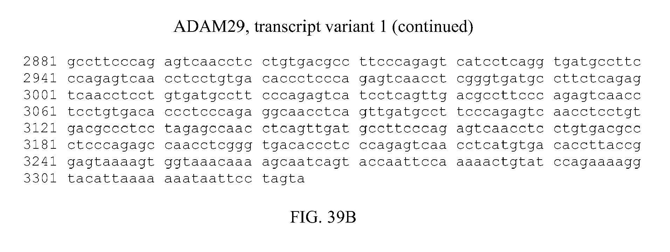

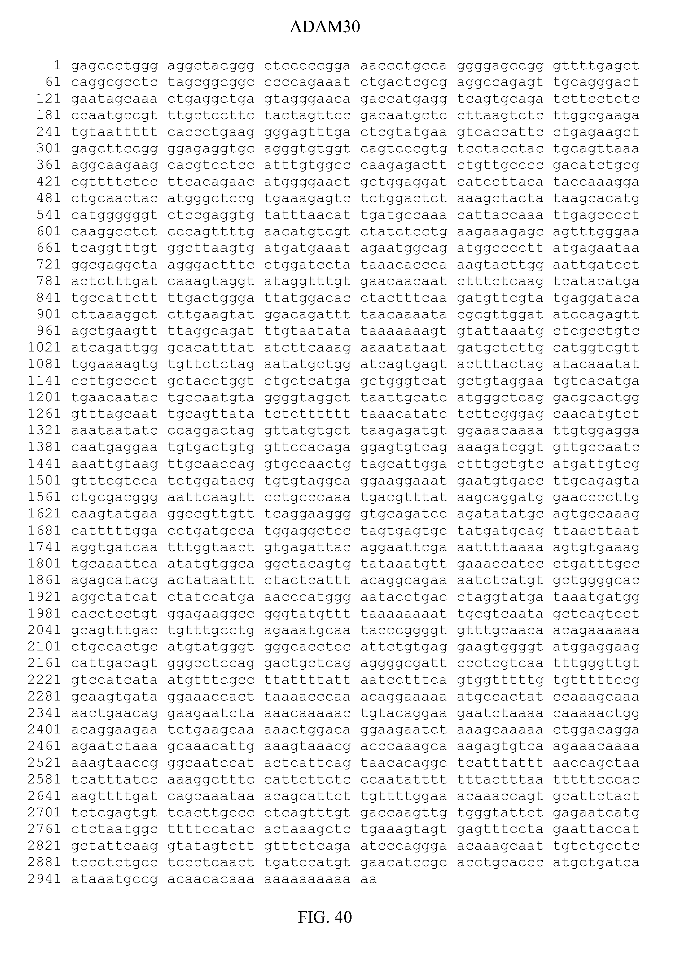

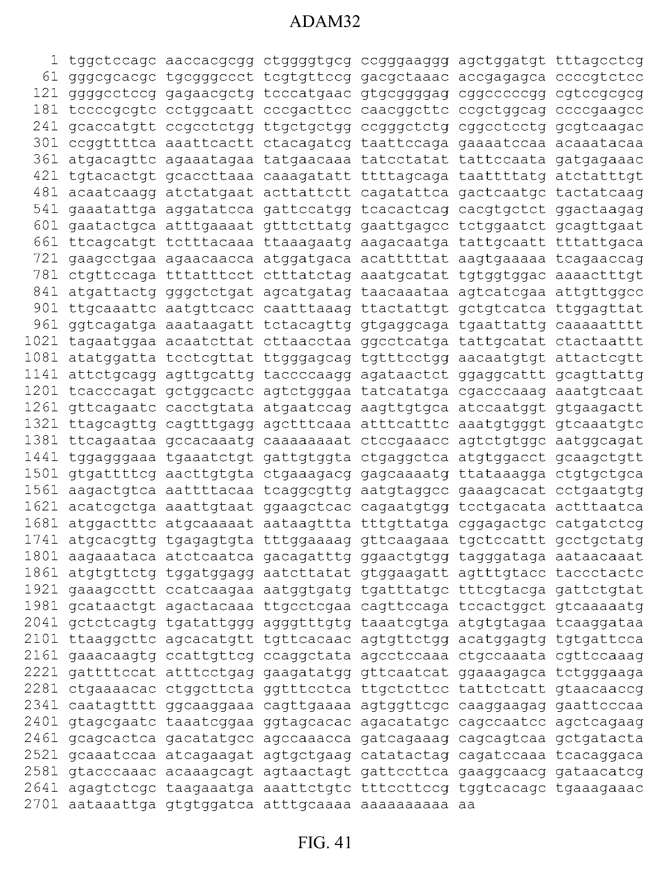

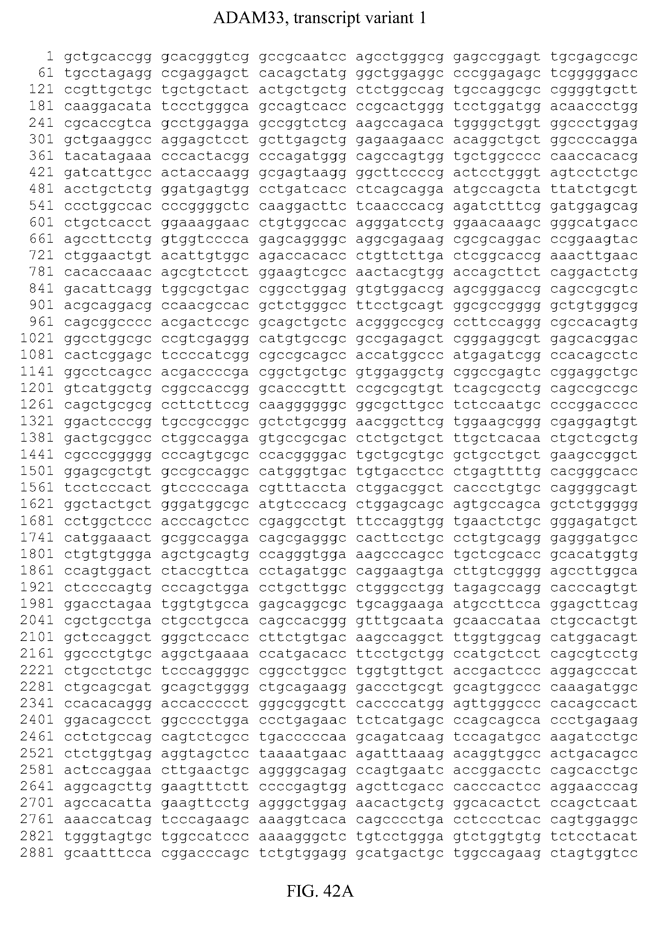

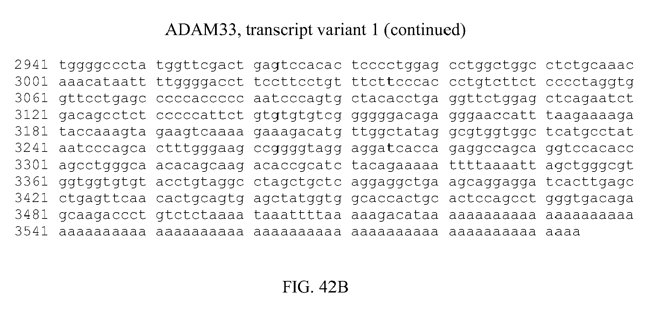

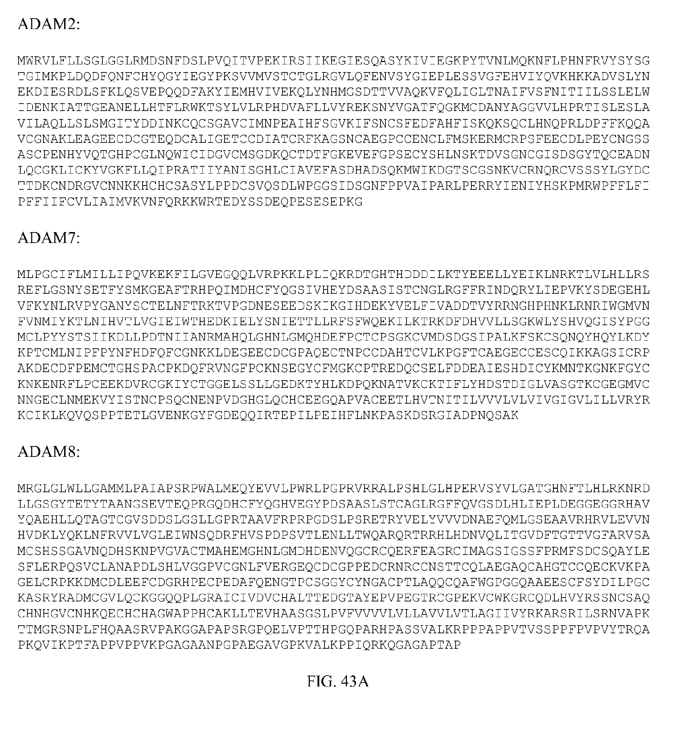

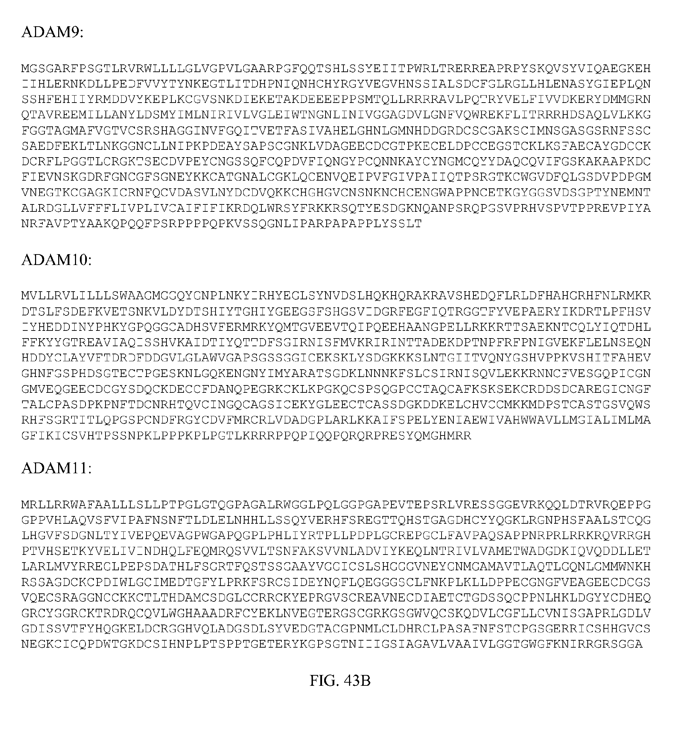

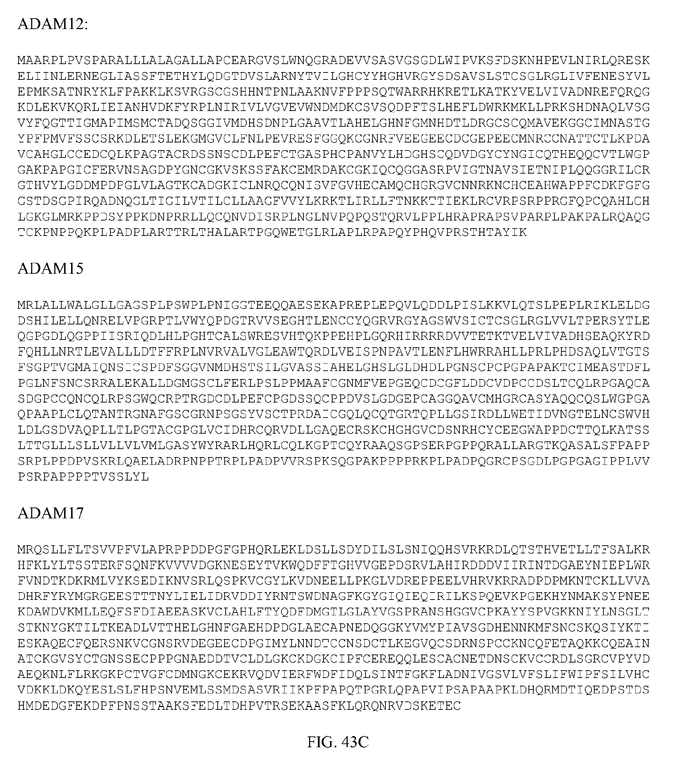

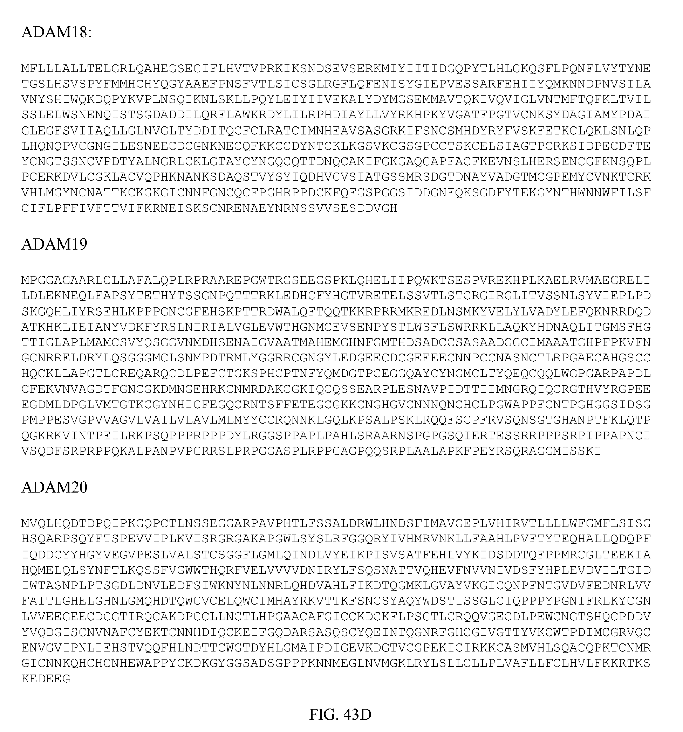

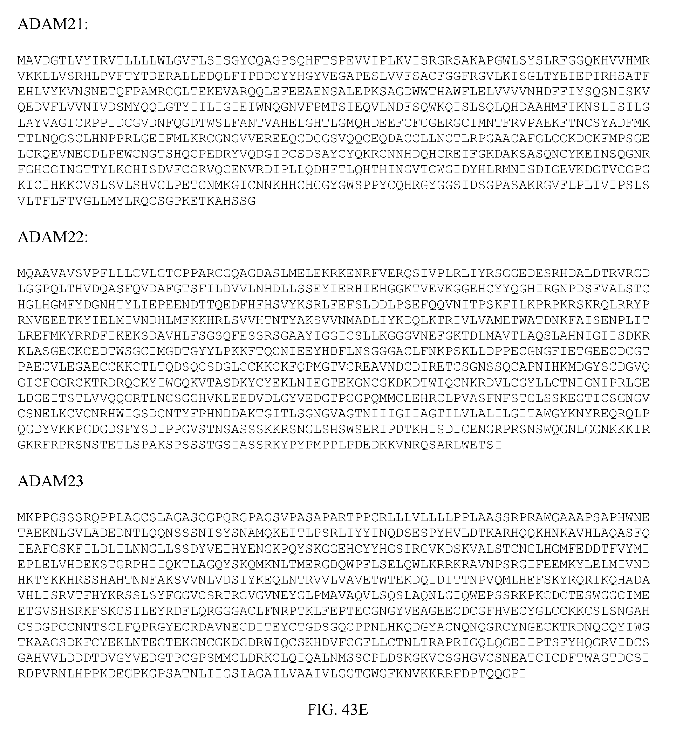

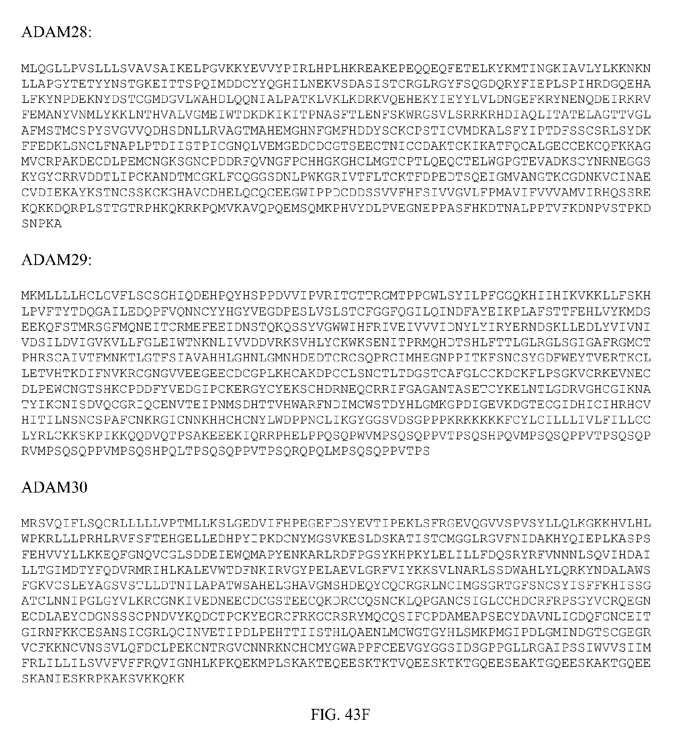

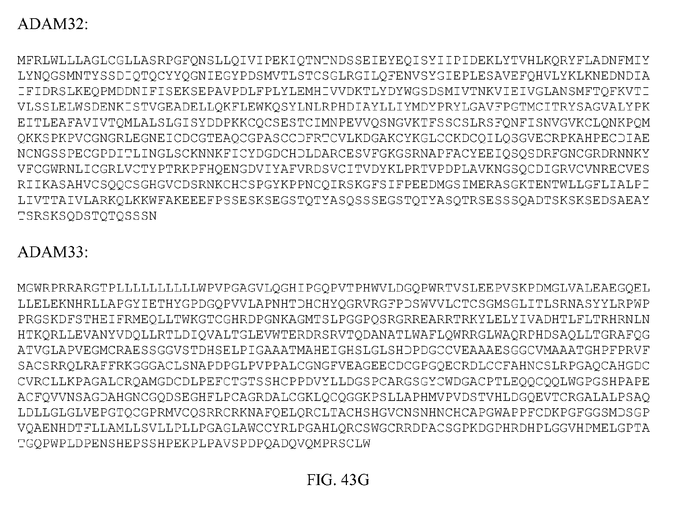

Nucleic acid sequences corresponding to ADAM RNA transcripts are provided in FIGs. 20-42. Amino acid sequences for the corresponding ADAM polypeptides are provided in FIG. 43.

Unless otherwise defined, all technical and scientific terms used herein have the same meaning as commonly understood by one of ordinary skill in the art to which this invention belongs.

The inventions illustratively described herein may suitably be practiced in the absence of any element or elements, limitation or limitations, not specifically disclosed herein. Thus, for example, the terms "comprising," "including," "containing," etc. shall be read expansively and without limitation. Additionally, the terms and expressions employed herein have been used as terms of description and not of limitation, and there is no intention in the use of such terms and expressions of excluding any equivalents of the features shown and described or portions thereof, but it is recognized that various modifications are possible within the scope of the invention claimed.

Thus, it should be understood that although the present invention has been specifically disclosed by preferred embodiments and optional features, modification, improvement and variation of the inventions embodied therein herein disclosed may be resorted to by those skilled in the art, and that such modifications, improvements and variations are considered to be within the scope of this invention. The materials, methods, and examples provided here are representative of preferred embodiments, are exemplary, and are not intended as limitations on the scope of the invention.

The invention has been described broadly and generically herein. Each of the narrower species and subgeneric groupings falling within the generic disclosure also form part of the invention. This includes the generic description of the invention with a proviso or negative limitation removing any subject matter from the genus, regardless of whether or not the excised material is specifically recited herein.

All publications, patent applications, patents, and other references mentioned herein are expressly incorporated by reference in their entirety, including all formulas and figures, to the same extent as if each were incorporated by reference individually. In case of conflict, the present specification, including definitions, will control.

Other embodiments are set forth within the following claims.

### Cited References

1. Edwards, D.R., M.M. Handsley, and C.J. Pennington, The ADAM metalloproteinases. Mol Aspects Med, 2008. 29(5): p. 258-89.
2. Mochizuki, S. and Y. Okada, ADAMs in cancer cell proliferation and progression. Cancer Sci, 2007. 98(5): p. 621-8.
3. Reiss, K., A. Ludwig, and P. Saftig, Breaking up the tie: disintegrin-like metalloproteinases as regulators of cell migration in inflammation and invasion. Pharmacol Ther, 2006. 111(3): p. 985-1006.
4. Tousseyn, T., et al., (Make) stick and cut loose--disintegrin metalloproteases in development and disease. Birth Defects Res C Embryo Today, 2006. 78(1): p. 24-46.
5. Arribas, J., J.J. Bech-Serra, and B. Santiago-Josefat, ADAMs, cell migration and cancer. Cancer Metastasis Rev, 2006. 25(1): p. 57-68.
6. Blanchot-Jossic, F., et al., Up-regulated expression of ADAM 17 in human colon carcinoma: co-expression with EGFR in neoplastic and endothelial cells. J Pathol, 2005. 207(2): p. 156-63.
7. Lendeckel, U., et al., Increased expression of ADAM family members in human breast cancer and breast cancer cell lines. J Cancer Res Clin Oncol, 2005. 131(1): p. 41-8.
8. Mazzocca, A., et al., A secreted form of ADAM9 promotes carcinoma invasion through tumor-stromal interactions. Cancer Res, 2005. 65(11): p. 4728-38.
9. McGowan, P.M., et al., ADAM-17 predicts adverse outcome in patients with breast cancer. Ann Oncol, 2008. 19(6): p. 1075-81.
10. McGowan, P.M., et al., ADAM-17 expression in breast cancer correlates with variables of tumor progression. Clin Cancer Res, 2007. 13(8): p. 2335-43.
11. Mitsui, Y., et al., ADAM28 is overexpressed in human breast carcinomas: implications for carcinoma cell proliferation through cleavage of insulin-like growth factor binding protein-3. Cancer Res, 2006. 66(20): p. 9913-20.
12. Najy, A.J., K.C. Day, and M.L. Day, ADAM15 supports prostate cancer metastasis by modulating tumor cell endothelial cell interaction. Cancer Res, 2008. 68(4): p. 1092-9.
13. O'Shea, C., et al., Expression of ADAM-9 mRNA and protein in human breast cancer. Int J Cancer, 2003. 105(6): p.754-61.
14. Ringel, J., et al., Aberrant expression of a disintegrin and metalloproteinase 17/tumor necrosis factor-alpha converting enzyme increases the malignant potential in human pancreatic ductal adenocarcinoma. Cancer Res, 2006. 66(18): p. 9045-53.
15. Wildeboer, D., et al., Metalloproteinase disintegrins ADAM8 and ADAM19 are highly regulated in human primary brain tumors and their expression levels and activities are associated with invasiveness. J Neuropathol Exp Neurol, 2006. 65(5): p. 516-27.
16. D'Abaco, G.M., et al., ADAM22, expressed in normal brain but not in high-grade gliomas, inhibits cellular proliferation via the disintegrin domain. Neurosurgery, 2006. 58(1): p. 179-86; discussion 179-86.
17. Takeda, S., Three-dimensional domain architecture of the ADAM family proteinases. Semin Cell Dev Biol, 2008.
18. Bessette et al., Efficient folding of proteins with multiple disulfide bonds in the Escherichia coli cytoplasm. Proc. Natl. Acad. Sci (USA) 1999, 96(24):13703-8.
19. Calvete, J.J., et al., Snake venom disintegrins: novel dimeric disintegrins and structural diversification by disulphide bond engineering. Biochem J, 2003. 372(Pt 3): p. 725-34.
20. Juarez, P., et al., Evolution of snake venom disintegrins by positive darwinian selection. Mol Biol Evol, 2008. 25(11): p. 2391-407.
21. McLane, M.A., T. Joerger, and A. Mahmoud, Disintegrins in health and disease. Front Biosci, 2008. 13: p. 6617-37.
22. Shimaoka, M. and T.A. Springer, Therapeutic antagonists and conformational regulation of integrin function. Nat Rev Drug Discov, 2003. 2(9): p. 703-16.
23. Silva, R., et al., Integrins: the keys to unlocking angiogenesis. Arterioscler Thromb Vase Biol, 2008. 28(10): p. 1703-13.
24. Hood, J.D. and D.A. Cheresh, Role of integrins in cell invasion and migration. Nat Rev Cancer, 2002. 2(2): p. 91-100.
25. Mizejewski, G.J., Role of integrins in cancer: survey of expression patterns. Proc Soc Exp Biol Med, 1999. 222(2): p.124-38.
26. Swenson, S., et al., Intravenous liposomal delivery of the snake venom disintegrin contortrostatin limits breast cancer progression. Mol Cancer Ther, 2004. 3(4): p. 499-511.
27. Minea, R., et al., Development of a novel recombinant disintegrin, contortrostatin, as an effective anti-tumor and antiangiogenic agent. Pathophysiol Haemost Thromb, 2005. 34(4-5): p. 177-83.
28. Igarashi, T., et al., Crystal structures of catrocollastatin/VAP2B reveal a dynamic, modular architecture of ADAM/adamalysin/reprolysin family proteins. FEBS Lett, 2007. 581(13): p. 2416-22. Page 9 of 43 Page 14
29. Prinz, W.A., et al., The role of the thioredoxin and glutaredoxin pathways in reducing protein disulfide bonds in the Escherichia coli cytoplasm. J Biol Chem, 1997. 272(25): p. 15661-7.
30. LaVallie, E.R., et al., A thioredoxin gene fusion expression system that circumvents inclusion body formation in the E. coli cytoplasm. Biotechnology (N Y), 1993. 11(2): p. 187-93.
31. Yoneda, T., et al., A bone-seeking clone exhibits different biological properties from the MDA-MB-231 parental human breast cancer cells and a brain-seeking clone in vivo and in vitro. J Bone Miner Res, 2001. 16(8): p. 1486-95.
32. Yuan, X., et al., Isolation of cancer stem cells from adult glioblastoma multiforme. Oncogene, 2004. 23(58): p. 400.
33. Alfandari, D., C. McCusker, and H. Cousin, ADAM function in embryogenesis. Semin Cell Dev Biol, 2008.
34. Duffy M.J. et al., Role of ADAMs in cancer formation and progression. Clin Cancer Res. 2009 Feb 15;15(4):1140-4.
35. Protopapa, E. et al., Vascular density and the response of breast carcinomas to mastectomy and adjuvant chemotherapy. Eur J Cancer, 1993. 29A(10): p. 1391-3.
36. Fox, S.B. and A.L. Harris, Histological quantitation of tumour angiogenesis. Apmis, 2004. 112(7-8): p. 413-30.

### FURTHER EMBODIMENTS

1. A Modified ADAM-derived Polypeptide (MAP), comprising an ADAM-derived Polypeptide (AP) that is modified at the following amino acid residues:
   a) a first cysteine C-terminal to a Cys-Asp-Cys (CDC) motif;
   b) two contiguous amino acids C-terminal to said first cysteine; and,
   c) a cysteine C-terminal to a tripeptide motif,
      and wherein the modification of said amino acid residues is independently selected from the group consisting of:
      a) deletion;
      b) substitution; and,
      c) chemical modification,
   and wherein ADAM means "A Disintegrin and Metalloproteinase" and, wherein, said AP comprises a distintegrin-like domain from an ADAM comprising (i) the Cys-Asp-Cys (CDC) motif, (ii) the tripeptide motif as indicated in FIG. 1 and (iii) lacking all or substantially all of the ADAM metalloprotease domain, cysteine-rich domains, and interdomain segments, and wherein said ADAM is not ADAM 17.
2. The MAP of clause 1 wherein said MAP is derived from an ADAM selected from the group consisting of: ADAM15, ADAM28, ADAM1, ADAM2, ADAM3, ADAM6, ADAM7, ADAM8, ADAM9, ADAM10, ADAM11, ADAM12, ADAM18, ADAM19, ADAM20, ADAM21, ADAM22, ADAM23, ADAM29, ADAM30, ADAM32, ADAM33.
3. The MAP of clause 1 wherein said MAP is selected from the group consisting of:
   MAP 15, MAP28, MAP1, MAP2, MAP3, MAP6, MAP7, MAP8, MAP9, MAP10, MAP11, MAP12, MAP18, MAP19, MAP20, MAP21, MAP22, MAP23, MAP29, MAP30, MAP32, MAP33.
4. The MAP of clause 1 wherein said modifications of said amino acid residues are deletions.
5. A fusion protein comprising an N-tenninal segment encoding thioredoxin and a C-terminal segment encoding a modified ADAM-derived polypeptide (MAP) as specified in clause 1.
6. The MAP of clause 1 wherein:
   a) said MAP is capable of inhibiting the movement of HUVEC or MDA-MB-435 cells through a reconstituted basement membrane;
   b) said MAP is capable of increasing the level of phosphorylation of FAK in MDA-MB-435 cells; or,
   c) said MAP is capable of inhibiting tube formation of HUVECs in culture.
7. A nucleic acid encoding a MAP of clause 1.
8. The nucleic acid of clause 7, further comprising a nucleic acid sequence encoding thioredoxin 5' to the nucleic acid encoding said MAP, wherein said nucleic acid encodes a thioredoxin-MAP fusion protein.
9. An expression vector comprising the nucleic acid of clause 7.
10. Prokaryotic host cells transformed with said expression vector of clause 9, wherein said expression vector is under inducible control, wherein said host also carries stable mutations in thioredoxin reductase B (*trxB*) gene and/or the glutathione reductase (*gor*) gene and wherein said *trxB* and *gor* mutations are selectable to maintain the expression vector and *trxB* and *gor* mutations in said host cells during growth.
11. A method of treating an individual suffering from cancer, said method comprising administering to said individual an effective amount of at least one MAP as specified in clause 1.
12. The method of clause 11 wherein said cancer is an integrin expressing cancer.
13. The method of clause 12 wherein said cancer is selected from the group consisting of breast cancer, colorectal cancer, basal cell carcinoma, adenocarcinoma, gastrointestinal cancer, colon cancer, liver cancer, bladder cancer, pancreatic cancer, ovarian cancer, cervical cancer, lung cancer, skin cancer, prostate cancer, renal cell carcinoma, central nervous system (CNS) cancer, and leukemia.
14. The method of clause 13, wherein said gastrointestinal cancer is selected from the group consisting of lip cancer, mouth cancer, esophageal cancer, small bowel cancer and stomach cancer.
15. The method of clause13, wherein said skin cancer is selected from the group consisting of squamous cell and basal cell cancer.
16. A method of inhibiting the binding of an integrin to a ligand comprising contacting a cell that expresses the integrin with an effective amount of a MAP according to clause1.
17. The method of clause16 wherein said MAP is selected from the group consisting of MAP15, MAP28, MAP1, MAP2, MAP3, MAP6, MAP7, MAP8, MAP9, MAP10, MAP11, MAP12, MAP18, MAP19, MAP20, MAP21, MAP22, MAP23, MAP29, MAP30, MAP32, MAP33.
18. The method of clause 16 wherein said MAP further comprises a fusion of an N-terminal segment ofthioredoxin.
19. The method of clause 16 wherein:
   a) said MAP is capable of inhibiting the movement of HUVEC or MDA-MB-435 cells through a reconstituted basement membrane;
   b) said MAP is capable of increasing the level of phosphorylation of FAK in MDA-MB-435 cells; or,
   c) said MAP is capable of inhibiting tube formation of HUVECs in culture.
20. A method of determining the presence of cancer cells in an individual, said method comprising contacting said cancer cells with at least one MAP according to clause 1 and detecting said at least one MAP.
21. The method of clause 20 wherein said cancer is an integrin expressing cancer.
22. The method of clause 20 wherein said cancer is selected from the group consisting of breast cancer, colorectal cancer, basal cell carcinoma, adenocarcinoma, gastrointestinal cancer, colon cancer, liver cancer, bladder cancer, pancreatic cancer, ovarian cancer, cervical cancer, lung cancer, skin cancer, prostate cancer, renal cell carcinoma, central nervous system (CNS) cancer, and leukemia.
23. The method of clause 22, wherein said gastrointestinal cancer is selected from the group consisting of lip cancer, mouth cancer, esophageal cancer, small bowel cancer and stomach cancer.
24. The method of clause 22, wherein said skin cancer is selected from the group consisting of squamous cell and basal cell cancer.
25. The method of clause 20, wherein said MAP is labeled.
26. The method of clause 25, wherein said label is a Positron Emmission Tomography probe or a fluorescent probe.
27. A method of preparing an artificial ECM scaffold comprising coating said artificial ECM scaffold with a MAP according to clause 1.
28. The method of clause 27, further comprising the introduction of stem cell precursors to the artificial ECM scaffold.
29. The method of clause 27, where said artificial ECM scaffold comprises a urinary bladder scaffold, an esophageal scaffold or an anal scaffold.
30. A method of expressing a MAPs of clause 1 in prokaryotic host cells, said method comprising:
   a) growing the prokaryotic host cells of claim 10, wherein said expression vector has an antibiotic resistance gene which makes it selectable on a first antibiotic, and wherein said *trxB* and *gor* mutations are selectable on at least one additional antibiotic to maintain the expression vector and *trxB* and *gor* mutations in said host cells during growth, in the presence of the first and said at least one additional antibiotic to obtain a sufficient number of cells suitable to seed a reactor in which host cells will be grown and the fusion protein expression induced; and
   b) seeding the reactor with the cells of step a) and growing the cells and inducing expression of the fusion protein, wherein said cells in the reactor are grown in the presence of the first antibiotic and in the absence of said at least one additional antibiotic.
31. The method of clause 30 wherein the host cells express mutant products of both the *trxB* and *gor* genes.
32. The method of clause 31 wherein said host cells are mutant in both *trxB* and *gor* genes.
33. The method of clause 31 wherein the *trxB* and *gor* genes are selectable on different antibiotics.
34. The method of clause 30 wherein the thioredoxin portion of the fusion protein has the sequence:
   MSDKIIHLTDDSFDTDVLKADGAILVDFWAEWCGPCKMIAPILDEIADEYQGKLTVAKL NIDQNPGTAPKYG IRGIPTLLLFKNGEVAATKVGALSKGQLKEF LDANLA.
35. The method of claim 30 wherein the host is deficient in any one or more of *ompT* or *lon* gene products.
36. The method of claim 30 wherein a sequence encoding a cleavage site is located between the sequence encoding thioredoxin and the sequence encoding the disulfide rich protein.
37. The method of claim 30 wherein the fusion protein further includes a peptide sequence which is a ligand for a receptor.
38. The method of claim 30 wherein said prokaryotic host cell is an Origami strain.
39. A stent coated with the composition of claim 1.
40. A Modified ADAM-derived Polypeptide (MAP), comprising an ADAM-derived Polypeptide (AP) comprising a distintegrin-like domain from an ADAM comprising (i) the Cys-Asp-Cys (CDC) motif, (ii) the tripeptide motif as indicated in FIG. 1 and (iii) lacking all or substantially all of the ADAM metalloprotease domain, cysteine-rich domains, and interdomain segments, and further comprising one or more amino acid modifications that result in disruption of the interdomain disulfide linkages with the first cysteine C-terminal to the CDC motif and interdomain disulfide linkages with the cysteine C-terminal to the tripeptide motif, and wherein ADAM means "A Disintegrin and Metalloproteinase."
41. A polypeptide according to any of the polypeptide sequences in FIG. 2
42. A nucleic acid encoding a polypeptide according to claim 41.

## Claims

1. A Modified ADAM-derived Polypeptide (MAP), comprising an ADAM-derived Polypeptide (AP) that is modified at the following amino acid residues:
a) a first cysteine C-terminal to a Cys-Asp-Cys (CDC) motif;
b) two contiguous amino acids C-terminal to said first cysteine; and,
c) a cysteine C-terminal to a tripeptide motif,
and wherein the modification of said amino acid residues is independently selected from the group consisting of:
a) deletion;
b) substitution; and,
c) chemical modification,
and wherein ADAM means "A Disintegrin and Metalloproteinase" and, wherein, said AP comprises a distintegrin-like domain from an ADAM comprising (i) the Cys-Asp-Cys (CDC) motif, (ii) the tripeptide motif as indicated in FIG. 1 and (iii) lacking all or substantially all of the ADAM metalloprotease domain, cysteine-rich domains, and interdomain segments, and wherein said MAP is derived from an ADAM selected from the group consisting of: ADAM15, ADAM28, ADAM1, ADAM2, ADAM3, ADAM6, ADAM7, ADAM8, ADAM9, ADAM10, ADAM11, ADAM12, ADAM18, ADAM19, ADAM20, ADAM21, ADAM22, ADAM23, ADAM29, ADAM30, ADAM32, ADAM33 and/or wherein said MAP is selected from the group consisting of: MAP15, MAP28, MAP1, MAP2, MAP3, MAP6, MAP7, MAP8, MAP9, MAP10, MAP11, MAP12, MAP18, MAP19, MAP20, MAP21, MAP22, MAP23, MAP29, MAP30, MAP32, MAP33.

2. A fusion protein comprising an N-terminal segment encoding thioredoxin and a C-terminal segment encoding a modified ADAM-derived polypeptide (MAP) as specified in claim 1.

3. A nucleic acid encoding a MAP of any preceding claim, optionally further comprising a nucleic acid sequence encoding thioredoxin 5' to the nucleic acid encoding said MAP, wherein said nucleic acid encodes a thioredoxin-MAP fusion protein.

4. An expression vector comprising the nucleic acid of claim 3.

5. Prokaryotic host cells transformed with said expression vector of claim 4, wherein said expression vector is under inducible control, wherein said host also carries stable mutations in thioredoxin reductase B (*trxB*) gene and/or the glutathione reductase (*gor*) gene and wherein said *trxB* and *gor* mutations are selectable to maintain the expression vector and *trxB* and *gor* mutations in said host cells during growth.

6. A composition comprising a MAP of claim 1, for use in the preparation of a medicament to inhibiting the binding of an integrin to a ligand or to treat cancer, such as integrin expressing cancer, e.g., breast cancer, colorectal cancer, basal cell carcinoma, adenocarcinoma, colon cancer, liver cancer, bladder cancer, pancreatic cancer, ovarian cancer, cervical cancer, lung cancer, prostate cancer, renal cell carcinoma, central nervous system (CNS) cancer, leukemia, gastrointestinal cancer, e.g., lip cancer, mouth cancer, esophageal cancer, small bowel cancer and stomach cancer, and skin cancer, e.g., squamous cell and basal cell cancer.

7. An in vitro method of inhibiting the binding of an integrin to a ligand comprising contacting a cell that expresses the integrin with an effective amount of a MAP according to any one of claims 1 or 2.

8. An in vitro method of determining the presence of cancer cells in an individual, said method comprising contacting said cancer cells isolated from the patient with at least one MAP according to any one of claims 1 or 2 and detecting said at least one MAP, preferably wherein said cancer is an integrin expressing cancer, such as breast cancer, colorectal cancer, basal cell carcinoma, adenocarcinoma, gastrointestinal cancer (e.g., lip cancer, mouth cancer, esophageal cancer, small bowel cancer and stomach cancer), colon cancer, liver cancer, bladder cancer, pancreatic cancer, ovarian cancer, cervical cancer, lung cancer, skin cancer (e.g., squamous cell and basal cell cancer), prostate cancer, renal cell carcinoma, central nervous system (CNS) cancer, and leukemia and wherein the MAP is optionally labeled.

9. Use of: at least one MAP, a nucleic acid encoding the MAP or the expression vector comprising the nucleic acid encoding the MAP of any one of claims 1 to 7 in the preparation of an artificial ECM scaffold comprising coating said artificial ECM scaffold with the MAP, optionally further comprising the introduction of stem cell precursors to the artificial ECM scaffold, and further optionally where said artificial ECM scaffold comprises a urinary bladder scaffold, an esophageal scaffold or an anal scaffold.

10. A method of expressing a MAPs of claim 1 in prokaryotic host cells, said method comprising:
a) growing the prokaryotic host cells of claim 5, wherein said expression vector has an antibiotic resistance gene which makes it selectable on a first antibiotic, and wherein said *trxB* and *gor* mutations are selectable on at least one additional antibiotic to maintain the expression vector and *trxB* and *gor* mutations in said host cells during growth, in the presence of the first and said at least one additional antibiotic to obtain a sufficient number of cells suitable to seed a reactor in which host cells will be grown and wherein the MAP of claim 1 comprises a fusion of an N-terminal segment of thioreoxin; and
b) seeding the reactor with the cells of step a) and growing the cells and, wherein said cells in the reactor are grown in the presence of the first antibiotic and in the absence of said at least one additional antibiotic.

11. The method of claim 10 wherein the host cells express mutant products of both the *trxB* and *gor* genes or wherein said host cells are mutant in both *trxB* and *gor* genes, and optionally wherein the *trxB* and *gor* genes are selectable on different antibiotics, orwherein the host is deficient in any one or more of *ompT* or *lon* gene products.

12. The method of claim 10 wherein the thioredoxin portion of the fusion protein has the sequence:
MSDKIIHLTDDSFDTDVLKADGAILVDFWAEWCGPCKMIAPILDEIADEYQGKLTVAKLN IDQNPGTAPKYG IRGIPTLLLFKNGEVAATKVGALSKGQLKEF LDANLA.

13. The method of claim 10 further comprising a sequence encoding a cleavage site is located between the sequence encoding thioredoxin and a sequence encoding a disulfide rich protein, or alternatively wherein the fusion protein further comprises a peptide sequence which is a ligand for a receptor and/or wherein said prokaryotic host cell is an Origami strain.

14. A stent coated with MAP of any of claims 1 or 2.

15. A Modified ADAM-derived Polypeptide (MAP), comprising an ADAM-derived Polypeptide (AP) comprising a distintegrin-like domain from an ADAM comprising (i) the Cys-Asp-Cys (CDC) motif, (ii) the tripeptide motif as indicated in FIG. 1 and (iii) lacking all or substantially all of the ADAM metalloprotease domain, cysteine-rich domains, and interdomain segments, and further comprising one or more amino acid modifications that result in disruption of the interdomain disulfide linkages with the first cysteine C-terminal to the CDC motif and interdomain disulfide linkages with the cysteine C-terminal to the tripeptide motif, and wherein ADAM means "A Disintegrin and Metalloproteinase", and wherein said MAP is derived from an ADAM selected from the group consisting of: ADAM15, ADAM28, ADAM1, ADAM2, ADAM3, ADAM6, ADAM7, ADAM8, ADAM9, ADAM10, ADAM11, ADAM12, ADAM 18, ADAM 19, ADAM20, ADAM21, ADAM22, ADAM23, ADAM29, ADAM30, ADAM32, ADAM33 and/or wherein said MAP is selected from the group consisting of: MAP15, MAP28, MAP1, MAP2, MAP3, MAP6, MAP7, MAP8, MAP9, MAP10, MAP11, MAP12, MAP18, MAP19, MAP20, MAP21, MAP22, MAP23, MAP29, MAP30, MAP32, MAP33.

16. A polypeptide according to any of the polypeptide sequences of MAP15, MAP28, MAP1, MAP2, MAP3, MAP6, MAP7, MAP8, MAP9, MAP10, MAP11, MAP12, MAP18, MAP19, MAP20, MAP21, MAP22, MAP23, MAP29, MAP30, MAP32, MAP33.

17. A nucleic acid encoding a polypeptide according to claim 16.


SEQUENCE LISTING

<110> UNIVERSITY OF SOUTHERN CALIFORNIA

<120> MODIFIED ADAM DISINTEGRIN DOMAIN POLYPEPTIDES AND USES THEREOF

<130> 075405-1502

<140> PCT/US2011/024243
<141> 2011-02-09

<150> 61/303,631
<151> 2010-02-11

<160> 188

<170> PatentIn version 3.5

<210> 1
<211> 109
<212> PRT
<213> Artificial Sequence

<220>
<223> Description of Artificial Sequence: Synthetic
      polypeptide

<400> 1
Met Ser Asp Lys Ile Ile His Leu Thr Asp Asp Ser Phe Asp Thr Asp
1               5                   10                  15

Val Leu Lys Ala Asp Gly Ala Ile Leu Val Asp Phe Trp Ala Glu Trp
            20                  25                  30

Cys Gly Pro Cys Lys Met Ile Ala Pro Ile Leu Asp Glu Ile Ala Asp
        35                  40                  45

Glu Tyr Gln Gly Lys Leu Thr Val Ala Lys Leu Asn Ile Asp Gln Asn
    50                  55                  60

Pro Gly Thr Ala Pro Lys Tyr Gly Ile Arg Gly Ile Pro Thr Leu Leu
65                  70                  75                  80

Leu Phe Lys Asn Gly Glu Val Ala Ala Thr Lys Val Gly Ala Leu Ser
                85                  90                  95

Lys Gly Gln Leu Lys Glu Phe Leu Asp Ala Asn Leu Ala
            100                 105

<210> 2
<211> 76
<212> PRT
<213> Homo sapiens

<400> 2
Thr Glu Gln Cys Asp Cys Gly Ser Leu Cys Gln His His Ala Cys Cys

1               5                   10                  15

Asp Glu Asn Cys Ile Leu Lys Ala Lys Ala Glu Cys Ser Asp Gly Pro
            20                  25                  30

Cys Cys His Lys Cys Lys Phe His Arg Lys Gly Tyr Pro Cys Cys Pro
        35                  40                  45

Ser Ser Arg Ser Cys Asp Leu Pro Glu Phe Cys Asn Gly Thr Ser Ala
    50                  55                  60

Leu Cys Pro Asn Asn Arg His Lys Gln Asp Gly Ser
65                  70                  75

<210> 3
<211> 81
<212> PRT
<213> Homo sapiens

<400> 3
Gly Glu Glu Cys Asp Cys Gly Thr Glu Gln Asp Cys Ala Leu Ile Gly
1               5                   10                  15

Glu Thr Cys Cys Asp Ile Ala Thr Cys Arg Phe Lys Ala Gly Ser Asn
            20                  25                  30

Cys Ala Glu Gly Pro Cys Cys Glu Asn Cys Leu Phe Met Ser Lys Glu
        35                  40                  45

Arg Met Cys Arg Pro Ser Phe Glu Glu Cys Asp Leu Pro Glu Tyr Cys
    50                  55                  60

Asn Gly Ser Ser Ala Ser Cys Pro Glu Asn His Tyr Val Gln Thr Gly
65                  70                  75                  80

His

<210> 4
<211> 81
<212> PRT
<213> Homo sapiens

<400> 4
Thr Glu Gln Cys Asp Cys Gly Tyr Lys Glu Ala Cys Thr His Lys Lys
1               5                   10                  15

Cys Cys Asn Pro Ala Asp Cys Thr Leu Val Arg Ser Ala Glu Cys Gly
            20                  25                  30

Thr Gly Ser Cys Cys Asn Asn Lys Thr Cys Thr Ile His Glu Arg Gly
        35                  40                  45

His Val Cys Arg Lys Ser Val Asp Met Cys Asp Phe Pro Glu Tyr Cys
    50                  55                  60

Asn Gly Thr Ser Glu Phe Cys Val Pro Asp Val Lys Ala Ala Asp Leu
65                  70                  75                  80

Glu

<210> 5
<211> 78
<212> PRT
<213> Homo sapiens

<400> 5
Arg Glu Glu Cys Asp Cys Gly Ser Phe Lys Gln Cys Tyr Ala Ser Tyr
1               5                   10                  15

Cys Cys Gln Ser Asp Cys His Leu Thr Pro Gly Ser Ile Cys His Ile
            20                  25                  30

Gly Glu Cys Cys Thr Asn Cys Ser Phe Ser Pro Pro Gly Thr Leu Cys
        35                  40                  45

Arg Pro Ile Gln Asn Ile Cys Asp Leu Pro Glu Tyr Cys His Gly Thr
    50                  55                  60

Thr Val Thr Cys Pro Ala Asn Val Tyr Met Gln Asp Gly Thr
65                  70                  75

<210> 6
<211> 78
<212> PRT
<213> Homo sapiens

<400> 6
Gly Glu Glu Cys Asp Cys Gly Pro Ala Gln Glu Cys Thr Asn Pro Cys
1               5                   10                  15

Cys Asp Ala His Thr Cys Val Leu Lys Pro Gly Phe Thr Cys Ala Glu
            20                  25                  30

Gly Glu Cys Cys Glu Ser Cys Gln Ile Lys Lys Ala Gly Ser Ile Cys
        35                  40                  45

Arg Pro Ala Lys Asp Glu Cys Asp Phe Pro Glu Met Cys Thr Gly His
    50                  55                  60

Ser Pro Ala Cys Pro Lys Asp Gln Phe Arg Val Asn Gly Phe
65                  70                  75

<210> 7
<211> 78
<212> PRT
<213> Homo sapiens

<400> 7
Gly Glu Gln Cys Asp Cys Gly Pro Pro Glu Asp Cys Arg Asn Arg Cys
1               5                   10                  15

Cys Asn Ser Thr Thr Cys Gln Leu Ala Glu Gly Ala Gln Cys Ala His
            20                  25                  30

Gly Thr Cys Cys Gln Glu Cys Lys Val Lys Pro Ala Gly Glu Leu Cys
        35                  40                  45

Arg Pro Lys Lys Asp Met Cys Asp Leu Glu Glu Phe Cys Asp Gly Arg
    50                  55                  60

His Pro Glu Cys Pro Glu Asp Ala Phe Gln Glu Asn Gly Thr
65                  70                  75

<210> 8
<211> 79
<212> PRT
<213> Homo sapiens

<400> 8
Gly Glu Glu Cys Asp Cys Gly Thr Pro Lys Glu Cys Glu Leu Asp Pro
1               5                   10                  15

Cys Cys Glu Gly Ser Thr Cys Lys Leu Lys Ser Phe Ala Glu Cys Ala
            20                  25                  30

Tyr Gly Asp Cys Cys Lys Asp Cys Arg Phe Leu Pro Gly Gly Thr Leu
        35                  40                  45

Cys Arg Gly Lys Thr Ser Glu Cys Asp Val Pro Glu Tyr Cys Asn Gly
    50                  55                  60

Ser Ser Gln Phe Cys Gln Pro Asp Val Phe Ile Gln Asn Gly Tyr
65                  70                  75

<210> 9
<211> 86
<212> PRT
<213> Homo sapiens

<400> 9

Gly Glu Glu Cys Asp Cys Gly Tyr Ser Asp Gln Cys Lys Asp Glu Cys
1               5                   10                  15

Cys Phe Asp Ala Asn Gln Pro Glu Gly Arg Lys Cys Lys Leu Lys Pro
            20                  25                  30

Gly Lys Gln Cys Ser Pro Ser Gln Gly Pro Cys Cys Thr Ala Gln Cys
        35                  40                  45

Ala Phe Lys Ser Lys Ser Glu Lys Cys Arg Asp Asp Ser Asp Cys Ala
    50                  55                  60

Arg Glu Gly Ile Cys Asn Gly Phe Thr Ala Leu Cys Pro Ala Ser Asp
65                  70                  75                  80

Pro Lys Pro Asn Phe Thr
                85

<210> 10
<211> 79
<212> PRT
<213> Homo sapiens

<400> 10
Gly Glu Glu Cys Asp Cys Gly Ser Val Gln Glu Cys Ser Arg Ala Gly
1               5                   10                  15

Gly Asn Cys Cys Lys Lys Cys Thr Leu Thr His Asp Ala Met Cys Ser
            20                  25                  30

Asp Gly Leu Cys Cys Arg Arg Cys Lys Tyr Glu Pro Arg Gly Val Ser
        35                  40                  45

Cys Arg Glu Ala Val Asn Glu Cys Asp Ile Ala Glu Thr Cys Thr Gly
    50                  55                  60

Asp Ser Ser Gln Cys Pro Pro Asn Leu His Lys Leu Asp Gly Tyr
65                  70                  75

<210> 11
<211> 78
<212> PRT
<213> Homo sapiens

<400> 11
Gly Glu Glu Cys Asp Cys Gly Glu Pro Glu Glu Cys Met Asn Arg Cys
1               5                   10                  15

Cys Asn Ala Thr Thr Cys Thr Leu Lys Pro Asp Ala Val Cys Ala His
            20                  25                  30

Gly Leu Cys Cys Glu Asp Cys Gln Leu Lys Pro Ala Gly Thr Ala Cys
        35                  40                  45

Arg Asp Ser Ser Asn Ser Cys Asp Leu Pro Glu Phe Cys Thr Gly Ala
    50                  55                  60

Ser Pro His Cys Pro Ala Asn Val Tyr Leu His Asp Gly His
65                  70                  75

<210> 12
<211> 79
<212> PRT
<213> Homo sapiens

<400> 12
Gly Glu Gln Cys Asp Cys Gly Phe Leu Asp Asp Cys Val Asp Pro Cys
1               5                   10                  15

Cys Asp Ser Leu Thr Cys Gln Leu Arg Pro Gly Ala Gln Cys Ala Ser
            20                  25                  30

Asp Gly Pro Cys Cys Gln Asn Cys Gln Leu Arg Pro Ser Gly Trp Gln
        35                  40                  45

Cys Arg Pro Thr Arg Gly Asp Cys Asp Leu Pro Glu Phe Cys Pro Gly
    50                  55                  60

Asp Ser Ser Gln Cys Pro Pro Asp Val Ser Leu Gly Asp Gly Glu
65                  70                  75

<210> 13
<211> 80
<212> PRT
<213> Homo sapiens

<400> 13
Gly Glu Glu Cys Asp Pro Gly Ile Met Tyr Leu Asn Asn Asp Thr Cys
1               5                   10                  15

Cys Asn Ser Asp Cys Thr Leu Lys Glu Gly Val Gln Cys Ser Asp Arg
            20                  25                  30

Asn Ser Pro Cys Cys Lys Asn Cys Gln Phe Glu Thr Ala Gln Lys Lys
        35                  40                  45

Cys Gln Glu Ala Ile Asn Ala Thr Cys Lys Gly Val Ser Tyr Cys Thr
    50                  55                  60

Gly Asn Ser Ser Glu Cys Pro Pro Pro Gly Asn Ala Glu Asp Asp Thr
65                  70                  75                  80

<210> 14
<211> 81
<212> PRT
<213> Homo sapiens

<400> 14
Asn Glu Glu Cys Asp Cys Gly Asn Lys Asn Glu Cys Gln Phe Lys Lys
1               5                   10                  15

Cys Cys Asp Tyr Asn Thr Cys Lys Leu Lys Gly Ser Val Lys Cys Gly
            20                  25                  30

Ser Gly Pro Cys Cys Thr Ser Lys Cys Glu Leu Ser Ile Ala Gly Thr
        35                  40                  45

Pro Cys Arg Lys Ser Ile Asp Pro Glu Cys Asp Phe Thr Glu Tyr Cys
    50                  55                  60

Asn Gly Thr Ser Ser Asn Cys Val Pro Asp Thr Tyr Ala Leu Asn Gly
65                  70                  75                  80

Arg

<210> 15
<211> 78
<212> PRT
<213> Homo sapiens

<400> 15
Gly Glu Glu Cys Asp Cys Gly Glu Glu Glu Glu Cys Asn Asn Pro Cys
1               5                   10                  15

Cys Asn Ala Ser Asn Cys Thr Leu Arg Pro Gly Ala Glu Cys Ala His
            20                  25                  30

Gly Ser Cys Cys His Gln Cys Lys Leu Leu Ala Pro Gly Thr Leu Cys
        35                  40                  45

Arg Glu Gln Ala Arg Gln Cys Asp Leu Pro Glu Phe Cys Thr Gly Lys
    50                  55                  60

Ser Pro His Cys Pro Thr Asn Phe Tyr Gln Met Asp Gly Thr
65                  70                  75

<210> 16
<211> 78
<212> PRT
<213> Homo sapiens

<400> 16
Gly Glu Glu Cys Asp Cys Gly Thr Ile Arg Gln Cys Ala Lys Asp Pro
1               5                   10                  15

Cys Cys Leu Leu Asn Cys Thr Leu His Pro Gly Ala Ala Cys Ala Phe
            20                  25                  30

Gly Ile Cys Cys Lys Asp Cys Lys Phe Leu Pro Ser Gly Thr Leu Cys
        35                  40                  45

Arg Gln Gln Val Gly Glu Cys Asp Leu Pro Glu Trp Cys Asn Gly Thr
    50                  55                  60

Ser His Gln Cys Pro Asp Asp Val Tyr Val Gln Asp Gly Ile
65                  70                  75

<210> 17
<211> 78
<212> PRT
<213> Homo sapiens

<400> 17
Glu Glu Gln Cys Asp Cys Gly Ser Val Gln Gln Cys Glu Gln Asp Ala
1               5                   10                  15

Cys Cys Leu Leu Asn Cys Thr Leu Arg Pro Gly Ala Ala Cys Ala Phe
            20                  25                  30

Gly Leu Cys Cys Lys Asp Cys Lys Phe Met Pro Ser Gly Glu Leu Cys
        35                  40                  45

Arg Gln Glu Val Asn Glu Cys Asp Leu Pro Glu Trp Cys Asn Gly Thr
    50                  55                  60

Ser His Gln Cys Pro Glu Asp Arg Tyr Val Gln Asp Gly Ile
65                  70                  75

<210> 18
<211> 79
<212> PRT
<213> Homo sapiens

<400> 18
Gly Glu Glu Cys Asp Cys Gly Thr Pro Ala Glu Cys Val Leu Glu Gly
1               5                   10                  15

Ala Glu Cys Cys Lys Lys Cys Thr Leu Thr Gln Asp Ser Gln Cys Ser
            20                  25                  30

Asp Gly Leu Cys Cys Lys Lys Cys Lys Phe Gln Pro Met Gly Thr Val
        35                  40                  45

Cys Arg Glu Ala Val Asn Asp Cys Asp Ile Arg Glu Thr Cys Ser Gly
    50                  55                  60

Asn Ser Ser Gln Cys Ala Pro Asn Ile His Lys Met Asp Gly Tyr
65                  70                  75

<210> 19
<211> 78
<212> PRT
<213> Homo sapiens

<400> 19
Gly Glu Glu Cys Asp Cys Gly Phe His Val Glu Cys Tyr Gly Leu Cys
1               5                   10                  15

Cys Lys Lys Cys Ser Leu Ser Asn Gly Ala His Cys Ser Asp Gly Pro
            20                  25                  30

Cys Cys Asn Asn Thr Ser Cys Leu Phe Gln Pro Arg Gly Tyr Glu Cys
        35                  40                  45

Arg Asp Ala Val Asn Glu Cys Asp Ile Thr Glu Tyr Cys Thr Gly Asp
    50                  55                  60

Ser Gly Gln Cys Pro Pro Asn Leu His Lys Gln Asp Gly Tyr
65                  70                  75

<210> 20
<211> 78
<212> PRT
<213> Homo sapiens

<400> 20
Gly Glu Asp Cys Asp Cys Gly Thr Ser Glu Glu Cys Thr Asn Ile Cys
1               5                   10                  15

Cys Asp Ala Lys Thr Cys Lys Ile Lys Ala Thr Phe Gln Cys Ala Leu
            20                  25                  30

Gly Glu Cys Cys Glu Lys Cys Gln Phe Lys Lys Ala Gly Met Val Cys
        35                  40                  45

Arg Pro Ala Lys Asp Glu Cys Asp Leu Pro Glu Met Cys Asn Gly Lys
    50                  55                  60

Ser Gly Asn Cys Pro Asp Asp Arg Phe Gln Val Asn Gly Phe
65                  70                  75

<210> 21

<211> 78
<212> PRT
<213> Homo sapiens

<400> 21
Gly Glu Glu Cys Asp Cys Gly Pro Leu Lys His Cys Ala Lys Asp Pro
1               5                   10                  15

Cys Cys Leu Ser Asn Cys Thr Leu Thr Asp Gly Ser Thr Cys Ala Phe
            20                  25                  30

Gly Leu Cys Cys Lys Asp Cys Lys Phe Leu Pro Ser Gly Lys Val Cys
        35                  40                  45

Arg Lys Glu Val Asn Glu Cys Asp Leu Pro Glu Trp Cys Asn Gly Thr
    50                  55                  60

Ser His Lys Cys Pro Asp Asp Phe Tyr Val Glu Asp Gly Ile
65                  70                  75

<210> 22
<211> 78
<212> PRT
<213> Homo sapiens

<400> 22
Asn Glu Glu Cys Asp Cys Gly Ser Thr Glu Glu Cys Gln Lys Asp Arg
1               5                   10                  15

Cys Cys Gln Ser Asn Cys Lys Leu Gln Pro Gly Ala Asn Cys Ser Ile
            20                  25                  30

Gly Leu Cys Cys His Asp Cys Arg Phe Arg Pro Ser Gly Tyr Val Cys
        35                  40                  45

Arg Gln Glu Gly Asn Glu Cys Asp Leu Ala Glu Tyr Cys Asp Gly Asn
    50                  55                  60

Ser Ser Ser Cys Pro Asn Asp Val Tyr Lys Gln Asp Gly Thr
65                  70                  75

<210> 23
<211> 80
<212> PRT
<213> Homo sapiens

<400> 23
Asn Glu Ile Cys Asp Cys Gly Thr Glu Ala Gln Cys Gly Pro Ala Ser
1               5                   10                  15

Cys Cys Asp Phe Arg Thr Cys Val Leu Lys Asp Gly Ala Lys Cys Tyr
            20                  25                  30

Lys Gly Leu Cys Cys Lys Asp Cys Gln Ile Leu Gln Ser Gly Val Glu
        35                  40                  45

Cys Arg Pro Lys Ala His Pro Glu Cys Asp Ile Ala Glu Asn Cys Asn
    50                  55                  60

Gly Ser Ser Pro Glu Cys Gly Pro Asp Ile Thr Leu Ile Asn Gly Leu
65                  70                  75                  80

<210> 24
<211> 78
<212> PRT
<213> Homo sapiens

<400> 24
Gly Glu Glu Cys Asp Cys Gly Pro Gly Gln Glu Cys Arg Asp Leu Cys
1               5                   10                  15

Cys Phe Ala His Asn Cys Ser Leu Arg Pro Gly Ala Gln Cys Ala His
            20                  25                  30

Gly Asp Cys Cys Val Arg Cys Leu Leu Lys Pro Ala Gly Ala Leu Cys
        35                  40                  45

Arg Gln Ala Met Gly Asp Cys Asp Leu Pro Glu Phe Cys Thr Gly Thr
    50                  55                  60

Ser Ser His Cys Pro Pro Asp Val Tyr Leu Leu Asp Gly Ser
65                  70                  75

<210> 25
<211> 70
<212> PRT
<213> Trimeresurus flavoviridis

<400> 25
Gly Glu Glu Cys Asp Cys Gly Ser Pro Ser Asn Pro Cys Cys Asp Ala
1               5                   10                  15

Ala Thr Cys Lys Leu Arg Pro Gly Ala Gln Cys Ala Asp Gly Leu Cys
            20                  25                  30

Cys Asp Gln Cys Arg Phe Lys Lys Lys Arg Thr Ile Cys Arg Ile Ala
        35                  40                  45

Arg Gly Asp Phe Pro Asp Asp Arg Cys Thr Gly Gln Ser Ala Asp Cys
    50                  55                  60

Pro Arg Trp Asn Asp Leu

65                  70

<210> 26
<211> 72
<212> PRT
<213> Artificial Sequence

<220>
<223> Description of Artificial Sequence: Synthetic
      polypeptide

<400> 26
Gly Glu Gln Cys Asp Cys Gly Ser Leu His Ala Cys Cys Asp Glu Asn
1               5                   10                  15

Cys Ile Leu Lys Ala Lys Ala Glu Cys Ser Asp Gly Pro Cys Cys His
            20                  25                  30

Lys Cys Lys Phe His Arg Lys Gly Tyr Pro Cys Arg Pro Ser Ser Arg
        35                  40                  45

Ser Asp Leu Pro Glu Phe Cys Asn Gly Thr Ser Ala Leu Cys Pro Asn
    50                  55                  60

Asn Arg His Lys Gln Asp Gly Ser
65                  70

<210> 27
<211> 77
<212> PRT
<213> Artificial Sequence

<220>
<223> Description of Artificial Sequence: Synthetic
      polypeptide

<400> 27
Gly Glu Glu Cys Asp Cys Gly Thr Glu Gln Asp Ile Gly Glu Thr Cys
1               5                   10                  15

Cys Asp Ile Ala Thr Cys Arg Phe Lys Ala Gly Ser Asn Cys Ala Glu
            20                  25                  30

Gly Pro Cys Cys Glu Asn Cys Leu Phe Met Ser Lys Glu Arg Met Cys
        35                  40                  45

Arg Pro Ser Phe Glu Glu Asp Leu Pro Glu Tyr Cys Asn Gly Ser Ser
    50                  55                  60

Ala Ser Cys Pro Glu Asn His Tyr Val Gln Thr Gly His
65                  70                  75

<210> 28
<211> 77
<212> PRT
<213> Artificial Sequence

<220>
<223> Description of Artificial Sequence: Synthetic
      polypeptide

<400> 28
Gly Glu Gln Cys Asp Cys Gly Tyr Lys Glu Ala Lys Lys Cys Cys Asn
1               5                   10                  15

Pro Ala Asp Cys Thr Leu Val Arg Ser Ala Glu Cys Gly Thr Gly Ser
            20                  25                  30

Cys Cys Asn Asn Lys Thr Cys Thr Ile His Glu Arg Gly His Val Cys
        35                  40                  45

Arg Lys Ser Val Asp Met Asp Phe Pro Glu Tyr Cys Asn Gly Thr Ser
    50                  55                  60

Glu Phe Cys Val Pro Asp Val Lys Ala Ala Asp Leu Glu
65                  70                  75

<210> 29
<211> 74
<212> PRT
<213> Artificial Sequence

<220>
<223> Description of Artificial Sequence: Synthetic
      polypeptide

<400> 29
Gly Glu Glu Cys Asp Cys Gly Ser Phe Lys Gln Ser Tyr Cys Cys Gln
1               5                   10                  15

Ser Asp Cys His Leu Thr Pro Gly Ser Ile Cys His Ile Gly Glu Cys
            20                  25                  30

Cys Thr Asn Cys Ser Phe Ser Pro Pro Gly Thr Leu Cys Arg Pro Ile
        35                  40                  45

Gln Asn Ile Asp Leu Pro Glu Tyr Cys His Gly Thr Thr Val Thr Cys
    50                  55                  60

Pro Ala Asn Val Tyr Met Gln Asp Gly Thr
65                  70

<210> 30
<211> 74
<212> PRT

<213> Artificial Sequence

<220>
<223> Description of Artificial Sequence: Synthetic
      polypeptide

<400> 30
Gly Glu Glu Cys Asp Cys Gly Pro Ala Gln Glu Pro Cys Cys Asp Ala
1               5                   10                  15

His Thr Cys Val Leu Lys Pro Gly Phe Thr Cys Ala Glu Gly Glu Cys
            20                  25                  30

Cys Glu Ser Cys Gln Ile Lys Lys Ala Gly Ser Ile Cys Arg Pro Ala
        35                  40                  45

Lys Asp Glu Asp Phe Pro Glu Met Cys Thr Gly His Ser Pro Ala Cys
    50                  55                  60

Pro Lys Asp Gln Phe Arg Val Asn Gly Phe
65                  70

<210> 31
<211> 74
<212> PRT
<213> Artificial Sequence

<220>
<223> Description of Artificial Sequence: Synthetic
      polypeptide

<400> 31
Gly Glu Gln Cys Asp Cys Gly Pro Pro Glu Asp Arg Cys Cys Asn Ser
1               5                   10                  15

Thr Thr Cys Gln Leu Ala Glu Gly Ala Gln Cys Ala His Gly Thr Cys
            20                  25                  30

Cys Gln Glu Cys Lys Val Lys Pro Ala Gly Glu Leu Cys Arg Pro Lys
        35                  40                  45

Lys Asp Met Asp Leu Glu Glu Phe Cys Asp Gly Arg His Pro Glu Cys
    50                  55                  60

Pro Glu Asp Ala Phe Gln Glu Asn Gly Thr
65                  70

<210> 32
<211> 75
<212> PRT
<213> Artificial Sequence

<220>

<223> Description of Artificial Sequence: Synthetic
      polypeptide

<400> 32
Gly Glu Glu Cys Asp Cys Gly Thr Pro Lys Glu Asp Pro Cys Cys Glu
1               5                   10                  15

Gly Ser Thr Cys Lys Leu Lys Ser Phe Ala Glu Cys Ala Tyr Gly Asp
            20                  25                  30

Cys Cys Lys Asp Cys Arg Phe Leu Pro Gly Gly Thr Leu Cys Arg Gly
        35                  40                  45

Lys Thr Ser Glu Asp Val Pro Glu Tyr Cys Asn Gly Ser Ser Gln Phe
    50                  55                  60

Cys Gln Pro Asp Val Phe Ile Gln Asn Gly Tyr
65                  70                  75

<210> 33
<211> 82
<212> PRT
<213> Artificial Sequence

<220>
<223> Description of Artificial Sequence: Synthetic
      polypeptide

<400> 33
Gly Glu Glu Cys Asp Cys Gly Tyr Ser Asp Gln Glu Cys Cys Phe Asp
1               5                   10                  15

Ala Asn Gln Pro Glu Gly Arg Lys Cys Lys Leu Lys Pro Gly Lys Gln
            20                  25                  30

Cys Ser Pro Ser Gln Gly Pro Cys Cys Thr Ala Gln Cys Ala Phe Lys
        35                  40                  45

Ser Lys Ser Glu Lys Cys Arg Asp Asp Ser Asp Ala Arg Glu Gly Ile
    50                  55                  60

Cys Asn Gly Phe Thr Ala Leu Cys Pro Ala Ser Asp Pro Lys Pro Asn
65                  70                  75                  80

Phe Thr

<210> 34
<211> 75
<212> PRT
<213> Artificial Sequence

<220>
<223> Description of Artificial Sequence: Synthetic
      polypeptide

<400> 34
Gly Glu Glu Cys Asp Cys Gly Ser Val Gln Glu Ala Gly Gly Asn Cys
1               5                   10                  15

Cys Lys Lys Cys Thr Leu Thr His Asp Ala Met Cys Ser Asp Gly Leu
            20                  25                  30

Cys Cys Arg Arg Cys Lys Tyr Glu Pro Arg Gly Val Ser Cys Arg Glu
        35                  40                  45

Ala Val Asn Glu Asp Ile Ala Glu Thr Cys Thr Gly Asp Ser Ser Gln
    50                  55                  60

Cys Pro Pro Asn Leu His Lys Leu Asp Gly Tyr
65                  70                  75

<210> 35
<211> 74
<212> PRT
<213> Artificial Sequence

<220>
<223> Description of Artificial Sequence: Synthetic
      polypeptide

<400> 35
Gly Glu Glu Cys Asp Cys Gly Glu Pro Glu Glu Arg Cys Cys Asn Ala
1               5                   10                  15

Thr Thr Cys Thr Leu Lys Pro Asp Ala Val Cys Ala His Gly Leu Cys
            20                  25                  30

Cys Glu Asp Cys Gln Leu Lys Pro Ala Gly Thr Ala Cys Arg Asp Ser
        35                  40                  45

Ser Asn Ser Asp Leu Pro Glu Phe Cys Thr Gly Ala Ser Pro His Cys
    50                  55                  60

Pro Ala Asn Val Tyr Leu His Asp Gly His
65                  70

<210> 36
<211> 75
<212> PRT
<213> Artificial Sequence

<220>
<223> Description of Artificial Sequence: Synthetic
      polypeptide

<400> 36
Gly Glu Gln Cys Asp Cys Gly Phe Leu Asp Asp Pro Cys Cys Asp Ser
1               5                   10                  15

Leu Thr Cys Gln Leu Arg Pro Gly Ala Gln Cys Ala Ser Asp Gly Pro
            20                  25                  30

Cys Cys Gln Asn Cys Gln Leu Arg Pro Ser Gly Trp Gln Cys Arg Pro
        35                  40                  45

Thr Arg Gly Asp Asp Leu Pro Glu Phe Cys Pro Gly Asp Ser Ser Gln
    50                  55                  60

Cys Pro Pro Asp Val Ser Leu Gly Asp Gly Glu
65                  70                  75

<210> 37
<211> 77
<212> PRT
<213> Artificial Sequence

<220>
<223> Description of Artificial Sequence: Synthetic
      polypeptide

<400> 37
Gly Glu Glu Cys Asp Cys Gly Ile Met Tyr Leu Asp Thr Cys Cys Asn
1               5                   10                  15

Ser Asp Cys Thr Leu Lys Glu Gly Val Gln Cys Ser Asp Arg Asn Ser
            20                  25                  30

Pro Cys Cys Lys Asn Cys Gln Phe Glu Thr Ala Gln Lys Lys Cys Gln
        35                  40                  45

Glu Ala Ile Asn Ala Thr Lys Gly Val Ser Tyr Cys Thr Gly Asn Ser
    50                  55                  60

Ser Glu Cys Pro Pro Pro Gly Asn Ala Glu Asp Asp Thr
65                  70                  75

<210> 38
<211> 77
<212> PRT
<213> Artificial Sequence

<220>
<223> Description of Artificial Sequence: Synthetic
      polypeptide

<400> 38
Gly Glu Glu Cys Asp Cys Gly Asn Lys Asn Glu Lys Lys Cys Cys Asp

1               5                   10                  15

Tyr Asn Thr Cys Lys Leu Lys Gly Ser Val Lys Cys Gly Ser Gly Pro
            20                  25                  30

Cys Cys Thr Ser Lys Cys Glu Leu Ser Ile Ala Gly Thr Pro Cys Arg
        35                  40                  45

Lys Ser Ile Asp Pro Glu Asp Phe Thr Glu Tyr Cys Asn Gly Thr Ser
    50                  55                  60

Ser Asn Cys Val Pro Asp Thr Tyr Ala Leu Asn Gly Arg
65                  70                  75

<210> 39
<211> 74
<212> PRT
<213> Artificial Sequence

<220>
<223> Description of Artificial Sequence: Synthetic
      polypeptide

<400> 39
Gly Glu Glu Cys Asp Cys Gly Glu Glu Glu Glu Pro Cys Cys Asn Ala
1               5                   10                  15

Ser Asn Cys Thr Leu Arg Pro Gly Ala Glu Cys Ala His Gly Ser Cys
            20                  25                  30

Cys His Gln Cys Lys Leu Leu Ala Pro Gly Thr Leu Cys Arg Glu Gln
        35                  40                  45

Ala Arg Gln Asp Leu Pro Glu Phe Cys Thr Gly Lys Ser Pro His Cys
    50                  55                  60

Pro Thr Asn Phe Tyr Gln Met Asp Gly Thr
65                  70

<210> 40
<211> 74
<212> PRT
<213> Artificial Sequence

<220>
<223> Description of Artificial Sequence: Synthetic
      polypeptide

<400> 40
Gly Glu Glu Cys Asp Cys Gly Thr Ile Arg Gln Asp Pro Cys Cys Leu
1               5                   10                  15

Leu Asn Cys Thr Leu His Pro Gly Ala Ala Cys Ala Phe Gly Ile Cys
            20                  25                  30

Cys Lys Asp Cys Lys Phe Leu Pro Ser Gly Thr Leu Cys Arg Gln Gln
        35                  40                  45

Val Gly Glu Asp Leu Pro Glu Trp Cys Asn Gly Thr Ser His Gln Cys
    50                  55                  60

Pro Asp Asp Val Tyr Val Gln Asp Gly Ile
65                  70

<210> 41
<211> 74
<212> PRT
<213> Artificial Sequence

<220>
<223> Description of Artificial Sequence: Synthetic
      polypeptide

<400> 41
Gly Glu Gln Cys Asp Cys Gly Ser Val Gln Gln Asp Ala Cys Cys Leu
1               5                   10                  15

Leu Asn Cys Thr Leu Arg Pro Gly Ala Ala Cys Ala Phe Gly Leu Cys
            20                  25                  30

Cys Lys Asp Cys Lys Phe Met Pro Ser Gly Glu Leu Cys Arg Gln Glu
        35                  40                  45

Val Asn Glu Asp Leu Pro Glu Trp Cys Asn Gly Thr Ser His Gln Cys
    50                  55                  60

Pro Glu Asp Arg Tyr Val Gln Asp Gly Ile
65                  70

<210> 42
<211> 75
<212> PRT
<213> Artificial Sequence

<220>
<223> Description of Artificial Sequence: Synthetic
      polypeptide

<400> 42
Gly Glu Glu Cys Asp Cys Gly Thr Pro Ala Glu Glu Gly Ala Glu Cys
1               5                   10                  15

Cys Lys Lys Cys Thr Leu Thr Gln Asp Ser Gln Cys Ser Asp Gly Leu
            20                  25                  30

Cys Cys Lys Lys Cys Lys Phe Gln Pro Met Gly Thr Val Cys Arg Glu
        35                  40                  45

Ala Val Asn Asp Asp Ile Arg Glu Thr Cys Ser Gly Asn Ser Ser Gln
    50                  55                  60

Cys Ala Pro Asn Ile His Lys Met Asp Gly Tyr
65                  70                  75

<210> 43
<211> 74
<212> PRT
<213> Artificial Sequence

<220>
<223> Description of Artificial Sequence: Synthetic
      polypeptide

<400> 43
Gly Glu Glu Cys Asp Cys Gly Phe His Val Glu Leu Cys Cys Lys Lys
1               5                   10                  15

Cys Ser Leu Ser Asn Gly Ala His Cys Ser Asp Gly Pro Cys Cys Asn
            20                  25                  30

Asn Thr Ser Cys Leu Phe Gln Pro Arg Gly Tyr Glu Cys Arg Asp Ala
        35                  40                  45

Val Asn Glu Asp Ile Thr Glu Tyr Cys Thr Gly Asp Ser Gly Gln Cys
    50                  55                  60

Pro Pro Asn Leu His Lys Gln Asp Gly Tyr
65                  70

<210> 44
<211> 74
<212> PRT
<213> Artificial Sequence

<220>
<223> Description of Artificial Sequence: Synthetic
      polypeptide

<400> 44
Gly Glu Asp Cys Asp Cys Gly Thr Ser Glu Glu Ile Cys Cys Asp Ala
1               5                   10                  15

Lys Thr Cys Lys Ile Lys Ala Thr Phe Gln Cys Ala Leu Gly Glu Cys
            20                  25                  30

Cys Glu Lys Cys Gln Phe Lys Lys Ala Gly Met Val Cys Arg Pro Ala
        35                  40                  45

Lys Asp Glu Asp Leu Pro Glu Met Cys Asn Gly Lys Ser Gly Asn Cys
    50                  55                  60

Pro Asp Asp Arg Phe Gln Val Asn Gly Phe
65                  70

<210> 45
<211> 74
<212> PRT
<213> Artificial Sequence

<220>
<223> Description of Artificial Sequence: Synthetic
      polypeptide

<400> 45
Gly Glu Glu Cys Asp Cys Gly Pro Leu Lys His Asp Pro Cys Cys Leu
1               5                   10                  15

Ser Asn Cys Thr Leu Thr Asp Gly Ser Thr Cys Ala Phe Gly Leu Cys
            20                  25                  30

Cys Lys Asp Cys Lys Phe Leu Pro Ser Gly Lys Val Cys Arg Lys Glu
        35                  40                  45

Val Asn Glu Asp Leu Pro Glu Trp Cys Asn Gly Thr Ser His Lys Cys
    50                  55                  60

Pro Asp Asp Phe Tyr Val Glu Asp Gly Ile
65                  70

<210> 46
<211> 74
<212> PRT
<213> Artificial Sequence

<220>
<223> Description of Artificial Sequence: Synthetic
      polypeptide

<400> 46
Gly Glu Glu Cys Asp Cys Gly Ser Thr Glu Glu Asp Arg Cys Cys Gln
1               5                   10                  15

Ser Asn Cys Lys Leu Gln Pro Gly Ala Asn Cys Ser Ile Gly Leu Cys
            20                  25                  30

Cys His Asp Cys Arg Phe Arg Pro Ser Gly Tyr Val Cys Arg Gln Glu
        35                  40                  45

Gly Asn Glu Asp Leu Ala Glu Tyr Cys Asp Gly Asn Ser Ser Ser Cys

50                  55                  60

Pro Asn Asp Val Tyr Lys Gln Asp Gly Thr
65                  70

<210> 47
<211> 76
<212> PRT
<213> Artificial Sequence

<220>
<223> Description of Artificial Sequence: Synthetic
      polypeptide

<400> 47
Gly Glu Ile Cys Asp Cys Gly Thr Glu Ala Gln Ala Ser Cys Cys Asp
1               5                   10                  15

Phe Arg Thr Cys Val Leu Lys Asp Gly Ala Lys Cys Tyr Lys Gly Leu
            20                  25                  30

Cys Cys Lys Asp Cys Gln Ile Leu Gln Ser Gly Val Glu Cys Arg Pro
        35                  40                  45

Lys Ala His Pro Glu Asp Ile Ala Glu Asn Cys Asn Gly Ser Ser Pro
    50                  55                  60

Glu Cys Gly Pro Asp Ile Thr Leu Ile Asn Gly Leu
65                  70                  75

<210> 48
<211> 74
<212> PRT
<213> Artificial Sequence

<220>
<223> Description of Artificial Sequence: Synthetic
      polypeptide

<400> 48
Gly Glu Glu Cys Asp Cys Gly Pro Gly Gln Glu Leu Cys Cys Phe Ala
1               5                   10                  15

His Asn Cys Ser Leu Arg Pro Gly Ala Gln Cys Ala His Gly Asp Cys
            20                  25                  30

Cys Val Arg Cys Leu Leu Lys Pro Ala Gly Ala Leu Cys Arg Gln Ala
        35                  40                  45

Met Gly Asp Asp Leu Pro Glu Phe Cys Thr Gly Thr Ser Ser His Cys
    50                  55                  60

Pro Pro Asp Val Tyr Leu Leu Asp Gly Ser
65                  70

<210> 49
<211> 90
<212> PRT
<213> Unknown

<220>
<223> Description of Unknown: Snake venom VAP1 polypeptide

<400> 49
Ser Pro Ala Val Cys Gly Asn Tyr Phe Val Glu Val Gly Glu Glu Cys
1               5                   10                  15

Asp Cys Gly Ser Pro Arg Thr Cys Arg Asp Pro Cys Cys Asp Ala Thr
            20                  25                  30

Thr Cys Lys Leu Arg Gln Gly Ala Gln Cys Ala Glu Gly Leu Cys Cys
        35                  40                  45

Asp Gln Cys Arg Phe Lys Gly Ala Gly Thr Glu Cys Arg Ala Ala Lys
    50                  55                  60

Asp Glu Cys Asp Met Ala Asp Val Cys Thr Gly Arg Ser Ala Glu Cys
65                  70                  75                  80

Thr Asp Arg Phe Gln Arg Asn Gly Gln Pro
                85                  90

<210> 50
<211> 90
<212> PRT
<213> Unknown

<220>
<223> Description of Unknown: Snake venom Catrocollastatin
      polypeptide

<400> 50
Ser Pro Pro Val Cys Gly Asn Glu Leu Leu Glu Val Gly Glu Glu Cys
1               5                   10                  15

Asp Cys Gly Thr Pro Glu Asn Cys Gln Asn Glu Cys Cys Asp Ala Ala
            20                  25                  30

Thr Cys Lys Leu Lys Ser Gly Ser Gln Cys Gly His Gly Asp Cys Cys
        35                  40                  45

Glu Gln Cys Lys Phe Ser Lys Ser Gly Thr Glu Cys Arg Ala Ser Met
    50                  55                  60

Ser Glu Cys Asp Pro Ala Glu His Cys Thr Gly Gln Ser Ser Glu Cys
65                  70                  75                  80

Pro Ala Asp Val Phe His Lys Asn Gly Gln
                85                  90

<210> 51
<211> 83
<212> PRT
<213> Unknown

<220>
<223> Description of Unknown: Snake venom Salmosin3 polypeptide

<400> 51
Ser Pro Pro Val Cys Gly Asn Tyr Tyr Pro Glu Val Gly Glu Asp Cys
1               5                   10                  15

Asp Cys Gly Pro Pro Ala Asn Cys Gln Asn Pro Cys Cys Asp Ala Ala
            20                  25                  30

Thr Cys Gly Leu Thr Thr Gly Ser Gln Cys Ala Glu Gly Leu Cys Cys
        35                  40                  45

Asp Gln Cys Arg Leu Lys Lys Ala Gly Thr Ile Cys Arg Lys Ala Arg
    50                  55                  60

Gly Asp Asn Pro Asp Asp Arg Cys Thr Gly Gln Ser Gly Val Cys Pro
65                  70                  75                  80

Arg Asn Thr

<210> 52
<211> 83
<212> PRT
<213> Unknown

<220>
<223> Description of Unknown: Snake venom Bitistatin polypeptide

<400> 52
Ser Pro Pro Val Cys Gly Asn Lys Ile Leu Glu Glu Gly Glu Glu Cys
1               5                   10                  15

Asp Cys Gly Ser Pro Ala Asn Cys Gln Asp Arg Cys Cys Asn Ala Ala
            20                  25                  30

Thr Cys Lys Leu Thr Pro Gly Ser Gln Cys Asn His Gly Glu Cys Cys
        35                  40                  45

Asp Gln Cys Lys Phe Lys Lys Ala Gly Thr Val Cys Arg Ile Ala Arg

50                  55                  60

Gly Asp Trp Asn Asp Asp Tyr Cys Thr Gly Lys Ser Ser Asp Cys Pro
65                  70                  75                  80

Trp Asn His

<210> 53
<211> 70
<212> PRT
<213> Trimeresurus flavoviridis

<400> 53
Gly Glu Glu Cys Asp Cys Gly Ser Pro Ser Asn Pro Cys Cys Asp Ala
1               5                   10                  15

Ala Thr Cys Lys Leu Arg Pro Gly Ala Gln Cys Ala Asp Gly Leu Cys
            20                  25                  30

Cys Asp Gln Cys Arg Phe Lys Lys Lys Arg Thr Ile Cys Arg Ile Ala
        35                  40                  45

Arg Gly Asp Phe Pro Asp Asp Arg Cys Thr Gly Gln Ser Ala Asp Cys
    50                  55                  60

Pro Arg Trp Asn Asp Leu
65                  70

<210> 54
<211> 213
<212> DNA
<213> Artificial Sequence

<220>
<223> Description of Artificial Sequence: Synthetic
      polynucleotide

<400> 54
gagcagtgtg actgtggttc tctacatgca tgctgtgatg aaaactgtat actgaaggcg      60

aaagcagagt gcagtgatgg tccatgttgt cataagtgta aatttcaccg taagggatat     120

ccttgcaggc cttctagtcg ttccgatctc ccagaatttt gcaatggtac atctgcatta     180

tgccccaaca acaggcataa gcaagatggc tca                                  213

<210> 55
<211> 231
<212> DNA
<213> Artificial Sequence

<220>
<223> Description of Artificial Sequence: Synthetic

polynucleotide

<400> 55
ggagaggagt gtgactgtgg gactgaacag gatattggag aaacatgctg tgatattgcc      60

acatgtagat ttaaagccgg ttcaaactgt gctgaaggac catgctgcga aaactgtcta     120

tttatgtcaa aagaaagaat gtgtaggcct tcctttgaag aagacctccc tgaatattgc     180

aatggatcat ctgcatcatg cccagaaaac cactatgttc agactgggca t              231


<210> 56
<211> 228
<212> DNA
<213> Artificial Sequence

<220>
<223> Description of Artificial Sequence: Synthetic
      polynucleotide

<400> 56
gagcagtgtg attgtggcta taaagaggca aaaaaatgct gtaatcctgc agattgtact      60

ctagttagat ctgcagaatg tggcactgga tcatgctgta acaataaaac ttgtacgatc     120

cacgaaagag gccatgtctg cagaaaaagt gtagatatgg attttccaga atattgcaat     180

ggaacatctg agttttgtgt acctgatgtg aaagctgctg atttagaa                  228


<210> 57
<211> 219
<212> DNA
<213> Artificial Sequence

<220>
<223> Description of Artificial Sequence: Synthetic
      polynucleotide

<400> 57
gaggaatgtg actgtggctc cttcaagcag agttattgct gccaaagtga ctgtcactta      60

acaccgggga gcatctgcca tataggagag tgctgtacaa actgcagctt ctccccacca     120

gggactctct gcagacctat ccaaaatata gaccttccag agtactgtca cgggaccacc     180

gtgacatgtc ccgcaaacgt ttatatgcaa gatggaacc                            219


<210> 58
<211> 222
<212> DNA
<213> Artificial Sequence

<220>
<223> Description of Artificial Sequence: Synthetic
      polynucleotide

<400> 58
ggtgaagagt gtgactgtgg ccctgctcag gagccttgct gtgatgcaca cacatgtgta      60

ctgaagccag gatttacttg tgcagaagga gaatgctgtg aatcttgtca gataaaaaaa     120

gcagggtcca tatgcagacc ggcgaaagat gaagattttc ctgagatgtg cactggccac 180

tcgcctgcct gtcctaagga ccagttcagg gtcaatggat tt 222

<210> 59
<211> 222
<212> DNA
<213> Artificial Sequence

<220>
<223> Description of Artificial Sequence: Synthetic
      polynucleotide

<400> 59
ggggagcagt gcgactgcgg cccccccgag gaccgctgct gcaactctac cacctgccag 60

ctggctgagg gggcccagtg tgcgcacggt acctgctgcc aggagtgcaa ggtgaagccg 120

gctggtgagc tgtgccgtcc caagaaggac atggacctcg aggagttctg tgacggccgg 180

caccctgagt gcccggaaga cgccttccag gagaacggca cg 222

<210> 60
<211> 225
<212> DNA
<213> Artificial Sequence

<220>
<223> Description of Artificial Sequence: Synthetic
      polynucleotide

<400> 60
ggggaagagt gtgactgtgg tactccaaag gaagaccctt gctgcgaagg aagtacctgt 60

aagcttaaat catttgctga gtgtgcatat ggtgactgtt gtaaagactg tcggttcctt 120

ccaggaggta ctttatgccg aggaaaaacc agtgaggatg ttccagagta ctgcaatggt 180

tcttctcagt tctgtcagcc agatgttttt attcagaatg gatat 225

<210> 61
<211> 246
<212> DNA
<213> Artificial Sequence

<220>
<223> Description of Artificial Sequence: Synthetic
      polynucleotide

<400> 61
ggtgaagaat gtgattgtgg ctatagtgac caggaatgct gcttcgatgc aaatcaacca 60

gagggaagaa aatgcaaact gaaacctggg aaacagtgca gtccaagtca aggtccttgt 120

tgtacagcac agtgtgcatt caagtcaaag tctgagaagt gtcgggatga ttcagacgca 180

agggaaggaa tatgtaatgg cttcacagct ctctgcccag catctgaccc taaaccaaac 240

ttcaca 246

<210> 62
<211> 225
<212> DNA
<213> Artificial Sequence

<220>
<223> Description of Artificial Sequence: Synthetic
      polynucleotide

<400> 62
ggggaggagt gcgactgcgg ctcggtgcag gaggcaggtg gcaactgctg caagaaatgc      60

accctgactc acgacgccat gtgcagcgac gggctctgct gtcgccgctg caagtacgaa     120

ccacggggtg tgtcctgccg agaggccgtg aacgaggaca tcgcggagac ctgcaccggg     180

gactctagcc agtgcccgcc taacctgcac aagctggacg gttac                     225


<210> 63
<211> 222
<212> DNA
<213> Artificial Sequence

<220>
<223> Description of Artificial Sequence: Synthetic
      polynucleotide

<400> 63
ggagaggagt gtgactgtgg ggagccagag gaacgctgct gcaatgccac cacctgtacc      60

ctgaagccgg acgctgtgtg cgcacatggg ctgtgctgtg aagactgcca gctgaagcct     120

gcaggaacag cgtgcaggga ctccagcaac tccgacctcc cagagttctg cacaggggcc     180

agccctcact gcccagccaa cgtgtacctg cacgatgggc ac                        222


<210> 64
<211> 225
<212> DNA
<213> Artificial Sequence

<220>
<223> Description of Artificial Sequence: Synthetic
      polynucleotide

<400> 64
ggcgagcagt gtgactgtgg cttcctggat gacccctgct gtgattcttt gacctgccag      60

ctgaggccag gtgcacagtg tgcatctgac ggaccctgtt gtcaaaattg ccagctgcgc     120

ccgtctggct ggcagtgtcg tcctaccaga ggggatgact tgcctgaatt ctgcccagga     180

gacagctccc agtgtccccc tgatgtcagc ctaggggatg gcgag                     225


<210> 65
<211> 231
<212> DNA
<213> Artificial Sequence

<220>
<223> Description of Artificial Sequence: Synthetic
      polynucleotide

<400> 65
ggagaagagt gtgattgtgg catcatgtat ctggacacct gctgcaacag cgactgcacg      60

ttgaaggaag gtgtccagtg cagtgacagg aacagtcctt gctgtaaaaa ctgtcagttt     120

gagactgccc agaagaagtg ccaggaggcg attaatgcta ctaaaggcgt gtcctactgc     180

acaggtaata gcagtgagtg cccgcctcca ggaaatgctg aagatgacac t              231


<210> 66
<211> 228
<212> DNA
<213> Artificial Sequence

<220>
<223> Description of Artificial Sequence: Synthetic
      polynucleotide

<400> 66
gaagaatgtg actgtggtaa taaaaatgaa aagaagtgct gtgattataa cacatgtaaa      60

ctgaagggct cagtaaaatg tggttctgga ccatgttgta catcaaagtg tgagttgtca     120

atagcaggca ctccatgtag aaagagtatt gatccagagg attttacaga gtactgcaat     180

ggaacctcta gtaattgtgt tcctgacact tatgcattga atggccgt                  228


<210> 67
<211> 222
<212> DNA
<213> Artificial Sequence

<220>
<223> Description of Artificial Sequence: Synthetic
      polynucleotide

<400> 67
ggggaagagt gtgactgtgg agaagaagag gaaccctgct gcaatgcctc taattgtacc      60

ctgaggccgg gggcggagtg tgctcacggc tcctgctgcc accagtgtaa gctgttggct     120

cctgggaccc tgtgccgcga gcaggccagg caggacctcc cggagttctg tacgggcaag     180

tctccccact gccctaccaa cttctaccag atggatggta cc                        222


<210> 68
<211> 222
<212> DNA
<213> Artificial Sequence

<220>
<223> Description of Artificial Sequence: Synthetic
      polynucleotide

<400> 68

ggggaggaat gtgactgtgg aaccatacgg caggatccct gttgtctgtt aaactgtact 60

ctacatcctg gggctgcttg tgcttttgga atatgttgca aagactgcaa atttctgcca 120

tcaggaactt tatgtagaca acaagttggt gaagaccttc cagagtggtg caatgggaca 180

tcccatcaat gcccagatga tgtgtatgtg caggacggga tc 222

<210> 69
<211> 219
<212> DNA
<213> Artificial Sequence

<220>
<223> Description of Artificial Sequence: Synthetic
      polynucleotide

<400> 69
gagcagtgtg actgtggatc cgtacagcag gacgcctgtt gtctgttgaa ctgcactcta 60

aggcctgggg ctgcctgtgc ttttgggctt tgttgcaaag actgcaagtt catgccatca 120

ggggaactct gtagacaaga ggtcaatgaa gaccttccag aatggtgcaa tggaacatct 180

catcagtgtc cagaagatag atatgtgcag gacgggatc 219

<210> 70
<211> 225
<212> DNA
<213> Artificial Sequence

<220>
<223> Description of Artificial Sequence: Synthetic
      polynucleotide

<400> 70
ggagaggagt gtgattgtgg aaccccggcc gaagaaggag cagagtgttg taagaaatgc 60

accttgactc aagactctca atgcagtgac ggtctttgct gtaaaaagtg caagtttcag 120

cctatgggca ctgtgtgccg agaagcagta aatgatgata ttcgtgaaac gtgctcagga 180

aattcaagcc agtgtgcccc taatattcat aaaatggatg gatat 225

<210> 71
<211> 222
<212> DNA
<213> Artificial Sequence

<220>
<223> Description of Artificial Sequence: Synthetic
      polynucleotide

<400> 71
ggggaggagt gtgattgtgg ttttcatgtg gaattatgct gtaagaaatg ttccctctcc 60

aacggggctc actgcagcga cgggccctgc tgtaacaata cctcatgtct ttttcagcca 120

cgagggtatg aatgccggga tgctgtgaac gaggatatta ctgaatattg tactggagac 180

tctggtcagt gcccaccaaa tcttcataag caagacggat at 222

<210> 72
<211> 222
<212> DNA
<213> Artificial Sequence

<220>
<223> Description of Artificial Sequence: Synthetic
      polynucleotide

<400> 72
ggagaggact gtgattgtgg gacatctgag gaaatttgct gtgatgctaa gacatgtaaa 60

atcaaagcaa cttttcaatg tgcattagga gaatgttgtg aaaaatgcca atttaaaaag 120

gctgggatgg tgtgcagacc agcaaaagat gaggacctgc ctgaaatgtg taatggtaaa 180

tctggtaatt gtcctgatga tagattccaa gtcaatggct tc 222

<210> 73
<211> 222
<212> DNA
<213> Artificial Sequence

<220>
<223> Description of Artificial Sequence: Synthetic
      polynucleotide

<400> 73
ggagaagagt gtgactgtgg acctttaaag catgatccct gctgtctgtc aaattgcact 60

ctgactgatg gttctacttg tgcttttggg ctttgttgca aagactgcaa gttcctacca 120

tcagggaaag tgtgtagaaa ggaggtcaat gaagatcttc cagagtggtg caatggtact 180

tcccataagt gcccagatga cttttatgtg gaagatggaa tt 222

<210> 74
<211> 219
<212> DNA
<213> Artificial Sequence

<220>
<223> Description of Artificial Sequence: Synthetic
      polynucleotide

<400> 74
gaggaatgtg actgtggttc cacagaggag gatcggtgtt gccaatcaaa ttgtaagttg 60

caaccaggtg ccaactgtag cattggactt tgctgtcatg attgtcggtt tcgtccatct 120

ggatacgtgt gtaggcagga aggaaatgaa gaccttgcag agtactgcga cgggaattca 180

agttcctgcc caaatgacgt ttataagcag gatggaacc 219

<210> 75
<211> 225
<212> DNA

<213> Artificial Sequence

<220>
<223> Description of Artificial Sequence: Synthetic
      polynucleotide

<400> 75
gaaatctgtg attgtggtac tgaggctcaa gcaagctgtt gtgattttcg aacttgtgta      60

ctgaaagacg gagcaaaatg ttataaagga ctgtgctgca aagactgtca aattttacaa     120

tcaggcgttg aatgtaggcc gaaagcacat cctgaagaca tcgctgaaaa ttgtaatgga     180

agctcaccag aatgtggtcc tgacataact ttaatcaatg gactt                     225


<210> 76
<211> 222
<212> DNA
<213> Artificial Sequence

<220>
<223> Description of Artificial Sequence: Synthetic
      polynucleotide

<400> 76
ggcgaggagt gtgactgcgg ccctggccag gagctctgct gctttgctca caactgctcg      60

ctgcgcccgg gggcccagtg cgcccacggg gactgctgcg tgcgctgcct gctgaagccg     120

gctggagcgc tgtgccgcca ggccatgggt gacgacctcc ctgagttttg cacgggcacc     180

tcctcccact gtcccccaga cgtttaccta ctggacggct ca                        222


<210> 77
<211> 54
<212> DNA
<213> Artificial Sequence

<220>
<223> Description of Artificial Sequence: Synthetic
      primer

<400> 77
gttccagatc tcgaaaacct gtattttcag ggcgagcagt gtgactgtgg ttct            54


<210> 78
<211> 45
<212> DNA
<213> Artificial Sequence

<220>
<223> Description of Artificial Sequence: Synthetic
      primer

<400> 78
gttattcgcc atggcttatg agccatcttg cttatgcctg ttgtt                      45


<210> 79
<211> 57

<212> DNA
<213> Artificial Sequence

<220>
<223> Description of Artificial Sequence: Synthetic
      primer

<400> 79
gttccagatc tcgaaaacct gtattttcag ggagaggagt gtgactgtgg gactgaa          57


<210> 80
<211> 45
<212> DNA
<213> Artificial Sequence

<220>
<223> Description of Artificial Sequence: Synthetic
      primer

<400> 80
gttattcgcc atggcttaat gcccagtctg aacatagtgg ttttc                       45


<210> 81
<211> 56
<212> DNA
<213> Artificial Sequence

<220>
<223> Description of Artificial Sequence: Synthetic
      primer

<400> 81
gttccgccat ggctgaaaac ctgtattttc agggtgagca gtgtgattgt ggctat           56


<210> 82
<211> 44
<212> DNA
<213> Artificial Sequence

<220>
<223> Description of Artificial Sequence: Synthetic
      primer

<400> 82
gttattcgga attcttattc taaatcagca gctttcacat cagg                        44


<210> 83
<211> 54
<212> DNA
<213> Artificial Sequence

<220>
<223> Description of Artificial Sequence: Synthetic
      primer

<400> 83
gttccagatc tcgaaaacct gtattttcag ggcgaggaat gtgactgtgg ctcc             54

<210> 84
<211> 45
<212> DNA
<213> Artificial Sequence

<220>
<223> Description of Artificial Sequence: Synthetic
      primer

<400> 84
gttattcgcc atggcttagg ttccatcttg catataaacg tttgc                        45


<210> 85
<211> 57
<212> DNA
<213> Artificial Sequence

<220>
<223> Description of Artificial Sequence: Synthetic
      primer

<400> 85
gttccagatc tcgaaaacct gtattttcag ggtgaagagt gtgactgtgg ccctgct           57


<210> 86
<211> 45
<212> DNA
<213> Artificial Sequence

<220>
<223> Description of Artificial Sequence: Synthetic
      primer

<400> 86
gttattcgcc atggcttaaa atccattgac cctgaactgg tcctt                        45


<210> 87
<211> 57
<212> DNA
<213> Artificial Sequence

<220>
<223> Description of Artificial Sequence: Synthetic
      primer

<400> 87
gttccagatc tcgaaaacct gtattttcag ggggagcagt gcgactgcgg ccccccc           57


<210> 88
<211> 45
<212> DNA
<213> Artificial Sequence

<220>
<223> Description of Artificial Sequence: Synthetic
      primer

<400> 88
gttattcgcc atggcttacg tgccgttctc ctggaaggcg tcttc                        45

<210> 89
<211> 57
<212> DNA
<213> Artificial Sequence

<220>
<223> Description of Artificial Sequence: Synthetic
      primer

<400> 89
gttccagatc tcgaaaacct gtattttcag ggggaagagt gtgactgtgg tactcca          57


<210> 90
<211> 45
<212> DNA
<213> Artificial Sequence

<220>
<223> Description of Artificial Sequence: Synthetic
      primer

<400> 90
gttattcgcc atggcttaat atccattctg aataaaaaca tctgg                      45


<210> 91
<211> 57
<212> DNA
<213> Artificial Sequence

<220>
<223> Description of Artificial Sequence: Synthetic
      primer

<400> 91
gttccagatc tcgaaaacct gtattttcag ggtgaagaat gtgattgtgg ctatagt          57


<210> 92
<211> 45
<212> DNA
<213> Artificial Sequence

<220>
<223> Description of Artificial Sequence: Synthetic
      primer

<400> 92
gttattcgcc atggcttatg tgaagtttgg tttagggtca gatgc                      45


<210> 93
<211> 57
<212> DNA
<213> Artificial Sequence

<220>
<223> Description of Artificial Sequence: Synthetic
      primer

<400> 93
gttccagatc tcgaaaacct gtattttcag ggggaggagt gcgactgcgg ctcggtg          57


<210> 94
<211> 45
<212> DNA
<213> Artificial Sequence

<220>
<223> Description of Artificial Sequence: Synthetic
      primer

<400> 94
gttattcgcc atggcttagt aaccgtccag cttgtgcagg ttagg                       45


<210> 95
<211> 57
<212> DNA
<213> Artificial Sequence

<220>
<223> Description of Artificial Sequence: Synthetic
      primer

<400> 95
gttccagatc tcgaaaacct gtattttcag ggagaggagt gtgactgtgg ggagcca          57


<210> 96
<211> 45
<212> DNA
<213> Artificial Sequence

<220>
<223> Description of Artificial Sequence: Synthetic
      primer

<400> 96
gttattcgcc atggcttagt gcccatcgtg caggtacacg ttggc                       45


<210> 97
<211> 57
<212> DNA
<213> Artificial Sequence

<220>
<223> Description of Artificial Sequence: Synthetic
      primer

<400> 97
gttccagatc tcgaaaacct gtattttcag ggcgagcagt gtgactgtgg cttcctg          57


<210> 98
<211> 45
<212> DNA
<213> Artificial Sequence

<220>
<223> Description of Artificial Sequence: Synthetic

primer

<400> 98
gttattcgcc atggcttact cgccatcccc taggctgaca tcagg                45

<210> 99
<211> 57
<212> DNA
<213> Artificial Sequence

<220>
<223> Description of Artificial Sequence: Synthetic
      primer

<400> 99
gttccagatc tcgaaaacct gtattttcag ggagaagagt gtgattgtgg catcatg   57

<210> 100
<211> 45
<212> DNA
<213> Artificial Sequence

<220>
<223> Description of Artificial Sequence: Synthetic
      primer

<400> 100
gttattcgcc atggcttaag tgtcatcttc agcatttcct ggagg                45

<210> 101
<211> 54
<212> DNA
<213> Artificial Sequence

<220>
<223> Description of Artificial Sequence: Synthetic
      primer

<400> 101
gttccagatc tcgaaaacct gtattttcag ggcgaagaat gtgactgtgg taat      54

<210> 102
<211> 45
<212> DNA
<213> Artificial Sequence

<220>
<223> Description of Artificial Sequence: Synthetic
      primer

<400> 102
gttattcgcc atggcttaac ggccattcaa tgcataagtg tcagg                45

<210> 103
<211> 57
<212> DNA
<213> Artificial Sequence

<220>
<223> Description of Artificial Sequence: Synthetic
      primer

<400> 103
gttccagatc tcgaaaacct gtattttcag ggggaagagt gtgactgtgg agaagaa          57


<210> 104
<211> 45
<212> DNA
<213> Artificial Sequence

<220>
<223> Description of Artificial Sequence: Synthetic
      primer

<400> 104
gttattcgcc atggcttagg taccatccat ctggtagaag ttggt                       45


<210> 105
<211> 57
<212> DNA
<213> Artificial Sequence

<220>
<223> Description of Artificial Sequence: Synthetic
      primer

<400> 105
gttccagatc tcgaaaacct gtattttcag ggggaggaat gtgactgtgg aaccata          57


<210> 106
<211> 45
<212> DNA
<213> Artificial Sequence

<220>
<223> Description of Artificial Sequence: Synthetic
      primer

<400> 106
gttattcgcc atggcttaga tcccgtcctg cacatacaca tcatc                       45


<210> 107
<211> 54
<212> DNA
<213> Artificial Sequence

<220>
<223> Description of Artificial Sequence: Synthetic
      primer

<400> 107
gttccagatc tcgaaaacct gtattttcag ggcgagcagt gtgactgtgg atcc             54


<210> 108
<211> 45
<212> DNA

<213> Artificial Sequence

<220>
<223> Description of Artificial Sequence: Synthetic
      primer

<400> 108
gttattcgcc atggcttaga tcccgtcctg cacatatcta tcttc                        45


<210> 109
<211> 57
<212> DNA
<213> Artificial Sequence

<220>
<223> Description of Artificial Sequence: Synthetic
      primer

<400> 109
gttccagatc tcgaaaacct gtattttcag ggagaggagt gtgattgtgg aaccccg           57


<210> 110
<211> 45
<212> DNA
<213> Artificial Sequence

<220>
<223> Description of Artificial Sequence: Synthetic
      primer

<400> 110
gttattcgcc atggcttaat atccatccat tttatgaata ttagg                        45


<210> 111
<211> 57
<212> DNA
<213> Artificial Sequence

<220>
<223> Description of Artificial Sequence: Synthetic
      primer

<400> 111
gttccagatc tcgaaaacct gtattttcag ggggaggagt gtgattgtgg ttttcat           57


<210> 112
<211> 45
<212> DNA
<213> Artificial Sequence

<220>
<223> Description of Artificial Sequence: Synthetic
      primer

<400> 112
gttattcgcc atggcttaat atccgtcttg cttatgaaga tttgg                        45


<210> 113

<211> 57
<212> DNA
<213> Artificial Sequence

<220>
<223> Description of Artificial Sequence: Synthetic
      primer

<400> 113
gttccagatc tcgaaaacct gtattttcag ggagaggact gtgattgtgg gacatct          57


<210> 114
<211> 45
<212> DNA
<213> Artificial Sequence

<220>
<223> Description of Artificial Sequence: Synthetic
      primer

<400> 114
gttattcgcc atggcttaga agccattgac ttggaatcta tcatc                       45


<210> 115
<211> 59
<212> DNA
<213> Artificial Sequence

<220>
<223> Description of Artificial Sequence: Synthetic
      primer

<400> 115
gttccgccat ggctgaaaac ctgtattttc agggagaaga gtgtgactgt ggaccttta        59


<210> 116
<211> 44
<212> DNA
<213> Artificial Sequence

<220>
<223> Description of Artificial Sequence: Synthetic
      primer

<400> 116
gttattcgga attcttaaat tccatcttcc acataaaagt catc                        44


<210> 117
<211> 54
<212> DNA
<213> Artificial Sequence

<220>
<223> Description of Artificial Sequence: Synthetic
      primer

<400> 117
gttccagatc tcgaaaacct gtattttcag ggcgaggaat gtgactgtgg ttcc             54

<210> 118
<211> 45
<212> DNA
<213> Artificial Sequence

<220>
<223> Description of Artificial Sequence: Synthetic
      primer

<400> 118
gttattcgcc atggcttagg ttccatcctg cttataaacg tcatt                        45


<210> 119
<211> 54
<212> DNA
<213> Artificial Sequence

<220>
<223> Description of Artificial Sequence: Synthetic
      primer

<400> 119
gttccagatc tcgaaaacct gtattttcag ggcgaaatct gtgattgtgg tact              54


<210> 120
<211> 45
<212> DNA
<213> Artificial Sequence

<220>
<223> Description of Artificial Sequence: Synthetic
      primer

<400> 120
gttattcgcc atggcttaaa gtccattgat taaagttatg tcagg                        45


<210> 121
<211> 57
<212> DNA
<213> Artificial Sequence

<220>
<223> Description of Artificial Sequence: Synthetic
      primer

<400> 121
gttccagatc tcgaaaacct gtattttcag ggcgaggagt gtgactgcgg ccctggc           57


<210> 122
<211> 45
<212> DNA
<213> Artificial Sequence

<220>
<223> Description of Artificial Sequence: Synthetic
      primer

<400> 122

gttattcgga attccttatg agccgtccag taggtaaacg tctgg                    45

<210> 123
<211> 229
<212> PRT
<213> Artificial Sequence

<220>
<223> Description of Artificial Sequence: Synthetic
      polypeptide

<400> 123
Met Ser Asp Lys Ile Ile His Leu Thr Asp Asp Ser Phe Asp Thr Asp
1               5                   10                  15

Val Leu Lys Ala Asp Gly Ala Ile Leu Val Asp Phe Trp Ala Glu Trp
            20                  25                  30

Cys Gly Pro Cys Lys Met Ile Ala Pro Ile Leu Asp Glu Ile Ala Asp
        35                  40                  45

Glu Tyr Gln Gly Lys Leu Thr Val Ala Lys Leu Asn Ile Asp Gln Asn
    50                  55                  60

Pro Gly Thr Ala Pro Lys Tyr Gly Ile Arg Gly Ile Pro Thr Leu Leu
65                  70                  75                  80

Leu Phe Lys Asn Gly Glu Val Ala Ala Thr Lys Val Gly Ala Leu Ser
                85                  90                  95

Lys Gly Gln Leu Lys Glu Phe Leu Asp Ala Asn Leu Ala Gly Ser Gly
            100                 105                 110

Ser Gly His Met His His His His His His Ser Ser Gly Leu Val Pro
        115                 120                 125

Arg Gly Ser Gly Met Lys Glu Thr Ala Ala Ala Lys Phe Glu Arg Gln
    130                 135                 140

His Met Asp Ser Pro Asp Leu Glu Asn Leu Tyr Phe Gln Gly Glu Gln
145                 150                 155                 160

Cys Asp Cys Gly Ser Leu His Ala Cys Cys Asp Glu Asn Cys Ile Leu
                165                 170                 175

Lys Ala Lys Ala Glu Cys Ser Asp Gly Pro Cys Cys His Lys Cys Lys
            180                 185                 190

Phe His Arg Lys Gly Tyr Pro Cys Arg Pro Ser Ser Arg Ser Asp Leu
        195                 200                 205

Pro Glu Phe Cys Asn Gly Thr Ser Ala Leu Cys Pro Asn Asn Arg His
    210                 215                 220

Lys Gln Asp Gly Ser
225

<210> 124
<211> 234
<212> PRT
<213> Artificial Sequence

<220>
<223> Description of Artificial Sequence: Synthetic
      polypeptide

<400> 124
Met Ser Asp Lys Ile Ile His Leu Thr Asp Asp Ser Phe Asp Thr Asp
1               5                   10                  15

Val Leu Lys Ala Asp Gly Ala Ile Leu Val Asp Phe Trp Ala Glu Trp
            20                  25                  30

Cys Gly Pro Cys Lys Met Ile Ala Pro Ile Leu Asp Glu Ile Ala Asp
        35                  40                  45

Glu Tyr Gln Gly Lys Leu Thr Val Ala Lys Leu Asn Ile Asp Gln Asn
    50                  55                  60

Pro Gly Thr Ala Pro Lys Tyr Gly Ile Arg Gly Ile Pro Thr Leu Leu
65                  70                  75                  80

Leu Phe Lys Asn Gly Glu Val Ala Ala Thr Lys Val Gly Ala Leu Ser
                85                  90                  95

Lys Gly Gln Leu Lys Glu Phe Leu Asp Ala Asn Leu Ala Gly Ser Gly
            100                 105                 110

Ser Gly His Met His His His His His His Ser Ser Gly Leu Val Pro
        115                 120                 125

Arg Gly Ser Gly Met Lys Glu Thr Ala Ala Ala Lys Phe Glu Arg Gln
    130                 135                 140

His Met Asp Ser Pro Asp Leu Glu Asn Leu Tyr Phe Gln Gly Glu Glu
145                 150                 155                 160

Cys Asp Cys Gly Thr Glu Gln Asp Ile Gly Glu Thr Cys Cys Asp Ile
                165                 170                 175

Ala Thr Cys Arg Phe Lys Ala Gly Ser Asn Cys Ala Glu Gly Pro Cys
            180                 185                 190

Cys Glu Asn Cys Leu Phe Met Ser Lys Glu Arg Met Cys Arg Pro Ser
        195                 200                 205

Phe Glu Glu Asp Leu Pro Glu Tyr Cys Asn Gly Ser Ser Ala Ser Cys
    210                 215                 220

Pro Glu Asn His Tyr Val Gln Thr Gly His
225                 230

<210> 125
<211> 244
<212> PRT
<213> Artificial Sequence

<220>
<223> Description of Artificial Sequence: Synthetic
      polypeptide

<400> 125
   Met Ser Asp Lys Ile Ile His Leu Thr Asp Asp Ser Phe Asp Thr Asp
   1               5                   10                  15
   Val Leu Lys Ala Asp Gly Ala Ile Leu Val Asp Phe Trp Ala Glu Trp
               20                  25                  30
   Cys Gly Pro Cys Lys Met Ile Ala Pro Ile Leu Asp Glu Ile Ala Asp
           35                  40                  45
   Glu Tyr Gln Gly Lys Leu Thr Val Ala Lys Leu Asn Ile Asp Gln Asn
       50                  55                  60
   Pro Gly Thr Ala Pro Lys Tyr Gly Ile Arg Gly Ile Pro Thr Leu Leu
   65                  70                  75                  80
   Leu Phe Lys Asn Gly Glu Val Ala Ala Thr Lys Val Gly Ala Leu Ser
                   85                  90                  95
   Lys Gly Gln Leu Lys Glu Phe Leu Asp Ala Asn Leu Ala Gly Ser Gly
               100                 105                 110
   Ser Gly His Met His His His His His His Ser Ser Gly Leu Val Pro
           115                 120                 125
   Arg Gly Ser Gly Met Lys Glu Thr Ala Ala Ala Lys Phe Glu Arg Gln
       130                 135                 140
   His Met Asp Ser Pro Asp Leu Gly Thr Asp Asp Asp Asp Lys Ala Met
   145                 150                 155                 160
   Ala Glu Asn Leu Tyr Phe Gln Gly Glu Gln Cys Asp Cys Gly Tyr Lys
                   165                 170                 175
   Glu Ala Lys Lys Cys Cys Asn Pro Ala Asp Cys Thr Leu Val Arg Ser
               180                 185                 190
   Ala Glu Cys Gly Thr Gly Ser Cys Cys Asn Asn Lys Thr Cys Thr Ile
           195                 200                 205
   His Glu Arg Gly His Val Cys Arg Lys Ser Val Asp Met Asp Phe Pro
       210                 215                 220
   Glu Tyr Cys Asn Gly Thr Ser Glu Phe Cys Val Pro Asp Val Lys Ala
   225                 230                 235                 240
   Ala Asp Leu Glu

<210> 126
<211> 231
<212> PRT
<213> Artificial Sequence

<220>
<223> Description of Artificial Sequence: Synthetic
      polypeptide

<400> 126
Met Ser Asp Lys Ile Ile His Leu Thr Asp Asp Ser Phe Asp Thr Asp
1               5                   10                  15

Val Leu Lys Ala Asp Gly Ala Ile Leu Val Asp Phe Trp Ala Glu Trp
            20                  25                  30

Cys Gly Pro Cys Lys Met Ile Ala Pro Ile Leu Asp Glu Ile Ala Asp
        35                  40                  45

Glu Tyr Gln Gly Lys Leu Thr Val Ala Lys Leu Asn Ile Asp Gln Asn
    50                  55                  60

Pro Gly Thr Ala Pro Lys Tyr Gly Ile Arg Gly Ile Pro Thr Leu Leu
65                  70                  75                  80

Leu Phe Lys Asn Gly Glu Val Ala Ala Thr Lys Val Gly Ala Leu Ser
                85                  90                  95

Lys Gly Gln Leu Lys Glu Phe Leu Asp Ala Asn Leu Ala Gly Ser Gly

100                 105                 110

Ser Gly His Met His His His His His His Ser Ser Gly Leu Val Pro
        115                 120                 125

Arg Gly Ser Gly Met Lys Glu Thr Ala Ala Ala Lys Phe Glu Arg Gln
    130                 135                 140

His Met Asp Ser Pro Asp Leu Glu Asn Leu Tyr Phe Gln Gly Glu Glu
145                 150                 155                 160

Cys Asp Cys Gly Ser Phe Lys Gln Ser Tyr Cys Cys Gln Ser Asp Cys
                165                 170                 175

His Leu Thr Pro Gly Ser Ile Cys His Ile Gly Glu Cys Cys Thr Asn
            180                 185                 190

Cys Ser Phe Ser Pro Pro Gly Thr Leu Cys Arg Pro Ile Gln Asn Ile
        195                 200                 205

Asp Leu Pro Glu Tyr Cys His Gly Thr Thr Val Thr Cys Pro Ala Asn
    210                 215                 220

Val Tyr Met Gln Asp Gly Thr
225                 230

<210> 127
<211> 231
<212> PRT
<213> Artificial Sequence

<220>
<223> Description of Artificial Sequence: Synthetic
      polypeptide

<400> 127
Met Ser Asp Lys Ile Ile His Leu Thr Asp Asp Ser Phe Asp Thr Asp
1               5                   10                  15

Val Leu Lys Ala Asp Gly Ala Ile Leu Val Asp Phe Trp Ala Glu Trp
            20                  25                  30

Cys Gly Pro Cys Lys Met Ile Ala Pro Ile Leu Asp Glu Ile Ala Asp
        35                  40                  45

Glu Tyr Gln Gly Lys Leu Thr Val Ala Lys Leu Asn Ile Asp Gln Asn
    50                  55                  60

Pro Gly Thr Ala Pro Lys Tyr Gly Ile Arg Gly Ile Pro Thr Leu Leu
65                  70                  75                  80

Leu Phe Lys Asn Gly Glu Val Ala Ala Thr Lys Val Gly Ala Leu Ser
                85                  90                  95

Lys Gly Gln Leu Lys Glu Phe Leu Asp Ala Asn Leu Ala Gly Ser Gly
            100                 105                 110

Ser Gly His Met His His His His His His Ser Ser Gly Leu Val Pro
        115                 120                 125

Arg Gly Ser Gly Met Lys Glu Thr Ala Ala Ala Lys Phe Glu Arg Gln
    130                 135                 140

His Met Asp Ser Pro Asp Leu Glu Asn Leu Tyr Phe Gln Gly Glu Glu
145                 150                 155                 160

Cys Asp Cys Gly Pro Ala Gln Glu Pro Cys Cys Asp Ala His Thr Cys
                165                 170                 175

Val Leu Lys Pro Gly Phe Thr Cys Ala Glu Gly Glu Cys Cys Glu Ser
            180                 185                 190

Cys Gln Ile Lys Lys Ala Gly Ser Ile Cys Arg Pro Ala Lys Asp Glu
        195                 200                 205

Asp Phe Pro Glu Met Cys Thr Gly His Ser Pro Ala Cys Pro Lys Asp
    210                 215                 220

Gln Phe Arg Val Asn Gly Phe
225                 230

<210> 128
<211> 231
<212> PRT
<213> Artificial Sequence

<220>
<223> Description of Artificial Sequence: Synthetic
      polypeptide

<400> 128
Met Ser Asp Lys Ile Ile His Leu Thr Asp Asp Ser Phe Asp Thr Asp
1               5                   10                  15

Val Leu Lys Ala Asp Gly Ala Ile Leu Val Asp Phe Trp Ala Glu Trp
            20                  25                  30

Cys Gly Pro Cys Lys Met Ile Ala Pro Ile Leu Asp Glu Ile Ala Asp
        35                  40                  45

Glu Tyr Gln Gly Lys Leu Thr Val Ala Lys Leu Asn Ile Asp Gln Asn
    50                  55                  60

Pro Gly Thr Ala Pro Lys Tyr Gly Ile Arg Gly Ile Pro Thr Leu Leu
65                  70                  75                  80

Leu Phe Lys Asn Gly Glu Val Ala Ala Thr Lys Val Gly Ala Leu Ser
                85                  90                  95

Lys Gly Gln Leu Lys Glu Phe Leu Asp Ala Asn Leu Ala Gly Ser Gly
            100                 105                 110

Ser Gly His Met His His His His His His Ser Ser Gly Leu Val Pro
        115                 120                 125

Arg Gly Ser Gly Met Lys Glu Thr Ala Ala Ala Lys Phe Glu Arg Gln
    130                 135                 140

His Met Asp Ser Pro Asp Leu Glu Asn Leu Tyr Phe Gln Gly Glu Gln
145                 150                 155                 160

Cys Asp Cys Gly Pro Pro Glu Asp Arg Cys Cys Asn Ser Thr Thr Cys
                165                 170                 175

Gln Leu Ala Glu Gly Ala Gln Cys Ala His Gly Thr Cys Cys Gln Glu
            180                 185                 190

Cys Lys Val Lys Pro Ala Gly Glu Leu Cys Arg Pro Lys Lys Asp Met
        195                 200                 205

Asp Leu Glu Glu Phe Cys Asp Gly Arg His Pro Glu Cys Pro Glu Asp
    210                 215                 220

Ala Phe Gln Glu Asn Gly Thr
225                 230

<210> 129
<211> 232
<212> PRT
<213> Artificial Sequence

<220>
<223> Description of Artificial Sequence: Synthetic
      polypeptide

<400> 129
Met Ser Asp Lys Ile Ile His Leu Thr Asp Asp Ser Phe Asp Thr Asp
1               5                   10                  15

Val Leu Lys Ala Asp Gly Ala Ile Leu Val Asp Phe Trp Ala Glu Trp
            20                  25                  30

Cys Gly Pro Cys Lys Met Ile Ala Pro Ile Leu Asp Glu Ile Ala Asp
        35                  40                  45

Glu Tyr Gln Gly Lys Leu Thr Val Ala Lys Leu Asn Ile Asp Gln Asn
    50                  55                  60

Pro Gly Thr Ala Pro Lys Tyr Gly Ile Arg Gly Ile Pro Thr Leu Leu
65                  70                  75                  80

Leu Phe Lys Asn Gly Glu Val Ala Ala Thr Lys Val Gly Ala Leu Ser
                85                  90                  95

Lys Gly Gln Leu Lys Glu Phe Leu Asp Ala Asn Leu Ala Gly Ser Gly
            100                 105                 110

Ser Gly His Met His His His His His His Ser Ser Gly Leu Val Pro
        115                 120                 125

Arg Gly Ser Gly Met Lys Glu Thr Ala Ala Ala Lys Phe Glu Arg Gln
    130                 135                 140

His Met Asp Ser Pro Asp Leu Glu Asn Leu Tyr Phe Gln Gly Glu Glu
145                 150                 155                 160

Cys Asp Cys Gly Thr Pro Lys Glu Asp Pro Cys Cys Glu Gly Ser Thr
                165                 170                 175

Cys Lys Leu Lys Ser Phe Ala Glu Cys Ala Tyr Gly Asp Cys Cys Lys
            180                 185                 190

Asp Cys Arg Phe Leu Pro Gly Gly Thr Leu Cys Arg Gly Lys Thr Ser
        195                 200                 205

Glu Asp Val Pro Glu Tyr Cys Asn Gly Ser Ser Gln Phe Cys Gln Pro
    210                 215                 220

Asp Val Phe Ile Gln Asn Gly Tyr
225                 230

<210> 130
<211> 239
<212> PRT
<213> Artificial Sequence

<220>
<223> Description of Artificial Sequence: Synthetic

polypeptide

<400> 130
Met Ser Asp Lys Ile Ile His Leu Thr Asp Asp Ser Phe Asp Thr Asp
1               5                   10                  15

Val Leu Lys Ala Asp Gly Ala Ile Leu Val Asp Phe Trp Ala Glu Trp
            20                  25                  30

Cys Gly Pro Cys Lys Met Ile Ala Pro Ile Leu Asp Glu Ile Ala Asp
        35                  40                  45

Glu Tyr Gln Gly Lys Leu Thr Val Ala Lys Leu Asn Ile Asp Gln Asn
    50                  55                  60

Pro Gly Thr Ala Pro Lys Tyr Gly Ile Arg Gly Ile Pro Thr Leu Leu
65                  70                  75                  80

Leu Phe Lys Asn Gly Glu Val Ala Ala Thr Lys Val Gly Ala Leu Ser
                85                  90                  95

Lys Gly Gln Leu Lys Glu Phe Leu Asp Ala Asn Leu Ala Gly Ser Gly
            100                 105                 110

Ser Gly His Met His His His His His His Ser Ser Gly Leu Val Pro
        115                 120                 125

Arg Gly Ser Gly Met Lys Glu Thr Ala Ala Ala Lys Phe Glu Arg Gln
    130                 135                 140

His Met Asp Ser Pro Asp Leu Glu Asn Leu Tyr Phe Gln Gly Glu Glu
145                 150                 155                 160

Cys Asp Cys Gly Tyr Ser Asp Gln Glu Cys Cys Phe Asp Ala Asn Gln
                165                 170                 175

Pro Glu Gly Arg Lys Cys Lys Leu Lys Pro Gly Lys Gln Cys Ser Pro
            180                 185                 190

Ser Gln Gly Pro Cys Cys Thr Ala Gln Cys Ala Phe Lys Ser Lys Ser
        195                 200                 205

Glu Lys Cys Arg Asp Asp Ser Asp Ala Arg Glu Gly Ile Cys Asn Gly
    210                 215                 220

Phe Thr Ala Leu Cys Pro Ala Ser Asp Pro Lys Pro Asn Phe Thr
225                 230                 235

<210> 131
<211> 232
<212> PRT
<213> Artificial Sequence

<220>
<223> Description of Artificial Sequence: Synthetic
      polypeptide

<400> 131
Met Ser Asp Lys Ile Ile His Leu Thr Asp Asp Ser Phe Asp Thr Asp
1               5                   10                  15

Val Leu Lys Ala Asp Gly Ala Ile Leu Val Asp Phe Trp Ala Glu Trp
            20                  25                  30

Cys Gly Pro Cys Lys Met Ile Ala Pro Ile Leu Asp Glu Ile Ala Asp
        35                  40                  45

Glu Tyr Gln Gly Lys Leu Thr Val Ala Lys Leu Asn Ile Asp Gln Asn
    50                  55                  60

Pro Gly Thr Ala Pro Lys Tyr Gly Ile Arg Gly Ile Pro Thr Leu Leu
65                  70                  75                  80

Leu Phe Lys Asn Gly Glu Val Ala Ala Thr Lys Val Gly Ala Leu Ser
                85                  90                  95

Lys Gly Gln Leu Lys Glu Phe Leu Asp Ala Asn Leu Ala Gly Ser Gly
            100                 105                 110

Ser Gly His Met His His His His His His Ser Ser Gly Leu Val Pro
        115                 120                 125

Arg Gly Ser Gly Met Lys Glu Thr Ala Ala Ala Lys Phe Glu Arg Gln
    130                 135                 140

His Met Asp Ser Pro Asp Leu Glu Asn Leu Tyr Phe Gln Gly Glu Glu
145                 150                 155                 160

Cys Asp Cys Gly Ser Val Gln Glu Ala Gly Gly Asn Cys Cys Lys Lys
                165                 170                 175

Cys Thr Leu Thr His Asp Ala Met Cys Ser Asp Gly Leu Cys Cys Arg
            180                 185                 190

Arg Cys Lys Tyr Glu Pro Arg Gly Val Ser Cys Arg Glu Ala Val Asn
        195                 200                 205

Glu Asp Ile Ala Glu Thr Cys Thr Gly Asp Ser Ser Gln Cys Pro Pro

210                 215                 220

Asn Leu His Lys Leu Asp Gly Tyr
225                 230

<210> 132
<211> 231
<212> PRT
<213> Artificial Sequence

<220>
<223> Description of Artificial Sequence: Synthetic
      polypeptide

<400> 132
Met Ser Asp Lys Ile Ile His Leu Thr Asp Asp Ser Phe Asp Thr Asp
1               5                   10                  15

Val Leu Lys Ala Asp Gly Ala Ile Leu Val Asp Phe Trp Ala Glu Trp
            20                  25                  30

Cys Gly Pro Cys Lys Met Ile Ala Pro Ile Leu Asp Glu Ile Ala Asp
        35                  40                  45

Glu Tyr Gln Gly Lys Leu Thr Val Ala Lys Leu Asn Ile Asp Gln Asn
    50                  55                  60

Pro Gly Thr Ala Pro Lys Tyr Gly Ile Arg Gly Ile Pro Thr Leu Leu
65                  70                  75                  80

Leu Phe Lys Asn Gly Glu Val Ala Ala Thr Lys Val Gly Ala Leu Ser
                85                  90                  95

Lys Gly Gln Leu Lys Glu Phe Leu Asp Ala Asn Leu Ala Gly Ser Gly
            100                 105                 110

Ser Gly His Met His His His His His His Ser Ser Gly Leu Val Pro
        115                 120                 125

Arg Gly Ser Gly Met Lys Glu Thr Ala Ala Ala Lys Phe Glu Arg Gln
    130                 135                 140

His Met Asp Ser Pro Asp Leu Glu Asn Leu Tyr Phe Gln Gly Glu Glu
145                 150                 155                 160

Cys Asp Cys Gly Glu Pro Glu Glu Arg Cys Cys Asn Ala Thr Thr Cys
                165                 170                 175

Thr Leu Lys Pro Asp Ala Val Cys Ala His Gly Leu Cys Cys Glu Asp
            180                 185                 190

Cys Gln Leu Lys Pro Ala Gly Thr Ala Cys Arg Asp Ser Ser Asn Ser
        195                 200                 205

Asp Leu Pro Glu Phe Cys Thr Gly Ala Ser Pro His Cys Pro Ala Asn
    210                 215                 220

Val Tyr Leu His Asp Gly His
225                 230

<210> 133
<211> 232
<212> PRT
<213> Artificial Sequence

<220>
<223> Description of Artificial Sequence: Synthetic
      polypeptide

<400> 133
Met Ser Asp Lys Ile Ile His Leu Thr Asp Asp Ser Phe Asp Thr Asp
1               5                   10                  15

Val Leu Lys Ala Asp Gly Ala Ile Leu Val Asp Phe Trp Ala Glu Trp
            20                  25                  30

Cys Gly Pro Cys Lys Met Ile Ala Pro Ile Leu Asp Glu Ile Ala Asp
        35                  40                  45

Glu Tyr Gln Gly Lys Leu Thr Val Ala Lys Leu Asn Ile Asp Gln Asn
    50                  55                  60

Pro Gly Thr Ala Pro Lys Tyr Gly Ile Arg Gly Ile Pro Thr Leu Leu
65                  70                  75                  80

Leu Phe Lys Asn Gly Glu Val Ala Ala Thr Lys Val Gly Ala Leu Ser
                85                  90                  95

Lys Gly Gln Leu Lys Glu Phe Leu Asp Ala Asn Leu Ala Gly Ser Gly
            100                 105                 110

Ser Gly His Met His His His His His His Ser Ser Gly Leu Val Pro
        115                 120                 125

Arg Gly Ser Gly Met Lys Glu Thr Ala Ala Ala Lys Phe Glu Arg Gln
    130                 135                 140

His Met Asp Ser Pro Asp Leu Glu Asn Leu Tyr Phe Gln Gly Glu Gln
145                 150                 155                 160

Cys Asp Cys Gly Phe Leu Asp Asp Pro Cys Cys Asp Ser Leu Thr Cys
                165                 170                 175

Gln Leu Arg Pro Gly Ala Gln Cys Ala Ser Asp Gly Pro Cys Cys Gln
            180                 185                 190

Asn Cys Gln Leu Arg Pro Ser Gly Trp Gln Cys Arg Pro Thr Arg Gly
        195                 200                 205

Asp Asp Leu Pro Glu Phe Cys Pro Gly Asp Ser Ser Gln Cys Pro Pro
    210                 215                 220

Asp Val Ser Leu Gly Asp Gly Glu
225                 230

<210> 134
<211> 234
<212> PRT
<213> Artificial Sequence

<220>
<223> Description of Artificial Sequence: Synthetic
      polypeptide

<400> 134
Met Ser Asp Lys Ile Ile His Leu Thr Asp Asp Ser Phe Asp Thr Asp
1               5                   10                  15

Val Leu Lys Ala Asp Gly Ala Ile Leu Val Asp Phe Trp Ala Glu Trp
            20                  25                  30

Cys Gly Pro Cys Lys Met Ile Ala Pro Ile Leu Asp Glu Ile Ala Asp
        35                  40                  45

Glu Tyr Gln Gly Lys Leu Thr Val Ala Lys Leu Asn Ile Asp Gln Asn
    50                  55                  60

Pro Gly Thr Ala Pro Lys Tyr Gly Ile Arg Gly Ile Pro Thr Leu Leu
65                  70                  75                  80

Leu Phe Lys Asn Gly Glu Val Ala Ala Thr Lys Val Gly Ala Leu Ser
                85                  90                  95

Lys Gly Gln Leu Lys Glu Phe Leu Asp Ala Asn Leu Ala Gly Ser Gly
            100                 105                 110

Ser Gly His Met His His His His His His Ser Ser Gly Leu Val Pro
        115                 120                 125

Arg Gly Ser Gly Met Lys Glu Thr Ala Ala Ala Lys Phe Glu Arg Gln
    130                 135                 140

His Met Asp Ser Pro Asp Leu Glu Asn Leu Tyr Phe Gln Gly Glu Glu
145                 150                 155                 160

Cys Asp Cys Gly Ile Met Tyr Leu Asp Thr Cys Cys Asn Ser Asp Cys
                165                 170                 175

Thr Leu Lys Glu Gly Val Gln Cys Ser Asp Arg Asn Ser Pro Cys Cys
            180                 185                 190

Lys Asn Cys Gln Phe Glu Thr Ala Gln Lys Lys Cys Gln Glu Ala Ile
        195                 200                 205

Asn Ala Thr Lys Gly Val Ser Tyr Cys Thr Gly Asn Ser Ser Glu Cys
    210                 215                 220

Pro Pro Pro Gly Asn Ala Glu Asp Asp Thr
225                 230

<210> 135
<211> 234
<212> PRT
<213> Artificial Sequence

<220>
<223> Description of Artificial Sequence: Synthetic
      polypeptide

<400> 135
Met Ser Asp Lys Ile Ile His Leu Thr Asp Asp Ser Phe Asp Thr Asp
1               5                   10                  15

Val Leu Lys Ala Asp Gly Ala Ile Leu Val Asp Phe Trp Ala Glu Trp
            20                  25                  30

Cys Gly Pro Cys Lys Met Ile Ala Pro Ile Leu Asp Glu Ile Ala Asp
        35                  40                  45

Glu Tyr Gln Gly Lys Leu Thr Val Ala Lys Leu Asn Ile Asp Gln Asn
    50                  55                  60

Pro Gly Thr Ala Pro Lys Tyr Gly Ile Arg Gly Ile Pro Thr Leu Leu
65                  70                  75                  80

Leu Phe Lys Asn Gly Glu Val Ala Ala Thr Lys Val Gly Ala Leu Ser
                85                  90                  95

Lys Gly Gln Leu Lys Glu Phe Leu Asp Ala Asn Leu Ala Gly Ser Gly

            100                 105                 110

Ser Gly His Met His His His His His His Ser Ser Gly Leu Val Pro
        115                 120                 125

Arg Gly Ser Gly Met Lys Glu Thr Ala Ala Ala Lys Phe Glu Arg Gln
    130                 135                 140

His Met Asp Ser Pro Asp Leu Glu Asn Leu Tyr Phe Gln Gly Glu Glu
145                 150                 155                 160

Cys Asp Cys Gly Asn Lys Asn Glu Lys Lys Cys Cys Asp Tyr Asn Thr
                165                 170                 175

Cys Lys Leu Lys Gly Ser Val Lys Cys Gly Ser Gly Pro Cys Cys Thr
            180                 185                 190

Ser Lys Cys Glu Leu Ser Ile Ala Gly Thr Pro Cys Arg Lys Ser Ile
        195                 200                 205

Asp Pro Glu Asp Phe Thr Glu Tyr Cys Asn Gly Thr Ser Ser Asn Cys
    210                 215                 220

Val Pro Asp Thr Tyr Ala Leu Asn Gly Arg
225                 230

<210> 136
<211> 231
<212> PRT
<213> Artificial Sequence

<220>
<223> Description of Artificial Sequence: Synthetic
polypeptide

<400> 136
   Met Ser Asp Lys Ile Ile His Leu Thr Asp Asp Ser Phe Asp Thr Asp
   1 5 10 15
   Val Leu Lys Ala Asp Gly Ala Ile Leu Val Asp Phe Trp Ala Glu Trp
   20 25 30
   Cys Gly Pro Cys Lys Met Ile Ala Pro Ile Leu Asp Glu Ile Ala Asp
   35 40 45
   Glu Tyr Gln Gly Lys Leu Thr Val Ala Lys Leu Asn Ile Asp Gln Asn
   50 55 60
   Pro Gly Thr Ala Pro Lys Tyr Gly Ile Arg Gly Ile Pro Thr Leu Leu
   65 70 75 80
   Leu Phe Lys Asn Gly Glu Val Ala Ala Thr Lys Val Gly Ala Leu Ser
                   85                  90                  95
   Lys Gly Gln Leu Lys Glu Phe Leu Asp Ala Asn Leu Ala Gly Ser Gly
               100                 105                 110
   Ser Gly His Met His His His His His His Ser Ser Gly Leu Val Pro
           115                 120                 125
   Arg Gly Ser Gly Met Lys Glu Thr Ala Ala Ala Lys Phe Glu Arg Gln
       130                 135                 140
   His Met Asp Ser Pro Asp Leu Glu Asn Leu Tyr Phe Gln Gly Glu Glu
   145                 150                 155                 160
   Cys Asp Cys Gly Glu Glu Glu Glu Pro Cys Cys Asn Ala Ser Asn Cys
                   165                 170                 175
   Thr Leu Arg Pro Gly Ala Glu Cys Ala His Gly Ser Cys Cys His Gln
               180                 185                 190
   Cys Lys Leu Leu Ala Pro Gly Thr Leu Cys Arg Glu Gln Ala Arg Gln
           195                 200                 205
   Asp Leu Pro Glu Phe Cys Thr Gly Lys Ser Pro His Cys Pro Thr Asn
       210                 215                 220
   Phe Tyr Gln Met Asp Gly Thr
   225                 230

<210> 137
<211> 231
<212> PRT
<213> Artificial Sequence

<220>
<223> Description of Artificial Sequence: Synthetic
      polypeptide

<400> 137
Met Ser Asp Lys Ile Ile His Leu Thr Asp Asp Ser Phe Asp Thr Asp
1               5                   10                  15

Val Leu Lys Ala Asp Gly Ala Ile Leu Val Asp Phe Trp Ala Glu Trp
            20                  25                  30

Cys Gly Pro Cys Lys Met Ile Ala Pro Ile Leu Asp Glu Ile Ala Asp
        35                  40                  45

Glu Tyr Gln Gly Lys Leu Thr Val Ala Lys Leu Asn Ile Asp Gln Asn
    50                  55                  60

Pro Gly Thr Ala Pro Lys Tyr Gly Ile Arg Gly Ile Pro Thr Leu Leu
65                  70                  75                  80

Leu Phe Lys Asn Gly Glu Val Ala Ala Thr Lys Val Gly Ala Leu Ser
                85                  90                  95

Lys Gly Gln Leu Lys Glu Phe Leu Asp Ala Asn Leu Ala Gly Ser Gly
            100                 105                 110

Ser Gly His Met His His His His His His Ser Ser Gly Leu Val Pro
        115                 120                 125

Arg Gly Ser Gly Met Lys Glu Thr Ala Ala Ala Lys Phe Glu Arg Gln
    130                 135                 140

His Met Asp Ser Pro Asp Leu Glu Asn Leu Tyr Phe Gln Gly Glu Glu
145                 150                 155                 160

Cys Asp Cys Gly Thr Ile Arg Gln Asp Pro Cys Cys Leu Leu Asn Cys
                165                 170                 175

Thr Leu His Pro Gly Ala Ala Cys Ala Phe Gly Ile Cys Cys Lys Asp
            180                 185                 190

Cys Lys Phe Leu Pro Ser Gly Thr Leu Cys Arg Gln Gln Val Gly Glu
        195                 200                 205

Asp Leu Pro Glu Trp Cys Asn Gly Thr Ser His Gln Cys Pro Asp Asp
    210                 215                 220

Val Tyr Val Gln Asp Gly Ile
225                 230

<210> 138
<211> 231
<212> PRT
<213> Artificial Sequence

<220>
<223> Description of Artificial Sequence: Synthetic
      polypeptide

<400> 138
Met Ser Asp Lys Ile Ile His Leu Thr Asp Asp Ser Phe Asp Thr Asp
1               5                   10                  15

Val Leu Lys Ala Asp Gly Ala Ile Leu Val Asp Phe Trp Ala Glu Trp
            20                  25                  30

Cys Gly Pro Cys Lys Met Ile Ala Pro Ile Leu Asp Glu Ile Ala Asp
        35                  40                  45

Glu Tyr Gln Gly Lys Leu Thr Val Ala Lys Leu Asn Ile Asp Gln Asn
    50                  55                  60

Pro Gly Thr Ala Pro Lys Tyr Gly Ile Arg Gly Ile Pro Thr Leu Leu
65                  70                  75                  80

Leu Phe Lys Asn Gly Glu Val Ala Ala Thr Lys Val Gly Ala Leu Ser
                85                  90                  95

Lys Gly Gln Leu Lys Glu Phe Leu Asp Ala Asn Leu Ala Gly Ser Gly
            100                 105                 110

Ser Gly His Met His His His His His His Ser Ser Gly Leu Val Pro
        115                 120                 125

Arg Gly Ser Gly Met Lys Glu Thr Ala Ala Ala Lys Phe Glu Arg Gln
    130                 135                 140

His Met Asp Ser Pro Asp Leu Glu Asn Leu Tyr Phe Gln Gly Glu Gln
145                 150                 155                 160

Cys Asp Cys Gly Ser Val Gln Gln Asp Ala Cys Cys Leu Leu Asn Cys
                165                 170                 175

Thr Leu Arg Pro Gly Ala Ala Cys Ala Phe Gly Leu Cys Cys Lys Asp
            180                 185                 190

Cys Lys Phe Met Pro Ser Gly Glu Leu Cys Arg Gln Glu Val Asn Glu
        195                 200                 205

Asp Leu Pro Glu Trp Cys Asn Gly Thr Ser His Gln Cys Pro Glu Asp
    210                 215                 220

Arg Tyr Val Gln Asp Gly Ile
225                 230

<210> 139
<211> 232
<212> PRT
<213> Artificial Sequence

<220>
<223> Description of Artificial Sequence: Synthetic

polypeptide

<400> 139
Met Ser Asp Lys Ile Ile His Leu Thr Asp Asp Ser Phe Asp Thr Asp
1               5                   10                  15

Val Leu Lys Ala Asp Gly Ala Ile Leu Val Asp Phe Trp Ala Glu Trp
            20                  25                  30

Cys Gly Pro Cys Lys Met Ile Ala Pro Ile Leu Asp Glu Ile Ala Asp
        35                  40                  45

Glu Tyr Gln Gly Lys Leu Thr Val Ala Lys Leu Asn Ile Asp Gln Asn
    50                  55                  60

Pro Gly Thr Ala Pro Lys Tyr Gly Ile Arg Gly Ile Pro Thr Leu Leu
65                  70                  75                  80

Leu Phe Lys Asn Gly Glu Val Ala Ala Thr Lys Val Gly Ala Leu Ser
                85                  90                  95

Lys Gly Gln Leu Lys Glu Phe Leu Asp Ala Asn Leu Ala Gly Ser Gly
            100                 105                 110

Ser Gly His Met His His His His His His Ser Ser Gly Leu Val Pro
        115                 120                 125

Arg Gly Ser Gly Met Lys Glu Thr Ala Ala Ala Lys Phe Glu Arg Gln
    130                 135                 140

His Met Asp Ser Pro Asp Leu Glu Asn Leu Tyr Phe Gln Gly Glu Glu
145                 150                 155                 160

Cys Asp Cys Gly Thr Pro Ala Glu Glu Gly Ala Glu Cys Cys Lys Lys
                165                 170                 175

Cys Thr Leu Thr Gln Asp Ser Gln Cys Ser Asp Gly Leu Cys Cys Lys
            180                 185                 190

Lys Cys Lys Phe Gln Pro Met Gly Thr Val Cys Arg Glu Ala Val Asn
        195                 200                 205

Asp Asp Ile Arg Glu Thr Cys Ser Gly Asn Ser Ser Gln Cys Ala Pro
    210                 215                 220

Asn Ile His Lys Met Asp Gly Tyr
225                 230

<210> 140
<211> 231
<212> PRT
<213> Artificial Sequence

<220>
<223> Description of Artificial Sequence: Synthetic
      polypeptide

<400> 140
Met Ser Asp Lys Ile Ile His Leu Thr Asp Asp Ser Phe Asp Thr Asp
1               5                   10                  15

Val Leu Lys Ala Asp Gly Ala Ile Leu Val Asp Phe Trp Ala Glu Trp
            20                  25                  30

Cys Gly Pro Cys Lys Met Ile Ala Pro Ile Leu Asp Glu Ile Ala Asp
        35                  40                  45

Glu Tyr Gln Gly Lys Leu Thr Val Ala Lys Leu Asn Ile Asp Gln Asn
    50                  55                  60

Pro Gly Thr Ala Pro Lys Tyr Gly Ile Arg Gly Ile Pro Thr Leu Leu
65                  70                  75                  80

Leu Phe Lys Asn Gly Glu Val Ala Ala Thr Lys Val Gly Ala Leu Ser
                85                  90                  95

Lys Gly Gln Leu Lys Glu Phe Leu Asp Ala Asn Leu Ala Gly Ser Gly
            100                 105                 110

Ser Gly His Met His His His His His His Ser Ser Gly Leu Val Pro
        115                 120                 125

Arg Gly Ser Gly Met Lys Glu Thr Ala Ala Ala Lys Phe Glu Arg Gln
    130                 135                 140

His Met Asp Ser Pro Asp Leu Glu Asn Leu Tyr Phe Gln Gly Glu Glu
145                 150                 155                 160

Cys Asp Cys Gly Phe His Val Glu Leu Cys Cys Lys Lys Cys Ser Leu
                165                 170                 175

Ser Asn Gly Ala His Cys Ser Asp Gly Pro Cys Cys Asn Asn Thr Ser
            180                 185                 190

Cys Leu Phe Gln Pro Arg Gly Tyr Glu Cys Arg Asp Ala Val Asn Glu
        195                 200                 205

Asp Ile Thr Glu Tyr Cys Thr Gly Asp Ser Gly Gln Cys Pro Pro Asn

210                 215                 220

Leu His Lys Gln Asp Gly Tyr
225                 230

<210> 141
<211> 231
<212> PRT
<213> Artificial Sequence

<220>
<223> Description of Artificial Sequence: Synthetic
      polypeptide

<400> 141
Met Ser Asp Lys Ile Ile His Leu Thr Asp Asp Ser Phe Asp Thr Asp
1               5                   10                  15

Val Leu Lys Ala Asp Gly Ala Ile Leu Val Asp Phe Trp Ala Glu Trp
            20                  25                  30

Cys Gly Pro Cys Lys Met Ile Ala Pro Ile Leu Asp Glu Ile Ala Asp
        35                  40                  45

Glu Tyr Gln Gly Lys Leu Thr Val Ala Lys Leu Asn Ile Asp Gln Asn
    50                  55                  60

Pro Gly Thr Ala Pro Lys Tyr Gly Ile Arg Gly Ile Pro Thr Leu Leu
65                  70                  75                  80

Leu Phe Lys Asn Gly Glu Val Ala Ala Thr Lys Val Gly Ala Leu Ser
                85                  90                  95

Lys Gly Gln Leu Lys Glu Phe Leu Asp Ala Asn Leu Ala Gly Ser Gly
            100                 105                 110

Ser Gly His Met His His His His His His Ser Ser Gly Leu Val Pro
        115                 120                 125

Arg Gly Ser Gly Met Lys Glu Thr Ala Ala Ala Lys Phe Glu Arg Gln
    130                 135                 140

His Met Asp Ser Pro Asp Leu Glu Asn Leu Tyr Phe Gln Gly Glu Asp
145                 150                 155                 160

Cys Asp Cys Gly Thr Ser Glu Glu Ile Cys Cys Asp Ala Lys Thr Cys
                165                 170                 175

Lys Ile Lys Ala Thr Phe Gln Cys Ala Leu Gly Glu Cys Cys Glu Lys
            180                 185                 190

Cys Gln Phe Lys Lys Ala Gly Met Val Cys Arg Pro Ala Lys Asp Glu
        195                 200                 205

Asp Leu Pro Glu Met Cys Asn Gly Lys Ser Gly Asn Cys Pro Asp Asp
    210                 215                 220

Arg Phe Gln Val Asn Gly Phe
225                 230

<210> 142
<211> 241
<212> PRT
<213> Artificial Sequence

<220>
<223> Description of Artificial Sequence: Synthetic
      polypeptide

<400> 142
Met Ser Asp Lys Ile Ile His Leu Thr Asp Asp Ser Phe Asp Thr Asp
1               5                   10                  15

Val Leu Lys Ala Asp Gly Ala Ile Leu Val Asp Phe Trp Ala Glu Trp
            20                  25                  30

Cys Gly Pro Cys Lys Met Ile Ala Pro Ile Leu Asp Glu Ile Ala Asp
        35                  40                  45

Glu Tyr Gln Gly Lys Leu Thr Val Ala Lys Leu Asn Ile Asp Gln Asn
    50                  55                  60

Pro Gly Thr Ala Pro Lys Tyr Gly Ile Arg Gly Ile Pro Thr Leu Leu
65                  70                  75                  80

Leu Phe Lys Asn Gly Glu Val Ala Ala Thr Lys Val Gly Ala Leu Ser
                85                  90                  95

Lys Gly Gln Leu Lys Glu Phe Leu Asp Ala Asn Leu Ala Gly Ser Gly
            100                 105                 110

Ser Gly His Met His His His His His His Ser Ser Gly Leu Val Pro
        115                 120                 125

Arg Gly Ser Gly Met Lys Glu Thr Ala Ala Ala Lys Phe Glu Arg Gln
    130                 135                 140

His Met Asp Ser Pro Asp Leu Gly Thr Asp Asp Asp Asp Lys Ala Met
145                 150                 155                 160

Ala Glu Asn Leu Tyr Phe Gln Gly Glu Glu Cys Asp Cys Gly Pro Leu
                165                 170                 175

Lys His Asp Pro Cys Cys Leu Ser Asn Cys Thr Leu Thr Asp Gly Ser
            180                 185                 190

Thr Cys Ala Phe Gly Leu Cys Cys Lys Asp Cys Lys Phe Leu Pro Ser
        195                 200                 205

Gly Lys Val Cys Arg Lys Glu Val Asn Glu Asp Leu Pro Glu Trp Cys
    210                 215                 220

Asn Gly Thr Ser His Lys Cys Pro Asp Asp Phe Tyr Val Glu Asp Gly
225                 230                 235                 240

Ile

<210> 143
<211> 231
<212> PRT
<213> Artificial Sequence

<220>
<223> Description of Artificial Sequence: Synthetic
      polypeptide

<400> 143
Met Ser Asp Lys Ile Ile His Leu Thr Asp Asp Ser Phe Asp Thr Asp
1               5                   10                  15

Val Leu Lys Ala Asp Gly Ala Ile Leu Val Asp Phe Trp Ala Glu Trp
            20                  25                  30

Cys Gly Pro Cys Lys Met Ile Ala Pro Ile Leu Asp Glu Ile Ala Asp
        35                  40                  45

Glu Tyr Gln Gly Lys Leu Thr Val Ala Lys Leu Asn Ile Asp Gln Asn
    50                  55                  60

Pro Gly Thr Ala Pro Lys Tyr Gly Ile Arg Gly Ile Pro Thr Leu Leu
65                  70                  75                  80

Leu Phe Lys Asn Gly Glu Val Ala Ala Thr Lys Val Gly Ala Leu Ser
                85                  90                  95

Lys Gly Gln Leu Lys Glu Phe Leu Asp Ala Asn Leu Ala Gly Ser Gly
            100                 105                 110

Ser Gly His Met His His His His His His Ser Ser Gly Leu Val Pro
        115                 120                 125

Arg Gly Ser Gly Met Lys Glu Thr Ala Ala Ala Lys Phe Glu Arg Gln
    130                 135                 140

His Met Asp Ser Pro Asp Leu Glu Asn Leu Tyr Phe Gln Gly Glu Glu
145                 150                 155                 160

Cys Asp Cys Gly Ser Thr Glu Glu Asp Arg Cys Cys Gln Ser Asn Cys
                165                 170                 175

Lys Leu Gln Pro Gly Ala Asn Cys Ser Ile Gly Leu Cys Cys His Asp
            180                 185                 190

Cys Arg Phe Arg Pro Ser Gly Tyr Val Cys Arg Gln Glu Gly Asn Glu
        195                 200                 205

Asp Leu Ala Glu Tyr Cys Asp Gly Asn Ser Ser Ser Cys Pro Asn Asp
    210                 215                 220

Val Tyr Lys Gln Asp Gly Thr
225                 230

<210> 144
<211> 233
<212> PRT
<213> Artificial Sequence

<220>
<223> Description of Artificial Sequence: Synthetic
      polypeptide

<400> 144
Met Ser Asp Lys Ile Ile His Leu Thr Asp Asp Ser Phe Asp Thr Asp
1               5                   10                  15

Val Leu Lys Ala Asp Gly Ala Ile Leu Val Asp Phe Trp Ala Glu Trp
            20                  25                  30

Cys Gly Pro Cys Lys Met Ile Ala Pro Ile Leu Asp Glu Ile Ala Asp
        35                  40                  45

Glu Tyr Gln Gly Lys Leu Thr Val Ala Lys Leu Asn Ile Asp Gln Asn
    50                  55                  60

Pro Gly Thr Ala Pro Lys Tyr Gly Ile Arg Gly Ile Pro Thr Leu Leu
65                  70                  75                  80

Leu Phe Lys Asn Gly Glu Val Ala Ala Thr Lys Val Gly Ala Leu Ser

85                  90                  95

Lys Gly Gln Leu Lys Glu Phe Leu Asp Ala Asn Leu Ala Gly Ser Gly
            100                 105                 110

Ser Gly His Met His His His His His His Ser Ser Gly Leu Val Pro
        115                 120                 125

Arg Gly Ser Gly Met Lys Glu Thr Ala Ala Ala Lys Phe Glu Arg Gln
    130                 135                 140

His Met Asp Ser Pro Asp Leu Glu Asn Leu Tyr Phe Gln Gly Glu Ile
145                 150                 155                 160

Cys Asp Cys Gly Thr Glu Ala Gln Ala Ser Cys Cys Asp Phe Arg Thr
                165                 170                 175

Cys Val Leu Lys Asp Gly Ala Lys Cys Tyr Lys Gly Leu Cys Cys Lys
            180                 185                 190

Asp Cys Gln Ile Leu Gln Ser Gly Val Glu Cys Arg Pro Lys Ala His
        195                 200                 205

Pro Glu Asp Ile Ala Glu Asn Cys Asn Gly Ser Ser Pro Glu Cys Gly
    210                 215                 220

Pro Asp Ile Thr Leu Ile Asn Gly Leu
225                 230

<210> 145
<211> 231
<212> PRT
<213> Artificial Sequence

<220>
<223> Description of Artificial Sequence: Synthetic
      polypeptide

<400> 145
Met Ser Asp Lys Ile Ile His Leu Thr Asp Asp Ser Phe Asp Thr Asp
1               5                   10                  15

Val Leu Lys Ala Asp Gly Ala Ile Leu Val Asp Phe Trp Ala Glu Trp
            20                  25                  30

Cys Gly Pro Cys Lys Met Ile Ala Pro Ile Leu Asp Glu Ile Ala Asp
        35                  40                  45

Glu Tyr Gln Gly Lys Leu Thr Val Ala Lys Leu Asn Ile Asp Gln Asn
    50                  55                  60

Pro Gly Thr Ala Pro Lys Tyr Gly Ile Arg Gly Ile Pro Thr Leu Leu
65                  70                  75                  80

Leu Phe Lys Asn Gly Glu Val Ala Ala Thr Lys Val Gly Ala Leu Ser
                85                  90                  95

Lys Gly Gln Leu Lys Glu Phe Leu Asp Ala Asn Leu Ala Gly Ser Gly
            100                 105                 110

Ser Gly His Met His His His His His His Ser Ser Gly Leu Val Pro
        115                 120                 125

Arg Gly Ser Gly Met Lys Glu Thr Ala Ala Ala Lys Phe Glu Arg Gln
    130                 135                 140

His Met Asp Ser Pro Asp Leu Glu Asn Leu Tyr Phe Gln Gly Glu Glu
145                 150                 155                 160

Cys Asp Cys Gly Pro Gly Gln Glu Leu Cys Cys Phe Ala His Asn Cys
                165                 170                 175

Ser Leu Arg Pro Gly Ala Gln Cys Ala His Gly Asp Cys Cys Val Arg
            180                 185                 190

Cys Leu Leu Lys Pro Ala Gly Ala Leu Cys Arg Gln Ala Met Gly Asp
        195                 200                 205

Asp Leu Pro Glu Phe Cys Thr Gly Thr Ser Ser His Cys Pro Pro Asp
    210                 215                 220

Val Tyr Leu Leu Asp Gly Ser
225                 230

<210> 146
<211> 2840
<212> DNA
<213> Homo sapiens

<400> 146
   aaactgccga aggccaggag agaagcagga agagaaacac ggcttgaagt tctgtaagaa      60
   tcagagttgg gcaagaactg ccaataaggg agccagcgta gatctgagta ctaaacacct     120
   gagcctccga gggtttgtgt gcgagggaga gctctgctga tggacatagg cccagaatca     180
   ttaactgatt tatttgggcc atgtcactgg tggcattatt aaaagactct gccaacatcc     240
   tgctttatgg aaaaaccaag tggccttgga agaggctaag ataaagtttc aaacttgggc     300
   tccacagaag tggaacttaa ggctggggct agtaccagga ccttcatgta tcaggttaga     360
   gattttgatg cttttggtga tttttgtgcc aagcatgtac tgtcacctgg gatcaatcta 420
   ttactctttc tatgaaataa ttattccaaa gaggctgaca gtccagggag gagatagccc 480
   agtggaagga ctgtcctact tgttacttat gcaaggccag aagcacctgg ttcatctgaa 540
   ggtgaagaga aaccattttg tgaataactt tccagtctac agttaccaca atggcctcct 600
   ggggcaagaa tcgcctttca tctcacatga ctgccactat gaaggctaca tagaaggagt 660
   gtcaggttct tttgtttctg tcaacatctg tgcaggtctc aggggcacat cctcctgatt 720
   aaggaggaaa aatcttacag cattgagccc atggactctt caagacggtt tgaacatgtg 780
   ttatacacca tggcacatca agcgtgagtc tcctgtggtg tctactagct ggcaacaagg 840
   gagcaggaag cctcatgatc tacaggcact gtcctacttg tggtcacaca aaaagtacgt 900
   ggagatgttt gtcgtggtca acaaccagcg gttccagatg tggggcagta acgtcaatga 960
   gacggtccag acagtagtgg atgtcattgc tctggccaac agcttcacta ggggaataaa 1020
   cacagaggtg gtgctggctg gaatggagat ttggaccgag ggggacctaa tagatgtcac 1080
   agtggacttg caaatcacac tcaggaattt caatcactgg agacaagaga tgctcttcca 1140
   tcgtgcaaaa cacgatgttg cccacatgat cgttgggcat caccctggac agaatatggg 1200
   ccaggccttt ctcagtggtg cctgctcaag cggttttgcg gcagctgttg aatccttcca 1260
   tcatgaagat gtgctgttgt ttgcagccct gatggcccat gagctcgggc acaacctggg 1320
   tattcagcac gaccactcgg cctgcttttg taaagataag cacttttgcc tcatgcatga 1380
   aaatatcaca aaagaaagtg gcttcagcag ctgcagctct gactacttct accagttcct 1440
   tcgagaacac aaagggccct gcctatttaa caagccacgg cccaggggcc gcaagcgtag 1500
   ggattctgcc tgtggaaatg gtgtggtgga ggacacggag cagtgtgact gtggttctct 1560
   atgtcagcat catgcatgct gtgatgaaaa ctgtatactg aaggcgaaag cagagtgcag 1620
   tgatggtcca tgttgtcata agtgtaaatt tcaccgtaag ggatatcctt gctgtccttc 1680
   tagtcgttcc tgtgatctcc cagaattttg caatggtaca tctgcattat gccccaacaa 1740
   caggcataag caagatggct caaaatgtca tacaatttac gagtgcctta aagttcattg 1800
   tatggaccct aataatcagt gcttacaatt atatggatat ggtgcaaaat cagcctcaca 1860
   agagtgttac aattcaatga acagcaaagg ggaccaattt ggaaactgtg gcatttctac 1920
   cagtcctggg tcacaatatg ttcggtgttc agatggtaat atattttgtg ggaaacttat 1980
   atgttcaggt attacaggct taccaaaaat caatctccaa catacaatga ttcaggtccc 2040
   tcagggagat ggctcatgtt ggagcatgga tgcctatatg agtactgaca ttcctgatga 2100
   aggagatgtg cacaatggca cttactgtgc accaaacaaa gtctgcctga attccgcctg 2160
   cacagataaa accccagtga tttctgcctg caacccagaa aaaacgtgta atgggaaggg 2220
   agtttgtaat gatttagggc actgccactg taatgaaggg catgcccccc ctgactgtgt 2280
   tactgcagga agtggaggta gtgtggacag tggccctcct ggtaagctag gtgggacacc 2340
   ttcaggagaa ggtgaaaatc acaatatgac tcattccaga cgtgaagaac atgctgtaga 2400
   catgatgata ttatcattca ttatactttt tataatatta ttattaagta caattatttg 2460
   atctgcttgc ttaaaaaatc accagaggct gccccggcag aagctcctcc agcagtggct 2520
   ccaccaccgg ccccagaaat aaagccagaa gcagcagaag tggccacaga agaaaaagaa 2580
   gagaaggagg aagaaaaaga agaggaggag gaggaagaag aggaggaaga atcagattcc 2640
   taaggttaga aatagggaga tgaagccaag tatatcaaac tcctcaagta ctgagtggga 2700
   atgagatgtt tggtgaagta gaaatagata gctttagtgg ctctgactca gatatactga 2760
   tgtagaagga aggattcttt cacttttatt atttattttg gtaattaaat ttacattaat 2820
   ttaatatgtt aaatatttta 2840

<210> 147
<211> 2657
<212> DNA
<213> Homo sapiens

<400> 147
gcgtcatctc gcgcttccaa ctgccctgta accaccaact gccattattc cggctgggac 60

ccaggacttc aagccatgtg gcgcgtcttg tttctgctca gcgggctcgg cgggctgcgg 120

atggacagta attttgatag tttacctgtg caaattacag ttccggagaa aatacggtca 180

ataataaagg aaggaattga atcgcaggca tcctacaaaa ttgtaattga agggaaacca 240

tatactgtga atttaatgca aaaaaacttt ttaccccata attttagagt ttacagttat 300

agtggcacag gaattatgaa accacttgac caagattttc agaatttctg ccactaccaa 360

gggtatattg aaggttatcc aaaatctgtg gtgatggtta gcacatgtac tggactcagg 420

ggcgtactac agtttgaaaa tgttagttat ggaatagaac ccctggagtc ttcagttggc 480

tttgaacatg taatttacca agtaaaacat aagaaagcag atgtttcctt atataatgag 540

aaggatattg aatcaagaga tctgtccttt aaattacaaa gcgtagagcc acagcaagat 600

tttgcaaagt atatagaaat gcatgttata gttgaaaaac aattgtataa tcatatgggg 660

tctgatacaa ctgttgtcgc tcaaaaagtt ttccagttga ttggattgac gaatgctatt 720

tttgtttcat ttaatattac aattattctg tcttcattgg agctttggat agatgaaaat 780

aaaattgcaa ccactggaga agctaatgag ttattacaca catttttaag atggaaaaca 840

tcttatcttg ttttacgtcc tcatgatgtg gcatttttac ttgtttacag agaaaagtca 900

aattatgttg gtgcaacctt tcaagggaag atgtgtgatg caaactatgc aggaggtgtt 960

gttctgcacc ccagaaccat aagtctggaa tcacttgcag ttattttagc tcaattattg 1020

agccttagta tggggatcac ttatgatgac attaacaaat gccagtgctc aggagctgtc 1080

tgcattatga atccagaagc aattcatttc agtggtgtga agatctttag taactgcagc 1140

ttcgaagact ttgcacattt tatttcaaag cagaagtccc agtgtcttca caatcagcct 1200

cgcttagatc cttttttcaa acagcaagca gtgtgtggta atgcaaagct ggaagcagga 1260

gaggagtgtg actgtgggac tgaacaggat tgtgccctta ttggagaaac atgctgtgat 1320

attgccacat gtagatttaa agccggttca aactgtgctg aaggaccatg ctgcgaaaac 1380

tgtctattta tgtcaaaaga aagaatgtgt aggccttcct ttgaagaatg cgacctccct 1440

gaatattgca atggatcatc tgcatcatgc ccagaaaacc actatgttca gactgggcat 1500

ccgtgtggac tgaatcaatg gatctgtata gatggagttt gtatgagtgg ggataaacaa 1560

tgtacagaca catttggcaa agaagtagag tttggccctt cagaatgtta ttctcacctt 1620

aattcaaaga ctgatgtatc tggaaactgt ggtataagtg attcaggata cacacagtgt 1680

gaagctgaca atctgcagtg cggaaaatta atatgtaaat atgtaggtaa atttttatta 1740

caaattccaa gagccactat tatttatgcc aacataagtg gacatctctg cattgctgtg 1800

gaatttgcca gtgatcatgc agacagccaa aagatgtgga taaaagatgg aacttcttgt 1860

ggttcaaata aggtttgcag gaatcaaaga tgtgtgagtt cttcatactt gggttatgat 1920

tgtactactg acaaatgcaa tgatagaggt gtatgcaata acaaaaagca ctgtcactgt 1980

agtgcttcat atttacctcc agattgctca gttcaatcag atctatggcc tggtgggagt 2040

attgacagtg gcaattttcc acctgtagct ataccagcca gactccctga aaggcgctac 2100

attgagaaca tttaccattc caaaccaatg agatggccat ttttcttatt cattcctttc 2160

tttattattt tctgtgtact gattgctata atggtgaaag ttaatttcca aaggaaaaaa 2220

tggagaactg aggactattc aagcgatgag caacctgaaa gtgagagtga acctaaaggg 2280

tagtctggac aacagagatg ccatgatatc acttcttcta gagtaattat ctgtgatgga 2340

tggacacaaa aaaatggaaa gaaaagaatg tacattacct ggtttcctgg gattcaaacc 2400

tgcatattgt gattttaatt tgaccagaaa atatgatata tatgtataat ttcacagata 2460

atttacttat ttaaaaatgc atgataatga gttttacatt acaaatttct gtttttttaa 2520

agttatctta cgctatttct gttggttagt agacactaat tctgtcagta ggggcatggt 2580

ataaggaaat atcataatgt aatgaggtgg tactatgatt aaaagccact gttacatttc 2640

aaaaaaaaaa aaaaaaa 2657

<210> 148
<211> 2642
<212> DNA
<213> Homo sapiens

<400> 148

gggatccacg gagctgggtc cccgccgcgc cccgcggacc ccacggccct tccgctccgc 60

tcggcctcca ccaccctggc ctcgagctgc agccgttcca aggacaacgg ccatccctgt 120

ggctgggccg ccggctacgc aatgctgtct ctcttgctga tcctctcagg cctgggccgg 180

ctgacctccg cgggcccacg ccacttattc catcaaaatt gaggggaaac catacacttc 240

ctgcttgaaa aacagtcatt tttacaccca catttcctgg tgtatttata caacaaatca 300

ggaacattgt atgcagattc ttcattttca aagggtcatt gcttttacca aggatatgct 360

gcagacattc caaaatcagt tgtggcacta cgcacctgtt ctggactcag aggattactg 420

cagttagata atatcagtta tggtattgaa ccattggaat cttcacctac atataagcat 480

gtagtttatc gaattaaaaa tgatgctatt ggtcattcct cctttcaaga aaattatcct 540

gtggctcaat atatagatca gtcctacagg attcttgtca aatcagacat aaattcaggt 600

gccatgctat cgaaaagaac tctgaaaata caaatcatta tggataaagc catgtatgct 660

tatatgggct ccacggtggc agttgcagtt gagaaagttt tccaaatctt tggtcttatc 720

aacactatgt tttcccagct taatatgaca gttatgctgt cttccttgga gatctggtca 780

gatcaaaata agatttcaac aagtggtcat gctgatgaaa tactacagcg atttttgcct 840

tggaaacaaa aatttttgtt tcaaaggtct catgatatga catacttatt aatttatagg 900

aaccattcta cttatgtggg agcaacatat cacggaatgg catgcgatcc aaagtttgca 960

acaggaattg ctctgtatcc aaagaaaatc actgcagagg ccttttcagt tgttatggca 1020

cagttgcttg gaattaatct gggattgaca tatgatgata tctacaattg ttactgtcca 1080

ggaactacat gcataatgaa tcctgatgcg atatgatccc atggtatgaa gttttttagc 1140

agttgcagca tggatggatt taaacggata gttctgcagc ctgaacttaa atgttttcag 1200

gataaaacag tttcaaaaat gacttaccga aaatcttcaa cttgtggcaa tggaattttg 1260

gaacctacag agcagtgtga ttgtggctat aaagagagac aggatttcac catgttggcc 1320

aggctagtct caaacttcgg acctcaggtt atccacctgc ctcagcctct caaggtgctg 1380

ggattacaaa ccatacacaa ccccattttg aaaaggagaa atagacaaga agaaatgggt 1440

aacaggcctc aagcaagtct gaaacccaag agagccaatg atggacattc agactcctga 1500

ataatttcct ttgatgacat gtcctgcatc ttctataaac aggcatgcac tcataaaaaa 1560

tgctgtaatc ctgcagattg tactctagtt agatctgcag aatgtggcac tggatcatgc 1620

tgtaacaata aaacttgtac gatccacgaa agaggccatg tctgcagaaa aagtgtagat 1680

atgtgtgatt ttccagaata ttgcaatgga acatctgagt tttgtgtacc tgatgtgaaa 1740

gctgctgatt tagaatactg cagtaataag actagctatt gctttaaagg agtatgcaga 1800

gaaaggatta gacagtgttc acagttattt ggaaaatttg ctaagtctgc taatcttctg 1860

tgtacagaag aagtgaattt tcaaaatgac aaatttggaa actgtggttc ccgttgtgat 1920

ttttttgata tcctttgtgg aaagattgtt tgtcactgga tacattcaga actagtacca 1980

atgacagact tagacataca atatacttac cttagaggtc acgtatgttt gtccgcacat 2040

gcaagaaatg gttcaaaaca atcggagacc tatacagaag atataactgc atgtggccaa 2100

cacaaggtat gtcactcccg agaatgcaga aattttagtg aactaaatat aacaaaatat 2160

actacaaatt gtggacagaa tgggatttgc aatgaacatt tccattgtca atgtgatcct 2220

ggttatgctc ctccagattg tgagccagca atgtcatcac caggaggaag tatcaatgat 2280

ggattttggc ttacagtaga aaaaagtgta cccttgcttc caaaacgacg tgctgctcct 2340

aaaaacaatg gtcttttgat cagtttctac attttttcac ctttgctcat tttaattgct 2400

attgttagtc ttaaatggaa taaaatgaag agatttcgga gcaaagtggg aacagtaagc 2460

agcagatcta tctcagaaga cagcggtagt aacagcagcc agtcacagag ttaaagtaac 2520

caagtgataa aatcaaagcc atcggatgaa gaaaggatat taccaagaaa ctcacgatta 2580

acttaaaact ttcaacttca cagaaaaaaa taaatcatat gactaaaagc ataaaaaaaa 2640

aa 2642

<210> 149
<211> 1842
<212> DNA
<213> Homo sapiens

<400> 149
   gttctctgtc agggtctccc tgggagggac gcagccaccg cagctggttg gggcctggct 60
   tcgcccagga cagtcctttc ctttcccatt gtctttggat gactatcgct gggctgggac 120
   atgaggcggg cagaggcgcg ggtcaccctt aggacccccc tcttgctgct ggggctctgg 180
   gcgctcctgg ctccggtccg gtgttctcaa ggccgtccct tgtggcacta tgcctcctcc 240
   gaggtggtga ttcccaggaa ggagacacac catagcaaag gccttcagtt tcccggctgg 300
   ctgtcctaca gcctgtgttt tggggtcaaa gacacgtcat tcacatgcgg aggaaacacc 360
   ttctttggcc tagacatctg ctggtgacaa ctcaggatga ccaaggagtc ttgcagatgg 420
   gtgaccccta catccctcca gactgctagt acctcggcta cctggaggag gtgcctctgt 480
   ccatggtcac cgtcgacacg tgctatgggg acctcagagg catcatgagg ctggacgacc 540
   ttgcgtacga aatcaaaccc ctccaggatt cccgcaggcc ggtgtgtttt tgaaacactt 600
   gctcctgtgt ataacgaaac catgacaacg gttcgctgtg gaaacctcat agtggagggg 660
   agggaggaat gtgactgtgg ctccttcaag cagtgttatg ccagttattg ctgccaaagt 720
   gactgtcact taacaccggg gagcatctgc catataggag agtgctgtac aaactgcagc 780
   ttctccccac cagggactct ctgcagacct atccaaaata tatgtgacct tccagagtac 840
   tgtcacggga ccaccgtgac atgtcccgca aacgtttata tgcaagatgg aaccccgtgc 900
   actgaagaag gctactgcta tcgtgggaac tgcactgatc gcaatgtgct ctgcaaggcg 960
   atctttggtg tcagtgctga ggatgctccc gaggtctgct atgacataaa tcttgaaagc 1020
   taccgatttg gacattgtat tagacaacaa acatatctca gctaccaggc ttgtgcagga 1080
   atagataagt tttgtggaag actgcagtgt accaatgtga cccatcttcc ccggctgcag 1140
   gaacgtgttt cattccatca ctcagtgaga ggagggtttc agtgttttgg actggatgaa 1200
   caccatgcaa cagacacgac tgatgttggg cgtgtgatag atggcactcc ttgtgttcat 1260
   ggaaacttct gtaataacgc ccagtgcaat gtgactatca cttcactggg ctacaactgc 1320
   caccctcaga agtgcggtca tagaggagtc tgcaacaaca gaaggaactg ccattgccat 1380
   ataggctggg atcctccact gtgcctaaga agaggtgctg gtgggagtgt caacagcggg 1440
   ccacctccaa aaagaacacg ttccgtcaaa caaagccagc aatcagtgat gtatctgaga 1500
   gtggtctttg gtcgtattta cgccttcata attgcactgc tctttgggac agccaaaaat 1560
   gtgcgaacta tcaggaccac caccgttaag gaagggacag ttactaaccc cgaataacac 1620
   taattcagcc tcccgatccc tgtaaagata cagagaatat aacagcaaaa tctatgaaac 1680
   aggatcaggg gaagggatgg caaagctcaa gtccacattt cttgaagtcc acaggaagca 1740
   cagggtcctg tttcacatca cagggaaacg ggaggcattg gcttctgtcc caggttcttg 1800
   taggtcgctg atgctcactc tgaaataaat cttcaaaaac ac 1842

<210> 150
<211> 2612
<212> DNA
<213> Homo sapiens

<400> 150
aaaactgccc acctatctgt gaggtgcttc atccctgcag tggaagtgag gaggaagaaa 60

ggtgaactcc ttttctcaag cacttctgct ctcctctacc agaatcactc agaatgcttc 120

ccgggtgtat attcttgatg attttactca ttcctcaggt taaagaaaag ttcatccttg 180

gagtagaggg tcaacaactg gttcgtccta aaaagcttcc tctgatacag aagcgagata 240

ctggacacac ccatgatgat gacatactga aaacgtatga agaagaattg ttgtatgaaa 300

taaaactaaa tagaaaaacc ttagtccttc atcttctaag atccagggag ttcctaggct 360

caaattacag tgaaacattc tactccatga aaggagaagc gttcaccagg catcctcaga 420

tcatggatca ttgtttttac caaggatcca tagtacacga atatgattca gctgccagta 480

tcagtacgtg taatggtcta aggggattct tcagaataaa cgaccaaaga tacctcattg 540

aaccagtgaa atactcagat gagggagaac atttggtgtt caaatataac ctgagggtgc 600

cgtatggtgc caattattcc tgtacagagc ttaattttac cagaaaaact gttccagggg 660

ataatgaatc tgaagaagac tccaaaataa aaggcatcca tgatgaaaag tatgttgaat 720

tgttcattgt tgctgatgat actgtgtatc gcagaaatgg tcatcctcac aataaactaa 780

ggaaccgaat ttggggaatg gtcaattttg tcaacatgat ttataaaacc ttaaacatcc 840

atgtgacgtt ggttggcatt gaaatatgga cacatgaaga taaaatagaa ctatattcaa 900

atatagaaac taccttattg cgtttttcat tttggcaaga aaagatcctt aaaacacgga 960

aggattttga tcatgttgta ttactcagtg ggaagtggct ctactcacat gtgcaaggaa 1020

tttcttatcc agggggtatg tgcctgccct attattccac cagtatcatt aaggatcttt 1080

tacctgacac aaacataatt gcaaacagaa tggcacatca actggggcat aaccttggga 1140

tgcagcatga cgagttccca tgcacctgtc cttcaggaaa atgcgtgatg gacagtgatg 1200

gaagcattcc tgcactgaaa ttcagtaaat gcagccaaaa ccaataccac cagtacttga 1260

aggattataa gccaacatgc atgctcaaca ttccatttcc ttacaatttt catgatttcc 1320

aattttgtgg aaacaagaag ttggatgagg gtgaagagtg tgactgtggc cctgctcagg 1380

agtgtactaa tccttgctgt gatgcacaca catgtgtact gaagccagga tttacttgtg 1440

cagaaggaga atgctgtgaa tcttgtcaga taaaaaaagc agggtccata tgcagaccgg 1500

cgaaagatga atgtgatttt cctgagatgt gcactggcca ctcgcctgcc tgtcctaagg 1560

accagttcag ggtcaatgga tttccttgca agaactcaga aggctactgt ttcatgggga 1620

aatgtccaac tcgtgaggat cagtgctctg aactatttga tgatgaggca atagagagtc 1680

atgatatctg ctacaagatg aatacaaaag gaaataaatt tggatactgc aaaaacaagg 1740

aaaacagatt tcttccctgt gaggagaaag atgtcagatg tggaaagatc tactgcactg 1800

gaggggagct ttcctctctc cttggagaag acaagactta tcaccttaag gatccccaga 1860

agaatgctac tgtcaaatgc aaaactattt ttttatacca tgattctaca gacattggcc 1920

tggtggcgtc aggaacaaaa tgtggagagg gaatggtgtg caacaatggt gaatgtctaa 1980

acatggaaaa ggtctatatc tcaaccaatt gcccctctca gtgcaatgaa aatcctgtgg 2040

atggccacgg actccagtgc cactgtgagg aaggacaggc acctgtagcc tgtgaagaaa 2100

ccttacatgt taccaatatc accatcttgg ttgttgtgct tgtcctggtt attgtcggta 2160

tcggagttct tatactatta gttcgttacc gaaaatgtat caagttgaag caagttcaga 2220

gcccacctac agaaaccctg ggagtggaga acaaaggata ctttggtgat gagcagcaga 2280

taaggactga gccaatcctg ccagaaattc atttcctaaa taaacctgca agtaaagatt 2340

caagaggaat cgcagatccc aatcaaagtg ccaagtgagc ttgaagttgg atatccaaaa 2400

tggccgtgca agcttaggct ggggattctg gatgcaacgt ctttacaacc ttacctagat 2460

atctgctact cacattttgg gtagtgtttc aaacgttctt tatccagaca gacaatgttt 2520

aagagaaaca acttatttct gttaatattt accggtagaa ttcacaccct ctatcataaa 2580

catatgctgc agaaaaaaaa aaaaaaaaaa aa 2612

<210> 151
<211> 3316
<212> DNA
<213> Homo sapiens

<400> 151
gcggaaaaga gcctcgggcc aggagcgcag gaaccagacc gtgtcccgcg gggctgtcac 60

ctccgcctct gctccccgac ccggccatgc gcggcctcgg gctctggctg ctgggcgcga 120

tgatgctgcc tgcgattgcc cccagccggc cctgggccct catggagcag tatgaggtcg 180

tgttgccgtg gcgtctgcca ggcccccgag tccgccgagc tctgccctcc cacttgggcc 240

tgcacccaga gagggtgagc tacgtccttg gggccacagg gcacaacttc accctccacc 300

tgcggaagaa cagggacctg ctgggctccg gctacacaga gacctatacg gctgccaatg 360

gctccgaggt gacggagcag cctcgcgggc aggaccactg cttctaccag ggccacgtag 420

aggggtaccc ggactcagcc gccagcctca gcacctgtgc cggcctcagg ggtttcttcc 480

aggtggggtc agacctgcac ctgatcgagc ccctggatga aggtggcgag ggcggacggc 540

acgccgtgta ccaggctgag cacctgctgc agacggccgg gacctgcggg gtcagcgacg 600

acagcctggg cagcctcctg ggaccccgga cggcagccgt cttcaggcct cggcccgggg 660

actctctgcc atcccgagag acccgctacg tggagctgta tgtggtcgtg gacaatgcag 720

agttccagat gctggggagc gaagcagccg tgcgtcatcg ggtgctggag gtggtgaatc 780

acgtggacaa gctatatcag aaactcaact tccgtgtggt cctggtgggc ctggagattt 840

ggaatagtca ggacaggttc cacgtcagcc ccgaccccag tgtcacactg gagaacctcc 900

tgacctggca ggcacggcaa cggacacggc ggcacctgca tgacaacgta cagctcatca 960

cgggtgtcga cttcaccggg actaccgtgg ggtttgccag ggtgtccgcc atgtgctccc 1020

acagctcagg ggctgtgaac caggaccaca gcaagaaccc cgtgggcgtg gcctgtacca 1080

tggcccatga gatgggccac aacctgggca tggaccatga tgagaacgtc cagggctgcc 1140

gctgccagga acgcttcgag gccggccgct gcatcatggc gggcagcatt ggctccagtt 1200

tccccaggat gttcagtgac tgcagccagg cctacctgga gagctttttg gagcggccgc 1260

agtcggtgtg cctcgccaac gcccctgacc tcagccacct ggtgggcggc cccgtgtgtg 1320

ggaacctgtt tgtggagcgt ggggagcagt gcgactgcgg cccccccgag gactgccgga 1380

accgctgctg caactctacc acctgccagc tggctgaggg ggcccagtgt gcgcacggta 1440

cctgctgcca ggagtgcaag gtgaagccgg ctggtgagct gtgccgtccc aagaaggaca 1500

tgtgtgacct cgaggagttc tgtgacggcc ggcaccctga gtgcccggaa gacgccttcc 1560

aggagaacgg cacgccctgc tccgggggct actgctacaa cggggcctgt cccacactgg 1620

cccagcagtg ccaggccttc tgggggccag gtgggcaggc tgccgaggag tcctgcttct 1680

cctatgacat cctaccaggc tgcaaggcca gccggtacag ggctgacatg tgtggcgttc 1740

tgcagtgcaa gggtgggcag cagcccctgg ggcgtgccat ctgcatcgtg gatgtgtgcc 1800

acgcgctcac cacagaggat ggcactgcgt atgaaccagt gcccgagggc acccggtgtg 1860

gaccagagaa ggtttgctgg aaaggacgtt gccaggactt acacgtttac agatccagca 1920

actgctctgc ccagtgccac aaccatgggg tgtgcaacca caagcaggag tgccactgcc 1980

acgcgggctg ggccccgccc cactgcgcga agctgctgac tgaggtgcac gcagcgtccg 2040

ggagcctccc cgtcttcgtg gtggtggttc tggtgctcct ggcagttgtg ctggtcaccc 2100

tggcaggcat catcgtctac cgcaaagccc ggagccgcat cctgagcagg aacgtggctc 2160

ccaagaccac aatggggcgc tccaaccccc tgttccacca ggctgccagc cgcgtgccgg 2220

ccaagggcgg ggctccagcc ccatccaggg gcccccaaga gctggtcccc accacccacc 2280

cgggccagcc cgcccgacac ccggcctcct cggtggctct gaagaggccg ccccctgctc 2340

ctccggtcac tgtgtccagc ccacccttcc cagttcctgt ctacacccgg caggcaccaa 2400

agcaggtcat caagccaacg ttcgcacccc cagtgccccc agtcaaaccc ggggctggtg 2460

cggccaaccc tggtccagct gagggtgctg ttggcccaaa ggttgccctg aagcccccca 2520

tccagaggaa gcaaggagcc ggagctccca cagcacccta ggggggcacc tgcgcctgtg 2580

tggaaatttg gagaagttgc ggcagagaag ccatgcgttc cagcattcca cggtccagct 2640

agtgccgctc agccctagac cctgactttg caggctcagc tgctgttcta acctcaggaa 2700

tgcatctacc tgagaggctc ctgctgtcca cgccctcagc caattccttc tccccgcctt 2760

ggccacgtgt agccccagct gtctgcaggc accaggctgg gatgagctgt gtgcttgcgg 2820

gtgcgtgtgt gtgtacgtgt ctccaggtgg ccgctggtct cccgctgtgt tcaggaggcc 2880

acatatacag cccctcccag ccacacctgc ccctgctctg gggcctgctg agccggctgc 2940

cctgggcacc cggttccagg cagcacagac gtggggcatc cccagaaaga ctccatccca 3000

ggaccaggtt cccctgcgtg ctcttcgaga gggtgtcagt gagcagactg caccccaagc 3060

tcccgactcc aggtcccctg atcttggggc ctgtttccca tgggattcaa gagggacagc 3120

cccagctttg tgtgtgttta agcttaggaa tcgcctttat ggaaagggct atgtgggaga 3180

gtcagctatc ttgtctggtt ttcttgagac ctcagatgtg tgttcagcag ggctgaaagc 3240

ttttattctt taataatgag aaatgtatat tttactaata aattattgac cgagttctgt 3300

aaaaaaaaaa aaaaaa 3316

<210> 152
<211> 4111
<212> DNA
<213> Homo sapiens

<400> 152
cggcagggtt ggaaaatgat ggaagaggcg gaggtggagg cgaccgagtg ctgagaggaa 60
cctgcggaat cggccgagat ggggtctggc gcgcgctttc cctcggggac ccttcgtgtc 120
cggtggttgc tgttgcttgg cctggtgggc ccagtcctcg gtgcggcgcg gccaggcttt 180
caacagacct cacatctttc ttcttatgaa attataactc cttggagatt aactagagaa 240
agaagagaag cccctaggcc ctattcaaaa caagtatctt atgttattca ggctgaagga 300
aaagagcata ttattcactt ggaaaggaac aaagaccttt tgcctgaaga ttttgtggtt 360
tatacttaca acaaggaagg gactttaatc actgaccatc ccaatataca gaatcattgt 420
cattatcggg gctatgtgga gggagttcat aattcatcca ttgctcttag cgactgtttt 480
ggactcagag gattgctgca tttagagaat gcgagttatg ggattgaacc cctgcagaac 540
agctctcatt ttgagcacat catttatcga atggatgatg tctacaaaga gcctctgaaa 600
tgtggagttt ccaacaagga tatagagaaa gaaactgcaa aggatgaaga ggaagagcct 660
cccagcatga ctcagctact tcgaagaaga agagctgtct tgccacagac ccggtatgtg 720
gagctgttca ttgtcgtaga caaggaaagg tatgacatga tgggaagaaa tcagactgct 780
gtgagagaag agatgattct cctggcaaac tacttggata gtatgtatat tatgttaaat 840
attcgaattg tgctagttgg actggagatt tggaccaatg gaaacctgat caacatagtt 900
gggggtgctg gtgatgtgct ggggaacttc gtgcagtggc gggaaaagtt tcttatcaca 960
cgtcggagac atgacagtgc acagctagtt ctaaagaaag gttttggtgg aactgcagga 1020
atggcatttg tgggaacagt gtgttcaagg agccacgcag gcgggattaa tgtgtttgga 1080
caaatcactg tggagacatt tgcttccatt gttgctcatg aattgggtca taatcttgga 1140
atgaatcacg atgatgggag agattgttcc tgtggagcaa agagctgcat catgaattca 1200
ggagcatcgg gttccagaaa ctttagcagt tgcagtgcag aggactttga gaagttaact 1260
ttaaataaag gaggaaactg ccttcttaat attccaaagc ctgatgaagc ctatagtgct 1320
ccctcctgtg gtaataagtt ggtggacgct ggggaagagt gtgactgtgg tactccaaag 1380
gaatgtgaat tggacccttg ctgcgaagga agtacctgta agcttaaatc atttgctgag 1440
tgtgcatatg gtgactgttg taaagactgt cggttccttc caggaggtac tttatgccga 1500
ggaaaaacca gtgagtgtga tgttccagag tactgcaatg gttcttctca gttctgtcag 1560
ccagatgttt ttattcagaa tggatatcct tgccagaata acaaagccta ttgctacaac 1620
ggcatgtgcc agtattatga tgctcaatgt caagtcatct ttggctcaaa agccaaggct 1680
gcccccaaag attgtttcat tgaagtgaat tctaaaggtg acagatttgg caattgtggt 1740
ttctctggca atgaatacaa gaagtgtgcc actgggaatg ctttgtgtgg aaagcttcag 1800
tgtgagaatg tacaagagat acctgtattt ggaattgtgc ctgctattat tcaaacgcct 1860

agtcgaggca ccaaatgttg gggtgtggat ttccagctag gatcagatgt tccagatcct 1920

gggatggtta acgaaggcac aaaatgtggt gctggaaaga tctgtagaaa cttccagtgt 1980

gtagatgctt ctgttctgaa ttatgactgt gatgttcaga aaaagtgtca tggacatggg 2040

gtatgtaata gcaataagaa ttgtcactgt gaaaatggct gggctccccc aaattgtgag 2100

actaaaggat acggaggaag tgtggacagt ggacctacat acaatgaaat gaatactgca 2160

ttgagggacg gacttctggt cttcttcttc ctaattgttc cccttattgt ctgtgctatt 2220

tttatcttca tcaagaggga tcaactgtgg agaagctact tcagaaagaa gagatcacaa 2280

acatatgagt cagatggcaa aaatcaagca aacccttcta gacagccggg gagtgttcct 2340

cgacatgttt ctccagtgac acctcccaga gaagttccta tatatgcaaa cagatttgca 2400

gtaccaacct atgcagccaa gcaacctcag cagttcccat caaggccacc tccaccacaa 2460

ccgaaagtat catctcaggg aaacttaatt cctgcccgtc ctgctcctgc acctccttta 2520

tatagttccc tcacttgatt tttttaacct tctttttgca aatgtcttca gggaactgag 2580

ctaatacttt ttttttttct tgatgttttc ttgaaaagcc tttctgttgc aactatgaat 2640

gaaaacaaaa caccacaaaa cagacttcac taacacagaa aaacagaaac tgagtgtgag 2700

agttgtgaaa tacaaggaaa tgcagtaaag ccagggaatt tacaataaca tttccgtttc 2760

catcattgaa taagtcttat tcagtcatcg gtgaggttaa tgcactaatc atggattttt 2820

tgaacatgtt attgcagtga ttctcaaatt aactgtattg gtgtaagatt tttgtcatta 2880

agtgtttaag tgttattctg aattttctac cttagttatc attaatgtag ttcctcattg 2940

aacatgtgat aatctaatac ctgtgaaaac tgactaatca gctgccaata atatctaata 3000

tttttcatca tgcacgaatt aataatcatc atactctaga atcttgtctg tcactcacta 3060

catgaataag caaatattgt cttcaaaaga atgcacaaga accacaatta agatgtcata 3120

ttattttgaa agtacaaaat atactaaaag agtgtgtgtg tattcacgca gttactcgct 3180

tccattttta tgacctttca actataggta ataactctta gagaaattaa tttaatatta 3240

gaatttctat tatgaatcat gtgaaagcat gacattcgtt cacaatagca ctattttaaa 3300

taaattataa gctttaaggt acgaagtatt taatagatct aatcaaatat gttgattcat 3360

ggctataata aagcaggagc aattataaaa tcttcaatca attgaacttt tacaaaacca 3420

cttgagaatt tcatgagcac tttaaaatct gaactttcaa agcttgctat taaatcattt 3480

agaatgttta catttactaa ggtgtgctgg gtcatgtaaa atattagaca ctaatatttt 3540

catagaaatt aggctggaga aagaaggaag aaatggtttt cttaaatacc tacaaaaaag 3600

ttactgtggt atctatgagt tatcatctta gctgtgttaa aaatgaattt ttactatggc 3660

agatatggta tggatcgtaa aattttaagc actaaaaatt ttttcataac ctttcataat 3720

aaagtttaat aataggttta ttaactgaat ttcattagtt ttttaaaagt gtttttggtt 3780

tgtgtatata tacatataca aatacaacat ttacaataaa taaaatactt gaaattctct 3840

tttgtgtctc ctagtagctt cctactcaac tatttataat ctcattaatt aaaaagttat 3900

aattttagat aaaaattcta gtcaaatttt tacagatatt atctcactaa ttttcagact 3960

tttgccaaag tgtgcacaat ggctttttgt taataaagaa cagattagtt ttgaagaagg 4020

caaaaatttc agttttctga agacagcatg ttattttaac aatcaagtat acatattaaa 4080

aattgtgagc aatctcaaaa aaaaaaaaaa a 4111

<210> 153
<211> 3927
<212> DNA
<213> Homo sapiens

<400> 153
gcggcggcag gcctagcagc acgggaaccg tcccccgcgc gcatgcgcgc gcccctgaag 60

cgcctggggg acgggtatgg gcgggaggta ggggcgcggc tccgcgtgcc agttgggtgc 120

ccgcgcgtca cgtggtgagg aaggaggcgg aggtctgagt ttcgaaggag ggggggagag 180

aagagggaac gagcaaggga aggaaagcgg ggaaaggagg aaggaaacga acgaggggga 240

gggaggtccc tgttttggag gagctaggag cgttgccggc ccctgaagtg gagcgagagg 300

gaggtgcttc gccgtttctc ctgccagggg aggtcccggc ttcccgtgga ggctccggac 360

caagcccctt cagcttctcc ctccggatcg atgtgctgct gttaacccgt gaggaggcgg 420

cggcggcggc agcggcagcg gaagatggtg ttgctgagag tgttaattct gctcctctcc 480

tgggcggcgg ggatgggagg tcagtatggg aatcctttaa ataaatatat cagacattat 540

gaaggattat cttacaatgt ggattcatta caccaaaaac accagcgtgc caaaagagca 600

gtctcacatg aagaccaatt tttacgtcta gatttccatg cccatggaag acatttcaac 660

ctacgaatga agagggacac ttcccttttc agtgatgaat ttaaagtaga aacatcaaat 720

aaagtacttg attatgatac ctctcatatt tacactggac atatttatgg tgaagaagga 780

agttttagcc atgggtctgt tattgatgga agatttgaag gattcatcca gactcgtggt 840

ggcacatttt atgttgagcc agcagagaga tatattaaag accgaactct gccatttcac 900

tctgtcattt atcatgaaga tgatattaac tatccccata aatacggtcc tcaggggggc 960

tgtgcagatc attcagtatt tgaaagaatg aggaaatacc agatgactgg tgtagaggaa 1020

gtaacacaga tacctcaaga agaacatgct gctaatggtc cagaacttct gaggaaaaaa 1080

cgtacaactt cagctgaaaa aaatacttgt cagctttata ttcagactga tcatttgttc 1140

tttaaatatt acggaacacg agaagctgtg attgcccaga tatccagtca tgttaaagcg 1200

attgatacaa tttaccagac cacagacttc tccggaatcc gtaacatcag tttcatggtg 1260

aaacgcataa gaatcaatac aactgctgat gagaaggacc ctacaaatcc tttccgtttc 1320

ccaaatattg gtgtggagaa gtttctggaa ttgaattctg agcagaatca tgatgactac 1380

tgtttggcct atgtcttcac agaccgagat tttgatgatg gcgtacttgg tctggcttgg 1440

gttggagcac cttcaggaag ctctggagga atatgtgaaa aaagtaaact ctattcagat 1500

ggtaagaaga agtccttaaa cactggaatt attactgttc agaactatgg gtctcatgta 1560

cctcccaaag tctctcacat tacttttgct cacgaagttg gacataactt tggatcccca 1620

catgattctg gaacagagtg cacaccagga gaatctaaga atttgggtca aaaagaaaat 1680

ggcaattaca tcatgtatgc aagagcaaca tctggggaca aacttaacaa caataaattc 1740

tcactctgta gtattagaaa tataagccaa gttcttgaga agaagagaaa caactgtttt 1800

gttgaatctg gccaacctat ttgtggaaat ggaatggtag aacaaggtga agaatgtgat 1860

tgtggctata gtgaccagtg taaagatgaa tgctgcttcg atgcaaatca accagaggga 1920

agaaaatgca aactgaaacc tgggaaacag tgcagtccaa gtcaaggtcc ttgttgtaca 1980

gcacagtgtg cattcaagtc aaagtctgag aagtgtcggg atgattcaga ctgtgcaagg 2040

gaaggaatat gtaatggctt cacagctctc tgcccagcat ctgaccctaa accaaacttc 2100

acagactgta ataggcatac acaagtgtgc attaatgggc aatgtgcagg ttctatctgt 2160

gagaaatatg gcttagagga gtgtacgtgt gccagttctg atggcaaaga tgataaagaa 2220

ttatgccatg tatgctgtat gaagaaaatg gacccatcaa cttgtgccag tacagggtct 2280

gtgcagtgga gtaggcactt cagtggtcga accatcaccc tgcaacctgg atccccttgc 2340

aacgatttta gaggttactg tgatgttttc atgcggtgca gattagtaga tgctgatggt 2400

cctctagcta ggcttaaaaa agcaattttt agtccagagc tctatgaaaa cattgctgaa 2460

tggattgtgg ctcattggtg ggcagtatta cttatgggaa ttgctctgat catgctaatg 2520

gctggattta ttaagatatg cagtgttcat actccaagta gtaatccaaa gttgcctcct 2580

cctaaaccac ttccaggcac tttaaagagg aggagacctc cacagcccat tcagcaaccc 2640

cagcgtcagc ggccccgaga gagttatcaa atgggacaca tgagacgcta actgcagctt 2700

ttgccttggt tcttcctagt gcctacaatg ggaaaacttc actccaaaga gaaacctatt 2760

aagtcatcat ctccaaacta aaccctcaca agtaacagtt gaagaaaaaa tggcaagaga 2820

tcatatcctc agaccaggtg gaattactta aattttaaag cctgaaaatt ccaatttggg 2880

ggtgggaggt ggaaaaggaa cccaattttc ttatgaacag atatttttaa cttaatggca 2940

caaagtctta gaatattatt atgtgccccg tgttccctgt tcttcgttgc tgcattttct 3000

tcacttgcag gcaaacttgg ctctcaataa actttaccca caaattgaaa taaatatatt 3060

tttttcaact gccaatcaag gctaggaggc tcgaccacct caacattgga gacatcactt 3120

gccaatgtac ataccttgtt atatgcagac atgtatttct tacgtacact gtacttctgt 3180

gtgcaattgt aaacagaaat tgcaatatgg atgtttcttt gtattataaa atttttccgc 3240

tcttaattaa aaattactgt ttaattgaca tactcaggat aacagagaat ggtggtattc 3300

agtggtccag gattctgtaa tgctttacac aggcagtttt gaaatgaaaa tcaatttacc 3360

tttctgttac gatggagttg gttttgatac tcattttttc tttatcacat ggctgctacg 3420

ggcacaagtg actatactga agaacacagt taagtgttgt gcaaactgga catagcagca 3480

catactactt cagagttcat gatgtagatg tctggtttct gcttacgtct tttaaacttt 3540

ctaattcaat tccatttttc aattaatagg tgaaatttta ttcatgcttt gatagaaatt 3600

atgtcaatga aatgattctt tttatttgta gcctacttat ttgtgttttt catatatctg 3660

aaatatgcta attatgtttt ctgtctgata tggaaaagaa aagctgtgtc tttatcaaaa 3720

tatttaaacg gttttttcag catatcatca ctgatcattg gtaaccacta aagatgagta 3780

atttgcttaa gtagtagtta aaattgtaga taggccttct gacatttttt ttcctaaaat 3840

ttttaacagc attgaaggtg aaacagcaca atgtcccatt ccaaatttat ttttgaaaca 3900

gatgtaaata attggcattt taaagag 3927

<210> 154
<211> 4402
<212> DNA
<213> Homo sapiens

<400> 154
atgaggctgc tgcggcgctg ggcgttcgcg gctctgctgc tgtcgctgct ccccacgccc 60

ggtcttggga cccaaggtcc tgctggagct ctgcgatggg ggggcttacc ccagctggga 120

ggcccaggag cccctgaggt cacggaaccc agccgtctgg ttagggagag ctccggggga 180

gaggtccgaa agcagcagct ggacacaagg gtccgccagg agccaccagg gggcccgcct 240

gtccatctgg cccaggtgag tttcgtcatc ccagccttca actcaaactt caccctggac 300

ctggagctga accaccacct cctctcctcg caatacgtgg agcgccactt cagccgggag 360

gggacaaccc agcacagcac cggggctgga gaccactgct actaccaggg gaagctccgg 420

gggaacccgc actccttcgc cgccctctcc acctgccagg ggctgcatgg ggtcttctct 480

gatgggaact tgacttacat cgtggagccc caagaggtgg ctggaccttg gggagcccct 540

cagggacccc ttccccacct catttaccgg acccctctcc tcccagatcc cctcggatgc 600

agggaaccag gctgcctgtt tgctgtgcct gcccagtcgg ctcctccaaa ccggccgagg 660

ctgagaagga aaaggcaggt ccgccggggc caccctacag tgcacagtga aaccaagtat 720

gtggagctaa ttgtgatcaa cgaccaccag ctgttcgagc agatgcgaca gtcggtggtc 780

ctcaccagca actttgccaa gtccgtggtg aacctggccg atgtgatata caaggagcag 840

ctcaacactc gcatcgtcct ggttgccatg gaaacatggg cagatgggga caagatccag 900

gtgcaggatg acctcctgga gaccctggcc cggctcatgg tctaccgacg ggagggtctg 960

cctgagccca gtgatgccac ccacctcttc tcgggcagga ccttccagag cacgagcagc 1020

ggggcagcct acgtgggggg catatgctcc ctgtcccacg gcgggggtgt gaacgagtac 1080

ggcaacatgg gggcgatggc cgtgaccctt gcccagacgc tgggacagaa cctgggcatg 1140

atgtggaaca aacaccggag ctcggcaggg gactgcaagt gtccagacat ctggctgggc 1200

tgcatcatgg aggacactgg gttctacctg ccccgcaagt tctcgcgctg tagcatcgac 1260

gagtacaacc agtttctgca ggagggtggt ggcagctgcc tcttcaacaa gcccctcaag 1320

ctcctggacc ccccagagtg cgggaacggc ttcgtggagg caggggagga gtgcgactgc 1380

ggctcggtgc aggagtgcag ccgcgcaggt ggcaactgct gcaagaaatg caccctgact 1440

cacgacgcca tgtgcagcga cgggctctgc tgtcgccgct gcaagtacga accacggggt 1500

gtgtcctgcc gagaggccgt gaacgagtgc gacatcgcgg agacctgcac cggggactct 1560

agccagtgcc cgcctaacct gcacaagctg gacggttact actgtgacca tgagcagggc 1620

cgctgctacg gaggtcgctg caaaacccgg gaccggcagt gccaggttct ttggggccat 1680

gcggctgctg atcgcttctg ctacgagaag ctgaatgtgg aggggacgga gcgtgggagc 1740

tgtgggcgca agggatctgg ctgggtccag tgcagtaagc aggacgtgct gtgtggcttc 1800

ctcctctgtg tcaacatctc tggagctcct cggctagggg acctggtggg agacatcagt 1860

agtgtcacct tctaccacca gggcaaggag ctggactgca ggggaggcca cgtgcagctg 1920

gcggacggct ctgacctgag ctatgtggag gatggcacag cctgcgggcc taacatgttg 1980

tgcctggacc atcgctgcct gccagcttct gccttcaact tcagcacctg ccccggcagt 2040

ggggagcgcc ggatttgctc ccaccacggg gtctgcagca atgaagggaa gtgcatctgt 2100

cagccagact ggacaggcaa agactgcagt atccataacc ccctgcccac gtccccaccc 2160

acgggggaga cggagagata taaaggtccc agcggcacca acatcatcat tggctccatt 2220

gctggggctg tcctggttgc agccatcgtc ctgggcggca cgggctgggg atttaaaaac 2280

attcgccgag gaaggtccgg aggggcctaa gtgccaccct cctccctcca agcctggcac 2340

ccaccgtctc ggccctgaac cacgaggctg cccccatcca gccacggagg gaggcaccat 2400

gcaaatgtct tccaggtcca aacccttcaa ctcctggctc cgcaggggtt tgggtggggg 2460

ctgtggccct gcccttggca ccaccagggt ggaccaggcc tggagggcac ttcctccaca 2520

gtcccccacc cacctcctgc ggctcagcct tgcacaccca ctgccccgtg tgaatgtagc 2580

ttccacctca tggattgcca cagctcaact cgggggcgcc tggagggatg cccccaggca 2640

gccaccagtg gacctagcct ggatggcccc tccttgcaac caggcagctg agaccagggt 2700

cttacctctc tgggacctag gggacggggc ctgacatcta catttttaa aactgaatct 2760

taatcgatga atgtaaactc gggggtgctg gggccagggc agatgtgggg atgttttgac 2820

atttacagga gggcccggag aaactgaggt atggccatgc cctagaccct ccccaaggat 2880

gaccacaccc gaagtcctgt cactgagcac agtcaggggc tgggcatccc agcttgcccc 2940

cgcttagccc cgctgagctt ggaggaagta tgagtgctga ttcaaaccaa agctgcctgt 3000

gccatgccca aggcctaggt tatgggtacg gcaaccacat gtcccagatc gtctccaatt 3060

cgaaaacaac cgtcctgctg tccctgtcag gacacatgga ttttggcagg gcgggggggg 3120

ttctagaaaa tatagttcct ataataaaat ggcaccttcc ccctttcaag aagggtgatt 3180

ctggggccga ctcagggttt aggtgccccc tggtgtggcc tagatgtccc cacctgggcc 3240

atctttctgg ggaggactct cccaggtagg gaaggccaga ggtggcccag tgcctggagg 3300

gttagggtct ctgcctggga tatgcaagag gaagtaggaa agggaggtct catggatgat 3360

cctaggctgc tagaagtcct taaggcccca tctagtccat tccactccct acccccattc 3420

cagagccgag tagtaagttt acagatgttt cccccattac gtacccccac ccatccctgc 3480

tgcagcgagc ctgagagcca ggtagagcca ggcacagctc ctcagtcttc tcacacagtc 3540

ctgccggtgg ccttccctca tgacccttgc ttgggagggt ggagcactgg ctccttgacc 3600

ctaaaaggta gctggcaggg gcaagatggg ggccagctac ctaatggatg aaagccacaa 3660

gtgaatacag ttcttgtcac cagggttgcc ctgccctcac tcggcaggga gttctgacac 3720

cccagggccc gtgagctacc tgcttgagcc cctgtttctg gggcaccttc gaggaggcgt 3780

tgtggagggc atcgccccct gtttattcac aacaccctca ggggcaaaca ggcctgggac 3840

ccgctgacac cattttgggt agctggatgc acccgacagc agtggggtcc acacactgag 3900

ctccagctgg cactgcccac tcaagggctg agtggagggg cccctccggc cagctctgct 3960

ccaccagccc tgcaagctga tgcagggcgg gggaagggct gggtgttgca ctattgctgc 4020

gctgccttaa ggcatctgtc ctctggtggt gcacccgtgc acacaggtac agtgcatctg 4080

ggcacagctt ttggatccac acctctgcac aagtgtgaat acctctgcac atatgggcgt 4140

atctgtgtgt gctcgtgtat atggggtggg gaacatgaga cttcctgtga ccagtccacc 4200

ctggctccca gctgtctgta tcctcctgcc ccgccctggc gagtgcctac cctggcagaa 4260

cccagggagg agtggaggct gcctctgcct gggcctccac acagcatcct gtacatacgc 4320

cacctgggct gggggtgggg aggcagggcc aggagcatcg attaaagatc acatcctggg 4380

gcttccaggg agctcacacc aa 4402

<210> 155
<211> 6093
<212> DNA
<213> Homo sapiens

<400> 155
   acgccgccaa ctggcggggg tgcgggggag acaataattt gttccgcggt aataagaacg 60
   gtgactgctg gccgtggatc catttcacag gcctgccttc tctcactaac gctcttccta 120
   gtccccgggc caactcggac agtttgctca tttattgcaa cggtcaaggc tggcttgtgc 180
   cagaacggcg cgcgcgcgcg cacgcacgca cacacacggg gggaaacttt tttaaaaatg 240
   aaaggctaga agagctcagc ggcggcgcgg gcgctgcgcg agggctccgg agctgactcg 300
   ccgaggcagg aaatccctcc ggtcgcgacg cccggccccg gctcggcgcc cgcgtgggat 360
   ggtgcagcgc tcgccgccgg gcccgagagc tgctgcactg aaggccggcg acgatggcag 420
   cgcgcccgct gcccgtgtcc cccgcccgcg ccctcctgct cgccctggcc ggtgctctgc 480
   tcgcgccctg cgaggcccga ggggtgagct tatggaacca aggaagagct gatgaagttg 540
   tcagtgcctc tgttgggagt ggggacctct ggatcccagt gaagagcttc gactccaaga 600
   atcatccaga agtgctgaat attcgactac aacgggaaag caaagaactg atcataaatc 660
   tggaaagaaa tgaaggtctc attgccagca gtttcacgga aacccactat ctgcaagacg 720
   gtactgatgt ctccctcgct cgaaattaca cggtaattct gggtcactgt tactaccatg 780
   gacatgtacg gggatattct gattcagcag tcagtctcag cacgtgttct ggtctcaggg 840
   gacttattgt gtttgaaaat gaaagctatg tcttagaacc aatgaaaagt gcaaccaaca 900
   gatacaaact cttcccagcg aagaagctga aaagcgtccg gggatcatgt ggatcacatc 960
   acaacacacc aaacctcgct gcaaagaatg tgtttccacc accctctcag acatgggcaa 1020
   gaaggcataa aagagagacc ctcaaggcaa ctaagtatgt ggagctggtg atcgtggcag 1080
   acaaccgaga gtttcagagg caaggaaaag atctggaaaa agttaagcag cgattaatag 1140
   agattgctaa tcacgttgac aagttttaca gaccactgaa cattcggatc gtgttggtag 1200
   gcgtggaagt gtggaatgac atggacaaat gctctgtaag tcaggaccca ttcaccagcc 1260
   tccatgaatt tctggactgg aggaagatga agcttctacc tcgcaaatcc catgacaatg 1320
   cgcagcttgt cagtggggtt tatttccaag ggaccaccat cggcatggcc ccaatcatga 1380
   gcatgtgcac ggcagaccag tctgggggaa ttgtcatgga ccattcagac aatccccttg 1440
   gtgcagccgt gaccctggca catgagctgg gccacaattt cgggatgaat catgacacac 1500
   tggacagggg ctgtagctgt caaatggcgg ttgagaaagg aggctgcatc atgaacgctt 1560
   ccaccgggta cccatttccc atggtgttca gcagttgcag caggaaggac ttggagacca 1620
   gcctggagaa aggaatgggg gtgtgcctgt ttaacctgcc ggaagtcagg gagtctttcg 1680
   ggggccagaa gtgtgggaac agatttgtgg aagaaggaga ggagtgtgac tgtggggagc 1740
   cagaggaatg tatgaatcgc tgctgcaatg ccaccacctg taccctgaag ccggacgctg 1800
   tgtgcgcaca tgggctgtgc tgtgaagact gccagctgaa gcctgcagga acagcgtgca 1860
   gggactccag caactcctgt gacctcccag agttctgcac aggggccagc cctcactgcc 1920
   cagccaacgt gtacctgcac gatgggcact catgtcagga tgtggacggc tactgctaca 1980
   atggcatctg ccagactcac gagcagcagt gtgtcacgct ctggggacca ggtgctaaac 2040
   ctgcccctgg gatctgcttt gagagagtca attctgcagg tgatccttat ggcaactgtg 2100
   gcaaagtctc gaagagttcc tttgccaaat gcgagatgag agatgctaaa tgtggaaaaa 2160
   tccagtgtca aggaggtgcc agccggccag tcattggtac caatgccgtt tccatagaaa 2220
   caaacatccc cctgcagcaa ggaggccgga ttctgtgccg gggggaccac gtgtacttgg 2280
   gcgatgacat gccggaccca gggcttgtgc ttgcaggcac aaagtgtgca gatggaaaaa 2340
   tctgcctgaa tcgtcaatgt caaaatatta gtgtctttgg ggttcacgag tgtgcaatgc 2400
   agtgccacgg cagaggggtg tgcaacaaca ggaagaactg ccactgcgag gcccactggg 2460
   cacctccctt ctgtgacaag tttggctttg gaggaagcac agacagcggc cccatccggc 2520
   aagcagataa ccaaggttta accataggaa ttctggtgac catcctgtgt cttcttgctg 2580
   ccggatttgt ggtttatctc aaaaggaaga ccttgatacg actgctgttt acaaataaga 2640
   agaccaccat tgaaaaacta aggtgtgtgc gcccttcccg gccaccccgt ggcttccaac 2700
   cctgtcaggc tcacctcggc caccttggaa aaggcctgat gaggaagccg ccagattcct 2760
   acccaccgaa ggacaatccc aggagattgc tgcagtgtca gaatgttgac atcagcagac 2820
   ccctcaacgg cctgaatgtc cctcagcccc agtcaactca gcgagtgctt cctcccctcc 2880
   accgggctcc acgtgcacct agcgtccctg ccagacccct gccagccaag cctgcactta 2940
   ggcaggccca ggggacctgt aagccaaacc cccctcagaa gcctctgcct gcagatcctc 3000
   tggccagaac aactcggctc actcatgcct tggccaggac cccaggacaa tgggagactg 3060
   ggctccgcct ggcacccctc agacctgctc cacaatatcc acaccaagtg cccagatcca 3120
   cccacaccgc ctatattaag tgagaagccg acaccttttt tcaacagtga agacagaagt 3180
   ttgcactatc tttcagctcc agttggagtt ttttgtacca acttttagga ttttttttaa 3240
   tgtttaaaac atcattacta taagaacttt gagctactgc cgtcagtgct gtgctgtgct 3300
   atggtgctct gtctacttgc tcaggtactt gtaaattatt aatttatgca gaatgttgat 3360
   tacagtgcag tgcgctgtag taggcatttt taccatcact gagttttcca tggcaggaag 3420
   gcttgttgtg cttttagtat tttagtgaac ttgaaatatc ctgcttgatg ggattctgga 3480
   caggatgtgt ttgctttctg atcaaggcct tattggaaag cagtccccca actaccccca 3540
   gctgtgctta tggtaccaga tgcagctcaa gagatcccaa gtagaatctc agttgatttt 3600
   ctggattccc catctcaggc cagagccaag gggcttcagg tccaggctgt gtttggcttt 3660
   cagggaggcc ctgtgcccct tgacaactgg caggcaggct cccagggaca cctgggagaa 3720
   atctggcttc tggccaggaa gctttggtga gaacctgggt tgcagacagg aatcttaagg 3780
   tgtagccaca ccaggataga gactggaaca ctagacaagc cagaacttga ccctgagctg 3840
   accagccgtg agcatgtttg gaaggggtct gtagtgtcac tcaaggcggt gcttgataga 3900
   aatgccaagc acttcttttt ctcgctgtcc tttctagagc actgccacca gtaggttatt 3960
   tagcttggga aaggtggtgt ttctgtaaga aacctactgc ccaggcactg caaaccgcca 4020
   cctccctata ctgcttggag ctgagcaaat caccacaaac tgtaatacaa tgatcctgta 4080
   ttcagacaga tgaggctttc catgggacca caactatttt cagatgtgaa ccattaacca 4140
   gatctagtca atcaagtctg tttactgcaa ggttcaactt attaacaatt aggcagactc 4200
   tttatgcttg caaaaactac aaccaatgga atgtgatgtt catgggtata gttcatgtct 4260
   gctatcatta ttcgtagata ttggacaaag aaccttctct atggggcatc ctctttttcc 4320
   aacttggctg caggaatctt taaaagatgc ttttaacaga gtctgaacct atttcttaaa 4380
   cacttgcaac ctacctgttg agcatcacag aatgtgataa ggaaatcaac ttgcttatca 4440
   acttcctaaa tattatgaga tgctggcttg ggcagcatcc ccttgaactc ttcactcttc 4500
   aaatgcctga ctagggagcc atgtttcaca aggtctttaa agtgactaat ggcatgagaa 4560
   atacaaaaat actcagataa ggtaaaatgc catgatgcct ctgtcttctg gactggtttt 4620
   cacattagaa gacaattgac aacagttaca taattcactc tgagtgtttt atgagaaagc 4680
   cttcttttgg gggtcaacag ttttcctatg ctttgaaaca gaaaaatatg taccaagaat 4740
   cttggtttgc cttccagaaa acaaaactgc atttcacttt cccggtgttc cccactgtat 4800
   ctaggcaaca tagtattcat gactatggat aaactaaaca cgtgacacaa acacacacaa 4860
   aagggaaccc agctctaata cattccaact cgtatagcat gcatctgttt attctatagt 4920
   tattaagttc tttaaaatgt aaagccatgc tggaaaataa tactgctgag atacatacag 4980
   aattactgta actgattaca cttggtaatt gtactaaagc caaacatata tatactatta 5040
   aaaaggttta cagaatttta tggtgcatta cgtgggcatt gtctttttag atgcccaaat 5100
   ccttagatct ggcatgttag cccttcctcc aattataaga ggatatgaac tgagtttttc 5160
   ttttgttgtt tgttcttagc tgtaattcct atgcttctat ttcagagagc caggagagtt 5220
   tgatattaaa ggaggttaaa actgtgatct tatgccatgt catcaatggc cacttagggg 5280
   ccatggctga tgacacattc ttatctctac agtactaatg tgttattata gagccatgca 5340
   ttttatttct gaataagaac atatttaaac taatattccc ttacaatatg gacagtatta 5400
   atccttccaa gatgcagtat ttatcaagtg aagcatattt agcagcaaat tccattttaa 5460
   cataacttag gaaccaataa ccagggtgtt ttgtggttgg gggaggcacg gggtggagta 5520
   ttctttttta tatcctcaaa acaaaaaaaa tcaatactta tatttcaatg gcaatctagt 5580
   atttttttaa aagactgtat aggcatgaat aatagaggtg gtttgagttt tgtagggcca 5640
   tcacctggaa agtcaatgtg actagacaca aagtagccca gaggctactt ttcttcctac 5700
   agcttattat agttgtaggt tctatgacct cacttcatgg gttccaggca attccgctga 5760
   aaggtttgtc tcctgaaatt ttttaagttt gttttcctga cacatgtaat cagatgtgta 5820
   gcaaccgagg gaaacgaagc ctaacattct ccattgtgga aatacacaca ggaggttaca 5880
   tttcacagcg tggatttttc cagcttacac atgtgggatg acatcacaga aaccacaaaa 5940
   gcagcaaatt aaactgtagg agagtcaata ctcctgacga gtctcggggg gggggcattt 6000
   ttatgccttc ttaactttat gagaattctc aggctgaact ataggccatt gttcccaggc 6060
   aaatcaatac atcaatgcat cctcaaaaaa aaa 6093

<210> 156
<211> 2970
<212> DNA
<213> Homo sapiens

<400> 156
gcgcggcgct gctgggttct ccgaggcgac ctggccgccg gccgctcctc cgcgcgctgt 60

tccgcacttg ctgccctcgc ccggcccgga gcgccgctgc catgcggctg gcgctgctct 120

gggccctggg gctcctgggc gcgggcagcc ctctgccttc ctggccgctc ccaaatatag 180

gtggcactga ggagcagcag gcagagtcag agaaggcccc gagggagccc ttggagcccc 240

aggtccttca ggacgatctc ccaattagcc tcaaaaaggt gcttcagacc agtctgcctg 300

agcccctgag gatcaagttg gagctggacg gtgacagtca tatcctggag ctgctacaga 360

atagggagtt ggtcccaggc cgcccaaccc tggtgtggta ccagcccgat ggcactcggg 420

tggtcagtga gggacacact ttggagaact gctgctacca gggaagagtg cggggatatg 480

caggctcctg ggtgtccatc tgcacctgct ctgggctcag aggcttggtg gtcctgaccc 540

cagagagaag ctataccctg gagcaggggc ctggggacct tcagggtcct cccattattt 600

cgcgaatcca agatctccac ctgccaggcc acacctgtgc cctgagctgg cgggaatctg 660

tacacactca gaagccacca gagcaccccc tgggacagcg ccacattcgc cggaggcggg 720

atgtggtaac agagaccaag actgtggagt tggtgattgt ggctgatcac tcggaggccc 780

agaaataccg ggacttccag cacctgctaa accgcacact ggaagtggcc ctcttgctgg 840

acacattctt ccggcccctg aatgtacgag tggcactagt gggcctggag gcctggaccc 900

agcgtgacct ggtggagatc agcccaaacc cagctgtcac cctcgaaaac ttcctccact 960

ggcgcagggc acatttgctg cctcgattgc cccatgacag tgcccagctg gtgactggta 1020

cttcattctc tgggcctacg gtgggcatgg ccattcagaa ctccatctgt tctcctgact 1080

tctcaggagg tgtgaacatg gaccactcca ccagcatcct gggagtcgcc tcctccatag 1140

cccatgagtt gggccacagc ctgggcctgg accatgattt gcctgggaat agctgcccct 1200

gtccaggtcc agccccagcc aagacctgca tcatggaggc ctccacagac ttcctaccag 1260

gcctgaactt cagcaactgc agccgacggg ccctggagaa agccctcctg gatggaatgg 1320

gcagctgcct cttcgaacgg ctgcctagcc taccccctat ggctgctttc tgcggaaata 1380

tgtttgtgga gccgggcgag cagtgtgact gtggcttcct ggatgactgc gtcgatccct 1440

gctgtgattc tttgacctgc cagctgaggc caggtgcaca gtgtgcatct gacggaccct 1500

gttgtcaaaa ttgccagctg cgcccgtctg gctggcagtg tcgtcctacc agaggggatt 1560

gtgacttgcc tgaattctgc ccaggagaca gctcccagtg tccccctgat gtcagcctag 1620

gggatggcga gccctgcgct ggcgggcaag ctgtgtgcat gcacgggcgt tgtgcctcct 1680

atgcccagca gtgccagtca ctttggggac ctggagccca gcccgctgcg ccactttgcc 1740

tccagacagc taatactcgg ggaaatgctt ttgggagctg tgggcgcaac cccagtggca 1800

gttatgtgtc ctgcacccct agagatgcca tttgtgggca gctccagtgc cagacaggta 1860

ggacccagcc tctgctgggc tccatccggg atctactctg ggagacaata gatgtgaatg 1920

ggactgagct gaactgcagc tgggtgcacc tggacctggg cagtgatgtg gcccagcccc 1980

tcctgactct gcctggcaca gcctgtggcc ctggcctggt gtgtatagac catcgatgcc 2040

agcgtgtgga tctcctgggg gcacaggaat gtcgaagcaa atgccatgga catggggtct 2100

gtgacagcaa caggcactgc tactgtgagg agggctgggc accccctgac tgcaccactc 2160

agctcaaagc aaccagctcc ctgaccacag ggctgctcct cagcctcctg gtcttattgg 2220

tcctggtgat gcttggtgcc agctactggt accgtgcccg cctgcaccag cgactctgcc 2280

agctcaaggg acccacctgc cagtacaggg cagcccaatc tggtccctct gaacggccag 2340

gacctccgca gagggccctg ctggcacgag gcactaagca ggctagtgct ctcagcttcc 2400

cggccccccc ttccaggccg ctgccgcctg accctgtgtc caagagactc caggctgagc 2460

tggctgaccg acccaatccc cctacccgcc ctctgcccgc tgacccggtg gtgagaagcc 2520

cgaagtctca ggggccagcc aagcccccac ccccaaggaa gccactgcct gccgaccccc 2580

agggccggtg cccatcgggt gacctgcccg gcccaggggc tggaatcccg cccctagtgg 2640

taccctccag accagcgcca ccgcctccga cagtgtcctc gctctacctc tgacctctcc 2700

ggaggttccg ctgcctccaa gccggactta gggcttcaag aggcgggcgt gccctctgga 2760

gtcccctacc atgactgaag gcgccagaga ctggcggtgt cttaagactc cgggcaccgc 2820

cacgcgctgt caagcaacac tctgcggacc tgccggcgta gttgcagcgg gggcttgggg 2880

aggggctggg ggttggacgg gattgaggaa ggtccgcaca gcctgtctct gctcagttgc 2940

aataaacgtg acatcttggg agcgttcaaa 2970

<210> 157
<211> 3572
<212> DNA

<213> Homo sapiens

<400> 157
acctgcactt ctgggggcgt cgagcctggc ggtagaatct tcccagtagg cggcgcggga 60

gggaaaagag gattgagggg ctaggccggg cggatcccgt cctcccccga tgtgagcagt 120

tttccgaaac cccgtcaggc gaaggctgcc cagagaggtg gagtcggtag cggggccggg 180

aacatgaggc agtctctcct attcctgacc agcgtggttc ctttcgtgct ggcgccgcga 240

cctccggatg acccgggctt cggcccccac cagagactcg agaagcttga ttctttgctc 300

tcagactacg atattctctc tttatctaat atccagcagc attcggtaag aaaaagagat 360

ctacagactt caacacatgt agaaacacta ctaacttttt cagctttgaa aaggcatttt 420

aaattatacc tgacatcaag tactgaacgt ttttcacaaa atttcaaggt cgtggtggtg 480

gatggtaaaa acgaaagcga gtacactgta aaatggcagg acttcttcac tggacacgtg 540

gttggtgagc ctgactctag ggttctagcc cacataagag atgatgatgt tataatcaga 600

atcaacacag atggggccga atataacata gagccacttt ggagatttgt taatgatacc 660

aaagacaaaa gaatgttagt ttataaatct gaagatatca agaatgtttc acgtttgcag 720

tctccaaaag tgtgtggtta tttaaaagtg gataatgaag agttgctccc aaaagggtta 780

gtagacagag aaccacctga agagcttgtt catcgagtga aaagaagagc tgacccagat 840

cccatgaaga acacgtgtaa attattggtg gtagcagatc atcgcttcta cagatacatg 900

ggcagagggg aagagagtac aactacaaat tacttaatag agctaattga cagagttgat 960

gacatctatc ggaacacttc atgggataat gcaggtttta aaggctatgg aatacagata 1020

gagcagattc gcattctcaa gtctccacaa gaggtaaaac ctggtgaaaa gcactacaac 1080

atggcaaaaa gttacccaaa tgaagaaaag gatgcttggg atgtgaagat gttgctagag 1140

caatttagct ttgatatagc tgaggaagca tctaaagttt gcttggcaca ccttttcaca 1200

taccaagatt ttgatatggg aactcttgga ttagcttatg ttggctctcc cagagcaaac 1260

agccatggag gtgtttgtcc aaaggcttat tatagcccag ttgggaagaa aaatatctat 1320

ttgaatagtg gtttgacgag cacaaagaat tatggtaaaa ccatccttac aaaggaagct 1380

gacctggtta caactcatga attgggacat aattttggag cagaacatga tccggatggt 1440

ctagcagaat gtgccccgaa tgaggaccag ggagggaaat atgtcatgta tcccatagct 1500

gtgagtggcg atcacgagaa caataagatg ttttcaaact gcagtaaaca atcaatctat 1560

aagaccattg aaagtaaggc ccaggagtgt tttcaagaac gcagcaataa agtttgtggg 1620

aactcgaggg tggatgaagg agaagagtgt gatcctggca tcatgtatct gaacaacgac 1680

acctgctgca acagcgactg cacgttgaag gaaggtgtcc agtgcagtga caggaacagt 1740

ccttgctgta aaaactgtca gtttgagact gcccagaaga agtgccagga ggcgattaat 1800

gctacttgca aaggcgtgtc ctactgcaca ggtaatagca gtgagtgccc gcctccagga 1860

aatgctgaag atgacactgt ttgcttggat cttggcaagt gtaaggatgg gaaatgcatc 1920

cctttctgcg agagggaaca gcagctggag tcctgtgcat gtaatgaaac tgacaactcc 1980

tgcaaggtgt gctgcaggga cctttctggc cgctgtgtgc cctatgtcga tgctgaacaa 2040

aagaacttat ttttgaggaa aggaaagccc tgtacagtag gattttgtga catgaatggc 2100

aaatgtgaga aacgagtaca ggatgtaatt gaacgatttt gggatttcat tgaccagctg 2160

agcatcaata cttttggaaa gtttttagca gacaacatcg ttgggtctgt cctggttttc 2220

tccttgatat tttggattcc tttcagcatt cttgtccatt gtgtggataa gaaattggat 2280

aaacagtatg aatctctgtc tctgtttcac cccagtaacg tcgaaatgct gagcagcatg 2340

gattctgcat cggttcgcat tatcaaaccc tttcctgcgc cccagactcc aggccgcctg 2400

cagcctgccc ctgtgatccc ttcggcgcca gcagctccaa aactggacca ccagagaatg 2460

gacaccatcc aggaagaccc cagcacagac tcacatatgg acgaggatgg gtttgagaag 2520

gaccccttcc caaatagcag cacagctgcc aagtcatttg aggatctcac ggaccatccg 2580

gtcaccagaa gtgaaaaggc tgcctccttt aaactgcagc gtcagaatcg tgttgacagc 2640

aaagaaacag agtgctaatt tagttctcag ctcttctgac ttaagtgtgc aaaatatttt 2700

tatagatttg acctacaaat caatcacagc ttgtattttg tgaagactgg gaagtgactt 2760

agcagatgct ggtcatgtgt ttgaacttcc tgcaggtaaa cagttcttgt gtggtttggc 2820

ccttctcctt ttgaaaaggt aaggtgaagg tgaatctagc ttattttgag gctttcaggt 2880

tttagttttt aaaatatctt ttgacctgtg gtgcaaaagc agaaaataca gctggattgg 2940

gttatgaata tttacgtttt tgtaaattaa tcttttatat tgataacagc actgactagg 3000

gaaatgatca gttttttttt atacactgta atgaaccgct gaatatgagg catttggcat 3060

ttatttgtga tgacaactgg aatagttttt tttttttttt tttttttttg ccttcaacta 3120

aaaacaaagg agataaatct agtatacatt gtctctaaat tgtgggtcta tttctagtta 3180

ttacccagag tttttatgta gcagggaaaa tatatatcta aatttagaaa tcatttgggt 3240

taatatggct cttcataatt ctaagactaa tgctctctag aaacctaacc acctacctta 3300

cagtgagggc tatacatggt agccagttga atttatggaa tctaccaact gtttagggcc 3360

ctgatttgct gggcagtttt tctgtatttt ataagtatct tcatgtatcc ctgttactga 3420

tagggataca tgctcttaga aaattcacta ttggctggga gtggtggctc atgcctgtaa 3480

tcccagcact tggagaggct gaggttgcgc cactacactc cagcctgggt gacagagtga 3540

gactctgcct caaaaaaaaa aaaaaaaaaa aa 3572

<210> 158
<211> 2399

<212> DNA
<213> Homo sapiens

<400> 158
aacgctgctc aacggtctct gtccttggct gtggctcctg cgctctggct gagccatgtt 60

ccttctcctc gccctcctca ctgagcttgg aagactgcaa gcccacgaag gttctgaagg 120

aatatttctg catgtcacag ttccacggaa gattaagtca aatgacagtg aagtttcaga 180

gaggaagatg atttacatca ttacaattga tggacaacct tacactctac atctcggaaa 240

acaatcattc ttaccccaga actttttggt ttatacatat aatgaaactg gatctttgca 300

ttctgtgtct ccatatttta tgatgcattg ccattaccaa ggatatgctg ccgaatttcc 360

aaattcattt gtgacactca gtatatgttc tggtctcagg ggatttctcc agtttgaaaa 420

tatcagttat ggaattgaac cagtagaatc ttcagcaaga tttgagcata taatttatca 480

aatgaaaaat aatgatccaa atgtatccat tttagcagta aattacagtc atatttggca 540

gaaagaccag ccctacaaag ttcctttaaa ctcacagata aaaaatcttt caaaactatt 600

accccaatat ctggaaatat acattatagt ggaaaaagct ttgtatgatt atatgggatc 660

tgaaatgatg gctgtaacac aaaaaattgt ccaggttatt gggcttgtca acactatgtt 720

tacccagttc aaattgactg ttatactgtc ttccttggaa ttgtggtcaa atgaaaacca 780

gatttccacc agtggggatg ctgatgatat attacaaaga tttttggcat ggaaacggga 840

ctatctcatc ctacggcccc atgacatagc atacttactt gtttacagga aacatcctaa 900

atatgtggga gcaacatttc ctggcactgt atgcaataaa agctatgatg caggtattgc 960

tatgtatcca gatgcaatag gtttggaggg attttcggtt attatagctc aactgcttgg 1020

ccttaatgta ggattaacat atgatgacat cactcagtgt ttctgtctga gagctacatg 1080

catcatgaat catgaagcag tgagtgccag tggtagaaag atttttagca actgcagcat 1140

gcacgactat agatattttg tttcaaaatt tgagactaaa tgccttcaga agctttcaaa 1200

tttgcaacca ttacatcaaa atcaaccagt gtgtggtaat gggattttgg aatccaatga 1260

agaatgtgac tgtggtaata aaaatgaatg tcaatttaag aagtgctgtg attataacac 1320

atgtaaactg aagggctcag taaaatgtgg ttctggacca tgttgtacat caaagtgtga 1380

gttgtcaata gcaggcactc catgtagaaa gagtattgat ccagagtgtg attttacaga 1440

gtactgcaat ggaacctcta gtaattgtgt tcctgacact tatgcattga atggccgttt 1500

gtgcaagttg ggaactgcct attgctataa cggacaatgt caaactactg ataaccagtg 1560

tgccaagata tttggaaaag gtgctcaagg tgctccattt gcctgtttta aagaagttaa 1620

ttctctgcat gaaagatctg aaaactgtgg ttttaaaaat tcacaaccat taccttgtga 1680

acggaaggat gttctctgtg gaaaattagc ttgtgttcag ccacataaaa atgctaataa 1740

aagtgacgct caatctacag tttattcata tattcaagac catgtatgtg tatctatagc 1800

cactggttcc tccatgagat cagatggaac agacaatgcc tatgtggctg atggcaccat 1860

gtgtggtcca gaaatgtact gtgtaaataa aacctgcaga aaagttcatt taatgggata 1920

taactgtaat gccaccacaa aatgcaaagg gaaagggata tgtaataatt ttggtaattg 1980

tcaatgcttc cctggacata gacctccaga ttgtaaattc cagtttggtt ccccaggggg 2040

tagtattgat gatggaaatt ttcagaaatc tggtgacttt tatactgaaa aaggctacaa 2100

tacacactgg aacaactggt ttattctgag tttctgcatt tttctgccgt ttttcatagt 2160

tttcaccact gtgatcttta aaagaaatga aataagtaaa tcatgtaaca gagagaatgc 2220

agagtataat cgtaattcat ccgttgtatc agaaagcgat gacgtgggac attaatattg 2280

cacagaactt ccatagcaaa taacctaaag gaacgaatgt gctttattta taaccttacg 2340

ttatccccaa tgcattgtaa atgtcaaact tttggaaaat aaagcctgcg tgccctccc 2399

<210> 159
<211> 6506
<212> DNA
<213> Homo sapiens

<400> 159
agacgcgcct ccaccgccgg gcagtgggca ggtatggctg agggcgtgtg agcgccgagc 60

gctaagggcc gccgccacca tgccaggggg cgcaggcgcc gcccggctct gcttgctggc 120

gtttgccctg cagcccctcc ggccgcgggc ggcgcgggag cctggatgga caagaggaag 180

tgaggaaggc agccccaagc tgcagcatga acttatcata cctcagtgga agacttcaga 240

aagccccgtg agagaaaagc atccactcaa agctgagctc agggtaatgg ctgaggggcg 300

agaactgatc ctggacctgg agaagaatga gcaacttttt gctccttcct acacagaaac 360

ccattatact tcaagtggta accctcaaac caccacacgg aaattggagg atcactgctt 420

ttaccacggc acggtgaggg agacagaact gtccagcgtc acgctcagca cttgccgagg 480

aattagagga ctgattacgg tgagcagcaa cctcagctac gtcatcgagc ccctccctga 540

cagcaagggc caacacctta tttacagatc tgaacatctc aagccgcccc cgggaaactg 600

tgggttcgag cactccaagc ccaccaccag ggactgggct cttcagttta cacaacagac 660

caagaagcga cctcgcagga tgaaaaggga agatttaaac tccatgaagt atgtggagct 720

ttacctcgtg gctgattatt tagagtttca gaagaatcga cgagaccagg acgccaccaa 780

acacaagctc atagagatcg ccaactatgt tgataagttt taccgatcct tgaacatccg 840

gattgctctc gtgggcttgg aagtgtggac ccacgggaac atgtgtgaag tttcagagaa 900

tccatattct accctctggt cctttctcag ttggaggcgc aagctgcttg cccagaagta 960

ccatgacaac gcccaattaa tcacgggcat gtccttccac ggcaccacca tcggcctggc 1020

ccccctcatg gccatgtgct ctgtgtacca gtctggagga gtcaacatgg accactccga 1080

gaatgccatt ggcgtggctg ccaccatggc ccacgagatg ggccacaact ttggcatgac 1140

ccatgattct gcagattgct gctcggccag tgcggctgat ggtgggtgca tcatggcagc 1200

tgccactggg cacccctttc ccaaagtgtt caatggatgc aacaggaggg agctggacag 1260

gtatctgcag tcaggtggtg gaatgtgtct ctccaacatg ccagacacca ggatgttgta 1320

tggaggccgg aggtgtggga acgggtatct ggaagatggg gaagagtgtg actgtggaga 1380

agaagaggaa tgtaacaacc cctgctgcaa tgcctctaat tgtaccctga ggccgggggc 1440

ggagtgtgct cacggctcct gctgccacca gtgtaagctg ttggctcctg ggaccctgtg 1500

ccgcgagcag gccaggcagt gtgacctccc ggagttctgt acgggcaagt ctccccactg 1560

ccctaccaac ttctaccaga tggatggtac cccctgtgag ggcggccagg cctactgcta 1620

caacggcatg tgcctcacct accaggagca gtgccagcag ctgtggggac ccggagcccg 1680

acctgcccct gacctctgct tcgagaaggt gaatgtggca ggagacacct ttggaaactg 1740

tggaaaggac atgaatggtg aacacaggaa gtgcaacatg agagatgcga agtgtgggaa 1800

gatccagtgt cagagctctg aggcccggcc cctggagtcc aacgcggtgc ccattgacac 1860

cactatcatc atgaatggga ggcagatcca gtgccggggc acccacgtct accgaggtcc 1920

tgaggaggag ggtgacatgc tggacccagg gctggtgatg actggaacca agtgtggcta 1980

caaccatatt tgctttgagg ggcagtgcag gaacacctcc ttctttgaaa ctgaaggctg 2040

tgggaagaag tgcaatggcc atggggtctg taacaacaac cagaactgcc actgcctgcc 2100

gggctgggcc ccgcccttct gcaacacacc gggccacggg ggcagtatcg acagtgggcc 2160

tatgccccct gagagtgtgg gtcctgtggt agctggagtg ttggtggcca tcttggtgct 2220

ggcggtcctc atgctgatgt actactgctg cagacagaac aacaaactag gccaactcaa 2280

gccctcagct ctcccttcca agctgaggca acagttcagt tgtcccttca gggtttctca 2340

gaacagcggg actggtcatg ccaacccaac tttcaagctg cagacgcccc agggcaagcg 2400

aaaggtgatc aacactccgg aaatcctgcg gaagccctcc cagcctcctc cccggccccc 2460

tccagattat ctgcgtggtg ggtccccacc tgcaccactg ccagctcacc tgagcagggc 2520

tgctaggaac tccccagggc ccgggtctca aatagagagg acggagtcgt ccaggaggcc 2580

tcctccaagc cggccaattc cccccgcacc aaattgcatc gtttcccagg acttctccag 2640

gcctcggccg ccccagaagg cactcccggc aaacccagtg ccaggccgca ggagcctccc 2700

caggccagga ggtgcatccc cactgcggcc ccctggtgct ggccctcagc agtcccggcc 2760

tctggcagca cttgccccaa agtttccaga atacagatca cagagggctg gagggatgat 2820

tagctcgaaa atctagacct gtccaagggg cttctccctt tccttgagct ctctggacac 2880

tgcagaggac ccatggccat ggaaccctga agaagcatgt ctggccgcct ctgagctcct 2940

cccaccctcc tccaggaacc tccacatctc caaaaatctc cctgttgact cagtgcctcc 3000
tcggcttcct tggaagccca gagggactat gatctgatgg cctctaggtg ttgttttgtg 3060
caatatacag ccccaggtag ggaggggaga gtatgaggag ggtgactggc agcttctcct 3120
ccagactcct agccccgagg tgctgatgga gatgctcaag gccagcaagc ccctcaggcc 3180
agcacttcgc ttgcagaagc catccattca ctcctggggt gcagggcacg caagagagct 3240
tcccattgct tctgctctcc tcagaggtcc cgggctggat ggaggctggt acttacccac 3300
cccttttagc ttttagggat taaggaaggg tcaagccagc cactgctgtg gccctgccca 3360
gggcttggtt gagggaacgg cttctggctg tatggctgca tgtgacaagc cacgtcccct 3420
cccacctctc cccaaacccc tgcatccctg tattcacacg ggtcactctg actcagacag 3480
gtactattcg taggcagtgt agacagcagg aggagcaccg ggcttgggct tcctctgagc 3540
cgtgatgcca aaggttgcga ctcctgactc tggataattt ttagttgctc tttgttttct 3600
ctgccgcact ttcctggtgc cccacgcttt tctctcttcc ttcccctctc attctccctc 3660
taatgtgtgg tgctttggtg agcaaaccct cagcagtcct gaccttcggg tgaccaggtg 3720
cttgtgacct acaagtcaga gtcctctctc acagtcggcc actggatttc cctcactggc 3780
tctcaggagt gtgaccagag tagacttggg gcatggccat tggggtcata tgtttatttt 3840
tcattgtgtt ttgtgacctc agcagggtgg gggtcttcct ccttactcta agctaaatct 3900
aggtgaggtt tccccttagg gagcccagct atttacaaag tacacacgag ggagcaggct 3960
ggtcattgac ttcgggctgg accgttgccc tctgagcaga gaacagaccc atttctggga 4020
gctgcccgag atcactggag aaggcagcca gcagcagctg cactggaaca gtcagagcag 4080
ggagcctctt cctcaaccca gctttttgtc attcacttcc ttttgttctc tctctggtca 4140
ctgcccttac ctgaccctca cagaaagaga gctctgagca ggtgaggggg tctgcggtgg 4200
ctcctgtctt ccctgcagca gggaaggagg gccgtgtggt gctttgctag ataggacggt 4260
ttttgcaaag cacctggaga tgtttgctgg gagatagact cccactccac aaaggtgctg 4320
ggtggctctc cggacaggag ctggcctgac tctcactcct ctgaggcttt cctggggcct 4380
cctcccatcc tgccatgagc aattgtttgc tcttgaaaac ctcactgcaa ggctgaggct 4440
gagcttctga ttcaccaccc cagggcctcc ttatagttct ctgcacacaa taggtgcttc 4500
ttggatgttc ttgggtttgg aaataagtgg aaaatacggg atgtacccct gggggaaaag 4560
cctgggttgg gtttagaaag atctcaggaa aatgagtttc tcttccctca gggtggctgt 4620
gatacaggtt ccccatgtcc ttgccgtggg tcatccttgc tgtgggtcat ccttgctgtg 4680
gagatccatt ccccaccttt cctgtggccc aaccttttat ttaaatgtgc taccctctgc 4740
ctcaaggctt ggttcctgga aagtaaaggt gaaaacatcc cctttcaccc ctctgcaaaa 4800
caaacaagca acatcctcaa aacccaaccc catgcctcac agagcttcct gtggcttctc 4860

cagcctttct ccctcacatc aggaggtaga tagctctgaa atgacagcgc cacagccata 4920

gtgactgcat gagccatctg aacctgcagt ccaccctccc tggaaccaca ccagaaagag 4980

acctgggttg tcgttttctt gctttttgtt ttgttttgtt ttattatttt catatcacct 5040

ccatcccata aagttgtact gtgaactgga agatggtgga atgttttgga atttgataga 5100

ctttcggcaa ccagttctac taatgcttca ctcctggctc tgttcaggga ggctgcccag 5160

gaggaagact ggccattatg catccccttt tctttccagt gcccagtatg ctgttttgag 5220

gtgtcaaata caaataaatc tgggcttagg gaaggagaga ccttattcca aagcacgatt 5280

gcagaagggg aaagggaata ttgcaaaagg gagaggaagg ggccttatgg gaatagtgaa 5340

aaggctcaga ccgaccgatg gcaagatctg caagcgtctc aaagcccagg cagaaaagga 5400

cttttctttt attggaagaa gtaaacatgg ctagaaagaa ccacgttcag ggaatgacgt 5460

tgtgcccagc cttttttttt tttttttttt ttttgtctcc aggggagggg ctgtttgctg 5520

gctcaggctg aggatggccc aaagtccagg gtctggtggg gaggagggaa gcttaactca 5580

agtttgggtt agtgagttag caagctcttt gtgcagatgg ggatgtaggt aaatcttttt 5640

aaaagtgaaa ttaacctcct gccaatttta caacccaaga attttttttt aagggccttg 5700

gagccatctc taaaacaaac ctcaagggat ttagtgccct gtctccctgt ctctagaagc 5760

cttagcctgg gcacctggct caatcttgta actgcctgct agccatagat tcctttcagc 5820

cttgctgact tctccctata aaagtaaagc ctttttctgc cccagctctg agacacttgc 5880

agatcttaag gtctgagact tgctgatttt ctggttggag tgtttttttg tattgccata 5940

gtcccttccc cctgaagcaa tagcccctcc ccacctcctg caatacgcct ttccaatctt 6000

tattggaagt ctctccctgc ctacttccta atttattctt atttgacaga gggtatggaa 6060

gacttgcaat ttgaaaactg gggaccagtt ccaaagtcag taattgtgtt aaccacgtgt 6120

ataacagctc tgctggacac ccaagaaagc catgggaacg ccaactggaa aggtcccctt 6180

ccccagggga gcctgcgaag gagaggttct gtagaatcca agcccacatt tccaaagtca 6240

cccccaacgc gtcctctcac accgtccact gtgcgtttgt atgtgtctgg gatccagggc 6300

aatgtgaatt ttctttttat ttgggagatt gttcacggaa aacagatctt cttctctctt 6360

gtccacctat taattgttta caatatttgt acatctatgc aaaatacttg aatgggccat 6420

ggtgcctttt ttccttgtta gtatttaatt aaaaatgaat tgtttgtcat ttgcaatgtt 6480

aaaaaaaaaa aaaaaaaaaa aaaaaa 6506

<210> 160
<211> 2807
<212> DNA
<213> Homo sapiens

<400> 160
   gggagttcag cagtgtgttt aagactaggg tcatgcccac atcaatctca gattcccatt 60
   ttatctttct tttttggtat cactcctagt accaagtctt gtgtctgtca atatcctgtc 120
   caaaaaagaa aaacacacca aggaaaatta atataggaaa aatttaaaaa gttattagag 180
   gactgaaaat ataaaaatgg aacactgaaa gacacagagt ttttattttc agcactgcag 240
   ctctgatggt ccagctccac caggacacag atccccagat ccctaaaggt cagccatgca 300
   ccctgaacag ctcagaggga ggagccagac cagcagtgcc tcacaccttg ttctcttctg 360
   ctctagacag atggctccat aatgacagct tcataatggc agtgggtgag cccctggtgc 420
   acatcagggt cactcttctg ctgctctggt ttgggatgtt tttgtctatt tctggccact 480
   ctcaggccag gccctcccag tatttcactt ctccagaagt ggtgatccct ttgaaggtga 540
   tcagcagggg cagaggtgca aaggctcctg gatggctctc ctatagcctg cggtttgggg 600
   gacagagata cattgtccac atgagggtaa ataagctgtt gtttgctgca caccttcctg 660
   tgttcaccta cacagagcag catgccctgc tccaggatca gcccttcatc caggatgact 720
   gctactacca tggttatgtg gagggggtcc ctgagtcctt ggttgccctt agtacctgtt 780
   ctgggggctt tcttggaatg ctacagataa atgaccttgt ttatgaaatc aagccaatta 840
   gtgtttctgc cacatttgaa cacctagtat ataagataga cagtgatgat acacagtttc 900
   cacctatgag atgtgggtta acagaagaga aaatagcaca ccagatggag ttgcaattgt 960
   catataattt cactctgaag caaagttctt ttgtgggctg gtggacccat cagcggtttg 1020
   ttgagctggt agtggtcgtg gataatatta gatatctttt ctctcaaagt aatgcaacaa 1080
   cagtgcagca tgaagtattt aacgttgtca atatagtgga ttccttctat catcctttgg 1140
   aggttgatgt aattttgact ggaattgata tatggactgc atcaaatcca cttcctacca 1200
   gtggagacct agataatgtt ttagaggact tttctatttg gaagaattat aaccttaata 1260
   atcgactaca acatgatgtt gcacatcttt tcataaaaga cacacaaggc atgaagcttg 1320
   gtgttgccta tgttaaagga atatgccaga atccttttaa tactggagtt gatgtttttg 1380
   aagacaacag gttggtcgtt tttgcaatta ctttgggcca cgagcttggt cataatttgg 1440
   gtatgcaaca tgacacccag tggtgtgtgt gcgagctaca gtggtgcata atgcatgcct 1500
   atagaaaggt gacaactaaa tttagcaact gcagttatgc ccaatattgg gacagtacta 1560
   tcagtagtgg attatgtatt caaccgcctc catatccagg gaatatattt agactgaagt 1620
   actgtgggaa tctagtggtt gaagaagggg aggaatgtga ctgtggaacc atacggcagt 1680
   gtgcaaaaga tccctgttgt ctgttaaact gtactctaca tcctggggct gcttgtgctt 1740
   ttggaatatg ttgcaaagac tgcaaatttc tgccatcagg aactttatgt agacaacaag 1800
   ttggtgaatg tgaccttcca gagtggtgca atgggacatc ccatcaatgc ccagatgatg 1860
   tgtatgtgca ggacgggatc tcctgtaatg tgaatgcctt ctgctatgaa aagacgtgta 1920
   ataaccatga tatacaatgt aaagagattt ttggccaaga tgcaaggagt gcatctcaga 1980
   gttgctacca agaaatcaac acccaaggaa accgtttcgg tcactgtggt attgtaggca 2040
   caacatatgt aaaatgttgg acccctgata tcatgtgtgg gagggttcag tgtgaaaatg 2100
   tgggagtaat tcccaatctg atagagcatt ctacagtgca gcagtttcac ctcaatgaca 2160
   ccacttgctg gggcactgat tatcatttag ggatggctat acctgatatt ggtgaggtga 2220
   aagatggcac agtatgtggt ccagaaaaga tctgcatccg taagaagtgt gccagtatgg 2280
   ttcatctgtc acaagcctgt cagcctaaga cctgcaacat gaggggaatc tgcaacaaca 2340
   aacaacactg tcactgcaac catgaatggg cacccccata ctgcaaggac aaaggctatg 2400
   gaggtagtgc tgatagtggc ccacctccta agaacaacat ggaaggatta aatgtgatgg 2460
   gaaagttgcg ttacctgtca ctattgtgcc ttcttccttt ggttgctttt ttattatttt 2520
   gcttacatgt gctttttaag aaacgcacaa aaagtaaaga agatgaagaa ggataagaga 2580
   aatgggaaaa agaaggagac taaactttat acttcatttt taatatccaa ttttttaata 2640
   gaaaaatatg aagccatgtc tcactgttta aataaaactt catggacatt tcatgtcagg 2700
   attgcaagca ttagctatca cagcaaagga ttcctagcct attcttactt actctacagt 2760
   gtcttaagca atattaaagg ttccttttcc caaaaaaaaa aaaaaaa 2807

<210> 161
<211> 2406
<212> DNA
<213> Homo sapiens

<400> 161
atggcagtgg atgggaccct cgtgtacatc agggtcactc ttctgctgct ctggcttggg 60

gtatttttgt ctatttccgg ctactgtcag gctgggccct cccagcattt cacttccccg 120

gaagtggtga tccccttgaa ggtgatcagc aggggcagaa gtgcaaaggc tcctggatgg 180

ctctcctata gtctgcggtt tgggggccag aaacacgttg ttcatatgag ggtcaagaag 240

ctcttagttt ctagacacct cccagtgttc acctacacag atgagcgtgc actcctggag 300

gatcagctct tcatcccaga tgactgttac tatcatggtt acgtggaggg tgcccctgag 360

tctctggttg tgttcagtgc ttgttttggg ggctttcgag gagtattaaa aataagtggc 420

ctcacttatg aaattgaacc catcaggcac tctgccacat ttgaacacct ggtttacaaa 480

gtaaacagta atgagacaca attcccagct atgagatgtg gcttaacaga gaaggaagta 540

gcacgccaac agttggaatt tgaagaggct gagaactcag ctctggaacc aaaatctgct 600

ggtgactggt ggactcatgc atggtttctg gagctagttg ttgtggtgaa ccatgatttc 660

ttcatttact ctcaaagcaa catctcaaag gtgcaagagg atgtatttct tgttgtcaac 720

atagtggatt ccatgtatca gcagttaggt acttacataa ttttgattgg aattgaaatt 780

tggaatcaag gaaatgtttt cccaatgaca agcatagaac aggtcctgaa cgatttctct 840

caatggaaac aaatcagtct ttcccagcta cagcatgatg ctgcacatat gttcataaaa 900

aattcactta taagtatact tggcctagcc tatgttgcag gaatatgtcg tccacctatt 960

gattgtggag ttgataattt tcaaggagat acctggtctc tttttgccaa cactgtggcc 1020

catgagttag gtcatacgtt gggtatgcag catgatgaag aattctgttt ttgtggggaa 1080

agaggttgca tcatgaatac ttttagagtg ccagcagaga aattcaccaa ttgcagttac 1140

gctgatttta tgaagaccac cttaaaccag ggatcatgtc tgcataatcc tccaagattg 1200

ggggaaatct ttatgctaaa gcgctgtggg aatggtgtgg ttgaaagaga agagcagtgt 1260

gactgtggat ccgtacagca gtgtgaacaa gacgcctgtt gtctgttgaa ctgcactcta 1320

aggcctgggg ctgcctgtgc ttttgggctt tgttgcaaag actgcaagtt catgccatca 1380

ggggaactct gtagacaaga ggtcaatgaa tgtgaccttc cagaatggtg caatggaaca 1440

tctcatcagt gtccagaaga tagatatgtg caggacggga tcccctgtag tgacagtgcc 1500

tactgctatc aaaagaggtg taataaccat gaccagcatt gcagggagat ttttggtaaa 1560

gatgcaaaaa gtgcatctca gaattgctat aaagaaatca actctcaggg aaaccgtttt 1620

ggtcactgtg gtataaatgg cacaacatac ctaaaatgtc atatctctga tgtcttttgt 1680

gggagagttc aatgtgagaa tgtgagagac attcctcttc tccaagatca ttttactttg 1740

cagcacactc atatcaatgg tgtcacctgc tggggtattg actatcattt aaggatgaac 1800

atatctgaca ttggtgaagt gaaagatggt actgtgtgtg gcccaggaaa gatctgcatc 1860

cataagaagt gtgtcagtct gtctgtcttg tcacatgtct gccttcctga gacctgcaat 1920

atgaagggga tctgcaataa caaacatcac tgccactgtg gctatgggtg gtccccaccc 1980

tactgccagc acagaggcta tgggggcagt attgacagtg gcccagcatc tgcaaagaga 2040

ggagtttttt tgccgctgat tgtgattcct tctttgtctg ttttgacttt cctgtttact 2100

gtcgggcttc ttatgtatct acgacaatgt tctggtccca aagaaactaa ggctcattca 2160

tcaggttaag aaaatgtctc taacttaata ttccatgcat tagtacactt tagtctcttg 2220

gcagtagaaa cattagtaca tccctgaaac tgagcacatt tctgaccatt tccagaaagc 2280

tgcaaagatc ttcccttaca ttagtaccac aaacattgtc attaagttca agttattctt 2340

aacatgtttc tatctattgt ttattgtttt aagcagcaaa taaagctaca tccttccctc 2400

ccttta 2406

<210> 162
<211> 9334
<212> DNA
<213> Homo sapiens

<400> 162
actcgctcgc tccccccgcc agcggaagcg tccgcgaagc acaatgcagc actgagccgc 60
ggtggaggtt gcagcgccac ggccgccgca gcaccggccg gggctgggtg gaggtggccg 120
cggggacccc gggggcgcgg agcgagggaa acggactcgg cggcgccggc atgaggagct 180
gagcgtctcg ggcgaggcgg gctgacggca gcaccatgca ggcggcagtg gctgtgtccg 240
tgcccttctt gctgctctgt gtcctgggga cctgccctcc ggcgcgctgc ggccaggcag 300
gagacgcctc attgatggag ctagagaaga ggaaggaaaa ccgcttcgtg gagcgccaga 360
gcatcgtgcc actgcgcctc atctaccgct cgggcggcga agacgaaagt cggcacgacg 420
cgctcgacac gcgggtgcgg ggcgacctcg gtggcccgca gttgactcat gttgaccaag 480
caagcttcca ggttgatgcc tttggaacgt cattcattct cgatgtcgtg ctaaatcatg 540
atttgctgtc ctctgaatac atagagagac acattgaaca tggaggcaag actgtggaag 600
ttaaaggagg agagcactgt tactaccagg gccatatccg aggaaaccct gactcatttg 660
ttgcattgtc aacatgccac ggacttcatg ggatgttcta tgacgggaac cacacatatc 720
tcattgagcc agaagaaaat gacactactc aagaggattt ccattttcat tcagtttaca 780
aatccagact gtttgaattt tccttggatg atcttccatc tgaatttcag caagtaaaca 840
ttactccatc aaaatttatt ttgaagccaa gaccaaaaag gagtaaacgg cagcttcgtc 900
gatatcctcg taatgtagaa gaagaaacca aatacattga actgatgatt gtgaatgatc 960
accttatgtt taaaaaacat cggctttccg ttgtacatac caatacctat gcgaaatctg 1020
tggtgaacat ggcagattta atatataaag accaacttaa gaccaggata gtattggttg 1080
ctatggaaac ctgggcgact gacaacaagt ttgccatatc tgaaaatcca ttgatcaccc 1140
tacgtgagtt tatgaaatac aggagggatt ttatcaaaga gaaaagtgat gcagttcacc 1200
ttttttcggg aagtcaattt gagagtagcc ggagcggggc agcttatatt ggtgggattt 1260
gctcgttgct gaaaggagga ggcgtgaatg aatttgggaa aactgattta atggctgtta 1320
cacttgccca gtcattagcc cataatattg gtattatctc agacaaaaga aagttagcaa 1380
gtggtgaatg taaatgcgag gacacgtggt ccgggtgcat aatgggagac actggctatt 1440
atcttcctaa aaagttcacc cagtgtaata ttgaagagta tcatgacttc ctgaatagtg 1500
gaggtggtgc ctgccttttc aacaaacctt ctaagcttct tgatcctcct gagtgtggca 1560
atggcttcat tgaaactgga gaggagtgtg attgtggaac cccggccgaa tgtgtccttg 1620
aaggagcaga gtgttgtaag aaatgcacct tgactcaaga ctctcaatgc agtgacggtc 1680
tttgctgtaa aaagtgcaag tttcagccta tgggcactgt gtgccgagaa gcagtaaatg 1740
attgtgatat tcgtgaaacg tgctcaggaa attcaagcca gtgtgcccct aatattcata 1800
aaatggatgg atattcatgt gatggtgttc agggaatttg ctttggagga agatgcaaaa 1860

ccagagatag acaatgcaaa tacatttggg ggcaaaaggt gacagcatca gacaaatatt 1920

gctatgagaa actgaatatt gaagggacgg agaagggtaa ctgtgggaaa gacaaagaca 1980

catggataca gtgcaacaaa cgggatgtgc tttgtggtta ccttttgtgt accaatattg 2040

gcaatatccc aaggcttgga gaactcgatg gtgaaatcac atctacttta gttgtgcagc 2100

aaggaagaac attaaactgc agtggtgggc atgttaagct tgaagaagat gtagatcttg 2160

gctatgtgga agatgggaca ccttgtggtc cccaaatgat gtgcttagaa cacaggtgtc 2220

ttcctgtggc ttctttcaac tttagtactt gcttgagcag taaagaaggc actatttgct 2280

caggaaatgg agtttgcagt aatgagctga agtgtgtgtg taacagacac tggataggtt 2340

ctgattgcaa cacttacttc cctcacaatg atgatgcaaa gactggtatc actctgtctg 2400

gcaatggtgt tgctggcacc aatatcataa taggcataat tgctggcacc attttagtgc 2460

tggccctcat attaggaata actgcgtggg gttataaaaa ctatcgagaa cagagacagt 2520

taccccaggg agattatgta aaaaagcctg gagatggtga ctctttttat agcgacattc 2580

ctcccggagt cagcacaaac tcagcatcta gttctaagaa gaggtcaaat gggctctctc 2640

attcttggag tgaaaggatt ccagacacaa aacatatttc agacatctgt gaaaatgggc 2700

gacctcgaag taactcttgg caaggtaacc tgggaggcaa caaaaagaaa atcagaggca 2760

aaagatttag acctcggtct aattcaactg agactttatc tcctgccaag tctccttctt 2820

catcaactgg gtctattgcc tccagcagaa aataccctta cccaatgcct ccacttcctg 2880

atgaggacaa gaaagtgaac cgacaaagtg ccaggctatg ggagacatcc atttaagatc 2940

aactgtttac atgtgataca tcgaaaactg tttacttcaa cttttataga aacccaggct 3000

catggaatca ctgcaaatct atctgctctt cagacaatac gaagaccctc tgagatgcta 3060

cagaggagag gaagcggagt ttcacatctg gttaccattt tctttttgtc attggcttag 3120

gatttaacta accatgaaaa gaactactga aatattacac tataacatgg aacaataaag 3180

gtactggtat gttaatggat aatccgcatg acagataata tgtagaaata ttcataaagt 3240

taactcacat gacccaaatg tagcaagttt cctaaggtac aatagtggat tcagaacttg 3300

acgttctgag gcacatcctc actgtaaaca gtaatgctat atgcatgaag cttctgttta 3360

ttgttttcca tatttaagga aacaacatcc cataatagaa atgagcatgc agggctaagg 3420

catataggat ttttctgcag gactttaaag ctttgaaagg ccaatatccc ataggctaac 3480

tttaaacatg tatttttatt tttgttttgt tttttacttt tcatatttat attagcatac 3540

aaggacaatt gtatatatgt aacattttta aaattttaaa aaaatgcagc tgttacacac 3600

aagtgtattt tgccaaatgc ctaaaaattc cgtcacaatc acatcatcgt catccatcca 3660

gtgatcttca aagactataa gcaggtaatg taaatatagt ggtcaatgct gtaaatgtgt 3720

ctctccataa ttcgtatttg tccaaaacat gtgatatccc tttaacctgt gcacagtctg 3780

gaggcagttt tattgagtga gtagttgagt gagagaacaa aatgcccagc aaaacttctg 3840

gcctccacag tttgctgatg caggagccca ctctgctgcc gatgggctgc tccctggaaa 3900

tcatctatcc catccgtcca tcccatctca tcccagtgag ccagcccctc agcagtgtgt 3960

gctgctgaga ttaagcaagt gcaccaaaga cacatgagac aacgtactgc cagtgagctg 4020

gtagcttctg gtttgccaat actctaaccc tttgtggcct gaagttttgt tccttggcaa 4080

aactttgtag tcacccttat ctgtgaaatg aatgacttgt tttaacaaaa acaactttga 4140

cactgttgat taagaatatt tgtcatcact gggatgtgaa tcccaacaag tcattctgca 4200

ctgactctga agcaagatga cggaagattc tactgtttga gttgagcagc ttcaaccatt 4260

cataaagggt taagttagat ctaaaccaca gagatgcagc atctgcgaag ttacactaac 4320

agctggtggg ggaaagaatc tgatgctttg aaacatatat ttttaatcca taggaaaata 4380

agattctatt ttaaaagacc cttctttaaa accaaagttt gatagctgtt ataatggcag 4440

atctgtcatt ccaaaagaaa gctagctacc ctagtgaggc tggagattga tgggctcaat 4500

cacatggtct gctctgaagt atacttctga tttcctcatt tgctgtgtgt tcttaagagg 4560

aatggaatct gcagacaggt tttcagatat actctcttaa atttgatcct ctcccatagt 4620

atccatggac aaaatccata tcatcttcat atgtaatcaa tttgattaca tatcatgccc 4680

aaactgaagg gccgagttag aaggcagcat tttatactgt gtgtttatgt cttctgtaat 4740

ggacctctct tttgaaagtc tttgaaatgt ttggaaagca atttaatgat ccaaaattac 4800

ataatgtaaa acagcatttt tcatgacagg aagggatgtg attgttgata tgaccataaa 4860

gcgatccatc aggccaaggt ttgggtgagt gttgggcagt gttgccacat agctcaagcc 4920

aagaacagaa gggcagacag attgaaagat gtagcaaata aagtgacttt ttaccaagat 4980

cactgtaacg tgcactgaat gtttcctggt actgagtgta caaaaggcta atgctataag 5040

gtttattttg actataaaag agtttataga tttgaatata aggtgcctca tgtaaatatg 5100

cttcctagtt acaggtacac aaactctaat ttgaaagaat ccacaaatgg ctagctgaga 5160

agttaacaga tctctatcat tcgtctgtgt gtctttcttc cctcacctat ttttacatga 5220

aattattatt tcaaatgaaa atcatgatca tgtgcctttt gggagacatg gtttctctag 5280

aggtatatag tttgtaatta cccgccttgg taataacctt cctgtggaac tgactaaaaa 5340

tttttgtttt atttgtagag attatgattt gaggtataca gtaactttaa tgttctattc 5400

ttggaatcta ctctatctcc catgtttaaa cattcttaga gccatttgtg ggattcattg 5460

acaaatttat agtctcaagg gagagtttca agaagtatct atatgtatac tcatggttgg 5520

ggttctagag gcattacatc taaatattta tgacttttca tccaccgagg taggtagcat 5580

taaatataca agcacagcat tatttctctt taaatatcta ttttggagag aaactttgag 5640

gccgtatcac agtttatatc atgcaactaa tatttatatt tcccaatcca ctacaaaatc 5700

attgctaagc agaagagagc agttatttgg cctttatgtt ccattgtatg cctttttccc 5760

ctcttccagt tttatgcatt gataagacta cagattggca ccctgctgcc tgagaagttc 5820

catttatcca tagtcattgc agtgcagtaa accctgattt aaaggaacat ttaagtctct 5880

ccagtaagtg tgaacaggaa ggcatgggat agaagagagt ccttatggag tggactggcc 5940

acatgaactg tcctccagcc actctttatt tagatgggaa gtggcatggt cctctgaacg 6000

ctgcaggctg gaaatgatcc ccagggcaca tgcaatctgt ggaaaggaat acatcggaca 6060

gtgcaacatg gtgagggctt tcaatttgct gtggttgctg tatttttaac aagacacccg 6120

gagcttcgat taggaataac ctatcattag agttgttcct ttaaaaaatg ttgatgaata 6180

aagcaaatgt gggtttaaaa tgccaactta tttttcactt tgatattggt gctttattaa 6240

gtgataccat agtttctctc tcttttccct gttactttaa aaagaatttt aaaattgtgt 6300

ttgtatataa ggtcagtctg gcatagaatt tctgaaagta acattaatag gatttctaaa 6360

gaagttgtca gaaagacaaa attggatatt ctctgcactt tcagagaagg actatttatg 6420

tttgaatttc ataacaggct tacatttctt ttctattgat tcccttgcat atctacataa 6480

tttaatcctg tctattgtcc cagatctctt gtggctactt tacctgttac cttttttggg 6540

attttgttct ttatgtttta cagttacctt cggaccagaa gatatatggt atttttattc 6600

acttttttca gtgttttaaa ataaggtttg ttcatttatt cattttatcc attcatttcc 6660

tattgtcgta tagttctaac catatatata gtactttaaa tattttctta tgttattatt 6720

taacacaata ataaattctt atctaaagaa aagattattt tactttattt tacttaaagg 6780

aaagagaatt tattggcagg atattgagta gcttgtagag tatatggaag gggagagcca 6840

agttaggaac tggcaggagc gaggagggac aagacagctg cctgcacagt ctgataggac 6900

acatctggct gctgccactg gatgcccaca tgggcagcgt gttgtaaata tcaccctacc 6960

agttggtaag ttggcatgaa ttttccatga aacacttgac tgccatttgc agcatcatat 7020

ctacttttta tgcacctaat tatatttttc ctttcacttt tatgtttttt tctaaaactg 7080

ccacctaaat acctttccac tccgttaaat gtatggagtc aagataaatt tctcattctt 7140

gctctacagt ttaagatgta taagaagtaa acacttccgt ggaatagggc ttaatttggg 7200

gctcaaatgt tttgttttcc tcctgagatg tttttgaatg gataagacta ctctggtttt 7260

ggatgcagca acttttctcc tggcatggaa gaactaagga ctttgataaa cagtttccca 7320

ttgatagtat aatataatga ggggcccagt ctgctcttgt gaaaattggc tgagcggcag 7380

cagcccatcc cagctgtgtg tgagtagccc cgctgttaaa agtacacctg ctgggaattg 7440

ttggcacatg ctcatagcat atgctctgtg ggggaggtgc ttttaattcc aagaggagct 7500

tttctcccac atctgcggat gcagaacaaa gatgcagcag gactttagaa aacaggagtg 7560

tttctactga cattttattc attgctttgt tagtcccgca aatggagcaa cttctggttg 7620

tttgtaagca gaaaaacaaa acattcaaaa ccaaaaacct aattagtact tttgtccttt 7680

atatccctag gatgctctag ggagtagagg tggcaggcat ctttatcttc acccactggc 7740

acattagcag cttatgcaac ttaggaagtc actgggagat gagtcctaaa agcctgaatt 7800

tcaggatggg gaggagataa gaagcagaga actttgggag accatggggc atttagtcct 7860

ggcatagaca cagaatggat attcacagga agattcctga ggccagatat gcaggagagc 7920

acacattgtc acaaggggtg tggtgggaag agaggtgctg gatgcctaag acagacttct 7980

gtgcttggca gattgactga gtgttgactc tgaatacttt gtggtccagt ccataatagg 8040

ccagtcactc ctgcctgatg catccctgtt gcactgttca taaaacctgt atcatatggg 8100

gaagcttcaa ggctggcagg cttttcatca agtaatcatc attccatgtt cctacgtatc 8160

cttgaaatca aaaccaggca atatgtgtgt ttttggtttt gttttacaaa aaggcataca 8220

atgagacgaa ttcttaagat aatgcacata cagaatcatc aataaagttt caaagagttt 8280

atgaagaaaa ggtattttcc tttcttgtaa aaaatgttat atatatataa tattctgatc 8340

tgcattcacc ttagggctta gcctcttatt ttatgaattt tcaggctgtg atttgtgttg 8400

gatggctaag ctctaaaata atgcaaatag ccccaatctt taaatatagc ggtgctaagt 8460

tgaacaagta acacaataca gaaaatgctg atatgaatac ttagtaataa tacaaaagtt 8520

cctgctaaat tatatatgtg tatcactgcc tgactagaaa ccccactttt cttttctaat 8580

ccagcacaaa atcaaactct gattctacaa ccagtctttt taaagggcaa aaatgactca 8640

acacctttgt tttgtatgga aaactttttt ttttacacta actgcaaaac tgctttaaaa 8700

aaaggcttat tcaggataga taagcatgta ctcctttttt aacttgctgg aagacttgcc 8760

cctccaaact agcaccccca aaagacaact tctttcagaa acggggtgtt ttacctaaac 8820

atagtagctt acatgttagc cagcagtagg tcggcactag tgttttccac ggttatcacc 8880

tttgacaggt gatgtgcatc tatagatagt ggaagccacc ccatgaggag gtgttaatag 8940

cagcatggtt tcacttttgg taatcaggta atcatgtgta tatacttaga ttcgcattat 9000

tttaacattt ctctgctact ctgcacttca ggttcgttaa gctattttaa taattactgg 9060

ggttatggca aacaccaatg gaaatgtata tggcaactgc tttcctgagc aagtgtgatt 9120

tgttttatgg ctgttcaagt tataaaattg ttcttacatt gtaggtaaac aaaatcttga 9180

tgtttttaaa ggtcactgta acttaaggtt caaatttctg gcacagtttt attagtattc 9240

acttcggaag ctaataagat accatggttt tctatgttac tcccattgta acattagtaa 9300

agtgactttc aataaaagat ttatgttatt ttga 9334

<210> 163
<211> 3059
<212> DNA
<213> Homo sapiens

<400> 163
tcctctgcgt cccgccccgg gagtggctgc gaggctaggc gagccgggaa agggggcgcc 60

gcccagcccc gagccccgcg ccccgtgccc cgagcccgga gccccctgcc cgccgcggca 120

ccatgcgcgc cgagccggcg tgaccggctc cgcccgcggc cgccccgcag ctagcccggc 180

gctctcgccg gccacacgga gcggcgcccg ggagctatga gccatgaagc cgcccggcag 240

cagctcgcgg cagccgcccc tggcgggctg cagccttgcc ggcgcttcct gcggccccca 300

acgcggcccc gccggctcgg tgcctgccag cgccccggcc cgcacgccgc cctgccgcct 360

gcttctcgtc cttctcctgc tgcctccgct cgccgcctcg tcccggcccc gcgcctgggg 420

ggctgctgcg cccagcgctc cgcattggaa tgaaactgca gaaaaaaatt tgggagtcct 480

ggcagatgaa gacaatacat tgcaacagaa tagcagcagt aatatcagtt acagcaatgc 540

aatgcagaaa gaaatcacac tgccttcaag actcatatat tacatcaacc aagactcgga 600

aagcccttat cacgttcttg acacaaaggc aagacaccag caaaaacata ataaggctgt 660

ccatctggcc caggcaagct tccagattga agccttcggc tccaaattca ttcttgacct 720

catactgaac aatggtttgt tgtcttctga ttatgtggag attcactacg aaaatgggaa 780

accacagtac tctaagggtg gagagcactg ttactaccat ggaagcatca gaggcgtcaa 840

agactccaag gtggctctgt caacctgcaa tggacttcat ggcatgtttg aagatgatac 900

cttcgtgtat atgatagagc cactagagct ggttcatgat gagaaaagca caggtcgacc 960

acatataatc cagaaaacct tggcaggaca gtattctaag caaatgaaga atctcactat 1020

ggaaagaggt gaccagtggc cctttctctc tgaattacag tggttgaaaa gaaggaagag 1080

agcagtgaat ccatcacgtg gtatatttga agaaatgaaa tatttggaac ttatgattgt 1140

taatgatcac aaaacgtata agaagcatcg ctcttctcat gcacatacca acaactttgc 1200

aaagtccgtg gtcaaccttg tggattctat ttacaaggag cagctcaaca ccagggttgt 1260

cctggtggct gtagagacct ggactgagaa ggatcagatt gacatcacca ccaaccctgt 1320

gcagatgctc catgagttct caaaataccg gcagcgcatt aagcagcatg ctgatgctgt 1380

gcacctcatc tcgcgggtga catttcacta taagagaagc agtctgagtt actttggagg 1440

tgtctgttct cgcacaagag gagttggtgt gaatgagtat ggtcttccaa tggcagtggc 1500

acaagtatta tcgcagagcc tggctcaaaa ccttggaatc caatgggaac cttctagcag 1560

aaagccaaaa tgtgactgca cagaatcctg gggtggctgc atcatggagg aaacaggggt 1620

gtcccattct cgaaaatttt caaagtgcag cattttggag tatagagact ttttacagag 1680

aggaggtgga gcctgccttt tcaacaggcc aacaaagcta tttgagccca cggaatgtgg 1740

aaatggatac gtggaagctg gggaggagtg tgattgtggt tttcatgtgg aatgctatgg 1800

attatgctgt aagaaatgtt ccctctccaa cggggctcac tgcagcgacg ggccctgctg 1860

taacaatacc tcatgtcttt ttcagccacg agggtatgaa tgccgggatg ctgtgaacga 1920

gtgtgatatt actgaatatt gtactggaga ctctggtcag tgcccaccaa atcttcataa 1980

gcaagacgga tatgcatgca atcaaaatca gggccgctgc tacaatggcg agtgcaagac 2040

cagagacaac cagtgtcagt acatctgggg aacaaaggct gcagggtctg acaagttctg 2100

ctatgaaaag ctgaatacag aaggcactga gaagggaaac tgcgggaagg atggagaccg 2160

gtggattcag tgcagcaaac atgatgtgtt ctgtggattc ttactctgta ccaatcttac 2220

tcgagctcca cgtattggtc aacttcaggg tgagatcatt ccaacttcct tctaccatca 2280

aggccgggtg attgactgca gtggtgccca tgtagtttta gatgatgata cggatgtggg 2340

ctatgtagaa gatggaacgc catgtggccc gtctatgatg tgtttagatc ggaagtgcct 2400

acaaattcaa gccctaaata tgagcagctg tccactcgat tccaagggta aagtctgttc 2460

gggccatggg gtgtgtagta atgaagccac ctgcatttgt gatttcacct gggcagggac 2520

agattgcagt atccgggatc cagttaggaa ccttcacccc cccaaggatg aaggacccaa 2580

gggtcctagt gccaccaatc tcataatagg ctccatcgct ggtgccatcc tggtagcagc 2640

tattgtcctt gggggcacag gctggggatt taaaaatgtc aagaagagaa ggttcgatcc 2700

tactcagcaa ggccccatct gaatcagctg cgctggatgg acaccgcctt gcactgttgg 2760

attctgggta tgacatactc gcagcagtgt tactggaact attaagtttg taaacaaaac 2820

ctttgggtgg taatgactac ggagctaaag ttggggtgac aaggatgggg taaaagaaaa 2880

ctgtctcttt tggaaataat gtcaaagaac acctttcacc acctgtcagt aaacggggga 2940

gggggcaaaa gaccatgcta taaaaagaac tgttccagaa tctttttttt ccctaatgga 3000

cgaaggaaca acacacacac aaaaattaaa tgcaataaag gaatcattaa aaaaaaaaa 3059

<210> 164
<211> 3220
<212> DNA
<213> Homo sapiens

<400> 164
tcactggaga ggaggcaggg acagacccag cagcacccac ctgagcgaga agagcagaca 60

ccgtgctcct ggaatcaccc agcatgttgc aaggtctcct gccagtcagt ctcctcctct 120

ctgttgcagt aagtgctata aaagaactcc ctggggtgaa gaagtatgaa gtggtttatc 180

ctataagact tcatccactg cataaaagag aggccaaaga gccagagcaa caggaacaat 240

ttgaaactga attaaagtat aaaatgacaa ttaatggaaa aattgcagtg ctttatttga 300

aaaaaaacaa gaacctcctt gcaccaggct acacggaaac atattataat tccactggaa 360

aggagatcac cacaagccca caaattatgg atgattgtta ttatcaagga catattctta 420

atgaaaaggt ttctgacgct agcatcagca catgtagggg tctaaggggc tacttcagtc 480

agggggatca aagatacttt attgaacctt taagccccat acatcgggat ggacaggagc 540

atgcactctt caagtataac cctgatgaaa agaattatga cagcacctgt gggatggatg 600

gtgtgttgtg ggcccacgat ttgcagcaga acattgccct acctgccacc aaactagtaa 660

aattgaaaga caggaaggtt caggaacatg agaaatacat agaatattat ttggtcctgg 720

ataatggtga gtttaaaagg tacaatgaga atcaagatga gatcagaaag agggtatttg 780

agatggctaa ttatgtcaac atgctttata aaaagctcaa tactcatgtg gccttagttg 840

gtatggaaat ctggactgac aaggataaga taaagataac cccaaatgca agcttcacct 900

tggagaattt ttctaaatgg agggggagtg ttctctcaag aagaaagcgt catgatattg 960

ctcagttaat cacagcaaca gaacttgctg gaacgactgt gggtcttgca tttatgtcta 1020

caatgtgttc tccttattct gttggcgttg ttcaggacca cagcgataat cttcttagag 1080

ttgcagggac aatggcacat gaaatgggcc acaactttgg aatgtttcat gacgactatt 1140

cttgcaagtg tccttctaca atatgtgtga tggacaaagc actgagcttc tatataccca 1200

cagacttcag ttcctgcagc cgtctcagct atgacaagtt ttttgaagat aaattatcaa 1260

attgcctctt taatgctcca ttgcctacag atatcatatc cactccaatt tgtgggaacc 1320

agttggtgga aatgggagag gactgtgatt gtgggacatc tgaggaatgt accaatattt 1380

gctgtgatgc taagacatgt aaaatcaaag caacttttca atgtgcatta ggagaatgtt 1440

gtgaaaaatg ccaatttaaa aaggctggga tggtgtgcag accagcaaaa gatgagtgcg 1500

acctgcctga aatgtgtaat ggtaaatctg gtaattgtcc tgatgataga ttccaagtca 1560

atggcttccc ttgccatcac gggaagggcc actgcttgat ggggacatgc cccacactgc 1620

aggagcagtg cacagagctg tggggaccag gaactgaggt tgcagataag tcatgttaca 1680

acaggaatga aggtgggtca aagtacgggt actgtcgcag agtggatgac acactcattc 1740

cctgcaaagc aaatgatacc atgtgtggga agttgttctg tcaaggtggg tcggataatt 1800

tgccctggaa aggacggata gtgactttcc tgacatgtaa aacatttgat cctgaagaca 1860

caagtcaaga aataggcatg gtggccaatg gaactaagtg tggcgataac aaggtttgca 1920

ttaatgcaga atgtgtggat attgagaaag cctacaaatc aaccaattgc tcatctaagt 1980

gcaaaggaca tgctgtgtgt gaccatgagc tccagtgtca atgtgaggaa ggatggatcc 2040

ctcccgactg cgatgactcc tcagtggtct tccacttctc cattgtggtt ggggtgctgt 2100

tcccaatggc ggtcattttt gtggtggttg ctatggtaat ccggcaccag agctccagag 2160

aaaagcagaa gaaagatcag aggccactat ctaccactgg caccaggcca cacaaacaga 2220

agaggaaacc ccagatggta aaggctgttc aaccccaaga gatgagtcag atgaagcccc 2280

atgtgtatga tctgccagta gaaggcaatg agcccccagc ctcttttcat aaagacacaa 2340

acgcacttcc ccctactgtt ttcaaggata atccagtgtc tacacctaag gactcaaatc 2400

caaaagcatg aagcaacagc taagcaagaa ctaatggcta aattatcaac ttggaaaact 2460

ggaaaatctg gatggcagag aaatatacta tctatctcac cagtatttgc tctcgactca 2520

agaaggttaa cattttctga ttcatgttag actttgaaga gactaaagaa aattttcaag 2580

aggaacatat gcctgagaac ctttgcatga atttaaaatt tcaattatcc attcttataa 2640

gaaggaagat gattgtaaag aaatatctcc gaagttaaaa tctgtaatag gaattgattc 2700

attctctaat gaaaacaaaa cataaaaaca tcacactaat cttggaggaa taagaaaaat 2760

tgtacatcca ttaaatgtac aattgattgc aacatcttga ttgttttaac cattaacttg 2820

tcaaattaca atcacagtta agaaaatgat gtaaaattct gttttgtgga tctctttcct 2880

agattagctt ctgaaatcat tattagctat atcatttgag gttttctaca atttggtata 2940

actaagaatt taaaaatgtt ttatcatata tatttgtata attaattact ggcatggtta 3000

aagtggtttt cactttttaa atggagaaaa tttcagttaa attaatagga taaaccaggt 3060

tgcgaactgg tgacctgtag gccatgtttg cactgcaaat atatttggtc tgaatgatat 3120

tgatattgga cacatagtac ttttacatgt tttgaatgta ttgctaatat ttaaaaattg 3180

agagatcttg cataaacaat agattcccag ctttgtcaga 3220

<210> 165
<211> 3325
<212> DNA
<213> Homo sapiens

<400> 165
gtgacatcac atccactaac caaatggggt ggtgtgagta tctcctataa aataaaagct 60

ctcctgatgg cctgttcctg cacatttcct gaggacgccc tcgatcacaa gcagcttgct 120

ggatgaataa aggaaagaag gtttatatac actaataata gaaatgtttt gggatggttc 180

atgatttcga aaagcttcag agaaaataaa gatgcttaat gttagagtac aaaacattct 240

ccctgcagtc tcacgaactg tgaacaaaaa ctgaagtgaa aactcatagt gcataactcg 300

tcaatactcc tgtgatcgta taaccatcag caagaaaaca aatttgattg agcccccatc 360

cagtcctctt tgcgtggaat cagacctctt ttgcagtgga aaggagcaga gaatgtgact 420

ttcctgaaca acaggagcaa gaatcaatgc cagattctct tctctaaaaa taatcaattt 480

gttactacag tgctgcagct ctgatggttc aactctgcca aaagatggat ctttaatgat 540

tagcactaca cactgaccaa ctcagaagaa ggagccacac cacctgtgac tccagccctg 600

acttctgctc tggaccagtg tttccataac agggacttca aaatcactgt gatttgaagc 660

ctttttgaac atgaagatgt tactcctgct gcattgcctt ggggtgtttc tgtcctgttc 720

tggacacatc caggatgagc acccccaata tcacagccct ccggatgtgg tgattcctgt 780

gaggataact ggcaccacca gaggcatgac acctccaggc tggctctcct atatcctgcc 840

ctttggaggc cagaaacaca ttatccacat aaaggtcaag aagcttttgt tttccaaaca 900

cctccctgtg ttcacctaca cagaccaggg tgctatcctt gaggaccagc catttgtcca 960

gaataactgc tactatcatg gttatgtgga aggggaccca gaatccctgg tttccctcag 1020

tacctgtttt gggggttttc aaggaatatt acagataaat gactttgctt atgaaatcaa 1080

gcccctagca ttttctacca cgtttgaaca tctggtatac aagatggaca gtgaggagaa 1140

acaattttca accatgagat ccggatttat gcaaaatgaa ataacatgcc gaatggaatt 1200

tgaagaaatt gataattcca ctcagaagca aagttcttat gtgggctggt ggatccattt 1260

taggattgtt gaaattgtag tcgtcattga taattatctg tacattcgtt atgaaaggaa 1320

cgactcaaag ttgctggagg atctatatgt tattgttaat atagtggatt ccattttgga 1380

tgtcattggt gttaaggtgt tattatttgg tttggagatc tggaccaata aaaacctcat 1440

tgtagtagat gatgtaagga aatctgtgca cctgtattgc aagtggaagt cggagaacat 1500

tacgccccgg atgcaacatg acacctcaca tcttttcaca actctaggat taagagggtt 1560

aagtggcata ggagctttta gaggaatgtg tacaccacac cgtagttgtg caattgttac 1620

tttcatgaac aaaactttgg gcactttttc aattgcagtg gctcatcatc taggtcataa 1680

tttgggcatg aaccatgatg aggatacatg tcgttgttca caacctagat gcataatgca 1740

tgaaggcaac ccaccaataa ctaaatttag caattgtagt tatggtgatt tttgggaata 1800

tactgtagag aggacaaagt gtttgcttga aacagtacac acaaaggaca tctttaatgt 1860

gaagcgctgt gggaatggtg ttgttgaaga aggagaagag tgtgactgtg gacctttaaa 1920

gcattgtgca aaagatccct gctgtctgtc aaattgcact ctgactgatg gttctacttg 1980

tgcttttggg ctttgttgca aagactgcaa gttcctacca tcagggaaag tgtgtagaaa 2040

ggaggtcaat gaatgtgatc ttccagagtg gtgcaatggt acttcccata agtgcccaga 2100

tgacttttat gtggaagatg gaattccctg taaggagagg ggctactgct atgaaaagag 2160

ctgtcatgac cgcaatgaac agtgtaggag gatttttggt gcaggcgcaa atactgcaag 2220

tgagacttgc tacaaagaat tgaacacctt aggtgaccgt gttggtcact gtggtatcaa 2280

aaatgctaca tatataaagt gtaatatctc agatgtccag tgtggaagaa ttcagtgtga 2340

gaatgtgaca gaaattccca atatgagtga tcatactact gtgcattggg ctcgcttcaa 2400

tgacataatg tgctggagta ctgattacca tttggggatg aagggacctg atattggtga 2460

agtgaaagat ggaacagagt gtgggataga tcatatatgc atccacaggc actgtgtcca 2520

tataaccatc ttgaatagta attgctcacc tgcattttgt aacaagaggg gcatctgcaa 2580

caataaacat cactgccatt gcaattatct gtgggaccct cccaactgcc tgataaaagg 2640

ctatggaggt agtgttgaca gtggcccacc ccctaagaga aagaagaaaa agaagttctg 2700

ttatctgtgt atattgttgc ttattgtttt gtttatttta ttatgttgtc tttatcgact 2760

ttgtaaaaaa agtaaaccaa taaaaaagca gcaagatgtt caaactccat ctgcaaaaga 2820

agaggaaaaa attcagcgtc gacctcatga gttacctccc cagagtcaac cttgggtgat 2880

gccttcccag agtcaacctc ctgtgacgcc ttcccagagt catcctcagg tgatgccttc 2940

ccagagtcaa cctcctgtga caccctccca gagtcaacct cgggtgatgc cttctcagag 3000

tcaacctcct gtgatgcctt cccagagtca tcctcagttg acgccttccc agagtcaacc 3060

tcctgtgaca ccctcccaga ggcaacctca gttgatgcct tcccagagtc aacctcctgt 3120

gacgccctcc tagagccaac ctcagttgat gccttcccag agtcaacctc ctgtgacgcc 3180

ctcccagagc caacctcggg tgacaccctc ccagagtcaa cctcatgtga caccttaccg 3240

gagtaaaagt ggtaaacaaa agcaatcagt accaattcca aaaactgtat ccagaaaagg 3300

tacattaaaa aaataattcc tagta 3325

<210> 166
<211> 2972
<212> DNA
<213> Homo sapiens

<400> 166
gagccctggg aggctacggg ctcccccgga aaccctgcca ggggagccgg gttttgagct 60

caggcgcctc tagcggcggc ccccagaaat ctgactcgcg aggccagagt tgcagggact 120

gaatagcaaa ctgaggctga gtagggaaca gaccatgagg tcagtgcaga tcttcctctc 180

ccaatgccgt ttgctccttc tactagttcc gacaatgctc cttaagtctc ttggcgaaga 240

tgtaattttt caccctgaag gggagtttga ctcgtatgaa gtcaccattc ctgagaagct 300

gagcttccgg ggagaggtgc agggtgtggt cagtcccgtg tcctacctac tgcagttaaa 360

aggcaagaag cacgtcctcc atttgtggcc caagagactt ctgttgcccc gacatctgcg 420

cgttttctcc ttcacagaac atggggaact gctggaggat catccttaca taccaaagga 480

ctgcaactac atgggctccg tgaaagagtc tctggactct aaagctacta taagcacatg 540

catggggggt ctccgaggtg tatttaacat tgatgccaaa cattaccaaa ttgagcccct 600

caaggcctct cccagttttg aacatgtcgt ctatctcctg aagaaagagc agtttgggaa 660

tcaggtttgt ggcttaagtg atgatgaaat agaatggcag atggcccctt atgagaataa 720

ggcgaggcta agggactttc ctggatccta taaacaccca aagtacttgg aattgatcct 780

actctttgat caaagtaggt ataggtttgt gaacaacaat ctttctcaag tcatacatga 840

tgccattctt ttgactggga ttatggacac ctactttcaa gatgttcgta tgaggataca 900

cttaaaggct cttgaagtat ggacagattt taacaaaata cgcgttggat atccagagtt 960

agctgaagtt ttaggcagat ttgtaatata taaaaaaagt gtattaaatg ctcgcctgtc 1020

atcagattgg gcacatttat atcttcaaag aaaatataat gatgctcttg catggtcgtt 1080

tggaaaagtg tgttctctag aatatgctgg atcagtgagt actttactag atacaaatat 1140

ccttgcccct gctacctggt ctgctcatga gctgggtcat gctgtaggaa tgtcacatga 1200

tgaacaatac tgccaatgta ggggtaggct taattgcatc atgggctcag gacgcactgg 1260

gtttagcaat tgcagttata tctctttttt taaacatatc tcttcgggag caacatgtct 1320

aaataatatc ccaggactag gttatgtgct taagagatgt ggaaacaaaa ttgtggagga 1380

caatgaggaa tgtgactgtg gttccacaga ggagtgtcag aaagatcggt gttgccaatc 1440

aaattgtaag ttgcaaccag gtgccaactg tagcattgga ctttgctgtc atgattgtcg 1500

gtttcgtcca tctggatacg tgtgtaggca ggaaggaaat gaatgtgacc ttgcagagta 1560

ctgcgacggg aattcaagtt cctgcccaaa tgacgtttat aagcaggatg gaaccccttg 1620

caagtatgaa ggccgttgtt tcaggaaggg gtgcagatcc agatatatgc agtgccaaag 1680

catttttgga cctgatgcca tggaggctcc tagtgagtgc tatgatgcag ttaacttaat 1740

aggtgatcaa tttggtaact gtgagattac aggaattcga aattttaaaa agtgtgaaag 1800

tgcaaattca atatgtggca ggctacagtg tataaatgtt gaaaccatcc ctgatttgcc 1860

agagcatacg actataattt ctactcattt acaggcagaa aatctcatgt gctggggcac 1920

aggctatcat ctatccatga aacccatggg aatacctgac ctaggtatga taaatgatgg 1980

cacctcctgt ggagaaggcc gggtatgttt taaaaaaaat tgcgtcaata gctcagtcct 2040

gcagtttgac tgtttgcctg agaaatgcaa tacccggggt gtttgcaaca acagaaaaaa 2100

ctgccactgc atgtatgggt gggcacctcc attctgtgag gaagtggggt atggaggaag 2160

cattgacagt gggcctccag gactgctcag aggggcgatt ccctcgtcaa tttgggttgt 2220

gtccatcata atgtttcgcc ttattttatt aatcctttca gtggtttttg tgtttttccg 2280

gcaagtgata ggaaaccact taaaacccaa acaggaaaaa atgccactat ccaaagcaaa 2340

aactgaacag gaagaatcta aaacaaaaac tgtacaggaa gaatctaaaa caaaaactgg 2400

acaggaagaa tctgaagcaa aaactggaca ggaagaatct aaagcaaaaa ctggacagga 2460

agaatctaaa gcaaacattg aaagtaaacg acccaaagca aagagtgtca agaaacaaaa 2520

aaagtaaccg ggcaatccat actcattcag taacacaggc tcatttattt aaccagctaa 2580

tcatttatcc aaaggctttc cattcttctc ccaatatttt tttactttaa tttttcccac 2640

aagttttgat cagcaaataa acagcattct tgttttggaa acaaaccagt gcattctact 2700

tctcgagtgt tcacttgccc ctcagtttgt gaccaagttg tgggtattct gagaatcatg 2760

ctctaatggc ttttccatac actaaagctc tgaaagtagt gagtttccta gaattaccat 2820

gctattcaag gtatagtctt gtttctcaga atcccaggga acaaagcaat tgtctgcctc 2880

tccctctgcc tccctcaact tgatccatgt gaacatccgc acctgcaccc atgctgatca 2940

ataaatgccg acaacacaaa aaaaaaaaaa aa 2972

<210> 167
<211> 2742
<212> DNA
<213> Homo sapiens

<400> 167
tggctccagc aaccacgcgg ctggggtgcg ccgggaaggg agctggatgt tttagcctcg 60

gggcgcacgc tgcgggccct tcgtgttccg gacgctaaac accgagagca ccccgtctcc 120

ggggcctccg gagaacgctg tcccatgaac gtgcggggag cggcccccgg cgtccgcgcg 180

tccccgcgtc cctggcaatt cccgacttcc caacggcttc ccgctggcag ccccgaagcc 240

gcaccatgtt ccgcctctgg ttgctgctgg ccgggctctg cggcctcctg gcgtcaagac 300

ccggttttca aaattcactt ctacagatcg taattccaga gaaaatccaa acaaatacaa 360

atgacagttc agaaatagaa tatgaacaaa tatcctatat tattccaata gatgagaaac 420

tgtacactgt gcaccttaaa caaagatatt ttttagcaga taattttatg atctatttgt 480

acaatcaagg atctatgaat acttattctt cagatattca gactcaatgc tactatcaag 540

gaaatattga aggatatcca gattccatgg tcacactcag cacgtgctct ggactaagag 600

gaatactgca atttgaaaat gtttcttatg gaattgagcc tctggaatct gcagttgaat 660

ttcagcatgt tctttacaaa ttaaagaatg aagacaatga tattgcaatt tttattgaca 720

gaagcctgaa agaacaacca atggatgaca acatttttat aagtgaaaaa tcagaaccag 780

ctgttccaga tttatttcct ctttatctag aaatgcatat tgtggtggac aaaactttgt 840

atgattactg gggctctgat agcatgatag taacaaataa agtcatcgaa attgttggcc 900

ttgcaaattc aatgttcacc caatttaaag ttactattgt gctgtcatca ttggagttat 960

ggtcagatga aaataagatt tctacagttg gtgaggcaga tgaattattg caaaaatttt 1020

tagaatggaa acaatcttat cttaacctaa ggcctcatga tattgcatat ctactaattt 1080

atatggatta tcctcgttat ttgggagcag tgtttcctgg aacaatgtgt attactcgtt 1140

attctgcagg agttgcattg taccccaagg agataactct ggaggcattt gcagttattg 1200

tcacccagat gctggcactc agtctgggaa tatcatatga cgacccaaag aaatgtcaat 1260

gttcagaatc cacctgtata atgaatccag aagttgtgca atccaatggt gtgaagactt 1320

ttagcagttg cagtttgagg agctttcaaa atttcatttc aaatgtgggt gtcaaatgtc 1380

ttcagaataa gccacaaatg caaaaaaaat ctccgaaacc agtctgtggc aatggcagat 1440

tggagggaaa tgaaatctgt gattgtggta ctgaggctca atgtggacct gcaagctgtt 1500

gtgattttcg aacttgtgta ctgaaagacg gagcaaaatg ttataaagga ctgtgctgca 1560

aagactgtca aattttacaa tcaggcgttg aatgtaggcc gaaagcacat cctgaatgtg 1620

acatcgctga aaattgtaat ggaagctcac cagaatgtgg tcctgacata actttaatca 1680

atggactttc atgcaaaaat aataagttta tttgttatga cggagactgc catgatctcg 1740

atgcacgttg tgagagtgta tttggaaaag gttcaagaaa tgctccattt gcctgctatg 1800

aagaaataca atctcaatca gacagatttg ggaactgtgg tagggataga aataacaaat 1860

atgtgttctg tggatggagg aatcttatat gtggaagatt agtttgtacc taccctactc 1920

gaaagccttt ccatcaagaa aatggtgatg tgatttatgc tttcgtacga gattctgtat 1980

gcataactgt agactacaaa ttgcctcgaa cagttccaga tccactggct gtcaaaaatg 2040

gctctcagtg tgatattggg agggtttgtg taaatcgtga atgtgtagaa tcaaggataa 2100

ttaaggcttc agcacatgtt tgttcacaac agtgttctgg acatggagtg tgtgattcca 2160

gaaacaagtg ccattgttcg ccaggctata agcctccaaa ctgccaaata cgttccaaag 2220

gattttccat atttcctgag gaagatatgg gttcaatcat ggaaagagca tctgggaaga 2280

ctgaaaacac ctggcttcta ggtttcctca ttgctcttcc tattctcatt gtaacaaccg 2340

caatagtttt ggcaaggaaa cagttgaaaa agtggttcgc caaggaagag gaattcccaa 2400

gtagcgaatc taaatcggaa ggtagcacac agacatatgc cagccaatcc agctcagaag 2460

gcagcactca gacatatgcc agccaaacca gatcagaaag cagcagtcaa gctgatacta 2520

gcaaatccaa atcagaagat agtgctgaag catatactag cagatccaaa tcacaggaca 2580

gtacccaaac acaaagcagt agtaactagt gattccttca gaaggcaacg gataacatcg 2640

agagtctcgc taagaaatga aaattctgtc tttccttccg tggtcacagc tgaaagaaac 2700

aataaattga gtgtggatca atttgcaaaa aaaaaaaaaa aa 2742

<210> 168
<211> 3594
<212> DNA
<213> Homo sapiens

<400> 168
gctgcaccgg gcacgggtcg gccgcaatcc agcctgggcg gagccggagt tgcgagccgc 60

tgcctagagg ccgaggagct cacagctatg ggctggaggc cccggagagc tcgggggacc 120

ccgttgctgc tgctgctact actgctgctg ctctggccag tgccaggcgc cggggtgctt 180

caaggacata tccctgggca gccagtcacc ccgcactggg tcctggatgg acaaccctgg 240

cgcaccgtca gcctggagga gccggtctcg aagccagaca tggggctggt ggccctggag 300

gctgaaggcc aggagctcct gcttgagctg gagaagaacc acaggctgct ggccccagga 360

tacatagaaa cccactacgg cccagatggg cagccagtgg tgctggcccc caaccacacg 420

gatcattgcc actaccaagg gcgagtaagg ggcttccccg actcctgggt agtcctctgc 480

acctgctctg ggatgagtgg cctgatcacc ctcagcagga atgccagcta ttatctgcgt 540

ccctggccac cccgggctcc caaggacttc tcaacccacg agatctttcg gatggagcag 600

ctgctcacct ggaaaggaac ctgtggccac agggatcctg ggaacaaagc gggcatgacc 660

agccttcctg gtggtcccca gagcaggggc aggcgagaag cgcgcaggac ccggaagtac 720

ctggaactgt acattgtggc agaccacacc ctgttcttga ctcggcaccg aaacttgaac 780

cacaccaaac agcgtctcct ggaagtcgcc aactacgtgg accagcttct caggactctg 840

gacattcagg tggcgctgac cggcctggag gtgtggaccg agcgggaccg cagccgcgtc 900

acgcaggacg ccaacgccac gctctgggcc ttcctgcagt ggcgccgggg gctgtgggcg 960

cagcggcccc acgactccgc gcagctgctc acgggccgcg ccttccaggg cgccacagtg 1020

ggcctggcgc ccgtcgaggg catgtgccgc gccgagagct cgggaggcgt gagcacggac 1080

cactcggagc tccccatcgg cgccgcagcc accatggccc atgagatcgg ccacagcctc 1140

ggcctcagcc acgaccccga cggctgctgc gtggaggctg cggccgagtc cggaggctgc 1200

gtcatggctg cggccaccgg gcacccgttt ccgcgcgtgt tcagcgcctg cagccgccgc 1260

cagctgcgcg ccttcttccg caaggggggc ggcgcttgcc tctccaatgc cccggacccc 1320

ggactcccgg tgccgccggc gctctgcggg aacggcttcg tggaagcggg cgaggagtgt 1380

gactgcggcc ctggccagga gtgccgcgac ctctgctgct ttgctcacaa ctgctcgctg 1440

cgcccggggg cccagtgcgc ccacggggac tgctgcgtgc gctgcctgct gaagccggct 1500

ggagcgctgt gccgccaggc catgggtgac tgtgacctcc ctgagttttg cacgggcacc 1560

tcctcccact gtcccccaga cgtttaccta ctggacggct caccctgtgc caggggcagt 1620

ggctactgct gggatggcgc atgtcccacg ctggagcagc agtgccagca gctctggggg 1680

cctggctccc acccagctcc cgaggcctgt ttccaggtgg tgaactctgc gggagatgct 1740

catggaaact gcggccagga cagcgagggc cacttcctgc cctgtgcagg gagggatgcc 1800

ctgtgtggga agctgcagtg ccagggtgga aagcccagcc tgctcgcacc gcacatggtg 1860

ccagtggact ctaccgttca cctagatggc caggaagtga cttgtcgggg agccttggca 1920

ctccccagtg cccagctgga cctgcttggc ctgggcctgg tagagccagg cacccagtgt 1980

ggacctagaa tggtgtgcca gagcaggcgc tgcaggaaga atgccttcca ggagcttcag 2040

cgctgcctga ctgcctgcca cagccacggg gtttgcaata gcaaccataa ctgccactgt 2100

gctccaggct gggctccacc cttctgtgac aagccaggct ttggtggcag catggacagt 2160

ggccctgtgc aggctgaaaa ccatgacacc ttcctgctgg ccatgctcct cagcgtcctg 2220

ctgcctctgc tcccaggggc cggcctggcc tggtgttgct accgactccc aggagcccat 2280

ctgcagcgat gcagctgggg ctgcagaagg gaccctgcgt gcagtggccc caaagatggc 2340

ccacacaggg accaccccct gggcggcgtt caccccatgg agttgggccc cacagccact 2400

ggacagccct ggcccctgga ccctgagaac tctcatgagc ccagcagcca ccctgagaag 2460

cctctgccag cagtctcgcc tgacccccaa gcagatcaag tccagatgcc aagatcctgc 2520

ctctggtgag aggtagctcc taaaatgaac agatttaaag acaggtggcc actgacagcc 2580

actccaggaa cttgaactgc aggggcagag ccagtgaatc accggacctc cagcacctgc 2640

aggcagcttg gaagtttctt ccccgagtgg agcttcgacc cacccactcc aggaacccag 2700

agccacatta gaagttcctg agggctggag aacactgctg ggcacactct ccagctcaat 2760

aaaccatcag tcccagaagc aaaggtcaca cagcccctga cctccctcac cagtggaggc 2820

tgggtagtgc tggccatccc aaaagggctc tgtcctggga gtctggtgtg tctcctacat 2880

gcaatttcca cggacccagc tctgtggagg gcatgactgc tggccagaag ctagtggtcc 2940

tggggcccta tggttcgact gagtccacac tcccctggag cctggctggc ctctgcaaac 3000

aaacataatt ttggggacct tccttcctgt ttcttcccac cctgtcttct cccctaggtg 3060

gttcctgagc cccacccccc aatcccagtg ctacacctga ggttctggag ctcagaatct 3120

gacagcctct cccccattct gtgtgtgtcg gggggacaga gggaaccatt taagaaaaga 3180

taccaaagta gaagtcaaaa gaaagacatg ttggctatag gcgtggtggc tcatgcctat 3240

aatcccagca ctttgggaag ccggggtagg aggatcacca gaggccagca ggtccacacc 3300

agcctgggca acacagcaag acaccgcatc tacagaaaaa ttttaaaatt agctgggcgt 3360

ggtggtgtgt acctgtaggc ctagctgctc aggaggctga agcaggagga tcacttgagc 3420

ctgagttcaa cactgcagtg agctatggtg gcaccactgc actccagcct gggtgacaga 3480

gcaagaccct gtctctaaaa taaattttaa aaagacataa aaaaaaaaaa aaaaaaaaaa 3540

aaaaaaaaaa aaaaaaaaaa aaaaaaaaaa aaaaaaaaaa aaaaaaaaaa aaaa 3594

<210> 169
<211> 735
<212> PRT
<213> Homo sapiens

<400> 169
Met Trp Arg Val Leu Phe Leu Leu Ser Gly Leu Gly Gly Leu Arg Met
1               5                   10                  15

Asp Ser Asn Phe Asp Ser Leu Pro Val Gln Ile Thr Val Pro Glu Lys
            20                  25                  30

Ile Arg Ser Ile Ile Lys Glu Gly Ile Glu Ser Gln Ala Ser Tyr Lys
        35                  40                  45

Ile Val Ile Glu Gly Lys Pro Tyr Thr Val Asn Leu Met Gln Lys Asn
    50                  55                  60

Phe Leu Pro His Asn Phe Arg Val Tyr Ser Tyr Ser Gly Thr Gly Ile
65                  70                  75                  80

Met Lys Pro Leu Asp Gln Asp Phe Gln Asn Phe Cys His Tyr Gln Gly
                85                  90                  95

Tyr Ile Glu Gly Tyr Pro Lys Ser Val Val Met Val Ser Thr Cys Thr
            100                 105                 110

Gly Leu Arg Gly Val Leu Gln Phe Glu Asn Val Ser Tyr Gly Ile Glu
        115                 120                 125

Pro Leu Glu Ser Ser Val Gly Phe Glu His Val Ile Tyr Gln Val Lys
    130                 135                 140

His Lys Lys Ala Asp Val Ser Leu Tyr Asn Glu Lys Asp Ile Glu Ser
145                 150                 155                 160

Arg Asp Leu Ser Phe Lys Leu Gln Ser Val Glu Pro Gln Gln Asp Phe
                165                 170                 175

Ala Lys Tyr Ile Glu Met His Val Ile Val Glu Lys Gln Leu Tyr Asn
            180                 185                 190

His Met Gly Ser Asp Thr Thr Val Val Ala Gln Lys Val Phe Gln Leu
        195                 200                 205

Ile Gly Leu Thr Asn Ala Ile Phe Val Ser Phe Asn Ile Thr Ile Ile
    210                 215                 220

Leu Ser Ser Leu Glu Leu Trp Ile Asp Glu Asn Lys Ile Ala Thr Thr
225                 230                 235                 240

Gly Glu Ala Asn Glu Leu Leu His Thr Phe Leu Arg Trp Lys Thr Ser
                245                 250                 255

Tyr Leu Val Leu Arg Pro His Asp Val Ala Phe Leu Leu Val Tyr Arg
            260                 265                 270

Glu Lys Ser Asn Tyr Val Gly Ala Thr Phe Gln Gly Lys Met Cys Asp
        275                 280                 285

Ala Asn Tyr Ala Gly Gly Val Val Leu His Pro Arg Thr Ile Ser Leu
    290                 295                 300

Glu Ser Leu Ala Val Ile Leu Ala Gln Leu Leu Ser Leu Ser Met Gly
305                 310                 315                 320

Ile Thr Tyr Asp Asp Ile Asn Lys Cys Gln Cys Ser Gly Ala Val Cys
                325                 330                 335

Ile Met Asn Pro Glu Ala Ile His Phe Ser Gly Val Lys Ile Phe Ser
            340                 345                 350

Asn Cys Ser Phe Glu Asp Phe Ala His Phe Ile Ser Lys Gln Lys Ser
        355                 360                 365

Gln Cys Leu His Asn Gln Pro Arg Leu Asp Pro Phe Phe Lys Gln Gln
    370                 375                 380

Ala Val Cys Gly Asn Ala Lys Leu Glu Ala Gly Glu Glu Cys Asp Cys
385                 390                 395                 400

Gly Thr Glu Gln Asp Cys Ala Leu Ile Gly Glu Thr Cys Cys Asp Ile
                405                 410                 415

Ala Thr Cys Arg Phe Lys Ala Gly Ser Asn Cys Ala Glu Gly Pro Cys
            420                 425                 430

Cys Glu Asn Cys Leu Phe Met Ser Lys Glu Arg Met Cys Arg Pro Ser
        435                 440                 445

Phe Glu Glu Cys Asp Leu Pro Glu Tyr Cys Asn Gly Ser Ser Ala Ser
    450                 455                 460

Cys Pro Glu Asn His Tyr Val Gln Thr Gly His Pro Cys Gly Leu Asn
465                 470                 475                 480

Gln Trp Ile Cys Ile Asp Gly Val Cys Met Ser Gly Asp Lys Gln Cys
                485                 490                 495

Thr Asp Thr Phe Gly Lys Glu Val Glu Phe Gly Pro Ser Glu Cys Tyr
            500                 505                 510

Ser His Leu Asn Ser Lys Thr Asp Val Ser Gly Asn Cys Gly Ile Ser
        515                 520                 525

Asp Ser Gly Tyr Thr Gln Cys Glu Ala Asp Asn Leu Gln Cys Gly Lys
    530                 535                 540

Leu Ile Cys Lys Tyr Val Gly Lys Phe Leu Leu Gln Ile Pro Arg Ala

545                 550                 555                 560

Thr Ile Ile Tyr Ala Asn Ile Ser Gly His Leu Cys Ile Ala Val Glu
                565                 570                 575

Phe Ala Ser Asp His Ala Asp Ser Gln Lys Met Trp Ile Lys Asp Gly
            580                 585                 590

Thr Ser Cys Gly Ser Asn Lys Val Cys Arg Asn Gln Arg Cys Val Ser
        595                 600                 605

Ser Ser Tyr Leu Gly Tyr Asp Cys Thr Thr Asp Lys Cys Asn Asp Arg
    610                 615                 620

Gly Val Cys Asn Asn Lys Lys His Cys His Cys Ser Ala Ser Tyr Leu
625                 630                 635                 640

Pro Pro Asp Cys Ser Val Gln Ser Asp Leu Trp Pro Gly Gly Ser Ile
                645                 650                 655

Asp Ser Gly Asn Phe Pro Pro Val Ala Ile Pro Ala Arg Leu Pro Glu
            660                 665                 670

Arg Arg Tyr Ile Glu Asn Ile Tyr His Ser Lys Pro Met Arg Trp Pro
        675                 680                 685

Phe Phe Leu Phe Ile Pro Phe Phe Ile Ile Phe Cys Val Leu Ile Ala
    690                 695                 700

Ile Met Val Lys Val Asn Phe Gln Arg Lys Lys Trp Arg Thr Glu Asp
705                 710                 715                 720

Tyr Ser Ser Asp Glu Gln Pro Glu Ser Glu Ser Glu Pro Lys Gly
                725                 730                 735

<210> 170
<211> 754
<212> PRT
<213> Homo sapiens

<400> 170
   Met Leu Pro Gly Cys Ile Phe Leu Met Ile Leu Leu Ile Pro Gln Val
   1               5                   10                  15
   Lys Glu Lys Phe Ile Leu Gly Val Glu Gly Gln Gln Leu Val Arg Pro
               20                  25                  30
   Lys Lys Leu Pro Leu Ile Gln Lys Arg Asp Thr Gly His Thr His Asp
           35                  40                  45
   Asp Asp Ile Leu Lys Thr Tyr Glu Glu Glu Leu Leu Tyr Glu Ile Lys
       50                  55                  60
   Leu Asn Arg Lys Thr Leu Val Leu His Leu Leu Arg Ser Arg Glu Phe
   65                  70                  75                  80
   Leu Gly Ser Asn Tyr Ser Glu Thr Phe Tyr Ser Met Lys Gly Glu Ala
                   85                  90                  95
   Phe Thr Arg His Pro Gln Ile Met Asp His Cys Phe Tyr Gln Gly Ser
               100                 105                 110
   Ile Val His Glu Tyr Asp Ser Ala Ala Ser Ile Ser Thr Cys Asn Gly
           115                 120                 125
   Leu Arg Gly Phe Phe Arg Ile Asn Asp Gln Arg Tyr Leu Ile Glu Pro
       130                 135                 140
   Val Lys Tyr Ser Asp Glu Gly Glu His Leu Val Phe Lys Tyr Asn Leu
   145                 150                 155                 160
   Arg Val Pro Tyr Gly Ala Asn Tyr Ser Cys Thr Glu Leu Asn Phe Thr
                   165                 170                 175
   Arg Lys Thr Val Pro Gly Asp Asn Glu Ser Glu Glu Asp Ser Lys Ile
               180                 185                 190
   Lys Gly Ile His Asp Glu Lys Tyr Val Glu Leu Phe Ile Val Ala Asp
           195                 200                 205
   Asp Thr Val Tyr Arg Arg Asn Gly His Pro His Asn Lys Leu Arg Asn
       210                 215                 220
   Arg Ile Trp Gly Met Val Asn Phe Val Asn Met Ile Tyr Lys Thr Leu
   225                 230                 235                 240
   Asn Ile His Val Thr Leu Val Gly Ile Glu Ile Trp Thr His Glu Asp
                   245                 250                 255
   Lys Ile Glu Leu Tyr Ser Asn Ile Glu Thr Thr Leu Leu Arg Phe Ser
               260                 265                 270
   Phe Trp Gln Glu Lys Ile Leu Lys Thr Arg Lys Asp Phe Asp His Val
           275                 280                 285
   Val Leu Leu Ser Gly Lys Trp Leu Tyr Ser His Val Gln Gly Ile Ser
   290                 295                 300
   Tyr Pro Gly Gly Met Cys Leu Pro Tyr Tyr Ser Thr Ser Ile Ile Lys
   305                 310                 315                 320
   Asp Leu Leu Pro Asp Thr Asn Ile Ile Ala Asn Arg Met Ala His Gln
                   325                 330                 335
   Leu Gly His Asn Leu Gly Met Gln His Asp Glu Phe Pro Cys Thr Cys
               340                 345                 350
   Pro Ser Gly Lys Cys Val Met Asp Ser Asp Gly Ser Ile Pro Ala Leu
           355                 360                 365
   Lys Phe Ser Lys Cys Ser Gln Asn Gln Tyr His Gln Tyr Leu Lys Asp
       370                 375                 380
   Tyr Lys Pro Thr Cys Met Leu Asn Ile Pro Phe Pro Tyr Asn Phe His
   385                 390                 395                 400
   Asp Phe Gln Phe Cys Gly Asn Lys Lys Leu Asp Glu Gly Glu Glu Cys
                   405                 410                 415
   Asp Cys Gly Pro Ala Gln Glu Cys Thr Asn Pro Cys Cys Asp Ala His
               420                 425                 430
   Thr Cys Val Leu Lys Pro Gly Phe Thr Cys Ala Glu Gly Glu Cys Cys
           435                 440                 445
   Glu Ser Cys Gln Ile Lys Lys Ala Gly Ser Ile Cys Arg Pro Ala Lys
       450                 455                 460
   Asp Glu Cys Asp Phe Pro Glu Met Cys Thr Gly His Ser Pro Ala Cys
   465                 470                 475                 480
   Pro Lys Asp Gln Phe Arg Val Asn Gly Phe Pro Cys Lys Asn Ser Glu
                   485                 490                 495
   Gly Tyr Cys Phe Met Gly Lys Cys Pro Thr Arg Glu Asp Gln Cys Ser
               500                 505                 510
   Glu Leu Phe Asp Asp Glu Ala Ile Glu Ser His Asp Ile Cys Tyr Lys
           515                 520                 525
   Met Asn Thr Lys Gly Asn Lys Phe Gly Tyr Cys Lys Asn Lys Glu Asn
       530                 535                 540
   Arg Phe Leu Pro Cys Glu Glu Lys Asp Val Arg Cys Gly Lys Ile Tyr
   545                 550                 555                 560
   Cys Thr Gly Gly Glu Leu Ser Ser Leu Leu Gly Glu Asp Lys Thr Tyr
                   565                 570                 575
   His Leu Lys Asp Pro Gln Lys Asn Ala Thr Val Lys Cys Lys Thr Ile
               580                 585                 590
   Phe Leu Tyr His Asp Ser Thr Asp Ile Gly Leu Val Ala Ser Gly Thr
           595                 600                 605
   Lys Cys Gly Glu Gly Met Val Cys Asn Asn Gly Glu Cys Leu Asn Met
       610                 615                 620
   Glu Lys Val Tyr Ile Ser Thr Asn Cys Pro Ser Gln Cys Asn Glu Asn
   625                 630                 635                 640
   Pro Val Asp Gly His Gly Leu Gln Cys His Cys Glu Glu Gly Gln Ala
                   645                 650                 655
   Pro Val Ala Cys Glu Glu Thr Leu His Val Thr Asn Ile Thr Ile Leu
               660                 665                 670
   Val Val Val Leu Val Leu Val Ile Val Gly Ile Gly Val Leu Ile Leu
           675                 680                 685
   Leu Val Arg Tyr Arg Lys Cys Ile Lys Leu Lys Gln Val Gln Ser Pro
       690                 695                 700
   Pro Thr Glu Thr Leu Gly Val Glu Asn Lys Gly Tyr Phe Gly Asp Glu
   705                 710                 715                 720
   Gln Gln Ile Arg Thr Glu Pro Ile Leu Pro Glu Ile His Phe Leu Asn
                   725                 730                 735
   Lys Pro Ala Ser Lys Asp Ser Arg Gly Ile Ala Asp Pro Asn Gln Ser
               740                 745                 750
   Ala Lys

<210> 171
<211> 824
<212> PRT
<213> Homo sapiens

<400> 171
Met Arg Gly Leu Gly Leu Trp Leu Leu Gly Ala Met Met Leu Pro Ala

1               5                   10                  15

Ile Ala Pro Ser Arg Pro Trp Ala Leu Met Glu Gln Tyr Glu Val Val
            20                  25                  30

Leu Pro Trp Arg Leu Pro Gly Pro Arg Val Arg Arg Ala Leu Pro Ser
        35                  40                  45

His Leu Gly Leu His Pro Glu Arg Val Ser Tyr Val Leu Gly Ala Thr
    50                  55                  60

Gly His Asn Phe Thr Leu His Leu Arg Lys Asn Arg Asp Leu Leu Gly
65                  70                  75                  80

Ser Gly Tyr Thr Glu Thr Tyr Thr Ala Ala Asn Gly Ser Glu Val Thr
                85                  90                  95

Glu Gln Pro Arg Gly Gln Asp His Cys Phe Tyr Gln Gly His Val Glu
            100                 105                 110

Gly Tyr Pro Asp Ser Ala Ala Ser Leu Ser Thr Cys Ala Gly Leu Arg
        115                 120                 125

Gly Phe Phe Gln Val Gly Ser Asp Leu His Leu Ile Glu Pro Leu Asp
    130                 135                 140

Glu Gly Gly Glu Gly Gly Arg His Ala Val Tyr Gln Ala Glu His Leu
145                 150                 155                 160

Leu Gln Thr Ala Gly Thr Cys Gly Val Ser Asp Asp Ser Leu Gly Ser
                165                 170                 175

Leu Leu Gly Pro Arg Thr Ala Ala Val Phe Arg Pro Arg Pro Gly Asp
            180                 185                 190

Ser Leu Pro Ser Arg Glu Thr Arg Tyr Val Glu Leu Tyr Val Val Val
        195                 200                 205

Asp Asn Ala Glu Phe Gln Met Leu Gly Ser Glu Ala Ala Val Arg His
    210                 215                 220

Arg Val Leu Glu Val Val Asn His Val Asp Lys Leu Tyr Gln Lys Leu
225                 230                 235                 240

Asn Phe Arg Val Val Leu Val Gly Leu Glu Ile Trp Asn Ser Gln Asp
                245                 250                 255

Arg Phe His Val Ser Pro Asp Pro Ser Val Thr Leu Glu Asn Leu Leu
            260                 265                 270

Thr Trp Gln Ala Arg Gln Arg Thr Arg Arg His Leu His Asp Asn Val
        275                 280                 285

Gln Leu Ile Thr Gly Val Asp Phe Thr Gly Thr Thr Val Gly Phe Ala
    290                 295                 300

Arg Val Ser Ala Met Cys Ser His Ser Ser Gly Ala Val Asn Gln Asp
305                 310                 315                 320

His Ser Lys Asn Pro Val Gly Val Ala Cys Thr Met Ala His Glu Met
                325                 330                 335

Gly His Asn Leu Gly Met Asp His Asp Glu Asn Val Gln Gly Cys Arg
            340                 345                 350

Cys Gln Glu Arg Phe Glu Ala Gly Arg Cys Ile Met Ala Gly Ser Ile
        355                 360                 365

Gly Ser Ser Phe Pro Arg Met Phe Ser Asp Cys Ser Gln Ala Tyr Leu
    370                 375                 380

Glu Ser Phe Leu Glu Arg Pro Gln Ser Val Cys Leu Ala Asn Ala Pro
385                 390                 395                 400

Asp Leu Ser His Leu Val Gly Gly Pro Val Cys Gly Asn Leu Phe Val
                405                 410                 415

Glu Arg Gly Glu Gln Cys Asp Cys Gly Pro Pro Glu Asp Cys Arg Asn
            420                 425                 430

Arg Cys Cys Asn Ser Thr Thr Cys Gln Leu Ala Glu Gly Ala Gln Cys
        435                 440                 445

Ala His Gly Thr Cys Cys Gln Glu Cys Lys Val Lys Pro Ala Gly Glu
    450                 455                 460

Leu Cys Arg Pro Lys Lys Asp Met Cys Asp Leu Glu Glu Phe Cys Asp
465                 470                 475                 480

Gly Arg His Pro Glu Cys Pro Glu Asp Ala Phe Gln Glu Asn Gly Thr
                485                 490                 495

Pro Cys Ser Gly Gly Tyr Cys Tyr Asn Gly Ala Cys Pro Thr Leu Ala
            500                 505                 510

Gln Gln Cys Gln Ala Phe Trp Gly Pro Gly Gly Gln Ala Ala Glu Glu
        515                 520                 525

Ser Cys Phe Ser Tyr Asp Ile Leu Pro Gly Cys Lys Ala Ser Arg Tyr
    530                 535                 540

Arg Ala Asp Met Cys Gly Val Leu Gln Cys Lys Gly Gly Gln Gln Pro
545                 550                 555                 560

Leu Gly Arg Ala Ile Cys Ile Val Asp Val Cys His Ala Leu Thr Thr
                565                 570                 575

Glu Asp Gly Thr Ala Tyr Glu Pro Val Pro Glu Gly Thr Arg Cys Gly
            580                 585                 590

Pro Glu Lys Val Cys Trp Lys Gly Arg Cys Gln Asp Leu His Val Tyr
        595                 600                 605

Arg Ser Ser Asn Cys Ser Ala Gln Cys His Asn His Gly Val Cys Asn
    610                 615                 620

His Lys Gln Glu Cys His Cys His Ala Gly Trp Ala Pro Pro His Cys
625                 630                 635                 640

Ala Lys Leu Leu Thr Glu Val His Ala Ala Ser Gly Ser Leu Pro Val
                645                 650                 655

Phe Val Val Val Val Leu Val Leu Leu Ala Val Val Leu Val Thr Leu
            660                 665                 670

Ala Gly Ile Ile Val Tyr Arg Lys Ala Arg Ser Arg Ile Leu Ser Arg
        675                 680                 685

Asn Val Ala Pro Lys Thr Thr Met Gly Arg Ser Asn Pro Leu Phe His
    690                 695                 700

Gln Ala Ala Ser Arg Val Pro Ala Lys Gly Gly Ala Pro Ala Pro Ser
705                 710                 715                 720

Arg Gly Pro Gln Glu Leu Val Pro Thr Thr His Pro Gly Gln Pro Ala
                725                 730                 735

Arg His Pro Ala Ser Ser Val Ala Leu Lys Arg Pro Pro Pro Ala Pro
            740                 745                 750

Pro Val Thr Val Ser Ser Pro Pro Phe Pro Val Pro Val Tyr Thr Arg
        755                 760                 765

Gln Ala Pro Lys Gln Val Ile Lys Pro Thr Phe Ala Pro Pro Val Pro
    770                 775                 780

Pro Val Lys Pro Gly Ala Gly Ala Ala Asn Pro Gly Pro Ala Glu Gly
785                 790                 795                 800

Ala Val Gly Pro Lys Val Ala Leu Lys Pro Pro Ile Gln Arg Lys Gln
                805                 810                 815

Gly Ala Gly Ala Pro Thr Ala Pro
            820

<210> 172
<211> 819
<212> PRT
<213> Homo sapiens

<400> 172
Met Gly Ser Gly Ala Arg Phe Pro Ser Gly Thr Leu Arg Val Arg Trp
1               5                   10                  15

Leu Leu Leu Leu Gly Leu Val Gly Pro Val Leu Gly Ala Ala Arg Pro
            20                  25                  30

Gly Phe Gln Gln Thr Ser His Leu Ser Ser Tyr Glu Ile Ile Thr Pro
        35                  40                  45

Trp Arg Leu Thr Arg Glu Arg Arg Glu Ala Pro Arg Pro Tyr Ser Lys
    50                  55                  60

Gln Val Ser Tyr Val Ile Gln Ala Glu Gly Lys Glu His Ile Ile His
65                  70                  75                  80

Leu Glu Arg Asn Lys Asp Leu Leu Pro Glu Asp Phe Val Val Tyr Thr
                85                  90                  95

Tyr Asn Lys Glu Gly Thr Leu Ile Thr Asp His Pro Asn Ile Gln Asn
            100                 105                 110

His Cys His Tyr Arg Gly Tyr Val Glu Gly Val His Asn Ser Ser Ile
        115                 120                 125

Ala Leu Ser Asp Cys Phe Gly Leu Arg Gly Leu Leu His Leu Glu Asn
    130                 135                 140

Ala Ser Tyr Gly Ile Glu Pro Leu Gln Asn Ser Ser His Phe Glu His
145                 150                 155                 160

Ile Ile Tyr Arg Met Asp Asp Val Tyr Lys Glu Pro Leu Lys Cys Gly
                165                 170                 175

Val Ser Asn Lys Asp Ile Glu Lys Glu Thr Ala Lys Asp Glu Glu Glu
            180                 185                 190

Glu Pro Pro Ser Met Thr Gln Leu Leu Arg Arg Arg Arg Ala Val Leu
        195                 200                 205

Pro Gln Thr Arg Tyr Val Glu Leu Phe Ile Val Val Asp Lys Glu Arg
    210                 215                 220

Tyr Asp Met Met Gly Arg Asn Gln Thr Ala Val Arg Glu Glu Met Ile
225                 230                 235                 240

Leu Leu Ala Asn Tyr Leu Asp Ser Met Tyr Ile Met Leu Asn Ile Arg
                245                 250                 255

Ile Val Leu Val Gly Leu Glu Ile Trp Thr Asn Gly Asn Leu Ile Asn
            260                 265                 270

Ile Val Gly Gly Ala Gly Asp Val Leu Gly Asn Phe Val Gln Trp Arg
        275                 280                 285

Glu Lys Phe Leu Ile Thr Arg Arg Arg His Asp Ser Ala Gln Leu Val
    290                 295                 300

Leu Lys Lys Gly Phe Gly Gly Thr Ala Gly Met Ala Phe Val Gly Thr
305                 310                 315                 320

Val Cys Ser Arg Ser His Ala Gly Gly Ile Asn Val Phe Gly Gln Ile
                325                 330                 335

Thr Val Glu Thr Phe Ala Ser Ile Val Ala His Glu Leu Gly His Asn
            340                 345                 350

Leu Gly Met Asn His Asp Asp Gly Arg Asp Cys Ser Cys Gly Ala Lys
        355                 360                 365

Ser Cys Ile Met Asn Ser Gly Ala Ser Gly Ser Arg Asn Phe Ser Ser
    370                 375                 380

Cys Ser Ala Glu Asp Phe Glu Lys Leu Thr Leu Asn Lys Gly Gly Asn
385                 390                 395                 400

Cys Leu Leu Asn Ile Pro Lys Pro Asp Glu Ala Tyr Ser Ala Pro Ser
                405                 410                 415

Cys Gly Asn Lys Leu Val Asp Ala Gly Glu Glu Cys Asp Cys Gly Thr
            420                 425                 430

Pro Lys Glu Cys Glu Leu Asp Pro Cys Cys Glu Gly Ser Thr Cys Lys
        435                 440                 445

Leu Lys Ser Phe Ala Glu Cys Ala Tyr Gly Asp Cys Cys Lys Asp Cys
    450                 455                 460

Arg Phe Leu Pro Gly Gly Thr Leu Cys Arg Gly Lys Thr Ser Glu Cys
465                 470                 475                 480

Asp Val Pro Glu Tyr Cys Asn Gly Ser Ser Gln Phe Cys Gln Pro Asp
                485                 490                 495

Val Phe Ile Gln Asn Gly Tyr Pro Cys Gln Asn Asn Lys Ala Tyr Cys
            500                 505                 510

Tyr Asn Gly Met Cys Gln Tyr Tyr Asp Ala Gln Cys Gln Val Ile Phe
        515                 520                 525

Gly Ser Lys Ala Lys Ala Ala Pro Lys Asp Cys Phe Ile Glu Val Asn
    530                 535                 540

Ser Lys Gly Asp Arg Phe Gly Asn Cys Gly Phe Ser Gly Asn Glu Tyr
545                 550                 555                 560

Lys Lys Cys Ala Thr Gly Asn Ala Leu Cys Gly Lys Leu Gln Cys Glu
                565                 570                 575

Asn Val Gln Glu Ile Pro Val Phe Gly Ile Val Pro Ala Ile Ile Gln
            580                 585                 590

Thr Pro Ser Arg Gly Thr Lys Cys Trp Gly Val Asp Phe Gln Leu Gly
        595                 600                 605

Ser Asp Val Pro Asp Pro Gly Met Val Asn Glu Gly Thr Lys Cys Gly
    610                 615                 620

Ala Gly Lys Ile Cys Arg Asn Phe Gln Cys Val Asp Ala Ser Val Leu
625                 630                 635                 640

Asn Tyr Asp Cys Asp Val Gln Lys Lys Cys His Gly His Gly Val Cys
                645                 650                 655

Asn Ser Asn Lys Asn Cys His Cys Glu Asn Gly Trp Ala Pro Pro Asn

660                 665                 670

Cys Glu Thr Lys Gly Tyr Gly Gly Ser Val Asp Ser Gly Pro Thr Tyr
        675                 680                 685

Asn Glu Met Asn Thr Ala Leu Arg Asp Gly Leu Leu Val Phe Phe Phe
    690                 695                 700

Leu Ile Val Pro Leu Ile Val Cys Ala Ile Phe Ile Phe Ile Lys Arg
705                 710                 715                 720

Asp Gln Leu Trp Arg Ser Tyr Phe Arg Lys Lys Arg Ser Gln Thr Tyr
                725                 730                 735

Glu Ser Asp Gly Lys Asn Gln Ala Asn Pro Ser Arg Gln Pro Gly Ser
            740                 745                 750

Val Pro Arg His Val Ser Pro Val Thr Pro Pro Arg Glu Val Pro Ile
        755                 760                 765

Tyr Ala Asn Arg Phe Ala Val Pro Thr Tyr Ala Ala Lys Gln Pro Gln
    770                 775                 780

Gln Phe Pro Ser Arg Pro Pro Pro Pro Gln Pro Lys Val Ser Ser Gln
785                 790                 795                 800

Gly Asn Leu Ile Pro Ala Arg Pro Ala Pro Ala Pro Pro Leu Tyr Ser
                805                 810                 815

Ser Leu Thr

<210> 173
<211> 748
<212> PRT
<213> Homo sapiens

<400> 173
Met Val Leu Leu Arg Val Leu Ile Leu Leu Leu Ser Trp Ala Ala Gly
1               5                   10                  15

Met Gly Gly Gln Tyr Gly Asn Pro Leu Asn Lys Tyr Ile Arg His Tyr
            20                  25                  30

Glu Gly Leu Ser Tyr Asn Val Asp Ser Leu His Gln Lys His Gln Arg
        35                  40                  45

Ala Lys Arg Ala Val Ser His Glu Asp Gln Phe Leu Arg Leu Asp Phe
    50                  55                  60

His Ala His Gly Arg His Phe Asn Leu Arg Met Lys Arg Asp Thr Ser
65                  70                  75                  80

Leu Phe Ser Asp Glu Phe Lys Val Glu Thr Ser Asn Lys Val Leu Asp
                85                  90                  95

Tyr Asp Thr Ser His Ile Tyr Thr Gly His Ile Tyr Gly Glu Glu Gly
            100                 105                 110

Ser Phe Ser His Gly Ser Val Ile Asp Gly Arg Phe Glu Gly Phe Ile
        115                 120                 125

Gln Thr Arg Gly Gly Thr Phe Tyr Val Glu Pro Ala Glu Arg Tyr Ile
    130                 135                 140

Lys Asp Arg Thr Leu Pro Phe His Ser Val Ile Tyr His Glu Asp Asp
145                 150                 155                 160

Ile Asn Tyr Pro His Lys Tyr Gly Pro Gln Gly Gly Cys Ala Asp His
                165                 170                 175

Ser Val Phe Glu Arg Met Arg Lys Tyr Gln Met Thr Gly Val Glu Glu
            180                 185                 190

Val Thr Gln Ile Pro Gln Glu Glu His Ala Ala Asn Gly Pro Glu Leu
        195                 200                 205

Leu Arg Lys Lys Arg Thr Thr Ser Ala Glu Lys Asn Thr Cys Gln Leu
    210                 215                 220

Tyr Ile Gln Thr Asp His Leu Phe Phe Lys Tyr Tyr Gly Thr Arg Glu
225                 230                 235                 240

Ala Val Ile Ala Gln Ile Ser Ser His Val Lys Ala Ile Asp Thr Ile
                245                 250                 255

Tyr Gln Thr Thr Asp Phe Ser Gly Ile Arg Asn Ile Ser Phe Met Val
            260                 265                 270

Lys Arg Ile Arg Ile Asn Thr Thr Ala Asp Glu Lys Asp Pro Thr Asn
        275                 280                 285

Pro Phe Arg Phe Pro Asn Ile Gly Val Glu Lys Phe Leu Glu Leu Asn
    290                 295                 300

Ser Glu Gln Asn His Asp Asp Tyr Cys Leu Ala Tyr Val Phe Thr Asp

305                 310                 315                 320

Arg Asp Phe Asp Asp Gly Val Leu Gly Leu Ala Trp Val Gly Ala Pro
                325                 330                 335

Ser Gly Ser Ser Gly Gly Ile Cys Glu Lys Ser Lys Leu Tyr Ser Asp
            340                 345                 350

Gly Lys Lys Lys Ser Leu Asn Thr Gly Ile Ile Thr Val Gln Asn Tyr
        355                 360                 365

Gly Ser His Val Pro Pro Lys Val Ser His Ile Thr Phe Ala His Glu
    370                 375                 380

Val Gly His Asn Phe Gly Ser Pro His Asp Ser Gly Thr Glu Cys Thr
385                 390                 395                 400

Pro Gly Glu Ser Lys Asn Leu Gly Gln Lys Glu Asn Gly Asn Tyr Ile
                405                 410                 415

Met Tyr Ala Arg Ala Thr Ser Gly Asp Lys Leu Asn Asn Asn Lys Phe
            420                 425                 430

Ser Leu Cys Ser Ile Arg Asn Ile Ser Gln Val Leu Glu Lys Lys Arg
        435                 440                 445

Asn Asn Cys Phe Val Glu Ser Gly Gln Pro Ile Cys Gly Asn Gly Met
    450                 455                 460

Val Glu Gln Gly Glu Glu Cys Asp Cys Gly Tyr Ser Asp Gln Cys Lys
465                 470                 475                 480

Asp Glu Cys Cys Phe Asp Ala Asn Gln Pro Glu Gly Arg Lys Cys Lys
                485                 490                 495

Leu Lys Pro Gly Lys Gln Cys Ser Pro Ser Gln Gly Pro Cys Cys Thr
            500                 505                 510

Ala Gln Cys Ala Phe Lys Ser Lys Ser Glu Lys Cys Arg Asp Asp Ser
        515                 520                 525

Asp Cys Ala Arg Glu Gly Ile Cys Asn Gly Phe Thr Ala Leu Cys Pro
    530                 535                 540

Ala Ser Asp Pro Lys Pro Asn Phe Thr Asp Cys Asn Arg His Thr Gln
545                 550                 555                 560

Val Cys Ile Asn Gly Gln Cys Ala Gly Ser Ile Cys Glu Lys Tyr Gly
                565                 570                 575

Leu Glu Glu Cys Thr Cys Ala Ser Ser Asp Gly Lys Asp Asp Lys Glu
            580                 585                 590

Leu Cys His Val Cys Cys Met Lys Lys Met Asp Pro Ser Thr Cys Ala
        595                 600                 605

Ser Thr Gly Ser Val Gln Trp Ser Arg His Phe Ser Gly Arg Thr Ile
    610                 615                 620

Thr Leu Gln Pro Gly Ser Pro Cys Asn Asp Phe Arg Gly Tyr Cys Asp
625                 630                 635                 640

Val Phe Met Arg Cys Arg Leu Val Asp Ala Asp Gly Pro Leu Ala Arg
                645                 650                 655

Leu Lys Lys Ala Ile Phe Ser Pro Glu Leu Tyr Glu Asn Ile Ala Glu
            660                 665                 670

Trp Ile Val Ala His Trp Trp Ala Val Leu Leu Met Gly Ile Ala Leu
        675                 680                 685

Ile Met Leu Met Ala Gly Phe Ile Lys Ile Cys Ser Val His Thr Pro
    690                 695                 700

Ser Ser Asn Pro Lys Leu Pro Pro Pro Lys Pro Leu Pro Gly Thr Leu
705                 710                 715                 720

Lys Arg Arg Arg Pro Pro Gln Pro Ile Gln Gln Pro Gln Arg Gln Arg
                725                 730                 735

Pro Arg Glu Ser Tyr Gln Met Gly His Met Arg Arg
            740                 745

<210> 174
<211> 769
<212> PRT
<213> Homo sapiens

<400> 174
Met Arg Leu Leu Arg Arg Trp Ala Phe Ala Ala Leu Leu Leu Ser Leu
1               5                   10                  15

Leu Pro Thr Pro Gly Leu Gly Thr Gln Gly Pro Ala Gly Ala Leu Arg
            20                  25                  30

Trp Gly Gly Leu Pro Gln Leu Gly Gly Pro Gly Ala Pro Glu Val Thr

        35                  40                  45

Glu Pro Ser Arg Leu Val Arg Glu Ser Ser Gly Gly Glu Val Arg Lys
    50                  55                  60

Gln Gln Leu Asp Thr Arg Val Arg Gln Glu Pro Pro Gly Gly Pro Pro
65                  70                  75                  80

Val His Leu Ala Gln Val Ser Phe Val Ile Pro Ala Phe Asn Ser Asn
                85                  90                  95

Phe Thr Leu Asp Leu Glu Leu Asn His His Leu Leu Ser Ser Gln Tyr
            100                 105                 110

Val Glu Arg His Phe Ser Arg Glu Gly Thr Thr Gln His Ser Thr Gly
        115                 120                 125

Ala Gly Asp His Cys Tyr Tyr Gln Gly Lys Leu Arg Gly Asn Pro His
    130                 135                 140

Ser Phe Ala Ala Leu Ser Thr Cys Gln Gly Leu His Gly Val Phe Ser
145                 150                 155                 160

Asp Gly Asn Leu Thr Tyr Ile Val Glu Pro Gln Glu Val Ala Gly Pro
                165                 170                 175

Trp Gly Ala Pro Gln Gly Pro Leu Pro His Leu Ile Tyr Arg Thr Pro
            180                 185                 190

Leu Leu Pro Asp Pro Leu Gly Cys Arg Glu Pro Gly Cys Leu Phe Ala
        195                 200                 205

Val Pro Ala Gln Ser Ala Pro Pro Asn Arg Pro Arg Leu Arg Arg Lys
    210                 215                 220

Arg Gln Val Arg Arg Gly His Pro Thr Val His Ser Glu Thr Lys Tyr
225                 230                 235                 240

Val Glu Leu Ile Val Ile Asn Asp His Gln Leu Phe Glu Gln Met Arg
                245                 250                 255

Gln Ser Val Val Leu Thr Ser Asn Phe Ala Lys Ser Val Val Asn Leu
            260                 265                 270

Ala Asp Val Ile Tyr Lys Glu Gln Leu Asn Thr Arg Ile Val Leu Val
        275                 280                 285

Ala Met Glu Thr Trp Ala Asp Gly Asp Lys Ile Gln Val Gln Asp Asp
    290                 295                 300

Leu Leu Glu Thr Leu Ala Arg Leu Met Val Tyr Arg Arg Glu Gly Leu
305                 310                 315                 320

Pro Glu Pro Ser Asp Ala Thr His Leu Phe Ser Gly Arg Thr Phe Gln
                325                 330                 335

Ser Thr Ser Ser Gly Ala Ala Tyr Val Gly Gly Ile Cys Ser Leu Ser
            340                 345                 350

His Gly Gly Gly Val Asn Glu Tyr Gly Asn Met Gly Ala Met Ala Val
        355                 360                 365

Thr Leu Ala Gln Thr Leu Gly Gln Asn Leu Gly Met Met Trp Asn Lys
    370                 375                 380

His Arg Ser Ser Ala Gly Asp Cys Lys Cys Pro Asp Ile Trp Leu Gly
385                 390                 395                 400

Cys Ile Met Glu Asp Thr Gly Phe Tyr Leu Pro Arg Lys Phe Ser Arg
                405                 410                 415

Cys Ser Ile Asp Glu Tyr Asn Gln Phe Leu Gln Glu Gly Gly Gly Ser
            420                 425                 430

Cys Leu Phe Asn Lys Pro Leu Lys Leu Leu Asp Pro Pro Glu Cys Gly
        435                 440                 445

Asn Gly Phe Val Glu Ala Gly Glu Glu Cys Asp Cys Gly Ser Val Gln
    450                 455                 460

Glu Cys Ser Arg Ala Gly Gly Asn Cys Cys Lys Lys Cys Thr Leu Thr
465                 470                 475                 480

His Asp Ala Met Cys Ser Asp Gly Leu Cys Cys Arg Arg Cys Lys Tyr
                485                 490                 495

Glu Pro Arg Gly Val Ser Cys Arg Glu Ala Val Asn Glu Cys Asp Ile
            500                 505                 510

Ala Glu Thr Cys Thr Gly Asp Ser Ser Gln Cys Pro Pro Asn Leu His
        515                 520                 525

Lys Leu Asp Gly Tyr Tyr Cys Asp His Glu Gln Gly Arg Cys Tyr Gly
    530                 535                 540

Gly Arg Cys Lys Thr Arg Asp Arg Gln Cys Gln Val Leu Trp Gly His
545                 550                 555                 560

Ala Ala Ala Asp Arg Phe Cys Tyr Glu Lys Leu Asn Val Glu Gly Thr
                565                 570                 575

Glu Arg Gly Ser Cys Gly Arg Lys Gly Ser Gly Trp Val Gln Cys Ser
            580                 585                 590

Lys Gln Asp Val Leu Cys Gly Phe Leu Leu Cys Val Asn Ile Ser Gly
        595                 600                 605

Ala Pro Arg Leu Gly Asp Leu Val Gly Asp Ile Ser Ser Val Thr Phe
    610                 615                 620

Tyr His Gln Gly Lys Glu Leu Asp Cys Arg Gly Gly His Val Gln Leu
625                 630                 635                 640

Ala Asp Gly Ser Asp Leu Ser Tyr Val Glu Asp Gly Thr Ala Cys Gly
                645                 650                 655

Pro Asn Met Leu Cys Leu Asp His Arg Cys Leu Pro Ala Ser Ala Phe
            660                 665                 670

Asn Phe Ser Thr Cys Pro Gly Ser Gly Glu Arg Arg Ile Cys Ser His
        675                 680                 685

His Gly Val Cys Ser Asn Glu Gly Lys Cys Ile Cys Gln Pro Asp Trp
    690                 695                 700

Thr Gly Lys Asp Cys Ser Ile His Asn Pro Leu Pro Thr Ser Pro Pro
705                 710                 715                 720

Thr Gly Glu Thr Glu Arg Tyr Lys Gly Pro Ser Gly Thr Asn Ile Ile
                725                 730                 735

Ile Gly Ser Ile Ala Gly Ala Val Leu Val Ala Ala Ile Val Leu Gly
            740                 745                 750

Gly Thr Gly Trp Gly Phe Lys Asn Ile Arg Arg Gly Arg Ser Gly Gly
        755                 760                 765

Ala

<210> 175
<211> 909

<212> PRT
<213> Homo sapiens

<400> 175
Met Ala Ala Arg Pro Leu Pro Val Ser Pro Ala Arg Ala Leu Leu Leu
1               5                   10                  15

Ala Leu Ala Gly Ala Leu Leu Ala Pro Cys Glu Ala Arg Gly Val Ser
            20                  25                  30

Leu Trp Asn Gln Gly Arg Ala Asp Glu Val Val Ser Ala Ser Val Gly
        35                  40                  45

Ser Gly Asp Leu Trp Ile Pro Val Lys Ser Phe Asp Ser Lys Asn His
    50                  55                  60

Pro Glu Val Leu Asn Ile Arg Leu Gln Arg Glu Ser Lys Glu Leu Ile
65                  70                  75                  80

Ile Asn Leu Glu Arg Asn Glu Gly Leu Ile Ala Ser Ser Phe Thr Glu
                85                  90                  95

Thr His Tyr Leu Gln Asp Gly Thr Asp Val Ser Leu Ala Arg Asn Tyr
            100                 105                 110

Thr Val Ile Leu Gly His Cys Tyr Tyr His Gly His Val Arg Gly Tyr
        115                 120                 125

Ser Asp Ser Ala Val Ser Leu Ser Thr Cys Ser Gly Leu Arg Gly Leu
    130                 135                 140

Ile Val Phe Glu Asn Glu Ser Tyr Val Leu Glu Pro Met Lys Ser Ala
145                 150                 155                 160

Thr Asn Arg Tyr Lys Leu Phe Pro Ala Lys Lys Leu Lys Ser Val Arg
                165                 170                 175

Gly Ser Cys Gly Ser His His Asn Thr Pro Asn Leu Ala Ala Lys Asn
            180                 185                 190

Val Phe Pro Pro Pro Ser Gln Thr Trp Ala Arg Arg His Lys Arg Glu
        195                 200                 205

Thr Leu Lys Ala Thr Lys Tyr Val Glu Leu Val Ile Val Ala Asp Asn
    210                 215                 220

Arg Glu Phe Gln Arg Gln Gly Lys Asp Leu Glu Lys Val Lys Gln Arg
225                 230                 235                 240

Leu Ile Glu Ile Ala Asn His Val Asp Lys Phe Tyr Arg Pro Leu Asn
                245                 250                 255

Ile Arg Ile Val Leu Val Gly Val Glu Val Trp Asn Asp Met Asp Lys
            260                 265                 270

Cys Ser Val Ser Gln Asp Pro Phe Thr Ser Leu His Glu Phe Leu Asp
        275                 280                 285

Trp Arg Lys Met Lys Leu Leu Pro Arg Lys Ser His Asp Asn Ala Gln
    290                 295                 300

Leu Val Ser Gly Val Tyr Phe Gln Gly Thr Thr Ile Gly Met Ala Pro
305                 310                 315                 320

Ile Met Ser Met Cys Thr Ala Asp Gln Ser Gly Gly Ile Val Met Asp
                325                 330                 335

His Ser Asp Asn Pro Leu Gly Ala Ala Val Thr Leu Ala His Glu Leu
            340                 345                 350

Gly His Asn Phe Gly Met Asn His Asp Thr Leu Asp Arg Gly Cys Ser
        355                 360                 365

Cys Gln Met Ala Val Glu Lys Gly Gly Cys Ile Met Asn Ala Ser Thr
    370                 375                 380

Gly Tyr Pro Phe Pro Met Val Phe Ser Ser Cys Ser Arg Lys Asp Leu
385                 390                 395                 400

Glu Thr Ser Leu Glu Lys Gly Met Gly Val Cys Leu Phe Asn Leu Pro
                405                 410                 415

Glu Val Arg Glu Ser Phe Gly Gly Gln Lys Cys Gly Asn Arg Phe Val
            420                 425                 430

Glu Glu Gly Glu Glu Cys Asp Cys Gly Glu Pro Glu Glu Cys Met Asn
        435                 440                 445

Arg Cys Cys Asn Ala Thr Thr Cys Thr Leu Lys Pro Asp Ala Val Cys
    450                 455                 460

Ala His Gly Leu Cys Cys Glu Asp Cys Gln Leu Lys Pro Ala Gly Thr
465                 470                 475                 480

Ala Cys Arg Asp Ser Ser Asn Ser Cys Asp Leu Pro Glu Phe Cys Thr
                485                 490                 495

Gly Ala Ser Pro His Cys Pro Ala Asn Val Tyr Leu His Asp Gly His
        500                 505                 510

Ser Cys Gln Asp Val Asp Gly Tyr Cys Tyr Asn Gly Ile Cys Gln Thr
        515                 520                 525

His Glu Gln Gln Cys Val Thr Leu Trp Gly Pro Gly Ala Lys Pro Ala
    530                 535                 540

Pro Gly Ile Cys Phe Glu Arg Val Asn Ser Ala Gly Asp Pro Tyr Gly
545                 550                 555                 560

Asn Cys Gly Lys Val Ser Lys Ser Ser Phe Ala Lys Cys Glu Met Arg
                565                 570                 575

Asp Ala Lys Cys Gly Lys Ile Gln Cys Gln Gly Gly Ala Ser Arg Pro
            580                 585                 590

Val Ile Gly Thr Asn Ala Val Ser Ile Glu Thr Asn Ile Pro Leu Gln
        595                 600                 605

Gln Gly Gly Arg Ile Leu Cys Arg Gly Thr His Val Tyr Leu Gly Asp
    610                 615                 620

Asp Met Pro Asp Pro Gly Leu Val Leu Ala Gly Thr Lys Cys Ala Asp
625                 630                 635                 640

Gly Lys Ile Cys Leu Asn Arg Gln Cys Gln Asn Ile Ser Val Phe Gly
                645                 650                 655

Val His Glu Cys Ala Met Gln Cys His Gly Arg Gly Val Cys Asn Asn
            660                 665                 670

Arg Lys Asn Cys His Cys Glu Ala His Trp Ala Pro Pro Phe Cys Asp
        675                 680                 685

Lys Phe Gly Phe Gly Gly Ser Thr Asp Ser Gly Pro Ile Arg Gln Ala
    690                 695                 700

Asp Asn Gln Gly Leu Thr Ile Gly Ile Leu Val Thr Ile Leu Cys Leu
705                 710                 715                 720

Leu Ala Ala Gly Phe Val Val Tyr Leu Lys Arg Lys Thr Leu Ile Arg
                725                 730                 735

Leu Leu Phe Thr Asn Lys Lys Thr Thr Ile Glu Lys Leu Arg Cys Val

740                 745                 750

Arg Pro Ser Arg Pro Pro Arg Gly Phe Gln Pro Cys Gln Ala His Leu
        755                 760                 765

Gly His Leu Gly Lys Gly Leu Met Arg Lys Pro Pro Asp Ser Tyr Pro
    770                 775                 780

Pro Lys Asp Asn Pro Arg Arg Leu Leu Gln Cys Gln Asn Val Asp Ile
785                 790                 795                 800

Ser Arg Pro Leu Asn Gly Leu Asn Val Pro Gln Pro Gln Ser Thr Gln
                805                 810                 815

Arg Val Leu Pro Pro Leu His Arg Ala Pro Arg Ala Pro Ser Val Pro
            820                 825                 830

Ala Arg Pro Leu Pro Ala Lys Pro Ala Leu Arg Gln Ala Gln Gly Thr
        835                 840                 845

Cys Lys Pro Asn Pro Pro Gln Lys Pro Leu Pro Ala Asp Pro Leu Ala
    850                 855                 860

Arg Thr Thr Arg Leu Thr His Ala Leu Ala Arg Thr Pro Gly Gln Trp
865                 870                 875                 880

Glu Thr Gly Leu Arg Leu Ala Pro Leu Arg Pro Ala Pro Gln Tyr Pro
                885                 890                 895

His Gln Val Pro Arg Ser Thr His Thr Ala Tyr Ile Lys
            900                 905

<210> 176
<211> 863
<212> PRT
<213> Homo sapiens

<400> 176
Met Arg Leu Ala Leu Leu Trp Ala Leu Gly Leu Leu Gly Ala Gly Ser
1               5                   10                  15

Pro Leu Pro Ser Trp Pro Leu Pro Asn Ile Gly Gly Thr Glu Glu Gln
            20                  25                  30

Gln Ala Glu Ser Glu Lys Ala Pro Arg Glu Pro Leu Glu Pro Gln Val
        35                  40                  45

Leu Gln Asp Asp Leu Pro Ile Ser Leu Lys Lys Val Leu Gln Thr Ser
    50                  55                  60

Leu Pro Glu Pro Leu Arg Ile Lys Leu Glu Leu Asp Gly Asp Ser His
65                  70                  75                  80

Ile Leu Glu Leu Leu Gln Asn Arg Glu Leu Val Pro Gly Arg Pro Thr
                85                  90                  95

Leu Val Trp Tyr Gln Pro Asp Gly Thr Arg Val Val Ser Glu Gly His
            100                 105                 110

Thr Leu Glu Asn Cys Cys Tyr Gln Gly Arg Val Arg Gly Tyr Ala Gly
        115                 120                 125

Ser Trp Val Ser Ile Cys Thr Cys Ser Gly Leu Arg Gly Leu Val Val
    130                 135                 140

Leu Thr Pro Glu Arg Ser Tyr Thr Leu Glu Gln Gly Pro Gly Asp Leu
145                 150                 155                 160

Gln Gly Pro Pro Ile Ile Ser Arg Ile Gln Asp Leu His Leu Pro Gly
                165                 170                 175

His Thr Cys Ala Leu Ser Trp Arg Glu Ser Val His Thr Gln Lys Pro
            180                 185                 190

Pro Glu His Pro Leu Gly Gln Arg His Ile Arg Arg Arg Arg Asp Val
        195                 200                 205

Val Thr Glu Thr Lys Thr Val Glu Leu Val Ile Val Ala Asp His Ser
    210                 215                 220

Glu Ala Gln Lys Tyr Arg Asp Phe Gln His Leu Leu Asn Arg Thr Leu
225                 230                 235                 240

Glu Val Ala Leu Leu Leu Asp Thr Phe Phe Arg Pro Leu Asn Val Arg
                245                 250                 255

Val Ala Leu Val Gly Leu Glu Ala Trp Thr Gln Arg Asp Leu Val Glu
            260                 265                 270

Ile Ser Pro Asn Pro Ala Val Thr Leu Glu Asn Phe Leu His Trp Arg
        275                 280                 285

Arg Ala His Leu Leu Pro Arg Leu Pro His Asp Ser Ala Gln Leu Val
    290                 295                 300

Thr Gly Thr Ser Phe Ser Gly Pro Thr Val Gly Met Ala Ile Gln Asn

305                 310                 315                 320

Ser Ile Cys Ser Pro Asp Phe Ser Gly Gly Val Asn Met Asp His Ser
                325                 330                 335

Thr Ser Ile Leu Gly Val Ala Ser Ser Ile Ala His Glu Leu Gly His
            340                 345                 350

Ser Leu Gly Leu Asp His Asp Leu Pro Gly Asn Ser Cys Pro Cys Pro
        355                 360                 365

Gly Pro Ala Pro Ala Lys Thr Cys Ile Met Glu Ala Ser Thr Asp Phe
    370                 375                 380

Leu Pro Gly Leu Asn Phe Ser Asn Cys Ser Arg Arg Ala Leu Glu Lys
385                 390                 395                 400

Ala Leu Leu Asp Gly Met Gly Ser Cys Leu Phe Glu Arg Leu Pro Ser
                405                 410                 415

Leu Pro Pro Met Ala Ala Phe Cys Gly Asn Met Phe Val Glu Pro Gly
            420                 425                 430

Glu Gln Cys Asp Cys Gly Phe Leu Asp Asp Cys Val Asp Pro Cys Cys
        435                 440                 445

Asp Ser Leu Thr Cys Gln Leu Arg Pro Gly Ala Gln Cys Ala Ser Asp
    450                 455                 460

Gly Pro Cys Cys Gln Asn Cys Gln Leu Arg Pro Ser Gly Trp Gln Cys
465                 470                 475                 480

Arg Pro Thr Arg Gly Asp Cys Asp Leu Pro Glu Phe Cys Pro Gly Asp
                485                 490                 495

Ser Ser Gln Cys Pro Pro Asp Val Ser Leu Gly Asp Gly Glu Pro Cys
            500                 505                 510

Ala Gly Gly Gln Ala Val Cys Met His Gly Arg Cys Ala Ser Tyr Ala
        515                 520                 525

Gln Gln Cys Gln Ser Leu Trp Gly Pro Gly Ala Gln Pro Ala Ala Pro
    530                 535                 540

Leu Cys Leu Gln Thr Ala Asn Thr Arg Gly Asn Ala Phe Gly Ser Cys
545                 550                 555                 560

Gly Arg Asn Pro Ser Gly Ser Tyr Val Ser Cys Thr Pro Arg Asp Ala
                565                 570                 575

Ile Cys Gly Gln Leu Gln Cys Gln Thr Gly Arg Thr Gln Pro Leu Leu
            580                 585                 590

Gly Ser Ile Arg Asp Leu Leu Trp Glu Thr Ile Asp Val Asn Gly Thr
        595                 600                 605

Glu Leu Asn Cys Ser Trp Val His Leu Asp Leu Gly Ser Asp Val Ala
    610                 615                 620

Gln Pro Leu Leu Thr Leu Pro Gly Thr Ala Cys Gly Pro Gly Leu Val
625                 630                 635                 640

Cys Ile Asp His Arg Cys Gln Arg Val Asp Leu Leu Gly Ala Gln Glu
                645                 650                 655

Cys Arg Ser Lys Cys His Gly His Gly Val Cys Asp Ser Asn Arg His
            660                 665                 670

Cys Tyr Cys Glu Glu Gly Trp Ala Pro Pro Asp Cys Thr Thr Gln Leu
        675                 680                 685

Lys Ala Thr Ser Ser Leu Thr Thr Gly Leu Leu Leu Ser Leu Leu Val
    690                 695                 700

Leu Leu Val Leu Val Met Leu Gly Ala Ser Tyr Trp Tyr Arg Ala Arg
705                 710                 715                 720

Leu His Gln Arg Leu Cys Gln Leu Lys Gly Pro Thr Cys Gln Tyr Arg
                725                 730                 735

Ala Ala Gln Ser Gly Pro Ser Glu Arg Pro Gly Pro Pro Gln Arg Ala
            740                 745                 750

Leu Leu Ala Arg Gly Thr Lys Gln Ala Ser Ala Leu Ser Phe Pro Ala
        755                 760                 765

Pro Pro Ser Arg Pro Leu Pro Pro Asp Pro Val Ser Lys Arg Leu Gln
    770                 775                 780

Ala Glu Leu Ala Asp Arg Pro Asn Pro Pro Thr Arg Pro Leu Pro Ala
785                 790                 795                 800

Asp Pro Val Val Arg Ser Pro Lys Ser Gln Gly Pro Ala Lys Pro Pro
                805                 810                 815

Pro Pro Arg Lys Pro Leu Pro Ala Asp Pro Gln Gly Arg Cys Pro Ser
            820                 825                 830

Gly Asp Leu Pro Gly Pro Gly Ala Gly Ile Pro Pro Leu Val Val Pro
        835                 840                 845

Ser Arg Pro Ala Pro Pro Pro Pro Thr Val Ser Ser Leu Tyr Leu
    850                 855                 860

<210> 177
<211> 824
<212> PRT
<213> Homo sapiens

<400> 177
Met Arg Gln Ser Leu Leu Phe Leu Thr Ser Val Val Pro Phe Val Leu
1               5                   10                  15

Ala Pro Arg Pro Pro Asp Asp Pro Gly Phe Gly Pro His Gln Arg Leu
            20                  25                  30

Glu Lys Leu Asp Ser Leu Leu Ser Asp Tyr Asp Ile Leu Ser Leu Ser
        35                  40                  45

Asn Ile Gln Gln His Ser Val Arg Lys Arg Asp Leu Gln Thr Ser Thr
    50                  55                  60

His Val Glu Thr Leu Leu Thr Phe Ser Ala Leu Lys Arg His Phe Lys
65                  70                  75                  80

Leu Tyr Leu Thr Ser Ser Thr Glu Arg Phe Ser Gln Asn Phe Lys Val
                85                  90                  95

Val Val Val Asp Gly Lys Asn Glu Ser Glu Tyr Thr Val Lys Trp Gln
            100                 105                 110

Asp Phe Phe Thr Gly His Val Val Gly Glu Pro Asp Ser Arg Val Leu
        115                 120                 125

Ala His Ile Arg Asp Asp Asp Val Ile Ile Arg Ile Asn Thr Asp Gly
    130                 135                 140

Ala Glu Tyr Asn Ile Glu Pro Leu Trp Arg Phe Val Asn Asp Thr Lys
145                 150                 155                 160

Asp Lys Arg Met Leu Val Tyr Lys Ser Glu Asp Ile Lys Asn Val Ser
                165                 170                 175

Arg Leu Gln Ser Pro Lys Val Cys Gly Tyr Leu Lys Val Asp Asn Glu
            180                 185                 190

Glu Leu Leu Pro Lys Gly Leu Val Asp Arg Glu Pro Pro Glu Glu Leu
        195                 200                 205

Val His Arg Val Lys Arg Arg Ala Asp Pro Asp Pro Met Lys Asn Thr
    210                 215                 220

Cys Lys Leu Leu Val Val Ala Asp His Arg Phe Tyr Arg Tyr Met Gly
225                 230                 235                 240

Arg Gly Glu Glu Ser Thr Thr Thr Asn Tyr Leu Ile Glu Leu Ile Asp
                245                 250                 255

Arg Val Asp Asp Ile Tyr Arg Asn Thr Ser Trp Asp Asn Ala Gly Phe
            260                 265                 270

Lys Gly Tyr Gly Ile Gln Ile Glu Gln Ile Arg Ile Leu Lys Ser Pro
        275                 280                 285

Gln Glu Val Lys Pro Gly Glu Lys His Tyr Asn Met Ala Lys Ser Tyr
    290                 295                 300

Pro Asn Glu Glu Lys Asp Ala Trp Asp Val Lys Met Leu Leu Glu Gln
305                 310                 315                 320

Phe Ser Phe Asp Ile Ala Glu Glu Ala Ser Lys Val Cys Leu Ala His
                325                 330                 335

Leu Phe Thr Tyr Gln Asp Phe Asp Met Gly Thr Leu Gly Leu Ala Tyr
            340                 345                 350

Val Gly Ser Pro Arg Ala Asn Ser His Gly Gly Val Cys Pro Lys Ala
        355                 360                 365

Tyr Tyr Ser Pro Val Gly Lys Lys Asn Ile Tyr Leu Asn Ser Gly Leu
    370                 375                 380

Thr Ser Thr Lys Asn Tyr Gly Lys Thr Ile Leu Thr Lys Glu Ala Asp
385                 390                 395                 400

Leu Val Thr Thr His Glu Leu Gly His Asn Phe Gly Ala Glu His Asp
                405                 410                 415

Pro Asp Gly Leu Ala Glu Cys Ala Pro Asn Glu Asp Gln Gly Gly Lys
            420                 425                 430

Tyr Val Met Tyr Pro Ile Ala Val Ser Gly Asp His Glu Asn Asn Lys
        435                 440                 445

Met Phe Ser Asn Cys Ser Lys Gln Ser Ile Tyr Lys Thr Ile Glu Ser
    450                 455                 460

Lys Ala Gln Glu Cys Phe Gln Glu Arg Ser Asn Lys Val Cys Gly Asn
465                 470                 475                 480

Ser Arg Val Asp Glu Gly Glu Glu Cys Asp Pro Gly Ile Met Tyr Leu
                485                 490                 495

Asn Asn Asp Thr Cys Cys Asn Ser Asp Cys Thr Leu Lys Glu Gly Val
            500                 505                 510

Gln Cys Ser Asp Arg Asn Ser Pro Cys Cys Lys Asn Cys Gln Phe Glu
        515                 520                 525

Thr Ala Gln Lys Lys Cys Gln Glu Ala Ile Asn Ala Thr Cys Lys Gly
    530                 535                 540

Val Ser Tyr Cys Thr Gly Asn Ser Ser Glu Cys Pro Pro Pro Gly Asn
545                 550                 555                 560

Ala Glu Asp Asp Thr Val Cys Leu Asp Leu Gly Lys Cys Lys Asp Gly
                565                 570                 575

Lys Cys Ile Pro Phe Cys Glu Arg Glu Gln Gln Leu Glu Ser Cys Ala
            580                 585                 590

Cys Asn Glu Thr Asp Asn Ser Cys Lys Val Cys Cys Arg Asp Leu Ser
        595                 600                 605

Gly Arg Cys Val Pro Tyr Val Asp Ala Glu Gln Lys Asn Leu Phe Leu
    610                 615                 620

Arg Lys Gly Lys Pro Cys Thr Val Gly Phe Cys Asp Met Asn Gly Lys
625                 630                 635                 640

Cys Glu Lys Arg Val Gln Asp Val Ile Glu Arg Phe Trp Asp Phe Ile
                645                 650                 655

Asp Gln Leu Ser Ile Asn Thr Phe Gly Lys Phe Leu Ala Asp Asn Ile
            660                 665                 670

Val Gly Ser Val Leu Val Phe Ser Leu Ile Phe Trp Ile Pro Phe Ser
        675                 680                 685

Ile Leu Val His Cys Val Asp Lys Lys Leu Asp Lys Gln Tyr Glu Ser
    690                 695                 700

Leu Ser Leu Phe His Pro Ser Asn Val Glu Met Leu Ser Ser Met Asp
705                 710                 715                 720

Ser Ala Ser Val Arg Ile Ile Lys Pro Phe Pro Ala Pro Gln Thr Pro
                725                 730                 735

Gly Arg Leu Gln Pro Ala Pro Val Ile Pro Ser Ala Pro Ala Ala Pro
            740                 745                 750

Lys Leu Asp His Gln Arg Met Asp Thr Ile Gln Glu Asp Pro Ser Thr
        755                 760                 765

Asp Ser His Met Asp Glu Asp Gly Phe Glu Lys Asp Pro Phe Pro Asn
    770                 775                 780

Ser Ser Thr Ala Ala Lys Ser Phe Glu Asp Leu Thr Asp His Pro Val
785                 790                 795                 800

Thr Arg Ser Glu Lys Ala Ala Ser Phe Lys Leu Gln Arg Gln Asn Arg
                805                 810                 815

Val Asp Ser Lys Glu Thr Glu Cys
            820

<210> 178
<211> 739
<212> PRT
<213> Homo sapiens

<400> 178
Met Phe Leu Leu Leu Ala Leu Leu Thr Glu Leu Gly Arg Leu Gln Ala
1               5                   10                  15

His Glu Gly Ser Glu Gly Ile Phe Leu His Val Thr Val Pro Arg Lys
            20                  25                  30

Ile Lys Ser Asn Asp Ser Glu Val Ser Glu Arg Lys Met Ile Tyr Ile
        35                  40                  45

Ile Thr Ile Asp Gly Gln Pro Tyr Thr Leu His Leu Gly Lys Gln Ser
    50                  55                  60

Phe Leu Pro Gln Asn Phe Leu Val Tyr Thr Tyr Asn Glu Thr Gly Ser
65                  70                  75                  80

Leu His Ser Val Ser Pro Tyr Phe Met Met His Cys His Tyr Gln Gly
                85                  90                  95

Tyr Ala Ala Glu Phe Pro Asn Ser Phe Val Thr Leu Ser Ile Cys Ser
            100                 105                 110

Gly Leu Arg Gly Phe Leu Gln Phe Glu Asn Ile Ser Tyr Gly Ile Glu
        115                 120                 125

Pro Val Glu Ser Ser Ala Arg Phe Glu His Ile Ile Tyr Gln Met Lys
    130                 135                 140

Asn Asn Asp Pro Asn Val Ser Ile Leu Ala Val Asn Tyr Ser His Ile
145                 150                 155                 160

Trp Gln Lys Asp Gln Pro Tyr Lys Val Pro Leu Asn Ser Gln Ile Lys
                165                 170                 175

Asn Leu Ser Lys Leu Leu Pro Gln Tyr Leu Glu Ile Tyr Ile Ile Val
            180                 185                 190

Glu Lys Ala Leu Tyr Asp Tyr Met Gly Ser Glu Met Met Ala Val Thr
        195                 200                 205

Gln Lys Ile Val Gln Val Ile Gly Leu Val Asn Thr Met Phe Thr Gln
    210                 215                 220

Phe Lys Leu Thr Val Ile Leu Ser Ser Leu Glu Leu Trp Ser Asn Glu
225                 230                 235                 240

Asn Gln Ile Ser Thr Ser Gly Asp Ala Asp Asp Ile Leu Gln Arg Phe
                245                 250                 255

Leu Ala Trp Lys Arg Asp Tyr Leu Ile Leu Arg Pro His Asp Ile Ala
            260                 265                 270

Tyr Leu Leu Val Tyr Arg Lys His Pro Lys Tyr Val Gly Ala Thr Phe
        275                 280                 285

Pro Gly Thr Val Cys Asn Lys Ser Tyr Asp Ala Gly Ile Ala Met Tyr
    290                 295                 300

Pro Asp Ala Ile Gly Leu Glu Gly Phe Ser Val Ile Ile Ala Gln Leu
305                 310                 315                 320

Leu Gly Leu Asn Val Gly Leu Thr Tyr Asp Asp Ile Thr Gln Cys Phe
                325                 330                 335

Cys Leu Arg Ala Thr Cys Ile Met Asn His Glu Ala Val Ser Ala Ser
            340                 345                 350

Gly Arg Lys Ile Phe Ser Asn Cys Ser Met His Asp Tyr Arg Tyr Phe
        355                 360                 365

Val Ser Lys Phe Glu Thr Lys Cys Leu Gln Lys Leu Ser Asn Leu Gln
    370                 375                 380

Pro Leu His Gln Asn Gln Pro Val Cys Gly Asn Gly Ile Leu Glu Ser
385                 390                 395                 400

Asn Glu Glu Cys Asp Cys Gly Asn Lys Asn Glu Cys Gln Phe Lys Lys
                405                 410                 415

Cys Cys Asp Tyr Asn Thr Cys Lys Leu Lys Gly Ser Val Lys Cys Gly
            420                 425                 430

Ser Gly Pro Cys Cys Thr Ser Lys Cys Glu Leu Ser Ile Ala Gly Thr
        435                 440                 445

Pro Cys Arg Lys Ser Ile Asp Pro Glu Cys Asp Phe Thr Glu Tyr Cys
    450                 455                 460

Asn Gly Thr Ser Ser Asn Cys Val Pro Asp Thr Tyr Ala Leu Asn Gly
465                 470                 475                 480

Arg Leu Cys Lys Leu Gly Thr Ala Tyr Cys Tyr Asn Gly Gln Cys Gln
                485                 490                 495

Thr Thr Asp Asn Gln Cys Ala Lys Ile Phe Gly Lys Gly Ala Gln Gly
            500                 505                 510

Ala Pro Phe Ala Cys Phe Lys Glu Val Asn Ser Leu His Glu Arg Ser
        515                 520                 525

Glu Asn Cys Gly Phe Lys Asn Ser Gln Pro Leu Pro Cys Glu Arg Lys
    530                 535                 540

Asp Val Leu Cys Gly Lys Leu Ala Cys Val Gln Pro His Lys Asn Ala
545                 550                 555                 560

Asn Lys Ser Asp Ala Gln Ser Thr Val Tyr Ser Tyr Ile Gln Asp His
                565                 570                 575

Val Cys Val Ser Ile Ala Thr Gly Ser Ser Met Arg Ser Asp Gly Thr

580                 585                 590

Asp Asn Ala Tyr Val Ala Asp Gly Thr Met Cys Gly Pro Glu Met Tyr
        595                 600                 605

Cys Val Asn Lys Thr Cys Arg Lys Val His Leu Met Gly Tyr Asn Cys
    610                 615                 620

Asn Ala Thr Thr Lys Cys Lys Gly Lys Gly Ile Cys Asn Asn Phe Gly
625                 630                 635                 640

Asn Cys Gln Cys Phe Pro Gly His Arg Pro Pro Asp Cys Lys Phe Gln
                645                 650                 655

Phe Gly Ser Pro Gly Gly Ser Ile Asp Asp Gly Asn Phe Gln Lys Ser
            660                 665                 670

Gly Asp Phe Tyr Thr Glu Lys Gly Tyr Asn Thr His Trp Asn Asn Trp
        675                 680                 685

Phe Ile Leu Ser Phe Cys Ile Phe Leu Pro Phe Phe Ile Val Phe Thr
    690                 695                 700

Thr Val Ile Phe Lys Arg Asn Glu Ile Ser Lys Ser Cys Asn Arg Glu
705                 710                 715                 720

Asn Ala Glu Tyr Asn Arg Asn Ser Ser Val Val Ser Glu Ser Asp Asp
                725                 730                 735

Val Gly His

<210> 179
<211> 918
<212> PRT
<213> Homo sapiens

<400> 179
Met Pro Gly Gly Ala Gly Ala Ala Arg Leu Cys Leu Leu Ala Phe Ala
1               5                   10                  15

Leu Gln Pro Leu Arg Pro Arg Ala Ala Arg Glu Pro Gly Trp Thr Arg
            20                  25                  30

Gly Ser Glu Glu Gly Ser Pro Lys Leu Gln His Glu Leu Ile Ile Pro
        35                  40                  45

Gln Trp Lys Thr Ser Glu Ser Pro Val Arg Glu Lys His Pro Leu Lys
    50                  55                  60

Ala Glu Leu Arg Val Met Ala Glu Gly Arg Glu Leu Ile Leu Asp Leu
65                  70                  75                  80

Glu Lys Asn Glu Gln Leu Phe Ala Pro Ser Tyr Thr Glu Thr His Tyr
                85                  90                  95

Thr Ser Ser Gly Asn Pro Gln Thr Thr Thr Arg Lys Leu Glu Asp His
            100                 105                 110

Cys Phe Tyr His Gly Thr Val Arg Glu Thr Glu Leu Ser Ser Val Thr
        115                 120                 125

Leu Ser Thr Cys Arg Gly Ile Arg Gly Leu Ile Thr Val Ser Ser Asn
    130                 135                 140

Leu Ser Tyr Val Ile Glu Pro Leu Pro Asp Ser Lys Gly Gln His Leu
145                 150                 155                 160

Ile Tyr Arg Ser Glu His Leu Lys Pro Pro Pro Gly Asn Cys Gly Phe
                165                 170                 175

Glu His Ser Lys Pro Thr Thr Arg Asp Trp Ala Leu Gln Phe Thr Gln
            180                 185                 190

Gln Thr Lys Lys Arg Pro Arg Arg Met Lys Arg Glu Asp Leu Asn Ser
        195                 200                 205

Met Lys Tyr Val Glu Leu Tyr Leu Val Ala Asp Tyr Leu Glu Phe Gln
    210                 215                 220

Lys Asn Arg Arg Asp Gln Asp Ala Thr Lys His Lys Leu Ile Glu Ile
225                 230                 235                 240

Ala Asn Tyr Val Asp Lys Phe Tyr Arg Ser Leu Asn Ile Arg Ile Ala
                245                 250                 255

Leu Val Gly Leu Glu Val Trp Thr His Gly Asn Met Cys Glu Val Ser
            260                 265                 270

Glu Asn Pro Tyr Ser Thr Leu Trp Ser Phe Leu Ser Trp Arg Arg Lys
        275                 280                 285

Leu Leu Ala Gln Lys Tyr His Asp Asn Ala Gln Leu Ile Thr Gly Met
    290                 295                 300

Ser Phe His Gly Thr Thr Ile Gly Leu Ala Pro Leu Met Ala Met Cys

305                 310                 315                 320

Ser Val Tyr Gln Ser Gly Gly Val Asn Met Asp His Ser Glu Asn Ala
                325                 330                 335

Ile Gly Val Ala Ala Thr Met Ala His Glu Met Gly His Asn Phe Gly
            340                 345                 350

Met Thr His Asp Ser Ala Asp Cys Cys Ser Ala Ser Ala Ala Asp Gly
        355                 360                 365

Gly Cys Ile Met Ala Ala Ala Thr Gly His Pro Phe Pro Lys Val Phe
    370                 375                 380

Asn Gly Cys Asn Arg Arg Glu Leu Asp Arg Tyr Leu Gln Ser Gly Gly
385                 390                 395                 400

Gly Met Cys Leu Ser Asn Met Pro Asp Thr Arg Met Leu Tyr Gly Gly
                405                 410                 415

Arg Arg Cys Gly Asn Gly Tyr Leu Glu Asp Gly Glu Glu Cys Asp Cys
            420                 425                 430

Gly Glu Glu Glu Glu Cys Asn Asn Pro Cys Cys Asn Ala Ser Asn Cys
        435                 440                 445

Thr Leu Arg Pro Gly Ala Glu Cys Ala His Gly Ser Cys Cys His Gln
    450                 455                 460

Cys Lys Leu Leu Ala Pro Gly Thr Leu Cys Arg Glu Gln Ala Arg Gln
465                 470                 475                 480

Cys Asp Leu Pro Glu Phe Cys Thr Gly Lys Ser Pro His Cys Pro Thr
                485                 490                 495

Asn Phe Tyr Gln Met Asp Gly Thr Pro Cys Glu Gly Gly Gln Ala Tyr
            500                 505                 510

Cys Tyr Asn Gly Met Cys Leu Thr Tyr Gln Glu Gln Cys Gln Gln Leu
        515                 520                 525

Trp Gly Pro Gly Ala Arg Pro Ala Pro Asp Leu Cys Phe Glu Lys Val
    530                 535                 540

Asn Val Ala Gly Asp Thr Phe Gly Asn Cys Gly Lys Asp Met Asn Gly
545                 550                 555                 560

Glu His Arg Lys Cys Asn Met Arg Asp Ala Lys Cys Gly Lys Ile Gln
                565                 570                 575

Cys Gln Ser Ser Glu Ala Arg Pro Leu Glu Ser Asn Ala Val Pro Ile
            580                 585                 590

Asp Thr Thr Ile Ile Met Asn Gly Arg Gln Ile Gln Cys Arg Gly Thr
        595                 600                 605

His Val Tyr Arg Gly Pro Glu Glu Glu Gly Asp Met Leu Asp Pro Gly
    610                 615                 620

Leu Val Met Thr Gly Thr Lys Cys Gly Tyr Asn His Ile Cys Phe Glu
625                 630                 635                 640

Gly Gln Cys Arg Asn Thr Ser Phe Phe Glu Thr Glu Gly Cys Gly Lys
                645                 650                 655

Lys Cys Asn Gly His Gly Val Cys Asn Asn Asn Gln Asn Cys His Cys
            660                 665                 670

Leu Pro Gly Trp Ala Pro Pro Phe Cys Asn Thr Pro Gly His Gly Gly
        675                 680                 685

Ser Ile Asp Ser Gly Pro Met Pro Pro Glu Ser Val Gly Pro Val Val
    690                 695                 700

Ala Gly Val Leu Val Ala Ile Leu Val Leu Ala Val Leu Met Leu Met
705                 710                 715                 720

Tyr Tyr Cys Cys Arg Gln Asn Asn Lys Leu Gly Gln Leu Lys Pro Ser
                725                 730                 735

Ala Leu Pro Ser Lys Leu Arg Gln Gln Phe Ser Cys Pro Phe Arg Val
            740                 745                 750

Ser Gln Asn Ser Gly Thr Gly His Ala Asn Pro Thr Phe Lys Leu Gln
        755                 760                 765

Thr Pro Gln Gly Lys Arg Lys Val Ile Asn Thr Pro Glu Ile Leu Arg
    770                 775                 780

Lys Pro Ser Gln Pro Pro Pro Arg Pro Pro Pro Asp Tyr Leu Arg Gly
785                 790                 795                 800

Gly Ser Pro Pro Ala Pro Leu Pro Ala His Leu Ser Arg Ala Ala Arg
                805                 810                 815

Asn Ser Pro Gly Pro Gly Ser Gln Ile Glu Arg Thr Glu Ser Ser Arg
            820                 825                 830

Arg Pro Pro Pro Ser Arg Pro Ile Pro Pro Ala Pro Asn Cys Ile Val
        835                 840                 845

Ser Gln Asp Phe Ser Arg Pro Arg Pro Pro Gln Lys Ala Leu Pro Ala
    850                 855                 860

Asn Pro Val Pro Gly Arg Arg Ser Leu Pro Arg Pro Gly Gly Ala Ser
865                 870                 875                 880

Pro Leu Arg Pro Pro Gly Ala Gly Pro Gln Gln Ser Arg Pro Leu Ala
                885                 890                 895

Ala Leu Ala Pro Lys Phe Pro Glu Tyr Arg Ser Gln Arg Ala Gly Gly
            900                 905                 910

Met Ile Ser Ser Lys Ile
        915

<210> 180
<211> 776
<212> PRT
<213> Homo sapiens

<400> 180
Met Val Gln Leu His Gln Asp Thr Asp Pro Gln Ile Pro Lys Gly Gln
1               5                   10                  15

Pro Cys Thr Leu Asn Ser Ser Glu Gly Gly Ala Arg Pro Ala Val Pro
            20                  25                  30

His Thr Leu Phe Ser Ser Ala Leu Asp Arg Trp Leu His Asn Asp Ser
        35                  40                  45

Phe Ile Met Ala Val Gly Glu Pro Leu Val His Ile Arg Val Thr Leu
    50                  55                  60

Leu Leu Leu Trp Phe Gly Met Phe Leu Ser Ile Ser Gly His Ser Gln
65                  70                  75                  80

Ala Arg Pro Ser Gln Tyr Phe Thr Ser Pro Glu Val Val Ile Pro Leu
                85                  90                  95

Lys Val Ile Ser Arg Gly Arg Gly Ala Lys Ala Pro Gly Trp Leu Ser
            100                 105                 110

Tyr Ser Leu Arg Phe Gly Gly Gln Arg Tyr Ile Val His Met Arg Val
        115                 120                 125

Asn Lys Leu Leu Phe Ala Ala His Leu Pro Val Phe Thr Tyr Thr Glu
    130                 135                 140

Gln His Ala Leu Leu Gln Asp Gln Pro Phe Ile Gln Asp Asp Cys Tyr
145                 150                 155                 160

Tyr His Gly Tyr Val Glu Gly Val Pro Glu Ser Leu Val Ala Leu Ser
                165                 170                 175

Thr Cys Ser Gly Gly Phe Leu Gly Met Leu Gln Ile Asn Asp Leu Val
            180                 185                 190

Tyr Glu Ile Lys Pro Ile Ser Val Ser Ala Thr Phe Glu His Leu Val
        195                 200                 205

Tyr Lys Ile Asp Ser Asp Asp Thr Gln Phe Pro Pro Met Arg Cys Gly
    210                 215                 220

Leu Thr Glu Glu Lys Ile Ala His Gln Met Glu Leu Gln Leu Ser Tyr
225                 230                 235                 240

Asn Phe Thr Leu Lys Gln Ser Ser Phe Val Gly Trp Trp Thr His Gln
                245                 250                 255

Arg Phe Val Glu Leu Val Val Val Val Asp Asn Ile Arg Tyr Leu Phe
            260                 265                 270

Ser Gln Ser Asn Ala Thr Thr Val Gln His Glu Val Phe Asn Val Val
        275                 280                 285

Asn Ile Val Asp Ser Phe Tyr His Pro Leu Glu Val Asp Val Ile Leu
    290                 295                 300

Thr Gly Ile Asp Ile Trp Thr Ala Ser Asn Pro Leu Pro Thr Ser Gly
305                 310                 315                 320

Asp Leu Asp Asn Val Leu Glu Asp Phe Ser Ile Trp Lys Asn Tyr Asn
                325                 330                 335

Leu Asn Asn Arg Leu Gln His Asp Val Ala His Leu Phe Ile Lys Asp
            340                 345                 350

Thr Gln Gly Met Lys Leu Gly Val Ala Tyr Val Lys Gly Ile Cys Gln
        355                 360                 365

Asn Pro Phe Asn Thr Gly Val Asp Val Phe Glu Asp Asn Arg Leu Val
    370                 375                 380

Val Phe Ala Ile Thr Leu Gly His Glu Leu Gly His Asn Leu Gly Met
385                 390                 395                 400

Gln His Asp Thr Gln Trp Cys Val Cys Glu Leu Gln Trp Cys Ile Met
                405                 410                 415

His Ala Tyr Arg Lys Val Thr Thr Lys Phe Ser Asn Cys Ser Tyr Ala
            420                 425                 430

Gln Tyr Trp Asp Ser Thr Ile Ser Ser Gly Leu Cys Ile Gln Pro Pro
        435                 440                 445

Pro Tyr Pro Gly Asn Ile Phe Arg Leu Lys Tyr Cys Gly Asn Leu Val
    450                 455                 460

Val Glu Glu Gly Glu Glu Cys Asp Cys Gly Thr Ile Arg Gln Cys Ala
465                 470                 475                 480

Lys Asp Pro Cys Cys Leu Leu Asn Cys Thr Leu His Pro Gly Ala Ala
                485                 490                 495

Cys Ala Phe Gly Ile Cys Cys Lys Asp Cys Lys Phe Leu Pro Ser Gly
            500                 505                 510

Thr Leu Cys Arg Gln Gln Val Gly Glu Cys Asp Leu Pro Glu Trp Cys
        515                 520                 525

Asn Gly Thr Ser His Gln Cys Pro Asp Asp Val Tyr Val Gln Asp Gly
    530                 535                 540

Ile Ser Cys Asn Val Asn Ala Phe Cys Tyr Glu Lys Thr Cys Asn Asn
545                 550                 555                 560

His Asp Ile Gln Cys Lys Glu Ile Phe Gly Gln Asp Ala Arg Ser Ala
                565                 570                 575

Ser Gln Ser Cys Tyr Gln Glu Ile Asn Thr Gln Gly Asn Arg Phe Gly
            580                 585                 590

His Cys Gly Ile Val Gly Thr Thr Tyr Val Lys Cys Trp Thr Pro Asp
        595                 600                 605

Ile Met Cys Gly Arg Val Gln Cys Glu Asn Val Gly Val Ile Pro Asn
    610                 615                 620

Leu Ile Glu His Ser Thr Val Gln Gln Phe His Leu Asn Asp Thr Thr
625                 630                 635                 640

Cys Trp Gly Thr Asp Tyr His Leu Gly Met Ala Ile Pro Asp Ile Gly
                645                 650                 655

Glu Val Lys Asp Gly Thr Val Cys Gly Pro Glu Lys Ile Cys Ile Arg
            660                 665                 670

Lys Lys Cys Ala Ser Met Val His Leu Ser Gln Ala Cys Gln Pro Lys
        675                 680                 685

Thr Cys Asn Met Arg Gly Ile Cys Asn Asn Lys Gln His Cys His Cys
    690                 695                 700

Asn His Glu Trp Ala Pro Pro Tyr Cys Lys Asp Lys Gly Tyr Gly Gly
705                 710                 715                 720

Ser Ala Asp Ser Gly Pro Pro Pro Lys Asn Asn Met Glu Gly Leu Asn
                725                 730                 735

Val Met Gly Lys Leu Arg Tyr Leu Ser Leu Leu Cys Leu Leu Pro Leu
            740                 745                 750

Val Ala Phe Leu Leu Phe Cys Leu His Val Leu Phe Lys Lys Arg Thr
        755                 760                 765

Lys Ser Lys Glu Asp Glu Glu Gly
    770                 775

<210> 181
<211> 722
<212> PRT
<213> Homo sapiens

<400> 181
Met Ala Val Asp Gly Thr Leu Val Tyr Ile Arg Val Thr Leu Leu Leu
1               5                   10                  15

Leu Trp Leu Gly Val Phe Leu Ser Ile Ser Gly Tyr Cys Gln Ala Gly
            20                  25                  30

Pro Ser Gln His Phe Thr Ser Pro Glu Val Val Ile Pro Leu Lys Val
        35                  40                  45

Ile Ser Arg Gly Arg Ser Ala Lys Ala Pro Gly Trp Leu Ser Tyr Ser
    50                  55                  60

Leu Arg Phe Gly Gly Gln Lys His Val Val His Met Arg Val Lys Lys
65                  70                  75                  80

Leu Leu Val Ser Arg His Leu Pro Val Phe Thr Tyr Thr Asp Glu Arg
                85                  90                  95

Ala Leu Leu Glu Asp Gln Leu Phe Ile Pro Asp Asp Cys Tyr Tyr His
            100                 105                 110

Gly Tyr Val Glu Gly Ala Pro Glu Ser Leu Val Val Phe Ser Ala Cys
        115                 120                 125

Phe Gly Gly Phe Arg Gly Val Leu Lys Ile Ser Gly Leu Thr Tyr Glu
    130                 135                 140

Ile Glu Pro Ile Arg His Ser Ala Thr Phe Glu His Leu Val Tyr Lys
145                 150                 155                 160

Val Asn Ser Asn Glu Thr Gln Phe Pro Ala Met Arg Cys Gly Leu Thr
                165                 170                 175

Glu Lys Glu Val Ala Arg Gln Gln Leu Glu Phe Glu Glu Ala Glu Asn
            180                 185                 190

Ser Ala Leu Glu Pro Lys Ser Ala Gly Asp Trp Trp Thr His Ala Trp
        195                 200                 205

Phe Leu Glu Leu Val Val Val Val Asn His Asp Phe Phe Ile Tyr Ser
    210                 215                 220

Gln Ser Asn Ile Ser Lys Val Gln Glu Asp Val Phe Leu Val Val Asn
225                 230                 235                 240

Ile Val Asp Ser Met Tyr Gln Gln Leu Gly Thr Tyr Ile Ile Leu Ile
                245                 250                 255

Gly Ile Glu Ile Trp Asn Gln Gly Asn Val Phe Pro Met Thr Ser Ile
            260                 265                 270

Glu Gln Val Leu Asn Asp Phe Ser Gln Trp Lys Gln Ile Ser Leu Ser
        275                 280                 285

Gln Leu Gln His Asp Ala Ala His Met Phe Ile Lys Asn Ser Leu Ile
    290                 295                 300

Ser Ile Leu Gly Leu Ala Tyr Val Ala Gly Ile Cys Arg Pro Pro Ile
305                 310                 315                 320

Asp Cys Gly Val Asp Asn Phe Gln Gly Asp Thr Trp Ser Leu Phe Ala
                325                 330                 335

Asn Thr Val Ala His Glu Leu Gly His Thr Leu Gly Met Gln His Asp
            340                 345                 350

Glu Glu Phe Cys Phe Cys Gly Glu Arg Gly Cys Ile Met Asn Thr Phe
        355                 360                 365

Arg Val Pro Ala Glu Lys Phe Thr Asn Cys Ser Tyr Ala Asp Phe Met
    370                 375                 380

Lys Thr Thr Leu Asn Gln Gly Ser Cys Leu His Asn Pro Pro Arg Leu
385                 390                 395                 400

Gly Glu Ile Phe Met Leu Lys Arg Cys Gly Asn Gly Val Val Glu Arg
                405                 410                 415

Glu Glu Gln Cys Asp Cys Gly Ser Val Gln Gln Cys Glu Gln Asp Ala
            420                 425                 430

Cys Cys Leu Leu Asn Cys Thr Leu Arg Pro Gly Ala Ala Cys Ala Phe
        435                 440                 445

Gly Leu Cys Cys Lys Asp Cys Lys Phe Met Pro Ser Gly Glu Leu Cys
    450                 455                 460

Arg Gln Glu Val Asn Glu Cys Asp Leu Pro Glu Trp Cys Asn Gly Thr
465                 470                 475                 480

Ser His Gln Cys Pro Glu Asp Arg Tyr Val Gln Asp Gly Ile Pro Cys
                485                 490                 495

Ser Asp Ser Ala Tyr Cys Tyr Gln Lys Arg Cys Asn Asn His Asp Gln
            500                 505                 510

His Cys Arg Glu Ile Phe Gly Lys Asp Ala Lys Ser Ala Ser Gln Asn
        515                 520                 525

Cys Tyr Lys Glu Ile Asn Ser Gln Gly Asn Arg Phe Gly His Cys Gly
    530                 535                 540

Ile Asn Gly Thr Thr Tyr Leu Lys Cys His Ile Ser Asp Val Phe Cys
545                 550                 555                 560

Gly Arg Val Gln Cys Glu Asn Val Arg Asp Ile Pro Leu Leu Gln Asp

                565                 570                 575

His Phe Thr Leu Gln His Thr His Ile Asn Gly Val Thr Cys Trp Gly
            580                 585                 590

Ile Asp Tyr His Leu Arg Met Asn Ile Ser Asp Ile Gly Glu Val Lys
        595                 600                 605

Asp Gly Thr Val Cys Gly Pro Gly Lys Ile Cys Ile His Lys Lys Cys
    610                 615                 620

Val Ser Leu Ser Val Leu Ser His Val Cys Leu Pro Glu Thr Cys Asn
625                 630                 635                 640

Met Lys Gly Ile Cys Asn Asn Lys His His Cys His Cys Gly Tyr Gly
                645                 650                 655

Trp Ser Pro Pro Tyr Cys Gln His Arg Gly Tyr Gly Gly Ser Ile Asp
            660                 665                 670

Ser Gly Pro Ala Ser Ala Lys Arg Gly Val Phe Leu Pro Leu Ile Val
        675                 680                 685

Ile Pro Ser Leu Ser Val Leu Thr Phe Leu Phe Thr Val Gly Leu Leu
    690                 695                 700

Met Tyr Leu Arg Gln Cys Ser Gly Pro Lys Glu Thr Lys Ala His Ser
705                 710                 715                 720

Ser Gly

<210> 182
<211> 906
<212> PRT
<213> Homo sapiens

<400> 182

<210> 183
<211> 832
<212> PRT
<213> Homo sapiens

<400> 183
Met Lys Pro Pro Gly Ser Ser Ser Arg Gln Pro Pro Leu Ala Gly Cys
1               5                   10                  15

Ser Leu Ala Gly Ala Ser Cys Gly Pro Gln Arg Gly Pro Ala Gly Ser
            20                  25                  30

Val Pro Ala Ser Ala Pro Ala Arg Thr Pro Pro Cys Arg Leu Leu Leu
        35                  40                  45

Val Leu Leu Leu Leu Pro Pro Leu Ala Ala Ser Ser Arg Pro Arg Ala
    50                  55                  60

Trp Gly Ala Ala Ala Pro Ser Ala Pro His Trp Asn Glu Thr Ala Glu
65                  70                  75                  80

Lys Asn Leu Gly Val Leu Ala Asp Glu Asp Asn Thr Leu Gln Gln Asn
                85                  90                  95

Ser Ser Ser Asn Ile Ser Tyr Ser Asn Ala Met Gln Lys Glu Ile Thr
            100                 105                 110

Leu Pro Ser Arg Leu Ile Tyr Tyr Ile Asn Gln Asp Ser Glu Ser Pro
        115                 120                 125

Tyr His Val Leu Asp Thr Lys Ala Arg His Gln Gln Lys His Asn Lys
    130                 135                 140

Ala Val His Leu Ala Gln Ala Ser Phe Gln Ile Glu Ala Phe Gly Ser
145                 150                 155                 160

Lys Phe Ile Leu Asp Leu Ile Leu Asn Asn Gly Leu Leu Ser Ser Asp
                165                 170                 175

Tyr Val Glu Ile His Tyr Glu Asn Gly Lys Pro Gln Tyr Ser Lys Gly
            180                 185                 190

Gly Glu His Cys Tyr Tyr His Gly Ser Ile Arg Gly Val Lys Asp Ser
        195                 200                 205

Lys Val Ala Leu Ser Thr Cys Asn Gly Leu His Gly Met Phe Glu Asp
    210                 215                 220

Asp Thr Phe Val Tyr Met Ile Glu Pro Leu Glu Leu Val His Asp Glu
225                 230                 235                 240

Lys Ser Thr Gly Arg Pro His Ile Ile Gln Lys Thr Leu Ala Gly Gln
                245                 250                 255

Tyr Ser Lys Gln Met Lys Asn Leu Thr Met Glu Arg Gly Asp Gln Trp
            260                 265                 270

Pro Phe Leu Ser Glu Leu Gln Trp Leu Lys Arg Arg Lys Arg Ala Val
        275                 280                 285

Asn Pro Ser Arg Gly Ile Phe Glu Glu Met Lys Tyr Leu Glu Leu Met
    290                 295                 300

Ile Val Asn Asp His Lys Thr Tyr Lys Lys His Arg Ser Ser His Ala
305                 310                 315                 320

His Thr Asn Asn Phe Ala Lys Ser Val Val Asn Leu Val Asp Ser Ile
                325                 330                 335

Tyr Lys Glu Gln Leu Asn Thr Arg Val Val Leu Val Ala Val Glu Thr
            340                 345                 350

Trp Thr Glu Lys Asp Gln Ile Asp Ile Thr Thr Asn Pro Val Gln Met
        355                 360                 365

Leu His Glu Phe Ser Lys Tyr Arg Gln Arg Ile Lys Gln His Ala Asp
    370                 375                 380

Ala Val His Leu Ile Ser Arg Val Thr Phe His Tyr Lys Arg Ser Ser
385                 390                 395                 400

Leu Ser Tyr Phe Gly Gly Val Cys Ser Arg Thr Arg Gly Val Gly Val
                405                 410                 415

Asn Glu Tyr Gly Leu Pro Met Ala Val Ala Gln Val Leu Ser Gln Ser
            420                 425                 430

Leu Ala Gln Asn Leu Gly Ile Gln Trp Glu Pro Ser Ser Arg Lys Pro
        435                 440                 445

Lys Cys Asp Cys Thr Glu Ser Trp Gly Gly Cys Ile Met Glu Glu Thr
    450                 455                 460

Gly Val Ser His Ser Arg Lys Phe Ser Lys Cys Ser Ile Leu Glu Tyr
465                 470                 475                 480

Arg Asp Phe Leu Gln Arg Gly Gly Gly Ala Cys Leu Phe Asn Arg Pro
                485                 490                 495

Thr Lys Leu Phe Glu Pro Thr Glu Cys Gly Asn Gly Tyr Val Glu Ala
            500                 505                 510

Gly Glu Glu Cys Asp Cys Gly Phe His Val Glu Cys Tyr Gly Leu Cys
        515                 520                 525

Cys Lys Lys Cys Ser Leu Ser Asn Gly Ala His Cys Ser Asp Gly Pro
    530                 535                 540

Cys Cys Asn Asn Thr Ser Cys Leu Phe Gln Pro Arg Gly Tyr Glu Cys
545                 550                 555                 560

Arg Asp Ala Val Asn Glu Cys Asp Ile Thr Glu Tyr Cys Thr Gly Asp
                565                 570                 575

Ser Gly Gln Cys Pro Pro Asn Leu His Lys Gln Asp Gly Tyr Ala Cys
            580                 585                 590

Asn Gln Asn Gln Gly Arg Cys Tyr Asn Gly Glu Cys Lys Thr Arg Asp
        595                 600                 605

Asn Gln Cys Gln Tyr Ile Trp Gly Thr Lys Ala Ala Gly Ser Asp Lys
    610                 615                 620

Phe Cys Tyr Glu Lys Leu Asn Thr Glu Gly Thr Glu Lys Gly Asn Cys
625                 630                 635                 640

Gly Lys Asp Gly Asp Arg Trp Ile Gln Cys Ser Lys His Asp Val Phe
                645                 650                 655

Cys Gly Phe Leu Leu Cys Thr Asn Leu Thr Arg Ala Pro Arg Ile Gly
            660                 665                 670

Gln Leu Gln Gly Glu Ile Ile Pro Thr Ser Phe Tyr His Gln Gly Arg
        675                 680                 685

Val Ile Asp Cys Ser Gly Ala His Val Val Leu Asp Asp Asp Thr Asp
    690                 695                 700

Val Gly Tyr Val Glu Asp Gly Thr Pro Cys Gly Pro Ser Met Met Cys
705                 710                 715                 720

Leu Asp Arg Lys Cys Leu Gln Ile Gln Ala Leu Asn Met Ser Ser Cys
                725                 730                 735

Pro Leu Asp Ser Lys Gly Lys Val Cys Ser Gly His Gly Val Cys Ser
            740                 745                 750

Asn Glu Ala Thr Cys Ile Cys Asp Phe Thr Trp Ala Gly Thr Asp Cys
        755                 760                 765

Ser Ile Arg Asp Pro Val Arg Asn Leu His Pro Pro Lys Asp Glu Gly
    770                 775                 780

Pro Lys Gly Pro Ser Ala Thr Asn Leu Ile Ile Gly Ser Ile Ala Gly
785                 790                 795                 800

Ala Ile Leu Val Ala Ala Ile Val Leu Gly Gly Thr Gly Trp Gly Phe
                805                 810                 815

Lys Asn Val Lys Lys Arg Arg Phe Asp Pro Thr Gln Gln Gly Pro Ile
            820                 825                 830

<210> 184
<211> 775
<212> PRT
<213> Homo sapiens

<400> 184
Met Leu Gln Gly Leu Leu Pro Val Ser Leu Leu Leu Ser Val Ala Val
1               5                   10                  15

Ser Ala Ile Lys Glu Leu Pro Gly Val Lys Lys Tyr Glu Val Val Tyr
            20                  25                  30

Pro Ile Arg Leu His Pro Leu His Lys Arg Glu Ala Lys Glu Pro Glu
        35                  40                  45

Gln Gln Glu Gln Phe Glu Thr Glu Leu Lys Tyr Lys Met Thr Ile Asn
    50                  55                  60

Gly Lys Ile Ala Val Leu Tyr Leu Lys Lys Asn Lys Asn Leu Leu Ala
65                  70                  75                  80

Pro Gly Tyr Thr Glu Thr Tyr Tyr Asn Ser Thr Gly Lys Glu Ile Thr
                85                  90                  95

Thr Ser Pro Gln Ile Met Asp Asp Cys Tyr Tyr Gln Gly His Ile Leu
            100                 105                 110

Asn Glu Lys Val Ser Asp Ala Ser Ile Ser Thr Cys Arg Gly Leu Arg
        115                 120                 125

Gly Tyr Phe Ser Gln Gly Asp Gln Arg Tyr Phe Ile Glu Pro Leu Ser
    130                 135                 140

Pro Ile His Arg Asp Gly Gln Glu His Ala Leu Phe Lys Tyr Asn Pro
145                 150                 155                 160

Asp Glu Lys Asn Tyr Asp Ser Thr Cys Gly Met Asp Gly Val Leu Trp
                165                 170                 175

Ala His Asp Leu Gln Gln Asn Ile Ala Leu Pro Ala Thr Lys Leu Val
            180                 185                 190

Lys Leu Lys Asp Arg Lys Val Gln Glu His Glu Lys Tyr Ile Glu Tyr
        195                 200                 205

Tyr Leu Val Leu Asp Asn Gly Glu Phe Lys Arg Tyr Asn Glu Asn Gln
    210                 215                 220

Asp Glu Ile Arg Lys Arg Val Phe Glu Met Ala Asn Tyr Val Asn Met
225                 230                 235                 240

Leu Tyr Lys Lys Leu Asn Thr His Val Ala Leu Val Gly Met Glu Ile
                245                 250                 255

Trp Thr Asp Lys Asp Lys Ile Lys Ile Thr Pro Asn Ala Ser Phe Thr
            260                 265                 270

Leu Glu Asn Phe Ser Lys Trp Arg Gly Ser Val Leu Ser Arg Arg Lys
        275                 280                 285

Arg His Asp Ile Ala Gln Leu Ile Thr Ala Thr Glu Leu Ala Gly Thr
    290                 295                 300

Thr Val Gly Leu Ala Phe Met Ser Thr Met Cys Ser Pro Tyr Ser Val
305                 310                 315                 320

Gly Val Val Gln Asp His Ser Asp Asn Leu Leu Arg Val Ala Gly Thr
                325                 330                 335

Met Ala His Glu Met Gly His Asn Phe Gly Met Phe His Asp Asp Tyr
            340                 345                 350

Ser Cys Lys Cys Pro Ser Thr Ile Cys Val Met Asp Lys Ala Leu Ser
        355                 360                 365

Phe Tyr Ile Pro Thr Asp Phe Ser Ser Cys Ser Arg Leu Ser Tyr Asp
    370                 375                 380

Lys Phe Phe Glu Asp Lys Leu Ser Asn Cys Leu Phe Asn Ala Pro Leu
385                 390                 395                 400

Pro Thr Asp Ile Ile Ser Thr Pro Ile Cys Gly Asn Gln Leu Val Glu
                405                 410                 415

Met Gly Glu Asp Cys Asp Cys Gly Thr Ser Glu Glu Cys Thr Asn Ile
            420                 425                 430

Cys Cys Asp Ala Lys Thr Cys Lys Ile Lys Ala Thr Phe Gln Cys Ala
        435                 440                 445

Leu Gly Glu Cys Cys Glu Lys Cys Gln Phe Lys Lys Ala Gly Met Val
    450                 455                 460

Cys Arg Pro Ala Lys Asp Glu Cys Asp Leu Pro Glu Met Cys Asn Gly
465                 470                 475                 480

Lys Ser Gly Asn Cys Pro Asp Asp Arg Phe Gln Val Asn Gly Phe Pro
                485                 490                 495

Cys His His Gly Lys Gly His Cys Leu Met Gly Thr Cys Pro Thr Leu
            500                 505                 510

Gln Glu Gln Cys Thr Glu Leu Trp Gly Pro Gly Thr Glu Val Ala Asp
        515                 520                 525

Lys Ser Cys Tyr Asn Arg Asn Glu Gly Gly Ser Lys Tyr Gly Tyr Cys

530                 535                 540

Arg Arg Val Asp Asp Thr Leu Ile Pro Cys Lys Ala Asn Asp Thr Met
545                 550                 555                 560

Cys Gly Lys Leu Phe Cys Gln Gly Gly Ser Asp Asn Leu Pro Trp Lys
                565                 570                 575

Gly Arg Ile Val Thr Phe Leu Thr Cys Lys Thr Phe Asp Pro Glu Asp
            580                 585                 590

Thr Ser Gln Glu Ile Gly Met Val Ala Asn Gly Thr Lys Cys Gly Asp
        595                 600                 605

Asn Lys Val Cys Ile Asn Ala Glu Cys Val Asp Ile Glu Lys Ala Tyr
    610                 615                 620

Lys Ser Thr Asn Cys Ser Ser Lys Cys Lys Gly His Ala Val Cys Asp
625                 630                 635                 640

His Glu Leu Gln Cys Gln Cys Glu Glu Gly Trp Ile Pro Pro Asp Cys
                645                 650                 655

Asp Asp Ser Ser Val Val Phe His Phe Ser Ile Val Val Gly Val Leu
            660                 665                 670

Phe Pro Met Ala Val Ile Phe Val Val Val Ala Met Val Ile Arg His
        675                 680                 685

Gln Ser Ser Arg Glu Lys Gln Lys Lys Asp Gln Arg Pro Leu Ser Thr
    690                 695                 700

Thr Gly Thr Arg Pro His Lys Gln Lys Arg Lys Pro Gln Met Val Lys
705                 710                 715                 720

Ala Val Gln Pro Gln Glu Met Ser Gln Met Lys Pro His Val Tyr Asp
                725                 730                 735

Leu Pro Val Glu Gly Asn Glu Pro Pro Ala Ser Phe His Lys Asp Thr
            740                 745                 750

Asn Ala Leu Pro Pro Thr Val Phe Lys Asp Asn Pro Val Ser Thr Pro
        755                 760                 765

Lys Asp Ser Asn Pro Lys Ala
    770                 775

<210> 185
<211> 820
<212> PRT
<213> Homo sapiens

<400> 185
Met Lys Met Leu Leu Leu Leu His Cys Leu Gly Val Phe Leu Ser Cys
1               5                   10                  15

Ser Gly His Ile Gln Asp Glu His Pro Gln Tyr His Ser Pro Pro Asp
            20                  25                  30

Val Val Ile Pro Val Arg Ile Thr Gly Thr Thr Arg Gly Met Thr Pro
        35                  40                  45

Pro Gly Trp Leu Ser Tyr Ile Leu Pro Phe Gly Gly Gln Lys His Ile
    50                  55                  60

Ile His Ile Lys Val Lys Lys Leu Leu Phe Ser Lys His Leu Pro Val
65                  70                  75                  80

Phe Thr Tyr Thr Asp Gln Gly Ala Ile Leu Glu Asp Gln Pro Phe Val
                85                  90                  95

Gln Asn Asn Cys Tyr Tyr His Gly Tyr Val Glu Gly Asp Pro Glu Ser
            100                 105                 110

Leu Val Ser Leu Ser Thr Cys Phe Gly Gly Phe Gln Gly Ile Leu Gln
        115                 120                 125

Ile Asn Asp Phe Ala Tyr Glu Ile Lys Pro Leu Ala Phe Ser Thr Thr
    130                 135                 140

Phe Glu His Leu Val Tyr Lys Met Asp Ser Glu Glu Lys Gln Phe Ser
145                 150                 155                 160

Thr Met Arg Ser Gly Phe Met Gln Asn Glu Ile Thr Cys Arg Met Glu
                165                 170                 175

Phe Glu Glu Ile Asp Asn Ser Thr Gln Lys Gln Ser Ser Tyr Val Gly
            180                 185                 190

Trp Trp Ile His Phe Arg Ile Val Glu Ile Val Val Val Ile Asp Asn
        195                 200                 205

Tyr Leu Tyr Ile Arg Tyr Glu Arg Asn Asp Ser Lys Leu Leu Glu Asp
    210                 215                 220

Leu Tyr Val Ile Val Asn Ile Val Asp Ser Ile Leu Asp Val Ile Gly

225                 230                 235                 240

Val Lys Val Leu Leu Phe Gly Leu Glu Ile Trp Thr Asn Lys Asn Leu
                245                 250                 255

Ile Val Val Asp Asp Val Arg Lys Ser Val His Leu Tyr Cys Lys Trp
            260                 265                 270

Lys Ser Glu Asn Ile Thr Pro Arg Met Gln His Asp Thr Ser His Leu
        275                 280                 285

Phe Thr Thr Leu Gly Leu Arg Gly Leu Ser Gly Ile Gly Ala Phe Arg
    290                 295                 300

Gly Met Cys Thr Pro His Arg Ser Cys Ala Ile Val Thr Phe Met Asn
305                 310                 315                 320

Lys Thr Leu Gly Thr Phe Ser Ile Ala Val Ala His His Leu Gly His
                325                 330                 335

Asn Leu Gly Met Asn His Asp Glu Asp Thr Cys Arg Cys Ser Gln Pro
            340                 345                 350

Arg Cys Ile Met His Glu Gly Asn Pro Pro Ile Thr Lys Phe Ser Asn
        355                 360                 365

Cys Ser Tyr Gly Asp Phe Trp Glu Tyr Thr Val Glu Arg Thr Lys Cys
    370                 375                 380

Leu Leu Glu Thr Val His Thr Lys Asp Ile Phe Asn Val Lys Arg Cys
385                 390                 395                 400

Gly Asn Gly Val Val Glu Glu Gly Glu Glu Cys Asp Cys Gly Pro Leu
                405                 410                 415

Lys His Cys Ala Lys Asp Pro Cys Cys Leu Ser Asn Cys Thr Leu Thr
            420                 425                 430

Asp Gly Ser Thr Cys Ala Phe Gly Leu Cys Cys Lys Asp Cys Lys Phe
        435                 440                 445

Leu Pro Ser Gly Lys Val Cys Arg Lys Glu Val Asn Glu Cys Asp Leu
    450                 455                 460

Pro Glu Trp Cys Asn Gly Thr Ser His Lys Cys Pro Asp Asp Phe Tyr
465                 470                 475                 480

Val Glu Asp Gly Ile Pro Cys Lys Glu Arg Gly Tyr Cys Tyr Glu Lys
                485                 490                 495

Ser Cys His Asp Arg Asn Glu Gln Cys Arg Arg Ile Phe Gly Ala Gly
            500                 505                 510

Ala Asn Thr Ala Ser Glu Thr Cys Tyr Lys Glu Leu Asn Thr Leu Gly
        515                 520                 525

Asp Arg Val Gly His Cys Gly Ile Lys Asn Ala Thr Tyr Ile Lys Cys
    530                 535                 540

Asn Ile Ser Asp Val Gln Cys Gly Arg Ile Gln Cys Glu Asn Val Thr
545                 550                 555                 560

Glu Ile Pro Asn Met Ser Asp His Thr Thr Val His Trp Ala Arg Phe
                565                 570                 575

Asn Asp Ile Met Cys Trp Ser Thr Asp Tyr His Leu Gly Met Lys Gly
            580                 585                 590

Pro Asp Ile Gly Glu Val Lys Asp Gly Thr Glu Cys Gly Ile Asp His
        595                 600                 605

Ile Cys Ile His Arg His Cys Val His Ile Thr Ile Leu Asn Ser Asn
    610                 615                 620

Cys Ser Pro Ala Phe Cys Asn Lys Arg Gly Ile Cys Asn Asn Lys His
625                 630                 635                 640

His Cys His Cys Asn Tyr Leu Trp Asp Pro Pro Asn Cys Leu Ile Lys
                645                 650                 655

Gly Tyr Gly Gly Ser Val Asp Ser Gly Pro Pro Pro Lys Arg Lys Lys
            660                 665                 670

Lys Lys Lys Phe Cys Tyr Leu Cys Ile Leu Leu Leu Ile Val Leu Phe
        675                 680                 685

Ile Leu Leu Cys Cys Leu Tyr Arg Leu Cys Lys Lys Ser Lys Pro Ile
    690                 695                 700

Lys Lys Gln Gln Asp Val Gln Thr Pro Ser Ala Lys Glu Glu Glu Lys
705                 710                 715                 720

Ile Gln Arg Arg Pro His Glu Leu Pro Pro Gln Ser Gln Pro Trp Val
                725                 730                 735

Met Pro Ser Gln Ser Gln Pro Pro Val Thr Pro Ser Gln Ser His Pro
                740                 745                 750

Gln Val Met Pro Ser Gln Ser Gln Pro Pro Val Thr Pro Ser Gln Ser
            755                 760                 765

Gln Pro Arg Val Met Pro Ser Gln Ser Gln Pro Pro Val Met Pro Ser
    770                 775                 780

Gln Ser His Pro Gln Leu Thr Pro Ser Gln Ser Gln Pro Pro Val Thr
785                 790                 795                 800

Pro Ser Gln Arg Gln Pro Gln Leu Met Pro Ser Gln Ser Gln Pro Pro
                805                 810                 815

Val Thr Pro Ser
            820

<210> 186
<211> 790
<212> PRT
<213> Homo sapiens

<400> 186
Met Arg Ser Val Gln Ile Phe Leu Ser Gln Cys Arg Leu Leu Leu Leu
1               5                   10                  15

Leu Val Pro Thr Met Leu Leu Lys Ser Leu Gly Glu Asp Val Ile Phe
            20                  25                  30

His Pro Glu Gly Glu Phe Asp Ser Tyr Glu Val Thr Ile Pro Glu Lys
        35                  40                  45

Leu Ser Phe Arg Gly Glu Val Gln Gly Val Val Ser Pro Val Ser Tyr
    50                  55                  60

Leu Leu Gln Leu Lys Gly Lys Lys His Val Leu His Leu Trp Pro Lys
65                  70                  75                  80

Arg Leu Leu Leu Pro Arg His Leu Arg Val Phe Ser Phe Thr Glu His
                85                  90                  95

Gly Glu Leu Leu Glu Asp His Pro Tyr Ile Pro Lys Asp Cys Asn Tyr
            100                 105                 110

Met Gly Ser Val Lys Glu Ser Leu Asp Ser Lys Ala Thr Ile Ser Thr
        115                 120                 125

Cys Met Gly Gly Leu Arg Gly Val Phe Asn Ile Asp Ala Lys His Tyr
    130                 135                 140

Gln Ile Glu Pro Leu Lys Ala Ser Pro Ser Phe Glu His Val Val Tyr
145                 150                 155                 160

Leu Leu Lys Lys Glu Gln Phe Gly Asn Gln Val Cys Gly Leu Ser Asp
                165                 170                 175

Asp Glu Ile Glu Trp Gln Met Ala Pro Tyr Glu Asn Lys Ala Arg Leu
            180                 185                 190

Arg Asp Phe Pro Gly Ser Tyr Lys His Pro Lys Tyr Leu Glu Leu Ile
        195                 200                 205

Leu Leu Phe Asp Gln Ser Arg Tyr Arg Phe Val Asn Asn Asn Leu Ser
    210                 215                 220

Gln Val Ile His Asp Ala Ile Leu Leu Thr Gly Ile Met Asp Thr Tyr
225                 230                 235                 240

Phe Gln Asp Val Arg Met Arg Ile His Leu Lys Ala Leu Glu Val Trp
                245                 250                 255

Thr Asp Phe Asn Lys Ile Arg Val Gly Tyr Pro Glu Leu Ala Glu Val
            260                 265                 270

Leu Gly Arg Phe Val Ile Tyr Lys Lys Ser Val Leu Asn Ala Arg Leu
        275                 280                 285

Ser Ser Asp Trp Ala His Leu Tyr Leu Gln Arg Lys Tyr Asn Asp Ala
    290                 295                 300

Leu Ala Trp Ser Phe Gly Lys Val Cys Ser Leu Glu Tyr Ala Gly Ser
305                 310                 315                 320

Val Ser Thr Leu Leu Asp Thr Asn Ile Leu Ala Pro Ala Thr Trp Ser
                325                 330                 335

Ala His Glu Leu Gly His Ala Val Gly Met Ser His Asp Glu Gln Tyr
            340                 345                 350

Cys Gln Cys Arg Gly Arg Leu Asn Cys Ile Met Gly Ser Gly Arg Thr
        355                 360                 365

Gly Phe Ser Asn Cys Ser Tyr Ile Ser Phe Phe Lys His Ile Ser Ser
    370                 375                 380

Gly Ala Thr Cys Leu Asn Asn Ile Pro Gly Leu Gly Tyr Val Leu Lys
385                 390                 395                 400

Arg Cys Gly Asn Lys Ile Val Glu Asp Asn Glu Glu Cys Asp Cys Gly
                405                 410                 415

Ser Thr Glu Glu Cys Gln Lys Asp Arg Cys Cys Gln Ser Asn Cys Lys
            420                 425                 430

Leu Gln Pro Gly Ala Asn Cys Ser Ile Gly Leu Cys Cys His Asp Cys
        435                 440                 445

Arg Phe Arg Pro Ser Gly Tyr Val Cys Arg Gln Glu Gly Asn Glu Cys
    450                 455                 460

Asp Leu Ala Glu Tyr Cys Asp Gly Asn Ser Ser Ser Cys Pro Asn Asp
465                 470                 475                 480

Val Tyr Lys Gln Asp Gly Thr Pro Cys Lys Tyr Glu Gly Arg Cys Phe
                485                 490                 495

Arg Lys Gly Cys Arg Ser Arg Tyr Met Gln Cys Gln Ser Ile Phe Gly
            500                 505                 510

Pro Asp Ala Met Glu Ala Pro Ser Glu Cys Tyr Asp Ala Val Asn Leu
        515                 520                 525

Ile Gly Asp Gln Phe Gly Asn Cys Glu Ile Thr Gly Ile Arg Asn Phe
    530                 535                 540

Lys Lys Cys Glu Ser Ala Asn Ser Ile Cys Gly Arg Leu Gln Cys Ile
545                 550                 555                 560

Asn Val Glu Thr Ile Pro Asp Leu Pro Glu His Thr Thr Ile Ile Ser
                565                 570                 575

Thr His Leu Gln Ala Glu Asn Leu Met Cys Trp Gly Thr Gly Tyr His
            580                 585                 590

Leu Ser Met Lys Pro Met Gly Ile Pro Asp Leu Gly Met Ile Asn Asp
        595                 600                 605

Gly Thr Ser Cys Gly Glu Gly Arg Val Cys Phe Lys Lys Asn Cys Val
    610                 615                 620

Asn Ser Ser Val Leu Gln Phe Asp Cys Leu Pro Glu Lys Cys Asn Thr
625                 630                 635                 640

Arg Gly Val Cys Asn Asn Arg Lys Asn Cys His Cys Met Tyr Gly Trp
                645                 650                 655

Ala Pro Pro Phe Cys Glu Glu Val Gly Tyr Gly Gly Ser Ile Asp Ser
            660                 665                 670

Gly Pro Pro Gly Leu Leu Arg Gly Ala Ile Pro Ser Ser Ile Trp Val
        675                 680                 685

Val Ser Ile Ile Met Phe Arg Leu Ile Leu Leu Ile Leu Ser Val Val
    690                 695                 700

Phe Val Phe Phe Arg Gln Val Ile Gly Asn His Leu Lys Pro Lys Gln
705                 710                 715                 720

Glu Lys Met Pro Leu Ser Lys Ala Lys Thr Glu Gln Glu Glu Ser Lys
                725                 730                 735

Thr Lys Thr Val Gln Glu Glu Ser Lys Thr Lys Thr Gly Gln Glu Glu
            740                 745                 750

Ser Glu Ala Lys Thr Gly Gln Glu Glu Ser Lys Ala Lys Thr Gly Gln
        755                 760                 765

Glu Glu Ser Lys Ala Asn Ile Glu Ser Lys Arg Pro Lys Ala Lys Ser
    770                 775                 780

Val Lys Lys Gln Lys Lys
785                 790

<210> 187
<211> 787
<212> PRT
<213> Homo sapiens

<400> 187
Met Phe Arg Leu Trp Leu Leu Leu Ala Gly Leu Cys Gly Leu Leu Ala
1               5                   10                  15

Ser Arg Pro Gly Phe Gln Asn Ser Leu Leu Gln Ile Val Ile Pro Glu
            20                  25                  30

Lys Ile Gln Thr Asn Thr Asn Asp Ser Ser Glu Ile Glu Tyr Glu Gln
        35                  40                  45

Ile Ser Tyr Ile Ile Pro Ile Asp Glu Lys Leu Tyr Thr Val His Leu
    50                  55                  60

Lys Gln Arg Tyr Phe Leu Ala Asp Asn Phe Met Ile Tyr Leu Tyr Asn
65                  70                  75                  80

Gln Gly Ser Met Asn Thr Tyr Ser Ser Asp Ile Gln Thr Gln Cys Tyr
                85                  90                  95

Tyr Gln Gly Asn Ile Glu Gly Tyr Pro Asp Ser Met Val Thr Leu Ser
            100                 105                 110

Thr Cys Ser Gly Leu Arg Gly Ile Leu Gln Phe Glu Asn Val Ser Tyr
        115                 120                 125

Gly Ile Glu Pro Leu Glu Ser Ala Val Glu Phe Gln His Val Leu Tyr
    130                 135                 140

Lys Leu Lys Asn Glu Asp Asn Asp Ile Ala Ile Phe Ile Asp Arg Ser
145                 150                 155                 160

Leu Lys Glu Gln Pro Met Asp Asp Asn Ile Phe Ile Ser Glu Lys Ser
                165                 170                 175

Glu Pro Ala Val Pro Asp Leu Phe Pro Leu Tyr Leu Glu Met His Ile
            180                 185                 190

Val Val Asp Lys Thr Leu Tyr Asp Tyr Trp Gly Ser Asp Ser Met Ile
        195                 200                 205

Val Thr Asn Lys Val Ile Glu Ile Val Gly Leu Ala Asn Ser Met Phe
    210                 215                 220

Thr Gln Phe Lys Val Thr Ile Val Leu Ser Ser Leu Glu Leu Trp Ser
225                 230                 235                 240

Asp Glu Asn Lys Ile Ser Thr Val Gly Glu Ala Asp Glu Leu Leu Gln
                245                 250                 255

Lys Phe Leu Glu Trp Lys Gln Ser Tyr Leu Asn Leu Arg Pro His Asp
            260                 265                 270

Ile Ala Tyr Leu Leu Ile Tyr Met Asp Tyr Pro Arg Tyr Leu Gly Ala
        275                 280                 285

Val Phe Pro Gly Thr Met Cys Ile Thr Arg Tyr Ser Ala Gly Val Ala
    290                 295                 300

Leu Tyr Pro Lys Glu Ile Thr Leu Glu Ala Phe Ala Val Ile Val Thr
305                 310                 315                 320

Gln Met Leu Ala Leu Ser Leu Gly Ile Ser Tyr Asp Asp Pro Lys Lys
                325                 330                 335

Cys Gln Cys Ser Glu Ser Thr Cys Ile Met Asn Pro Glu Val Val Gln
            340                 345                 350

Ser Asn Gly Val Lys Thr Phe Ser Ser Cys Ser Leu Arg Ser Phe Gln
        355                 360                 365

Asn Phe Ile Ser Asn Val Gly Val Lys Cys Leu Gln Asn Lys Pro Gln
    370                 375                 380

Met Gln Lys Lys Ser Pro Lys Pro Val Cys Gly Asn Gly Arg Leu Glu
385                 390                 395                 400

Gly Asn Glu Ile Cys Asp Cys Gly Thr Glu Ala Gln Cys Gly Pro Ala
                405                 410                 415

Ser Cys Cys Asp Phe Arg Thr Cys Val Leu Lys Asp Gly Ala Lys Cys
            420                 425                 430

Tyr Lys Gly Leu Cys Cys Lys Asp Cys Gln Ile Leu Gln Ser Gly Val
        435                 440                 445

Glu Cys Arg Pro Lys Ala His Pro Glu Cys Asp Ile Ala Glu Asn Cys
    450                 455                 460

Asn Gly Ser Ser Pro Glu Cys Gly Pro Asp Ile Thr Leu Ile Asn Gly
465                 470                 475                 480

Leu Ser Cys Lys Asn Asn Lys Phe Ile Cys Tyr Asp Gly Asp Cys His
                485                 490                 495

Asp Leu Asp Ala Arg Cys Glu Ser Val Phe Gly Lys Gly Ser Arg Asn
            500                 505                 510

Ala Pro Phe Ala Cys Tyr Glu Glu Ile Gln Ser Gln Ser Asp Arg Phe
        515                 520                 525

Gly Asn Cys Gly Arg Asp Arg Asn Asn Lys Tyr Val Phe Cys Gly Trp
    530                 535                 540

Arg Asn Leu Ile Cys Gly Arg Leu Val Cys Thr Tyr Pro Thr Arg Lys
545                 550                 555                 560

Pro Phe His Gln Glu Asn Gly Asp Val Ile Tyr Ala Phe Val Arg Asp

565                 570                 575

Ser Val Cys Ile Thr Val Asp Tyr Lys Leu Pro Arg Thr Val Pro Asp
            580                 585                 590

Pro Leu Ala Val Lys Asn Gly Ser Gln Cys Asp Ile Gly Arg Val Cys
        595                 600                 605

Val Asn Arg Glu Cys Val Glu Ser Arg Ile Ile Lys Ala Ser Ala His
    610                 615                 620

Val Cys Ser Gln Gln Cys Ser Gly His Gly Val Cys Asp Ser Arg Asn
625                 630                 635                 640

Lys Cys His Cys Ser Pro Gly Tyr Lys Pro Pro Asn Cys Gln Ile Arg
                645                 650                 655

Ser Lys Gly Phe Ser Ile Phe Pro Glu Glu Asp Met Gly Ser Ile Met
            660                 665                 670

Glu Arg Ala Ser Gly Lys Thr Glu Asn Thr Trp Leu Leu Gly Phe Leu
        675                 680                 685

Ile Ala Leu Pro Ile Leu Ile Val Thr Thr Ala Ile Val Leu Ala Arg
    690                 695                 700

Lys Gln Leu Lys Lys Trp Phe Ala Lys Glu Glu Glu Phe Pro Ser Ser
705                 710                 715                 720

Glu Ser Lys Ser Glu Gly Ser Thr Gln Thr Tyr Ala Ser Gln Ser Ser
                725                 730                 735

Ser Glu Gly Ser Thr Gln Thr Tyr Ala Ser Gln Thr Arg Ser Glu Ser
            740                 745                 750

Ser Ser Gln Ala Asp Thr Ser Lys Ser Lys Ser Glu Asp Ser Ala Glu
        755                 760                 765

Ala Tyr Thr Ser Arg Ser Lys Ser Gln Asp Ser Thr Gln Thr Gln Ser
    770                 775                 780

Ser Ser Asn
785

<210> 188
<211> 813
<212> PRT
<213> Homo sapiens

<400> 188
Met Gly Trp Arg Pro Arg Arg Ala Arg Gly Thr Pro Leu Leu Leu Leu
1               5                   10                  15

Leu Leu Leu Leu Leu Leu Trp Pro Val Pro Gly Ala Gly Val Leu Gln
            20                  25                  30

Gly His Ile Pro Gly Gln Pro Val Thr Pro His Trp Val Leu Asp Gly
        35                  40                  45

Gln Pro Trp Arg Thr Val Ser Leu Glu Glu Pro Val Ser Lys Pro Asp
    50                  55                  60

Met Gly Leu Val Ala Leu Glu Ala Glu Gly Gln Glu Leu Leu Leu Glu
65                  70                  75                  80

Leu Glu Lys Asn His Arg Leu Leu Ala Pro Gly Tyr Ile Glu Thr His
                85                  90                  95

Tyr Gly Pro Asp Gly Gln Pro Val Val Leu Ala Pro Asn His Thr Asp
            100                 105                 110

His Cys His Tyr Gln Gly Arg Val Arg Gly Phe Pro Asp Ser Trp Val
        115                 120                 125

Val Leu Cys Thr Cys Ser Gly Met Ser Gly Leu Ile Thr Leu Ser Arg
    130                 135                 140

Asn Ala Ser Tyr Tyr Leu Arg Pro Trp Pro Pro Arg Gly Ser Lys Asp
145                 150                 155                 160

Phe Ser Thr His Glu Ile Phe Arg Met Glu Gln Leu Leu Thr Trp Lys
                165                 170                 175

Gly Thr Cys Gly His Arg Asp Pro Gly Asn Lys Ala Gly Met Thr Ser
            180                 185                 190

Leu Pro Gly Gly Pro Gln Ser Arg Gly Arg Arg Glu Ala Arg Arg Thr
        195                 200                 205

Arg Lys Tyr Leu Glu Leu Tyr Ile Val Ala Asp His Thr Leu Phe Leu
    210                 215                 220

Thr Arg His Arg Asn Leu Asn His Thr Lys Gln Arg Leu Leu Glu Val
225                 230                 235                 240

Ala Asn Tyr Val Asp Gln Leu Leu Arg Thr Leu Asp Ile Gln Val Ala

245                 250                 255

Leu Thr Gly Leu Glu Val Trp Thr Glu Arg Asp Arg Ser Arg Val Thr
            260                 265                 270

Gln Asp Ala Asn Ala Thr Leu Trp Ala Phe Leu Gln Trp Arg Arg Gly
        275                 280                 285

Leu Trp Ala Gln Arg Pro His Asp Ser Ala Gln Leu Leu Thr Gly Arg
    290                 295                 300

Ala Phe Gln Gly Ala Thr Val Gly Leu Ala Pro Val Glu Gly Met Cys
305                 310                 315                 320

Arg Ala Glu Ser Ser Gly Gly Val Ser Thr Asp His Ser Glu Leu Pro
                325                 330                 335

Ile Gly Ala Ala Ala Thr Met Ala His Glu Ile Gly His Ser Leu Gly
            340                 345                 350

Leu Ser His Asp Pro Asp Gly Cys Cys Val Glu Ala Ala Ala Glu Ser
        355                 360                 365

Gly Gly Cys Val Met Ala Ala Ala Thr Gly His Pro Phe Pro Arg Val
    370                 375                 380

Phe Ser Ala Cys Ser Arg Arg Gln Leu Arg Ala Phe Phe Arg Lys Gly
385                 390                 395                 400

Gly Gly Ala Cys Leu Ser Asn Ala Pro Asp Pro Gly Leu Pro Val Pro
                405                 410                 415

Pro Ala Leu Cys Gly Asn Gly Phe Val Glu Ala Gly Glu Glu Cys Asp
            420                 425                 430

Cys Gly Pro Gly Gln Glu Cys Arg Asp Leu Cys Cys Phe Ala His Asn
        435                 440                 445

Cys Ser Leu Arg Pro Gly Ala Gln Cys Ala His Gly Asp Cys Cys Val
    450                 455                 460

Arg Cys Leu Leu Lys Pro Ala Gly Ala Leu Cys Arg Gln Ala Met Gly
465                 470                 475                 480

Asp Cys Asp Leu Pro Glu Phe Cys Thr Gly Thr Ser Ser His Cys Pro
                485                 490                 495

Pro Asp Val Tyr Leu Leu Asp Gly Ser Pro Cys Ala Arg Gly Ser Gly
        500                 505                 510

Tyr Cys Trp Asp Gly Ala Cys Pro Thr Leu Glu Gln Gln Cys Gln Gln
    515                 520                 525

Leu Trp Gly Pro Gly Ser His Pro Ala Pro Glu Ala Cys Phe Gln Val
530                 535                 540

Val Asn Ser Ala Gly Asp Ala His Gly Asn Cys Gly Gln Asp Ser Glu
545                 550                 555                 560

Gly His Phe Leu Pro Cys Ala Gly Arg Asp Ala Leu Cys Gly Lys Leu
                565                 570                 575

Gln Cys Gln Gly Gly Lys Pro Ser Leu Leu Ala Pro His Met Val Pro
            580                 585                 590

Val Asp Ser Thr Val His Leu Asp Gly Gln Glu Val Thr Cys Arg Gly
        595                 600                 605

Ala Leu Ala Leu Pro Ser Ala Gln Leu Asp Leu Leu Gly Leu Gly Leu
    610                 615                 620

Val Glu Pro Gly Thr Gln Cys Gly Pro Arg Met Val Cys Gln Ser Arg
625                 630                 635                 640

Arg Cys Arg Lys Asn Ala Phe Gln Glu Leu Gln Arg Cys Leu Thr Ala
                645                 650                 655

Cys His Ser His Gly Val Cys Asn Ser Asn His Asn Cys His Cys Ala
            660                 665                 670

Pro Gly Trp Ala Pro Pro Phe Cys Asp Lys Pro Gly Phe Gly Gly Ser
        675                 680                 685

Met Asp Ser Gly Pro Val Gln Ala Glu Asn His Asp Thr Phe Leu Leu
    690                 695                 700

Ala Met Leu Leu Ser Val Leu Leu Pro Leu Leu Pro Gly Ala Gly Leu
705                 710                 715                 720

Ala Trp Cys Cys Tyr Arg Leu Pro Gly Ala His Leu Gln Arg Cys Ser
                725                 730                 735

Trp Gly Cys Arg Arg Asp Pro Ala Cys Ser Gly Pro Lys Asp Gly Pro
            740                 745                 750

His Arg Asp His Pro Leu Gly Gly Val His Pro Met Glu Leu Gly Pro
        755                 760                 765

Thr Ala Thr Gly Gln Pro Trp Pro Leu Asp Pro Glu Asn Ser His Glu
    770                 775                 780

Pro Ser Ser His Pro Glu Lys Pro Leu Pro Ala Val Ser Pro Asp Pro
785                 790                 795                 800

Gln Ala Asp Gln Val Gln Met Pro Arg Ser Cys Leu Trp
                805                 810